# EUROPEAN PATENT SPECIFICATION

(11) **EP 1 246 623 B1**
(45) Date of publication and mention of the grant of the patent: **09.08.2006**
(21) Application number: 00988011.3
(22) Date of filing: 01.12.2000
(51) Int. Cl.: A61K 31/519, C07D 487/04, A61K 31/505

(54) **COMPOUNDS SPECIFIC TO ADENOSINE A1, A2A, AND A3 RECEPTOR AND USES THEREOF**
FÜR ADENOSIN A3, A2A UND A3 REZEPTOREN SPEZIFISCHE VERBINDUNGEN UND DEREN VERWENDUNGEN
COMPOSES SPECIFIQUES AUX RECEPTEURS A1, A2A ET A3 DE L'ADENOSINE ET LEURS UTILISATIONS

(30) Priority: 02.12.1999 US 454074; 02.12.1999 US 454254; 02.12.1999 US 454075
(43) Date of publication of application: 09.10.2002
(73) Proprietor: OSI Pharmaceuticals, Inc., Melville, New York 11747 (US)
(72) Inventor: CASTELHANO, Arlindo, L., New City, NY 10579 (US); MCKIBBEN, Bryan, White Plains, NY 10605 (US); WITTER, David, J., Putnam Valley, NY 10579 (US)
(74) Representative: Vossius & Partner
(86) International application number: PCT/US2000/032702
(87) International publication number: WO 2001/039777

(56) References cited:
- WO-A-93/20078
- US-A- 3 910 913
- MÜLLER C E ET AL: "JOURNAL OF MEDICINAL CHEMISTRY, AMERICAN CHEMICAL SOCIETY. WASHINGTON, US" JOURNAL OF MEDICINAL CHEMISTRY, AMERICAN CHEMICAL SOCIETY. WASHINGTON, US, vol. 39, 1996, pages 2482-2491, XP002106452 ISSN: 0022-2623
- DOOLEY M J ET AL: "Theoretical Structure-Activity Studies of Adenosine A1 Ligands: Requirements for Receptor Affinity" BIOORGANIC & MEDICINAL CHEMISTRY, ELSEVIER SCIENCE LTD, GB, vol. 4, no. 6, 1996, pages 923-934, XP002197561 ISSN: 0968-0896
- MÜLLER C E ET AL: "JOURNAL OF MEDICINAL CHEMISTRY, AMERICAN CHEMICAL SOCIETY. WASHINGTON, US" JOURNAL OF MEDICINAL CHEMISTRY, AMERICAN CHEMICAL SOCIETY. WASHINGTON, US, vol. 33, 1990, pages 2822-2828, XP002106454 ISSN: 0022-2623
- CAMPBELL R M ET AL: "Selective A1-adenosine receptor antagonists identified using yeast saccharomyces cerevisiae functional assays" BIOORGANIC & MEDICINAL CHEMISTRY LETTERS, OXFORD, GB, vol. 9, no. 16, 16 August 1999 (1999-08-16), pages 2413-2418, XP004174201 ISSN: 0960-894X
- ZHAO ET AL.: "Bioactivation of 6,7-Dimethyl-2,4-di-1-pyrrolidinyl-7H-pyrr olo[2,3-d]pyrimidine (U-89843) to Reactive Intermediates That Bind Covalently to Macromolecules and Produce Genotoxicity" CHEM. RES. TOXICOL., vol. 9, 1996, pages 1230-1239, XP002233447
- DHAINAUT ET AL.: "New Purines and Purine Analogs as Modulators of Multidrug Resistance" J. MED. CHEM., vol. 39, 1996, pages 4099-4108, XP002233448

## Description

Throughout this application, reference is made to compounds that specifically bind to i) adenosine A₁, receptors, ii) adenosine A₂ₐ receptors, and adenosine A₃ receptors.

Adenosine is an ubiquitous modulator of numerous physiological activities, particularly within the cardiovascular and nervous systems. The effects of adenosine appear to be mediated by specific cell surface receptor proteins. Adenosine modulates diverse physiological functions including induction of sedation, vasodilation, suppression of cardiac rate and contractility, inhibition of platelet aggregability, stimulation of gluconeagenesis and inhibition of lipolysis. In addition to its effects on adenylate cyclase, adenosine has been shown to open potassium channels, reduce flux through calcium channels, and inhibit or stimulate phosphoinositide turnover through receptor-mediated mechanisms (See for example, C.E. Muller and B. Stein "Adenosine Receptor Antagonists: Structures and Potential Therapeutic Applicacions," *Current Pharmaceutical Design,* 2:501 (1996) and C.E. Muller "A₁-Adenosine Receptor Antagonists," *Exp*. *Opin. Ther. Patents* 7(5) :419 (1997)).

Adenosine receptors belong to the superfamily of purine receptors which are currently subdivided into P₁ (adenosine) and P₂ (ATP, ADP, and other nucleotides) receptors. Four receptor subtypes for the nucleoside adenosine have been cloned so far from various species including humans. Two receptor subtypes (A₁ and A₂ₐ) exhibit affinity for adenosine in the nanomolar range while two other known subtypes A_{2b} and A₃ are low-affinity receptors, with affinity for adenosine in the low-micromolar range. A₁ and A₃ adenosine receptor activation can lead to an inhibition of adenylate cyclase activity, while A₂ₐ and A_{2b} activation causes a stimulation of adenylate cyclase.

A few A₁ antagonists have been developed for the treatment of cognitive disease, renal failure, and cardiac arrhythmias. It has been suggested that A₂ₐ antagonists may be beneficial for patients suffering from Morbus Parkinson (Parkinson's disease). Particularly in view of the potential for local delivery, adenosine receptor antagonists may be valuable for treatment of allergic inflammation and asthma. Available information (for example, Nyce & Metzger "DNA antisense Therapy for Asthma in an Animal Model" *Nature* (1997) 385: 721-5) indicates that in this pathophysiologic context, A₁ antagonists may block contraction of smooth muscle underlying respiratory epithelia, while A_{2b} or A₃ receptor antagonists may block mast cell degranulation, mitigating the release of histamine and other inflammatory mediators. A_{2b} receptors have been discovered throughout the gastrointestinal tract, especially in the colon and the intestinal epithelia. It has been suggested that A_{2b} receptors mediate cAMP response (Strohmeier *et al., J. Bio. Chem.* (1995) 270:2387-94).

Adenosine receptors have also been shown to exist on the retinas of various mammalian species including bovine, porcine, monkey, rat, guinea pig, mouse, rabbit and human (See, Blazynski *et al., Discrete Distributions of Adenosine Receptors in Mammalian Retina, Journal of Neurochemistry,* volume 54, pages 648-655 (1990); Woods et *al., Characterization of Adenosine* A₁*-Receptor Binding Sites in Bovine Retinal Membranes, Experimental Eye Research,* volume 53, pages 325-331 (1991); and Braas *et al*., *Endogenous adenosine and adenosine receptors localized to ganglion* cells *of the retina, Proceedings of the National Academy of Science,* volume 84, pages 3906-3910 (1987)). Recently, Williams reported the observation of adenosine transport sites in a cultured human retinal cell line (Williams *et al.*, *Nucleoside Transport Sites in a Cultured Human Retinal Cell Line Established By SV-40 T Antigen Gene, Current Eye* Research, volume 13, pages 109-118 (1994)).

Müller C. et al, Journal of Medicinal Chemistry, vol. 39, 1996, p. 2482-2491 discloses pyrrolo [2,3-d]pyrimidine derivatives as adenosine receptor antagonists.

Dooley M.J. et al, Bioorgonic & Medicinal Chemistry, vol.4, no. 6, 1996, p.923-934 discloses theoretical structure-activity studies of adenosine A1 Ligands, in particular regarding the requirements for receptor activity.

Müller C. et al., Journal of Medicinal Chemistry, vol. 33, 1990, p-2822-2828 pertains to the structure-activity relationships of patent A₁ selective adenosine receptor antagonists, in particular of 7-deaza-2-phenylademines.

Compounds which regulate the uptake of adenosine uptake have previously been suggested as potential therapeutic agents for the treatment of retinal and optic nerve head damage. In U.S. Patent No. 5,780,450 to Shade, Shade discusses the use of adenosine uptake inhibitors for treating eye disorders. Shade does not disclose the use of specific A₃ receptor inhibitors.

Additional adenosine receptor antagonists are needed as pharmacological tools and are of considerable interest as drugs for the above-referenced disease states and/or conditions.

The present invention is based on compounds which selectively bind to adenosine A₁ receptor, thereby treating a disease associated with A₁ adenosine receptor in a subject by administering to the subject a therapeutically effective amount of such compounds. The disease to be treated are associated with cognitive disease, renal failure, cardiac arrhythmias, respiratory epithelia, transmitter release, sedation, vasoconstriction, bradycardia, negative cardiac inotropy and dromotropy, branchoconstriction, neutropil chemotaxis, reflux condition, or ulcerative condition.

The present invention is based, at least in part, on the discovery that certain N-6 substituted 7-deazapurines, described *infra,* can be used to treat a N-6 substituted 7-deazapurine responsive state. Examples of such states include those in which the activity of the adenosine receptors is increased, *e.g.,* bronchitis, gastrointestinal disorders, or asthma. These states can be characterized in that adenosine receptor activation can lead to the inhibition or stimulation of adenylate cyclase activity. Compositions and methods of the invention include enantiomerically or diastereomerically pure N-6 substituted 7-deazapurines. Preferred N-6 substituted 7-deazapurines include those which have an acetamide, carboxamide, substituted cyclohexyl, *e.g*., cyclohexanol, or a urea moiety attached to the N-6 nitrogen through an alkylene chain.

The present invention pertains to the use of a therapeutically effective amount of a N-6 substituted 7-deazapurine for modulating an adenosine receptor(s) in a mammal , such that modulation of the adenosine receptor's activity occurs. Suitable adenosine receptors include the families of A₁, A₂, or A_{3.} In a preferred embodiment, the N-6 substituted 7-deazapurine is a adenosine receptor antagonist.

The invention further pertains to the use of a therapeutically effective amount of a N-6 substituted 7-deazapurine for treating N-6 substituted 7-deazapurine disorders, e.g., asthma, bronchitis, allergic rhinitis, chronic obstructive pulmonary disease, renal disorders, gastrointestinal disorders, and eye disorders, in a mammal, such that treatment of the disorder in the mammal occurs. Suitable N-6 substituted 7 deazapurines include those illustrated by the general formula I: 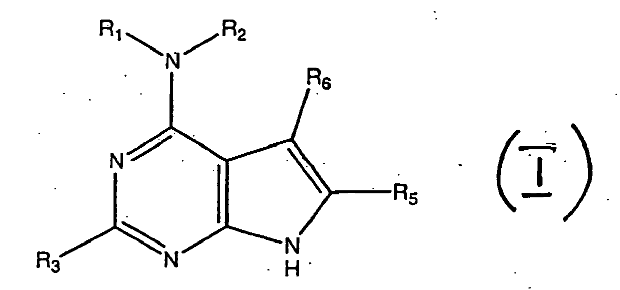 wherein R₁ is 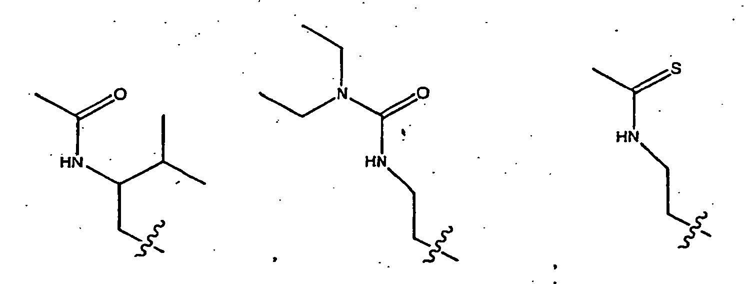 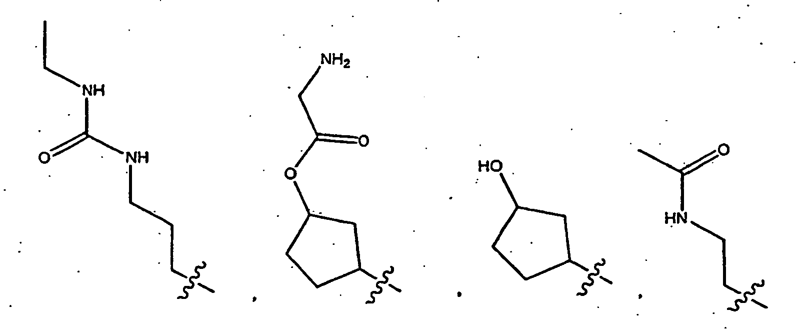 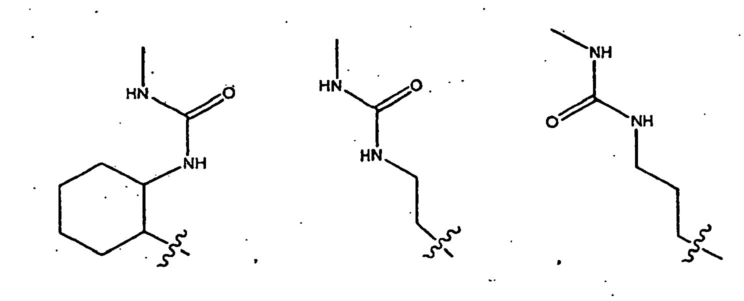 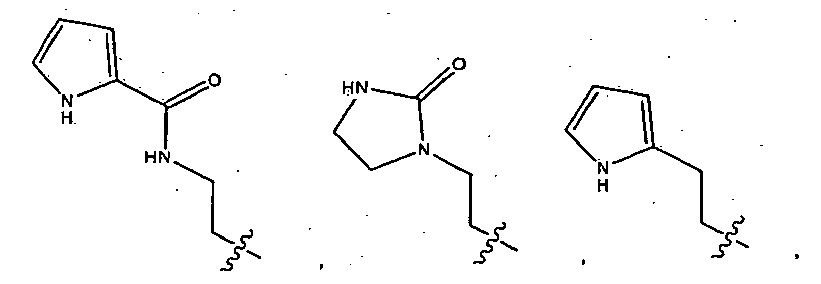 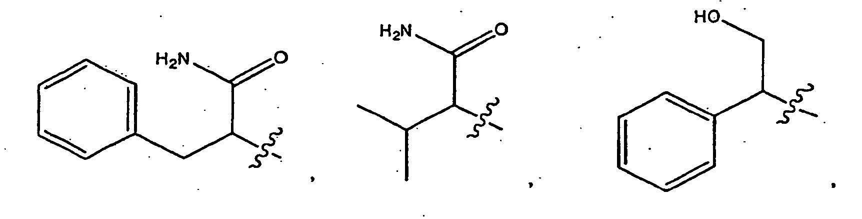 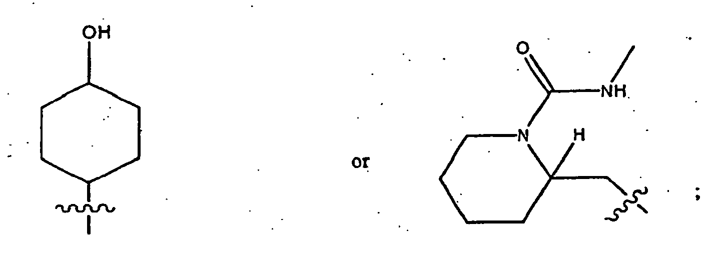 and
R2 is H; or
NR₁R₂ together are 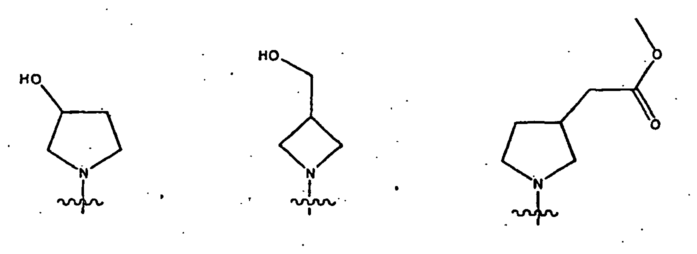 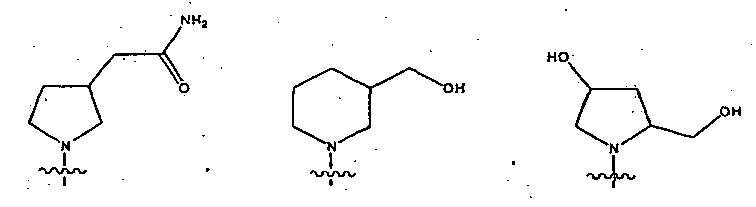 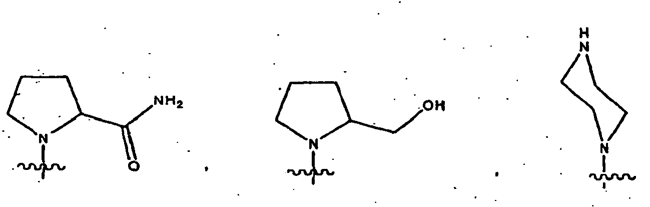 wherein R₃ is a substituted or unsubstituted phenyl, pyrrole, thiophene, furan, thiazole, imidazole, triazole, pyrazole, pyridine, 2 (1H) -pyridone, 4 (1H)-pyridone, pyrazine, pyrimidine, pyridazine, isothiazole, isoxazole, oxazole, tetrazole, naphthyl, tetralin, benzofuran, benzothiophene, 2,3-dihydroindole, 1H-indole, indolyl, benzopyrazole, 1,3-benzodioxole, benzoxazole, purine, coumarin, chromone, quinolyl, tetrahydroquinoline, isoquinoline, benzimidazole, quiriazoline, pteridine, 2(1H) - quinolone, 1 (2H)-isoquinolone, 1, 4-berizisoxazine, benzothiazole, quinoxaline, quinoline-N-oxide, isoquinoline-N-oxide, quinoxaline-N-oxidd, quinazoline-N-oxide; benzoxazine, phthalazine, or cinnoline; wherein R₅ is H, CH₃, substituted or unsubstituted alkyl, aryl or phenyl,
or 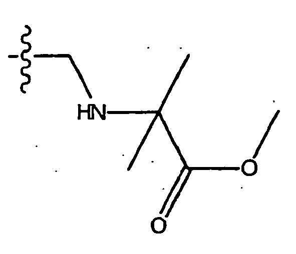 and
wherein R₆ is H, CH₃ substituted or unsubstituted alkyl, cycloalkyl,
wherein the substituent when present is halogen, hydroxyl, alkoxy, alkylcarbonyloxy, arylcarbonyloxy alkoxycarbonyloxy, aryloxycarbonyloxy, carboxylate, alkylcarbonyl alkoxycarbonyl, aminocarbonyl, alkylthiocarbonyl, phosphate, phosphohato, phospinato, cyano amino, acylamino, amidino, imino, sulfhydryl, alkylthio arylthio, thiocarboxylate sulfates, sulfonato, sulfamoyl, sulfonamido nitro trifluoromehyl, cyano, azido, heterocyclyl, or alkylaryl,
or a pharmaceutically acceptable salt thereof,
wherein when R₁ is 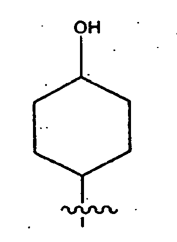 R3 is phenyl;
R₅ is 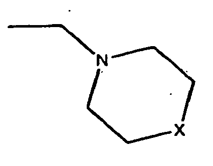 and R₆ is H;
wherein X is O or S; and
wherein when R₁ is 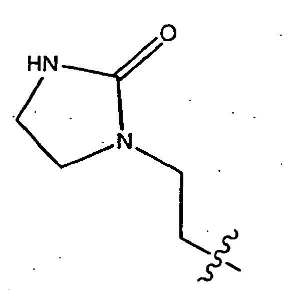 R₃ is phenyl;
R₅ is phenyl and
R₆ is H;
wherein when R₁ is 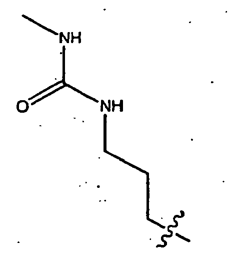 R₃ is 4-chlorophenyl;
R₅ and R₆ are each H;
wherein when R₁ is 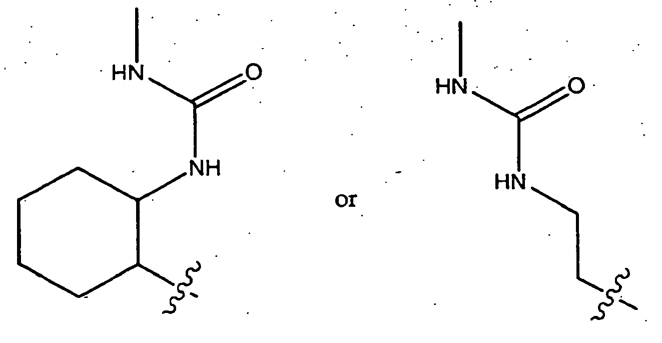 R₅ and R₆ are each independently H or alkyl;
wherein when R1 is 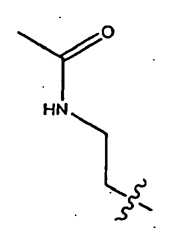 R3 is phenyl; and
R5 and R6 are both H, or
R3 is phenyl;
R5 is 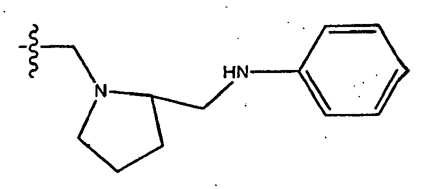 and R6 is H, or
R3 is 4-pyridyl;
R5 is CH3 and R6 is

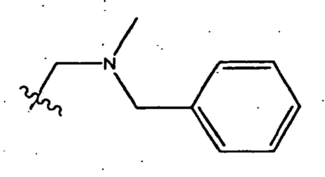

The invention further pertains to pharmaceutical compositions for treating a N-6 substituted 7-deazapurine responsive state in a mammal, *e.g.*, asthma, bronchitis, allergic rhinitis, chronic obstructive pulmonary disease, renal disorders, gastrointestinal disorders, and eye disorders. The pharmaceutical composition includes a therapeutically effective amount of a N-6 substituted 7-deazapurine and a pharmaceutically acceptable carrier.

The present invention also pertains to packaged pharmaceutical compositions for treating a N-6 substituted 7-deazapurine responsive state in a mammal. The packaged pharmaceutical composition includes a container holding a therapeutically effective amount of at least one N-6 substituted 7-deazapurine and instructions for using the N-6 substituted 7-deazapurine for treating a N-6 substituted 7-deazapurine responsive state in a mammal.

The deazapurines of the present invention may advantageously be selective A receptor antagonists. These compounds may be useful for numerous therapeutic uses such as, for example, the treatment of asthma, kidney failure associated with heart failure, and glaucoma. In a particularly preferred embodiment, the deazapurine is a water soluble prodrug that is capable of being metabolized in vivo to an active drug by, for example, esterase catalyzed hydrolysis.

In one aspect the invention features a method for inhibiting the activity of an adenosine receptor (*e.g*., A₃) in a cell in vitro, by contacting the cell with N-6 substituted 7-deazapurine (*e.g.,* preferably, an adenosine receptor antagonist).

In another aspect, the invention features the use of an effective amount of an N-6 substituted 7-deazapurine of formula I for treating damage to the eye of an animal(e.g., a human) . Preferably, the N-6 substituted 7-deazapurine is an antagonist of A₃ adenosine receptors in cells of the animal. The damage is to the retina or the optic nerve head and may be acute or chronic. The damage may be the result of, for example, glaucoma, edema, ischemia, hypoxia or trauma.

The invention also features a pharmaceutical composicion comprising a N-6 substituted compound of formula I. Preferably, the pharmaceutical preparation is an ophthalmic formulation (*e.g.*, an periocular, retrobulbar or intraocular injection formulation, a systemic formulation, or a surgical irrigating solution).

The invention further pertains to a method for inhibiting the activity of an adenosine receptor (*e.g.*, an A₂ₐ adenosine receptor) in a cell in vitro by contacting the cell with a compound of the invention. Preferably, the compound is an antagonist of the receptor.

The invention also pertains to the use of an effective amount of a compound of formula I (*e.g.*, an antagonise of A₂₂) for treating a gastrointestinal disorder (*e.g.*, diarrhea) or a respiratory disorder (*e.g.*, allergic rhinitis, chronic obstructive pulmonary disease) in an animal. Preferably, the animal is a human.

This invention also features a method for inhibiting the Activity of an A₁ adenosine receptor in a cell in vitro, which comprises contacting said cell with the above-mentioned compounds.

The features and other details of the invention will now be more particularly described and pointed out in the claims. It will be understood that the particular embodiments of the invention are shown by way of illustration and not as limitations of the invention. The principle features of this invention can be employed in various embodiments without departing from the scope of the invention.

The present invention pertains to the use of a therapeutically effective amount of a N-6 substituted 7-deazapurine, described *infra*, for treating a N-6 substituted 7-deazapurine responsive state in a mammal.

The language "N-6 substituted 7-deazapurine responsive state" is intended to include a disease state or condition characterized by its responsiveness to treatment with a N-6 substituted 7-deazapurine of the invention as described *infra, e.g.*, the treatment includes a significant diminishment of at least one symptom or effect of the state achieved with a N-6 substituted 7-deazapurine of the invention. Typically such states are associated with an increase of adenosine within a host such that the host often experiences physiological symptoms which include, but are not limited to, release of toxins, inflammation, coma, water retention, weight gain or weight loss, pancreatitis, emphysema, rheumatoid arthritis, osteoarthritis, multiple organ failure, infant and adult respiratory distress syndrome, allergic rhinitis, chronic obstructive pulmonary disease, eye disorders, gastrointestinal disorders, skin tumor promotion, immunodeficiency and asthma. (See for example, C.E. Muller and B. Stein "Adenosine Receptor Antagonists: Structures and Potential Therapeutic Applications," *Current Pharmaceutical Design,* **2**:501 (1996) and C.E. Muller "A₁-Adenosine Receptor Antagonists," Exp. *Opin. Ther. Patents* 7(5) :419 (1997) and I. Peoktistove. R. Polosa, S. T. Holgate and I. Biaggioni "Adenosine A₂₃ receptors: a novel therapeutic target in asthma?" Tips 19; 148 (1998)). The effects often associated with such symptoms include, but are not limited to, fever, shortness of breath, nausea, diarrhea, weakness, headache, and even death. In one embodiment, a N-6 substituted 7-deazapurine responsive state includes those disease states which are mediated by stimulation of adenosine receptors, *e.g.,* A₁, A₂ₐ, A_{2b}, A₃, etc., such that calcium concentrations in cells and/or activation of PLC (phospholipase C) is modulated. In a preferred embodiment, a N-6 substituted 7-deazapurine responsive state is associated with adenosine receptor(s), *e.g.*, the N-6 substituted 7-deazapurine acts as an antagonist. Examples of suitable responsive states which can be treated by the compounds of the invention, *e.g*., adenosine receptor subtypes which mediate biological effects, include central nervous system (CNS) effects, cardiovascular effects, renal effects, respiratory effects, immunological effects, gastro-intestinal effects and metabolic effects. The relative amount of adenosine in a subject can be associated with the effects listed below; that is increased levels of adenosine can trigger an effect, *e.g*., an undesired physiological response, *e.g*., an asthmatic attack.

CNS effects include decreased transmitter release (A₁), sedation (A₁), decreased locomotor activity (A₂ₐ), anticonvulsant activity, chemoreceptor stimulation (A₂) and hyperalgesia. Therapeutic applications of the inventive compounds include treatment of dementia, Alzheimer's disease and memory enhancement.

Cardiovascular effects include vasodilation (A₂ₐ), (A_{2b}) and (A₃), vasoconstriction (A₁), bradycardia (A₁), platelet inhibition (A₂ₐ), negative cardiac inotropy and dromotropy (A₁), arrhythmia, tachycardia and angiogenesis. Therapeutic applications of the inventive compounds include, for example, prevention of ischaemia-induced impairment of the heart and cardiotonics, myocardial tissue protection and restoration of cardiac function.

Renal effects include decreased GFR (A₁), mesangial cell contraction (A₁), antidiuresis (A₁) and inhibition of renin release (A₁). Suitable therapeutic applications of the inventive compounds include use of the inventive compounds as diuretic, natriuretic, potassium-sparing, kidney-protective/prevention of acute renal failure, antihypertensive, anti-oedematous and anti-nephritic agents.

Respiratory effects include bronchodilation (A₂), bronchoconstriction (A₁), chronic obstructive pulmonary disease, allergic rhinitis, mucus secretion and respiratory depression (A₂). Suitable therapeutic applications for the compounds of the invention include anti-asthmatic applications, treatment of lung disease after transplantation and respiratory disorders.

Immunological effects include immunosuapression (A₂), neutrophil chemotaxis (A₁), neutrophil superoxide generation (A₂ₐ) and mast cell degranulation (A_{2b} and A₃) Therapeutic applications of antagonists include allergic and non allergic inflammation, *e.g*., release of histamine and other inflammatory mediators.

Gastrointestinal effects include inhibition of acid secretion (A₁) therapeutic application may include reflux and ulcerative conditions Gastrointestinal effects also include colonic, intestinal and diarrheal disease, *e.g.,* diarrheal disease associated with intestinal inflammation (A_{2b}).

Eye disorders include retinal and optic nerve head injury and trauma related disorders (A₃). In a preferred embodiment, the eye disorder is glaucoma.

Other therapeutic applications of the compounds of the invention include treatment of obesity (lipolytic properties), hypertension, treatment of depression, sedative, anxiolytic, as antileptics and as laxatives, *e.g.*, effecting motility without causing diarrhea.

The term "disease state" is intended to include those conditions caused by or associated with unwanted levels of adenosine, adenylyl cyclase activity, increased physiological activity associated with aberrant stimulation of adenosine receptors and/or an increase in cAMP. In one embodiment, the disease state is, for example, asthma, chronic obstructive pulmonary disease, allergic rhinitis, bronchitis, renal disorders, gastrointestinal disorders, or eye disorders. Additional examples include chronic bronchitis and cystic fibrosis. Suitable examples of inflammatory diseases include non-lymphocytic leukemia, myocardial ischaemia, angina, infarction, cerebrovascular ischaemia, intermittent claudication, critical limb ischemia, venous hypertension, varicose veins, venous ulceration and arteriosclerosis. Impaired reperfusion states include, for example, any post-surgical trauma, such as reconstructive surgery, thrombolysis or angioplasty.

The language "treatment of a N-6 substituted 7-deazapurine responsive state" or "treating a N-6 substituted 7-deazapurine responsive state" is intended to include changes in a disease state or condition, as described above, such that physiological symptoms in a mammal can be significantly diminished or minimized. The language also includes control, prevention or inhibition of physiological symptoms or effects associated with an aberrant amount of adenosine. In one preferred embodiment, the control of the disease state or condition is such that the disease state or condition is eradicated. In another preferred embodiment, the control is selective such that aberrant levels of adenosine receptor activity are controlled while other physiologic systems and parameters are unaffected.

The term "N-6 substituted 7-deazapurine" is art recognized and is intended to include those compounds having the formula I: 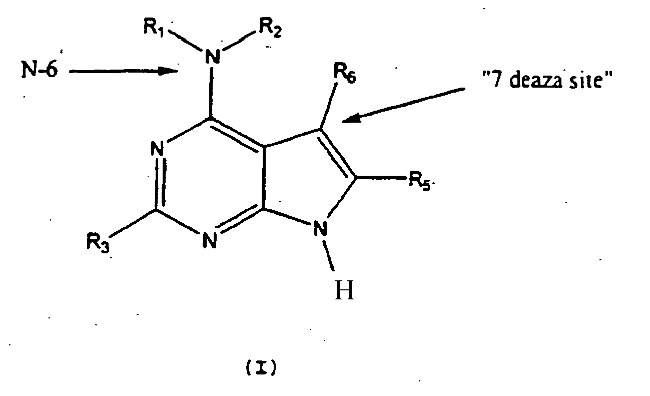 "N-substituted 7-deazapurine" includes pharmaceutically acceptable salts thereof.

The language "therapeutically effective amount" of an N-6 substituted 7-deazapurine, described *infra,* is that amount of a therapeutic compound necessary or sufficient to perform its intended function within a mammal, *e.g.*, treat a N-6 substituted 7-deazapurine responsive state, or a disease state in a mammal. An effective amount of the therapeutic compound can vary according to factors such as the amount of the causative agent already present in the mammal, the age, sex, and weight of the mammal, and the ability of the therapeutic compounds of the present invention to affect a N-6 substituted 7-deazapurine responsive state in the mammal. One of ordinary skill in the art would be able to study the aforementioned factors and make a determination regarding the effective amount of the therapeutic compound without undue experimentation. An *in vitro* or *in vivo* assay also can be used to determine an "effective amount" of the therapeutic compounds described *infra*. The ordinarily skilled artisan would select an appropriate amount of the therapeutic compound for use in the aforementioned assay or as a therapeutic treatment.

A therapeutically effective amount preferably diminishes at least one symptom or effect associated with the N-6 substituted 7-deazapurine responsive state or condition being treated by at least about 20%, (more preferably by at least about 40%, even more preferably by at least about 60%, and still more preferably by at least about 80%) relative to untreated subjects. Assays can be designed by one skilled in the art to measure the diminishment of such symptoms and/or effects. Any art recognized assay capable of measuring such parameters are intended to be included as part of this invention. For example, if asthma is the state being treated, then the volume of air expended from the lungs of a subject can be measured before and after treatment for measurement of increase in the volume using an art recognized technique. Likewise, if inflammation is the state being treated, then the area which is inflamed can be measured before and after treatment for measurement of diminishment in the area inflamed using an art recognized technique.

The term "cell" includes both prokaryotic and eukaryotic cells.

The term "animal" includes any organism with adenosine receptors or any organism susceptible to a N-6-substituted 7-deazapurine responsive state. Examples of animals include yeast, mammals, reptiles, and birds. It also includes transgenic animals.

The term "mammal" is art recognized and is intended to include an animal, more preferably a warm-blooded animal, most preferably cattle, sheep, pigs, horses, dogs, cats, rats, mice, and humans. Mammals susceptible to a N-6 substituted 7-deazapurine responsive state, inflammation, emphysema, asthma, central nervous system conditions, or acute respiratory distress syndrome, for example, are included as part of this invention.

In another aspect, the present invention pertains to the use of a therapeutically effective amount of a N-6 substituted 7-deazapurine for modulating an adenosine receptor(s) in a mammal. Suitable adenosine receptors include the families of A₁, A₂, or A₃. In a preferred embodiment, the N-6 substituted 7-deazapurine is an adenosine receptor antagonist.

The language "modulating an adenosine receptor" is intended to include those instances where a compound interacts with an adenosine receptor(s), causing increased, decreased or abnormal physiological activity associated with an adenosine receptor or subsequent cascade effects resulting from the modulation of the adenosine receptor. Physiological activities associated with adenosine receptors include induction of sedation, vasodilation, suppression of cardiac rate and contractility, inhibition of platelet aggregbility, stimulation of gluconeogenesis, inhibition of lipolysis, opening of potassium channels, reducing flux of calcium channels, etc.

The terms "modulate", "modulating" and "modulation" are intended to include preventing, eradicating, or inhibiting the resulting increase of undesired physiological activity associated with abnormal stimulation of an adenosine receptor, *e.g.*, in the context of the therapeutic methods of the invention. In another embodiment, the term modulate includes antagonistic effects, *e.g.*, diminishment of the activity or production of mediators of allergy and allergic inflammation which results from the overstimulation of adenosine receptor(s). For example, the therapeutic deazapurines of the invention can interact with an adenosine receptor to inhibit, for example, adenylate cyclase activity.

The language "condition characterized by aberrant adenosine receptor activity" is intended to include those diseases, disorders or conditions which are associated with aberrant stimulation of an adenosine receptor, in that the stimulation of the receptor causes a biochemical and or physiological chain of events that is directly or indirectly associated with the disease, disorder or condition. This stimulation of an adenosine receptor does not have to be the sole causative agent of the disease, disorder or condition but merely be responsible for causing some of the symptoms typically associated with the disease, disorder, or condition being treated. The aberrant stimulation of the receptor can be the sole factor or at least one other agent can be involved in the state being treated. Examples of conditions include those disease states listed supra, including inflammation, gastrointestinal disorders and those symptoms manifested by the presence of increased adenosine receptor activity. Preferred examples include those symptoms associated with asthma, allergic rhinitis, chronic obstructive pulmonary disease, emphysema, bronchitis, gastrointestinal disorders and glaucoma.

The language "treating or treatment of a condition characterized by aberrant adenosine receptor activity" is intended to include the alleviation of or diminishment of at least one symptom typically associated with the condition. The treatment also includes alleviation or diminishment of more than one symptom. Preferably, the treatment cures, *e.g.*, substantially eliminates, the symptoms associated with the condition.

The present invention pertains to compounds, N-6 substituted 7-deazapurines, having the formula I: 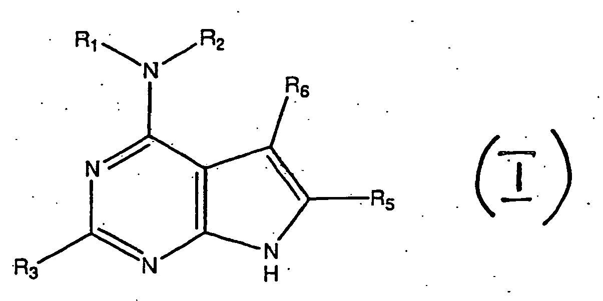 wherein R₁ is 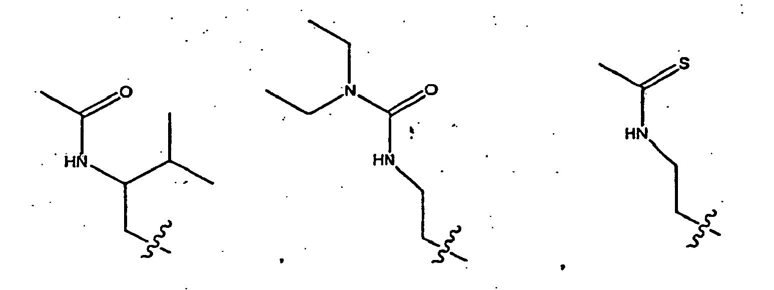 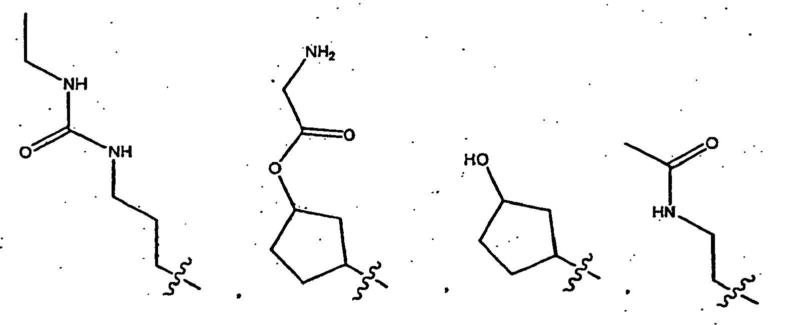 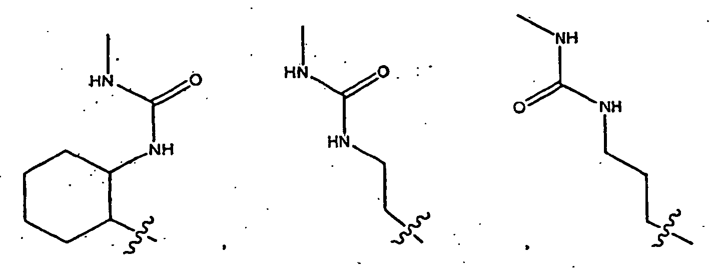 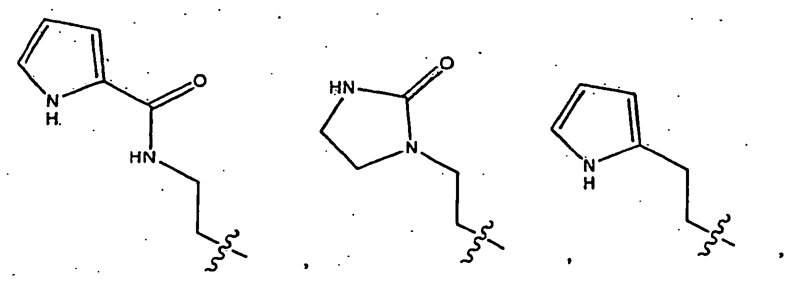 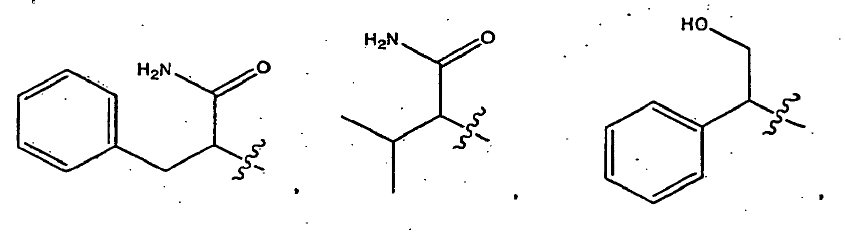 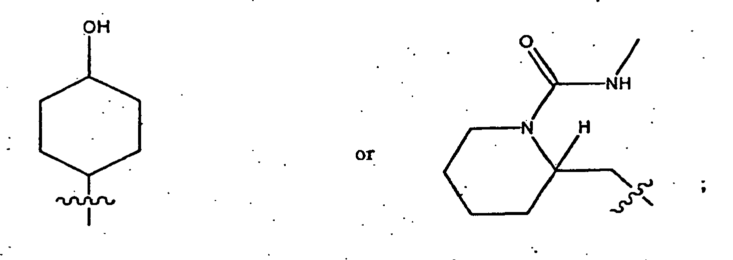 and
R2 is H; or
NR₁R₂ together are 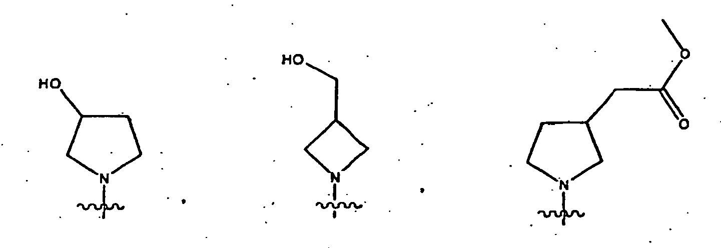 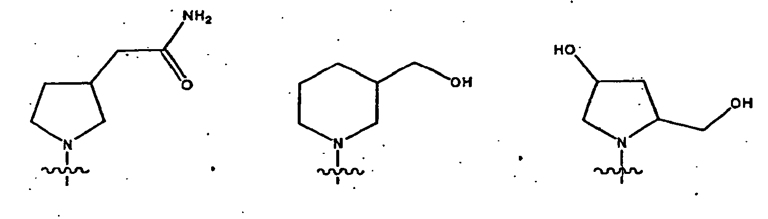 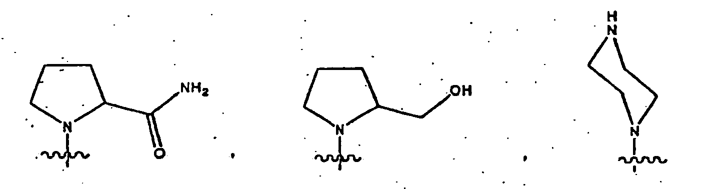 wherein R₃ is a substituted or unsubstituted phenyl, pyrrole, thiophene, furan, thiazole, imidazole, triazole, pyrazole, pyridine, 2(1H)-pyridone, 4 (1H)-pyridone, pyrazine, pyrimidine, pyridazine, isothiazole, isoxazole, oxazole, tetrazole, naphthyl, tetralin, benzofuran, benzothiophene, 2,3-dihydroindole, 1H-indole, indolyl, benzopyrazole, 1,3-benzodioxole, benzoxazole, purine, coumarin, chromone, quinolyl, tetrahydroquinoline, isoquinoline, benzimidazole, quinazoline, pteridine, 2(1H) - quinolone, 1(2H)-isoquinolone, 1,4-benzisoxazine, benzothiazole, quinoxaline, quinoline-N-oxide, isoquinoline-N-oxide, quinoxaline-N-oxide, quinazoline-N-oxide; benzoxazine, phthalazine, or cinnoline; wherein R₅ is H, CH₃, substituted or unsubstituted alkyl, aryl or phenyl,
or 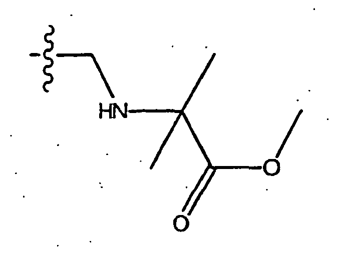 and
wherein R₆ is H, CH₃ substituted or unsubstituted alkyl, cycloalkyl,
wherein the substituent, when present, is halogen, hydroxyl, alkoxy, alkylcarbonyloxy, arylcarbonyloxy, alkoxycarbonyloxy, aryloxycarbonyloxy, carboxylate, alkylcarbonyl, alkoxycarbonyl, aminocarbonyl, alkylthiocarbonyl, phosphate, phosphonato, phosipinato, cyano, amino, acylamino, amidino, imino, sulfhydryl, alkylthio, arylthio, thiocarboxylate, sulfates, sulfonato, sulfamoyl, sulfonamido, nitro, trifluoromehyl, cyano, azido, heterocyclyl, or alkylaryl,
or a pharmaceutically acceptable salt thereof,
wherein when R₁ is 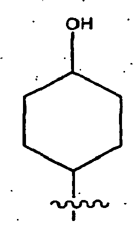 R3 is phenyl;
R₅ is 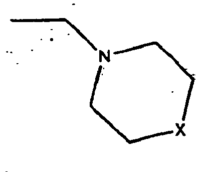 and R₆ is H;
wherein X is O or S; and
wherein when R₁ is 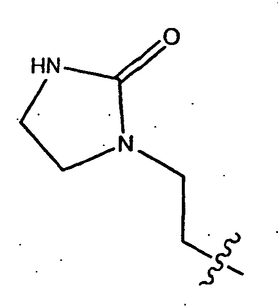 R₃ is phenyl;
R₅ is phenyl and
R₆ is H;
wherein when R₁ is 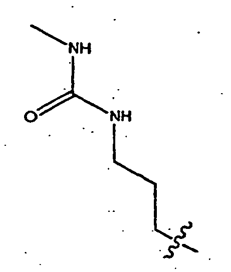 R₃ is 4-chlorophenyl;
R₅ and R₆ are each H;
wherein when R₁ is 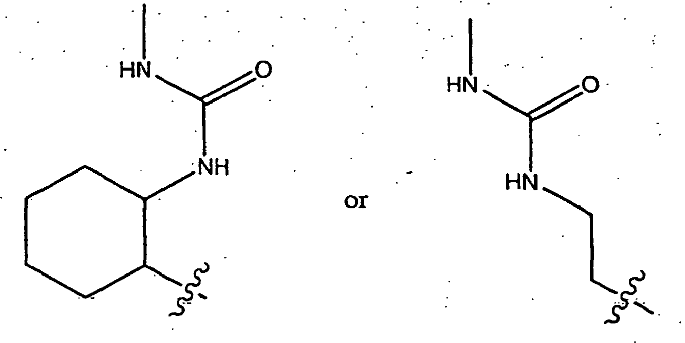 R₅ and R₆ are each independently H or alkyl;
wherein when R1 is 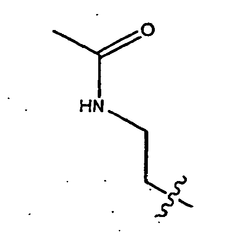 R3 is phenyl; and
R5 and R6 are both H, or
R3 is phenyl;
R5 is 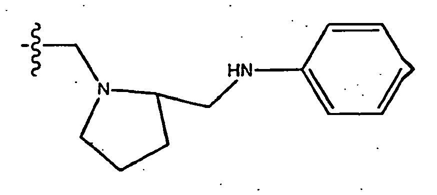 and R6 is H, or
R3 is 4-pyridyl;
R5 is CH3 and R6 is

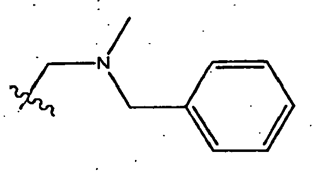

The compounds of the invention may comprise water-soluble prodrugs which are described in WO 99/33815, International Application No. PCT/US98/04595, filed March 9, 1998 and published July 8, 1999. The entire content of WO 99/33815 is expressly incorporated herein by reference. The water-soluble prodrugs are metabolized *in vivo* to an active drug, *e.g.*, by esterase catalyzed hydrolysis. Examples of potential prodrugs include deazapurines with, for example, R₁ as cycloalkyl substituted with -OC(O)(Z)NH₂, wherein Z is a side chain of a naturally or unnaturally occurring amino acid, or analog thereof, an α, β, γ, or ω amino acids, or a dipeptide. Preferred amino acid side chains include those of glycine, alanine, valine, leucine, isoleucine, lysine, α-methylalanine, aminocyclopropane carboxylic acid, azetidine-2-carboxylic acid, β-alanine, γ-aminobutyric acid, alanine-alanine, or glycine-alanine.

In one embodiment, the deazapurine is 4-(*cis*-3-hydroxycyclopentyl)amino-5,6-dimethyl-2-phenyl-7*H*-pyrrolo [2,3d]pyrimidine.

In another embodiment, the deazapurine is 4-(*cis*-3-(2-aminoacetoxy) cyclopentyl)amino-5,6-dimethyl-2-phenyl-7*H-*pyrrolo[2,3d] pyrimidine trifluoroacetic acid salt.

In yet another embodiment, the invention features a method for inhibiting the activity of an adenosine receptor (*e.g*., A₁, A_{2A}, A_{2B}, or, preferably, A₃) in a cell in vitro, by contacting the cell with N-6 substituted 7-deazapurine (*e.g*., preferably, an adenosine receptor antagonist).

In another aspect, the invention features the use of an effective amount of an N-6 substituted 7-deazapurine for treating damage to the eye of an animal (e.g., a human) . Preferably, the N-6 substituted 7-deazapurine is an antagonist of A₃ adenosine receptors in cells of the animal. The damage is to the retina or the optic nerve head and may be acute or chronic. The damage may be the result of, for example, glaucoma, edema, ischemia, hypoxia or trauma.

In another embodiment, the invention relates to a pharmaceutical composition containing an N-6 substituted 7-deazapurine of the invention and a pharmaceutically acceptable carrier.

The invention also pertains to the use of a therapeutically effective amount of a deazapurine of the invention for treating a N-6 substituted 7-deazapurine responsive state in an animal , such that treatment of a N-6 substituted 7-deazapurine responsive state in the animal occurs. Advantageously, the disease state may be a disorder mediated by adenosine. Examples of preferred disease states include: central nervous system disorders, cardiovascular disorders, renal disorders, inflammatory disorders, allergic disorders, gastrointestinal disorders, eye disorders, and respiratory disorders.

The term "alkyl" refers to the radical of saturated aliphatic groups, including straight-chain alkyl groups, branched-chain alkyl groups, cycloalkyl (alicyclic) groups, alkyl substituted cycloalkyl groups, and cycloalkyl substituted alkyl groups. The term alkyl further includes alkyl groups, which can further include oxygen, nitrogen, sulfur or phosphorous atoms replacing one or more carbons of the hydrocarbon backbone, *e.g.,* oxygen, nitrogen, sulfur or phosphorous atoms. In preferred embodiments, a straight chain or branched chain alkyl has 30 or fewer carbon atoms in its backbone (*e.g.,* C₁-C₃₀ for straight chain, C₃-C₃₀ for branched chain), and more preferably 20 or fewer. Likewise, preferred cycloalkyls have from 4-10 carbon atoms in their ring structure, and more preferably have 5, 6 or 7 carbons in the ring structure.

Moreover, the term alkyl as used throughout the specification and claims is intended to include both "unsubstituted alkyls" and "substituted alkyls", the latter of which refers to alkyl moieties having substituents replacing a hydrogen on one or more carbons of the hydrocarbon backbone. Such substituents can include, for example, halogen, hydroxyl, alkylcarbonyloxy, arylcarbonyloxy, alkoxycarbonyloxy, aryloxycarbonyloxy, carboxylate, alkylcarbonyl, alkoxycarbonyl, aminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, sulfonato, sulfamoyl, sulfonamido, nitro, trifluoromethyl, cyano, azido, heterocyclyl, alkylaryl, or an aromatic or heteroaromatic moiety. It will be understood by those skilled in the art that the moieties substituted on the hydrocarbon chain can themselves be substituted, if appropriate. Cycloalkyls can be further substituted, *e.g*., with the substituents described above. An "alkylaryl" moiety is an alkyl substituted with an aryl (*e.g*., phenylmethyl (benzyl)). The term "alkyl" also includes unsaturated aliphatic groups analogous in length and possible substitution to the alkyls described above, but that contain at least one double or triple bond respectively.

The term "aryl" as used herein, refers to the radical of aryl groups, including 5- and 6-membered single-ring aromatic groups that may include from zero to four heteroatoms, for example, benzene, pyrrole, furan, thiophene, imidazole, benzoxazole, benzothiazole, triazole, tetrazole, pyrazole, pyridine, pyrazine, pyridazine and pyrimidine, and the like. Aryl groups also include polycyclic fused aromatic groups such as naphthyl, quinolyl, indolyl, and the like. Those aryl groups having heteroatoms in the ring structure may also be referred to as "aryl heterocycles", "heteroaryls" or "heteroaromatics". The aromatic ring can be substituted at one or more ring positions with such substituents as described above, as for example, halogen, hydroxyl, alkoxy, alkylcarbonyloxy, arylcarbonyloxy, alkoxycarbonyloxy, aryloxycarbonyloxy, carboxylate, alkylcarbonyl, alkoxycarbonyl, aminocarbonyl, alkylthiocarbonyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, sulfonato, sulfamoyl, sulfonamido, nitro, trifluoromethyl, cyano, azido, heterocyclyl, alkylaryl, or an aromatic or heteroaromatic moiety Aryl groups can also be fused or bridged with alicyclic or heterocyclic rings which are not aromatic so as to form a polycycle (*e.g.*, tetralin).

The terms "alkenyl" and "alkynyl" refer to unsaturated aliphatic groups analogous in length and possible substitution to the alkyls described above, but that contain at least one double or triple bond respectively. For example, the invention contemplates cyano and propargyl groups.

Unless the number of carbons is otherwise specified, "lower alkyl" as used herein means an alkyl group, as defined above, but having from one to ten carbons, more preferably from one to six carbon atoms in its backbone structure, even more preferably one to three carbon atoms in its backbone structure. Likewise, "lower alkenyl" and "lower alkynyl" have similar chain lengths.

The terms "alkoxyalkyl" , "polyaminoalkyl" and "thioalkoxyalkyl" refer to alkyl groups, as described above which further include oxygen, nitrogen or sulfur atoms replacing one or more carbons of the hydrocarbon backbone, *e.g*., oxygen, nitrogen or sulfur atoms.

The terms "polycyclyl" or "polycyclic radical" refer to the radical of two or more cyclic rings (*e.g.*, cycloalkyls, cycloalkenyls, cycloalkynyls, aryls and/or heterocyclyls) in which two or more carbons are common to two adjoining rings, *e.g.*, the rings are "fused rings". Rings that are joined through non-adjacent atoms are termed "bridged" rings. Each of the rings of the polycycle can be substituted with such substituents as described above, as for example, halogen, hydroxyl, alkylcarbonyloxy, arylcarbonyloxy, alkoxycarbonyloxy, aryloxycarbonyloxy, carboxylate, alkylcarbonyl, alkoxycarbonyl, aminocarbonyl, alkylthiocarbonyl, alkoxyl, phosphate, phosphonato, phosphinato, cyano, amino (including alkyl amino, dialkylamino, arylamino, diarylamino, and alkylarylamino), acylamino (including alkylcarbonylamino, arylcarbonylamino, carbamoyl and ureido), amidino, imino, sulfhydryl, alkylthio, arylthio, thiocarboxylate, sulfates, sulfonato, sulfamoyl, sulfonamido, nitro, trifluoromethyl, cyano, azido, heterocyclyl, alkyl, alkylaryl, or an aromatic or heteroaromatic moiety

The term "heteroatom" as used herein means an atom of any element other than carbon or hydrogen. Preferred heteroatoms are nitrogen, oxygen, sulfur and phosphorus.

The term "amino acids" includes naturally and unnaturally occurring amino acids found in proteins such as glycine, alanine, valine, cysteine, leucine, isoleucine, serine, threonine, methionine, glutamic acid, aspartic acid, glutamine, asparagine, lysine, arginine, proline, histidine, phenylalanine, tyrosine, and tryptophan. Amino acid analogs include amino acids with lengthened or shortened side chains or variant side chains with appropriate functional groups. Amino acids also include D and L stereoisomers of an amino acid when the structure of the amino acid admits of stereoisomeric forms. The term "dipeptide" includes two or more amino acids linked together. Preferably, dipeptides are two amino acids linked via a peptide linkage. Particularly preferred dipeptides include, for example, alanine-alanine and glycine-alanine.

It will be noted that the structure of some of the compounds of this invention includes asymmetric carbon atoms and thus occur as racemates and racemic mixtures, single enantiomers, diastereomeric mixtures and individual diastereomers. All such isomeric forms of these compounds are expressly included in this invention. Each stereogenic carbon may be of the R or S configuration. It is to be understood accordingly that the isomers arising from such asymmetry (*e.g.*, all enantiomers and diastereomers) are included within the scope of this invention, unless indicated otherwise. Such isomers can be obtained in substantially pure form by classical separation techniques and by stereochemically controlled synthesis.

The invention further pertains to pharmaceutical compositions for treating a N-6 substituted 7-deazapurine responsive state in a mammal, *e.g*., respiratory disorders (*e.g*., asthma, bronchitis, chronic obstructive pulmonary disorder, and allergic rhinitis), renal disorders, gastrointestinal disorders, and eye disorders. The pharmaceutical composition includes a therapeutically effective amount of a N-6 substituted 7-deazapurine, described *supra,* and a pharmaceutically acceptable carrier. It is to be understood, that all of the deazapurines described above are included for therapeutic treatment. It is to be further understood that the deazapurines of the invention can be used alone or in combination with other deazapurines of the invention or in combination with additional therapeutic compounds, such as antibiotics, antiinflammatories, or anticancer agents, for example.

The term "antibiotic" is art recognized and is intended to include those substances produced by growing microorganisms and synthetic derivatives thereof, which eliminate or inhibit growth of pathogens and are selectively toxic to the pathogen while producing minimal or no deleterious effects upon the infected host subject. Suitable examples of antibiotics include, but are not limited to, the principle classes of aminoglycosides, cephalosporins, chloramphenicols, fuscidic acids, macrolides, penicillins, polymixins, tetracyclines and streptomycins.

The term "antiinflammatory" is art recognized and is intended to include those agents which act on body mechanisms, without directly antagonizing the causative agent of the inflammation such as glucocorticoids, aspirin, ibuprofen, NSAIDS, etc.

The term "anticancer agent" is art recognized and is intended to include those agents which diminish, eradicate, or prevent growth of cancer cells without, preferably, adversely affecting other physiological functions. Representative examples include cisplatin and cyclophosphamide.

When the compounds of the present invention are administered as pharmaceuticals, to humans and mammals, they can be given per se or as a pharmaceutical composition containing, for example, 0.1 to 99.5% (more preferably, 0.5 to 90%) of active ingredient in combination with a pharmaceutically acceptable carrier.

The phrase "pharmaceutically acceptable carrier" as used herein means a pharmaceutically acceptable material, composition or vehicle, such as a liquid or solid filler, diluent, excipient, solvent or encapsulating material, involved in carrying or transporting a compound(s) of the present invention within or to the subject such that it can performs its intended function. Typically, such compounds are carried or transported from one organ, or portion of the body, to another organ, or portion of the body. Each carrier must be "acceptable" in the sense of being compatible with the other ingredients of the formulation and not injurious to the patient. Some examples of materials which can serve as pharmaceutically acceptable carriers include: sugars, such as lactose, glucose and sucrose; starches, such as corn starch and potato starch; cellulose, and its derivatives, such as sodium carboxymethyl cellulose, ethyl cellulose and cellulose acetate; powdered tragacanth; malt; gelatin; talc; excipients, such as cocoa butter and suppository waxes; oils, such as peanut oil, cottonseed oil, safflower oil, sesame oil, olive oil, corn oil and soybean oil; glycols, such as propylene glycol; polyols, such as glycerin, sorbitol, mannitol and polyethylene glycol; esters, such as ethyl oleate and ethyl laurate; agar; buffering agents, such as magnesium hydroxide and aluminum hydroxide; alginic acid; pyrogen-free water; isotonic saline; Ringer's solution; ethyl alcohol; phosphate buffer solutions; and other non-toxic compatible substances employed in pharmaceutical formulations.

As set out above, certain embodiments of the present compounds can contain a basic functional group, such as amino or alkylamino, and are, thus, capable of forming pharmaceutically acceptable salts with pharmaceutically acceptable acids. The term "pharmaceutically acceptable salts" in this respect, refers to the relatively non-toxic, inorganic and organic acid addition salts of compounds of the present invention. These salts can be prepared *in situ* during the final isolation and purification of the compounds of the invention, or by separately reacting a purified compound of the invention in its free base form with a suitable organic or inorganic acid, and isolating the salt thus formed. Representative salts include the hydrobromide, hydrochloride, sulfate, bisulfate, phosphate, nitrate, acetate, valerate, oleate, palmitate, stearate, laurate, benzoate, lactate, phosphate, tosylate, citrate, maleate, fumarate, succinate, tartrate, napthylate, mesylate, glucoheptonate, lactobionate, and laurylsulphonate salts and the like. (*See, e.g., Berge et al*. (1977) "Pharmaceutical Salts", *J. Pharm. Sci.* 66:1-19).

In other cases, the compounds of the present invention may contain one or more acidic functional groups and, thus, are capable of forming pharmaceutically acceptable salts with pharmaceutically acceptable bases. The term "pharmaceutically acceptable salts" in these instances refers to the relatively non-toxic, inorganic and organic base addition salts of compounds of the present invention. These salts can likewise be prepared *in situ* during the final isolation and purification of the compounds, or by separately reacting the purified compound in its free acid form with a suitable base, such as the hydroxide, carbonate or bicarbonate of a pharmaceutically acceptable metal cation, with ammonia, or with a pharmaceutically acceptable organic primary, secondary or tertiary amine. Representative alkali or alkaline earth salts include the lithium, sodium, potassium, calcium, magnesium, and aluminum salts and the like. Representative organic amines useful for the formation of base addition salts include ethylamine, diethylamine, ethylenediamine, ethanolamine, diethanolamine, piperazine and the like.

The term "pharmaceutically acceptable esters" refers to the relatively non-toxic, esterified products of the compounds of the present invention. These esters can be prepared *in situ* during the final isolation and purification of the compounds, or by separately reacting the purified compound in its free acid form or hydroxyl with a suitable esterifying agent. Carboxylic acids can be converted into esters via treatment with an alcohol in the presence of a catalyst. Hydroxyl containing derivatives can be converted into esters via treatment with an esterifying agent such as alkanoyl halides. The term is further intended to include lower hydrocarbon groups capable of being solvated under physiological conditions, *e.g.*, alkyl esters, methyl, ethyl and propyl esters. (See, for example, Berge *et al., supra*.)

The invention further contemplates the use of prodrugs which are converted *in vivo* to the therapeutic compounds of the invention (see, *e.g.*, R.B. Silverman, 1992, "The Organic Chemistry of Drug Design and Drug Action", Academic Press, Chapter 8). Such prodrugs can be used to alter the biodistribution (*e.g.*, to allow compounds which would not typically enter the reactive site of the protease) or the pharmacokinetics of the therapeutic compound. For example, a carboxylic acid group, can be esterified, *e.g.,* with a methyl group or an ethyl group to yield an ester. When the ester is administered to a subject, the ester is cleaved, enzymatically or non-enzymatically, reductively or hydrolytically, to reveal the anionic group. An anionic group can be esterified with moieties (*e.g*., acyloxymethyl esters) which are cleaved to reveal an intermediate compound which subsequently decomposes to yield the active compound. In another embodiment, the prodrug is a reduced form of a sulfate or sulfonate, *e.g*., a thiol, which is oxidized *in vivo* to the therapeutic compound. Furthermore, an anionic moiety can be esterified to a group which is actively transported *in vivo*, or which is selectively taken up by target organs. The ester can be selected to allow specific targeting of the therapeutic moieties to particular reactive sites, as described below for carrier moieties.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: water soluble antioxidants, such as ascorbic acid, cysteins hydrochloride, sodium bisulfate, sodium metabisulfite, sodium sulfite and the like; oil-soluble antioxidants, such as ascorbyl palmitate, butylated hydroxyanisole (BHA), butylated hydroxytoluene (BHT), lecithin, propyl gallate, alpha-tocopherol, and the like; and metal chelating agents, such as citric acid, ethylenediamine tetraacetic acid (EDTA), sorbitol, tartaric acid, phosphoric acid, and the like.

Formulations of the present invention include those suitable for oral, nasal, topical, transdermal, buccal, sublingual, rectal, vaginal and/or parenteral administration. The formulations may conveniently be presented in unit dosage form and may be prepared by any methods well known in the art of pharmacy. The amount of active ingredient which can be combined with a carrier material to produce a single dosage form will generally be that amount of the compound which produces a therapeutic effect. Generally, out of one hundred per cent, this amount will range from about 1 per cent to about ninety-nine percent of active ingredient, preferably from about 5 per cent to about 70 per cent, most preferably from about 10 per cent to about 30 per cent.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

Formulations of the invention suitable for oral administration may be in the form of capsules, cachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

In solid dosage forms of the invention for oral administration (capsules, tablets, pills, dragees, powders, granules and the like), the active ingredient is mixed with one or more pharmaceutically acceptable carriers, such as sodium citrate or dicalcium phosphate, and/or any of the following: fillers or extenders, such as starches, lactose, sucrose, glucose, mannitol, and/or silicic acid; binders, such as, for example, carboxymethylcellulose, alainates, gelatin, polyvinyl pyrrolidone, sucrose and/or acacia; humectants, such as glycerol; disintegrating agents, such as agar-agar, calcium carbonate, potato or tapioca starch, alginic acid, certain silicates, and sodium carbonate; solution retarding agents, such as paraffin; absorption accelerators, such as quaternary ammonium compounds; wetting agents, such as, for example, cetyl alcohol and glycerol monostearate; absorbents, such as kaolin and bentonite clay; lubricants, such a talc, calcium stearate, magnesium stearate, solid polyethylene glycols, sodium lauryl sulfate, and mixtures thereof; and coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, as well as high molecular weight polyethylene glycols and the like.

A tablet may be made by compression or molding, optionally with one or more accessory ingredients. Compressed tablets may be prepared using binder (for example, gelatin or hydroxypropylmethyl cellulose), lubricant, inert diluent, preservative, disintegrant (for example, sodium search glycolate or cross-linked sodium carboxymethyl cellulose), surface-active or dispersing agent. Molded tablets may be made by molding in a suitable machine a mixture of the powdered compound moistened with an inert liquid diluent.

The tablets, and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulated so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally, in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the compounds of the invention include pharmaceutically acceptable emulsions, microemulsions, solutions, suspensions, syrups and elixirs. In addition to the active ingredient, the liquid dosage forms may contain inert dilutents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert dilutents, the oral compositions can also include adjuvants such as wetting agents, emulsifying and suspending agents, sweetening, flavoring, coloring, perfuming and preservative agents.

Suspensions, in addition to the active compounds, may contain suspending agents as, for example, ethoxylated isostearyl alcohols, polyoxyethylene sorbitol and sorbitan esters, microcrystalline cellulose, aluminum metahydroxide, bentonite, agar-agar and tragacanth, and mixtures thereof.

Formulations of the pharmaceutical compositions of the invention for rectal or vaginal administration may be presented as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active compound.

Formulations of the present invention which are suitable for vaginal administration also include pessaries, tampons, creams, gels, pastes, foams or spray formulations containing such carriers as are known in the art to be appropriate.

Dosage forms for the topical or transdermal administration of a compound of this invention include powders, sprays, ointments, pastes, creams, lotions, gels, solutions, patches and inhalants. The active compound may be mixed under sterile conditions with a pharmaceutically acceptable carrier, and with any preservatives, buffers, or propellants which may be required.

The ointments, pastes, creams and gels may contain, in addition to an active compound of this invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound of the present invention to the body. Such dosage forms can be made by dissolving or dispersing the compound in the proper medium. Absorption enhancers can also be used to increase the flux of the compound across the skin. The rate of such flux can be controlled by either providing a rate controlling membrane or dispersing the active compound in a polymer matrix or gel.

Ophthalmic formulations, eye ointments, powders, solutions and the like, are also contemplated as being within the scope of this invention. Preferably, the pharmaceutical preparation is an ophthalmic formulation (e.g., an periocular, retrobulbar or intraocular injection formulation, a systemic formulation, or a surgical irrigating solution).

The ophthalmic formulations of the present invention may include one or more deazapurines and a pharmaceutically acceptable vehicle. Various types of vehicles may be used. The vehicles will generally be aqueous in nature. Aqueous solutions are generally preferred, based on case of formulation, as well as a patient's ability to easily administer such compositions by means of instilling one to two drops of the solutions in the affected eyes. However, the deazapurines of the present invention may also be readily incorporated into other types of compositions, such as suspensions, viscous or semi-viscous gels or other types of solid or semi-solid compositions. The ophthalmic compositions of the present invention may also include various other ingredients, such as buffers, preservatives, co-solvents and viscosity building agents.

An appropriate buffer system (e.g., sodium phosphate, sodium acetate or sodium borate) may be added to prevent pH drift under storage conditions.

Ophthalmic products are typically packaged in multidose form. Preservatives are thus required to prevent microbial contamination during use. Suitable preservatives include: benzalkonium chloride, thimerosal, chlorobutanol, methyl paraben, propyl paraben, phenylethyl alcohol, edetate disodium, sorbic acid, polyquaternium-1, or other agents known to those skilled in the art. Such preservatives are typically employed at a level of from 0.001 to 1.0% weight/volume ("% w/v") .

When the deazapurines of the present invention are administered during intraocular surgical procedures, such as through retrobulbar or periocular injection and intraocular perfusion or injection, the use of balanced salt irrigating solutions as vehicles are most preferred. BSS® Sterile Irrigating Solution and BSS Plus® Sterile Intraocular Irrigating Solution (Alcon Laboratories, Inc., Fort Worth, Texas, USA) are examples of physiologically balanced intraocular irrigating solutions. The latter type of solution is described in U.S. Pat. No. 4,550,022 (Garabedian, *et al*.), the entire contents of which are hereby incorporated in the present specification by reference. Retrobulbar and periocular injections are known to those skilled in the art and are described in numerous publications including, for example, *Ophthalmic Surgery: Principles of Practice,* Ed., G. L. Spaeth. W. B. Sanders Co., Philadelphia, Pa.. U.S.A., pages 85-87 (1990).

As indicated above, use of deazapurines to prevent or reduce damage to retinal and optic nerve head tissues at the cellular level is a particularly important aspect of one embodiment of the invention. Ophthalmic conditions which may be treated include, but are not limited to, retinopathies, macular degeneration, ocular ischemia, glaucoma, and damage associated with injuries to ophthalmic tissues, such as ischemia reperfusion injuries, photochemical injuries, and injuries associated with ocular surgery, particularly injuries to the retina or optic nerve head by exposure to light or surgical instruments. The compounds may also be used as an adjunct to ophthalmic surgery, such as by vitreal or subconjunctival injection following ophthalmic surgery. The compounds may be used for acute treatment of temporary conditions, or may be administered chronically, especially in the case of degenerative disease. The compounds may also be used prophylactically, especially prior to ocular surgery or noninvasive ophthalmic procedures, or other types of surgery.

Pharmaceutical compositions of this invention suitable for parenteral administration comprise one or more compounds of the invention in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the compositions. In addition, prolonged absorption of the injectable pharmaceutical form may be brought about by the inclusion of agents which delay absorption such as aluminum monostearate and gelatin.

In some cases, in order to prolong the effect of a drug, it is desirable to slow the absorption of the drug from subcutaneous or intramuscular injection. This may be accomplished by the use of a liquid suspension of crystalline or amorphous material having poor water solubility. The rate of absorption of the drug then depends upon its rate of dissolution which, in turn, may depend upon crystal size and crystalline form. Alternatively, delayed absorption of a parenterally-administered drug form is accomplished by dissolving or suspending the drug in an oil vehicle.

Injectable depot forms are made by forming microencapsule matrices of the subject compounds in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions which are compatible with body tissue.

The preparations of the present invention may be given orally, parenterally, topically, or rectally. They are of course given by forms suitable for each administration route. For example, they are administered in tablets or capsule form, by injection, inhalation, eye lotion, ointment, suppository, etc. administration by injection, infusion or inhalation; topical by lotion or ointment; and rectal by suppositories. Oral administration is preferred.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

The phrases "systemic administration," "administered systematically," "peripheral administration" and "administered peripherally" as used herein mean the administration of a compound, drug or other material other than directly into the central nervous system, such that it enters the patient's system and, thus, is subject to metabolism and other like processes, for example, subcutaneous administration.

These compounds may be administered to humans and other animals for therapy by any suitable route of administration, including orally, nasally, as by, for example, a spray, rectally, intravaginally, parenterally, intracisternally, and topically, as by powders, ointments or drops, including buccally and sublingually.

Regardless of the route of administration selected, the compounds of the present invention, which may be used in a suitable hydrated form, and/or the pharmaceutical compositions of the present invention, are formulated into pharmaceutically acceptable dosage forms by conventional methods known to those of skill in the art.

Actual dosage levels of the active ingredients in the pharmaceutical compositions of this invention may be varied so as to obtain an amount of the active ingredient which is effective to achieve the desired therapeutic response for a particular patient, composition, and mode of administration, without being toxic to the patient.

The selected dosage level will depend upon a variety of factors including the activity of the particular compound of the present invention employed, or the ester, salt or amide thereof, the route of administration, the time of administration, the rate of excretion of the particular compound being employed, the duration of the treatment, other drugs, compounds and/or materials used in combination with the particular compound employed, the age, sex, weight, condition, general health and prior medical history of the patient being treated, and like factors well known in the medical arts.

A physician or veterinarian having ordinary skill in the art can readily determine and prescribe the effective amount of the pharmaceutical composition required For example, the physician or veterinarian could start doses of the compounds of the invention employed in the pharmaceutical composition at levels lower than that required in order to achieve the desired therapeutic effect and gradually increase the dosage until the desired effect is achieved.

In general, a suitable daily dose of a compound of the invention will be that amount of the compound which is the lowest dose effective to produce a therapeutic effect. Such an effective dose will generally depend upon the factors described above. Generally, intravenous and subcutaneous doses of the compounds of this invention for a patient, when used for the indicated analgesic effects, will range from about 0.0001 to about 200 mg per kilogram of body weight per day, more preferably from about 0.01 to about 150 mg per kg per day, and still more preferably from about 0.2 to about 140 mg per kg per day.

If desired, the effective daily dose of the active compound may be administered as two, three, four, five, six or more sub-doses administered separately at appropriate intervals throughout the day, optionally, in unit dosage forms.

While it is possible for a compound of the present invention to be administered alone, it is preferable to administer the compound as a pharmaceutical composition.

The present invention also pertains to packaged pharmaceutical compositions for treating a N-6 substituted 7 deazapurine responsive state, *e.g*., undesirable increased adenosine receptor activity in a mammal. The packaged pharmaceutical compositions include a container holding a therapeutically effective amount of at least one deazapurine as described *supra* and instructions for using the deazapurine for treating the deazapurine responsive state in the mammal.

The deazapurines of the invention can be prepared using standard methods for organic synthesis. Deazapurines can be purified by reverse phase HPLC, chromatography, recrystallization, etc. and their structures confirmed by mass spectral analysis, elemental analysis, IR and/or NMR spectroscopy.

Typically, synthesis of the intermediates as well as the deazapurines of the invention is performed in solution. The addition and removal of one or more protecting group is also typical practice and is known to those skilled in the art. Typical synthetic schemes for the preparation of deazapurine intermediates of the invention are outlined below in Scheme I.

The invention further pertains to a compound having the structure: 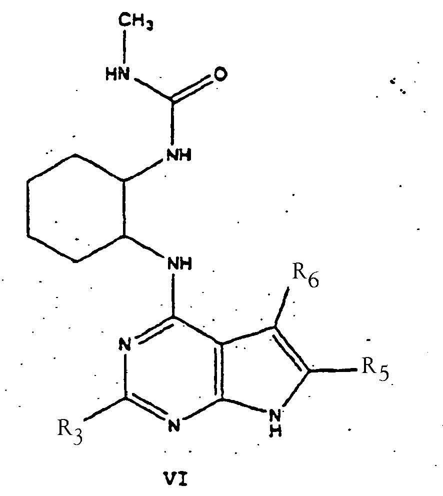 wherein R₃ is as defined above; wherein R₅ and R₆ are independently H, or alkyl.

In one embodiment of the compound, the compound has the structure:

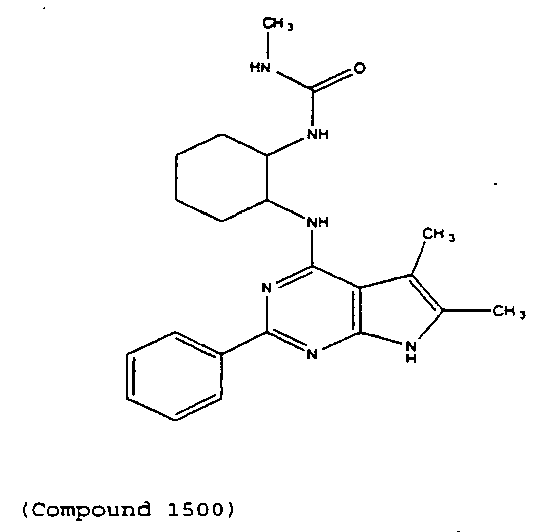

In one embodiment of the compound, the compound has the structure:

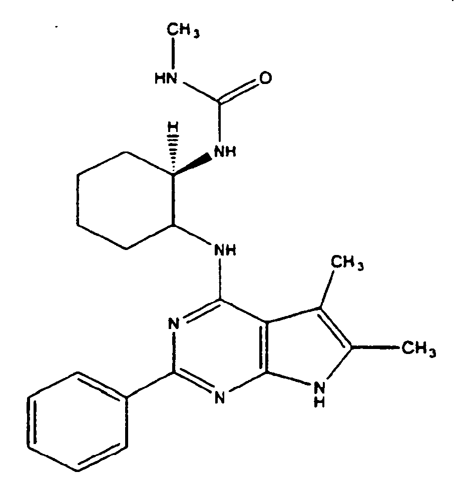

In another embodiment of the compound, the compound has the structure:

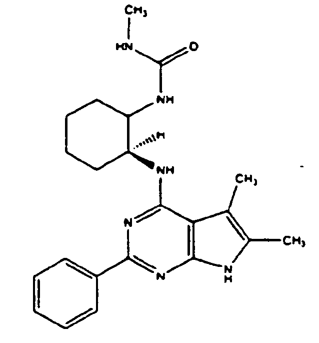

In another embodiment of compound 1500, the compound has the structure:

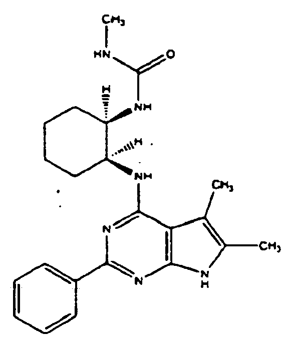

In a further embodiment of the compound, the compound has the structure:

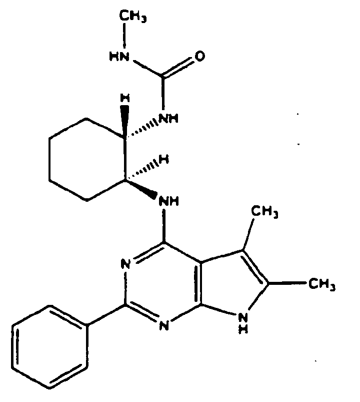

This invention also provides a compound having the structure :

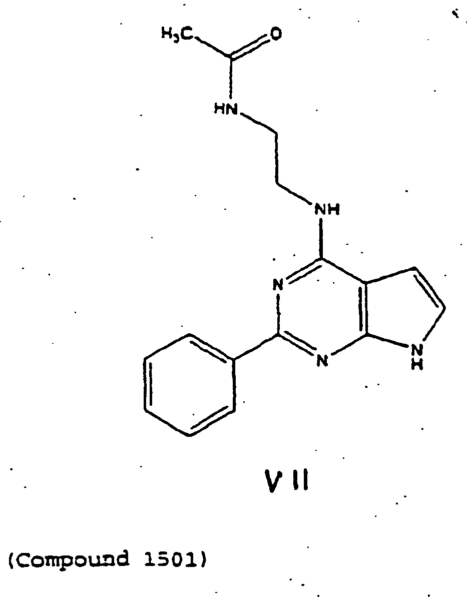

This invention further provides a compound having the structure : 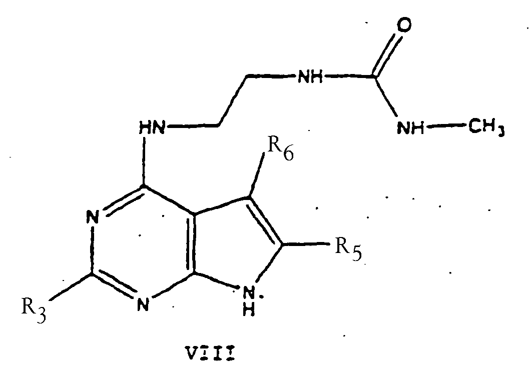 wherein R₃ is as defined above
wherein R₅ and R₆ are independently H, or alkyl.

In one embodiment of the compound, the compound has the structure;

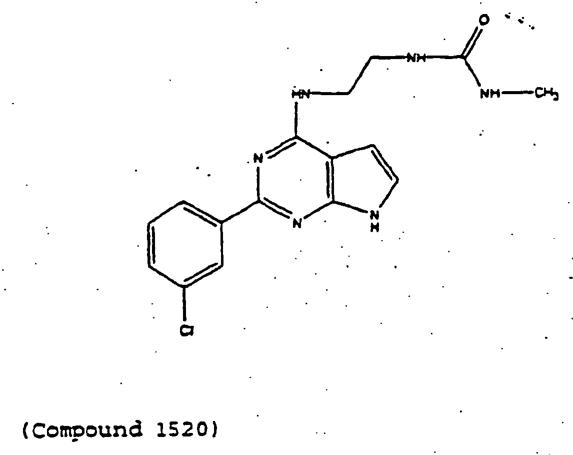

This invention also provides a compound having the structure : 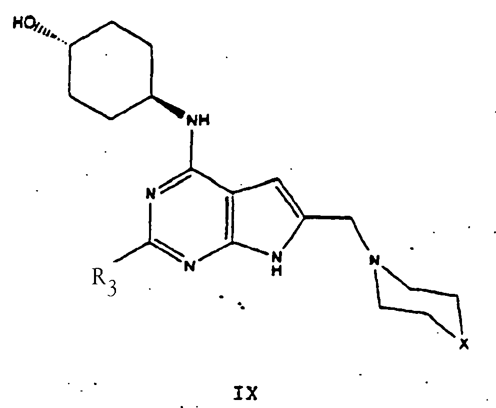 wherein R₃ is as defined above; wherein X is oxygen, or sulfur.

In one embodiment of the compound, the compound has the structure :

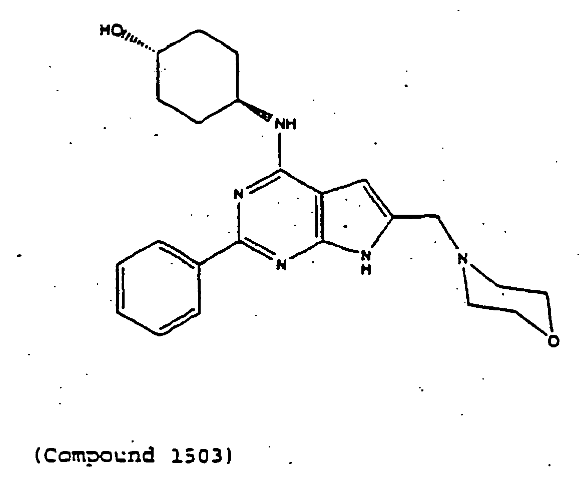

This invention also provides a compound having the structure : 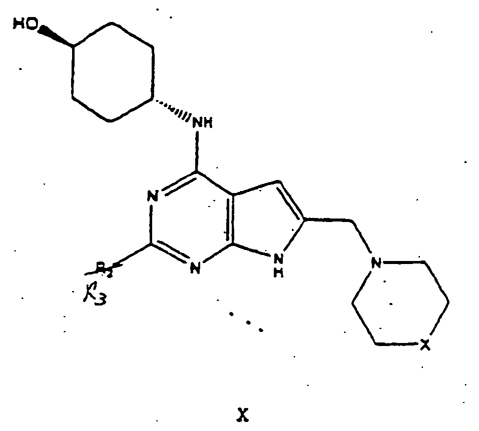 wherein R₃ is as defined above; wherein X is oxygen, or sulfur.

In one embodiment of the compound, the compound has the structure:

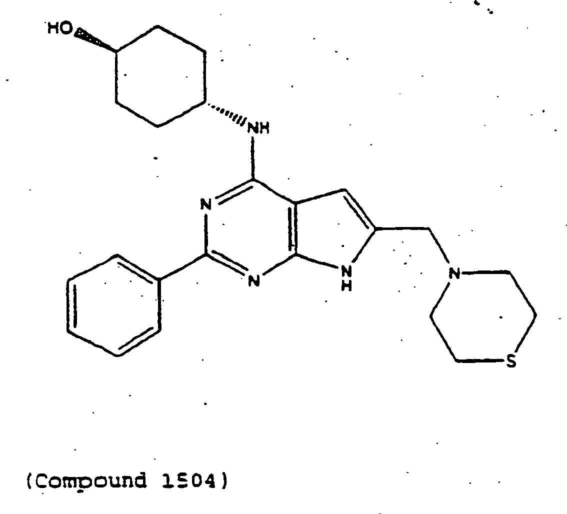

This invention further relates to the use of a therapeutically effective amount of a compound having the formula VI, VII, VIII, IX, or X for the manufacture of a medicament for treating a disease associated with A₁ adenosine receptor in a subject.

In one embodiment, the subject is a mammal. In another embodiment, the mammal is a human.

In another embodiment, the A₁ adenosine receptor is associated with cognitive disease, renal failure, cardiac arrhythmias, respiratory epithelia, transmitter release, sedation, vasoconstriction, bradycardia, negative cardiac inotropy and dromotropy, branchoconstriction, neutropil chemotaxis, reflux condition, or ulcerative condition.

This invention also provides a water-soluble prodrug of a compound having the structures VI, VII, VIII, IX, or X, wherein said water-soluble prodrug that is metabolized *in vivo* to an active drug which selectively inhibit A₁ adenosine receptor.

In one embodiment of the prodrug, said prodrug is metabolized *in vivo* by esterase catalyzed hydrolysis.

This invention also provides a pharmaceutical composition comprising the prodrug and a pharmaceutically acceptable carrier.

This invention further provides a method for inhibiting the activity of an A₁ adenosine receptor in a cell in vitro, which comprises contacting said cell with a compound having the structures VI, VII, VIII, IX, or X.

In one embodiment of the method, the compound is an antagonist of said A₁ adenosine receptor.

This invention also relates to the use of an effective amount of a compound having the structures VI, VII, VIII, IX, or X for the manufacture of a medicament for treating a gastrointestinal disorder in an subject.

In one embodiment, said disorder is diarrhea.

In another embodimen,t the subject is a human.

In another embodiment, the compound is an antagonist of A₁ adenosine receptors.

This invention also relates to the use of an effective amount of a compound having the structures VI, VII, VIII, IX, or X for the manufacture of a medicament for treating respiratory disorder in a subject.

In one embodiment, said disorder is asthma, chronic obstructive pulmonary disease, allergic rhinitis, or an upper-respiratory disorder.

In another embodiment, the subject is a human.

In another embodiment, said compound is an antagonist of A₁ adenosine receptors.

This invention further relates to the use of an effective amount of a compound having the structures VI, VII, VIII, IX, or X for the manufacture of a medicament for treating damage to the eye of a subject.

In one embodiment, said damage comprises retinal or optic nerve head damage.

In another embodiment, said damage is acute or chronic.

In another embodiment, wherein said damage is the result of glaucoma, edema, ischemia, hypoxia or trauma.

In another embodiment, the subject is a human.

In another embodiment, the compound is an antagonist of A₁ adenosine receptors.

This invention also provides a pharmaceutical composition comprising a therapeutically effective amount of a compound having the structures VI, VII, VIII, IX, or X, and a pharmaceutically acceptable carrier.

In another embodiment of the pharmaceutical composition, said therapeutically effective amount is effective to treat a respiratory disorder or a gastrointestinal disorder.

In another embodiment of the pharmaceutical composition, said gastrointestinal disorder is diarrhea.

In another embodiment of the pharmaceutical composition, said respiratory disorder is asthma, allergic rhinitis, or chronic obstructive pulmonary disease.

In another embodiment of the pharmaceutical composition, said pharmaceutical composition is an ophthalmic formulation.

In another embodiment of the pharmaceutical composition, said pharmaceutical composition is an periocular, retrobulbar or intraocular injection formulation.

In yet another embodiment of the pharmaceutical composition, said pharmaceutical composition is a systemic formulation.

In a further embodiment of the pharmaceutical preparation, said pharmaceutical composition is a surgical irrigating solution.

This invention also provides a packaged pharmaceutical composition for treating a disease associated with A₁ adenosine receptor in a subject, comprising: (a) a container holding a therapeutically effective amount of an adenosine A₁ specific compound; and (b) instructions for using said compound for treating said disease in a subject.

As used herein, "A compound is A₁ selective." means that a compound has a binding constant to adenosine Al receptor of at least ten time higher then that to adenosine A₂ₐ, A_{2b} or A₃.

This invention also provides a method of preparing the compound having structure I, comprising the steps of
a) reacting 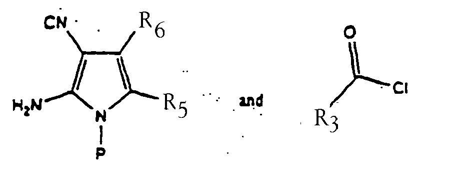 to provide 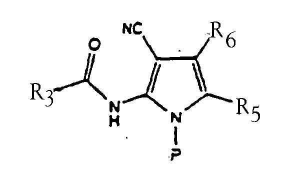 wherein P is a removable protecting group:
b) treating the product of step a) under cyclization conditions to provide 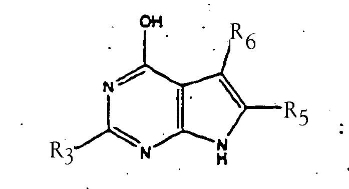
c) treating the product of step b) under suitable conditions to provide 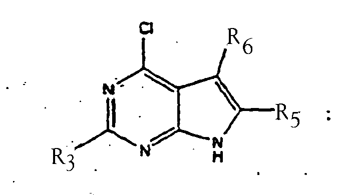 and
d) treating the chlorinated product of step c) with NH₂R₁ to provide 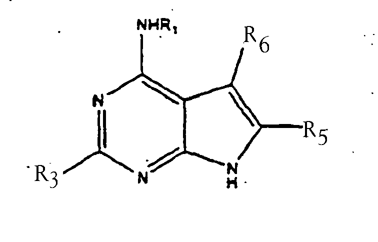 wherein R₁, R₃, R₅ and R₆ are as defined above.

Compounds represented by formula VI, VII, and VIII can be synthesized by any of the Schemes I-VIII. Compounds represented by formula IX, and X can be prepared by Scheme IX.

The invention is further illustrated by the following examples which in no way should be construed as being further limiting. The contents of all references, pending patent applications and published patent applications, cited throughout this application, including those referenced in the background section, are hereby incorporated by reference. It should be understood that the models used throughout the examples are accepted models and that the demonstration of efficacy in these models is predictive of efficacy in humans.

This invention will be better understood from the Experimental Details which follow. However, one skilled in the art will readily appreciate that the specific methods and results discussed are merely illustrative of the invention as described more fully in the claims which follow thereafter.

The deazapurines of the invention can be prepared using standard methods for organic synthesis. Deazapurines can be purified by reverse phase HPLC, chromatography, recrystallization, etc. and their structures confirmed by mass spectral analysis, elemental analysis, IR and/or NMR spectroscopy.

Typically, synthesis of the intermediates as well as the deazapurines of the invention is performed in solution. The addition and removal of one or more protecting group is also typical practice and is known to those skilled in the art. Typical synthetic schemes for the preparation of deazapurine intermediates of the invention are outlined below in Scheme I. 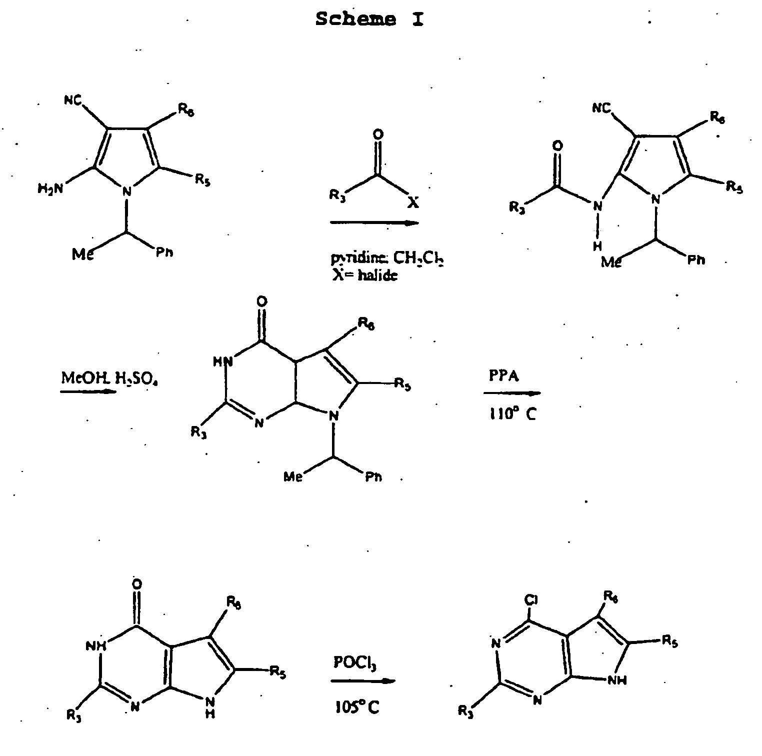 wherein R₃, R₅ and R₆ are as defined above.

In general, a protected 2-amino-3-cyano-pyrrole can be treated with an acyl halide to form a carboxyarnido-3-cyanopyrrole which can be treated with acidic methanol to effect ring closure to a pyrrolo[2,3d]pyrimidine-4(3*H*)-one (Muller, C.E. *et al*. J. Med. Chem. 40:4396 (1997)). Removal of the pyrrolo protecting group followed by treatment with a chlorinating reagent, *e.g*., phosphorous oxychloride, produced substituted or unsubstituted 4-chloro-7H-pyrrolo[2,3d]pyrimidines. Treatment of the chloropyrimidine with amines afforded 7-deazapurines.

For example, as shown in Scheme I, a N-(1-*dl*-phenylethyl)-2-amino-3-cyano-pyrrole was treated with an acyl halide in pyridine and dichloromethane. The resultant N-(1-*dl-*phenylethyl)-2-phenylcarboxyamido-3-cyano-pyrrole was treated with a 10:1 mixture of methanol/sulfuric acid to effect ring closure, resulting in a *dl*-7*H*-7-(1-phenylethyl)pyrrolo[2,3d]pyrimidine-4(3*H*)-one. Removal of the phenylethyl group by treatment of the pyrimidine with polyphosphoric acid (PPA) followed by POCl₃ afforded a key intermediate, the 4-chloro-7H-pyrrolo[2,3d]pyrimidine. Further treatment of the 4-chloro-7H-pyrrolo[2,3d]pyrimidine with various amines listed in Table 1 gives compounds of formula (I).

**TABLE 1**

| R | M⁺ + H | R | M⁺ - H |
|---|---|---|---|
| 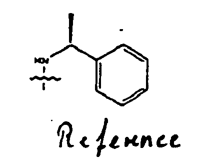 | 343.2 | 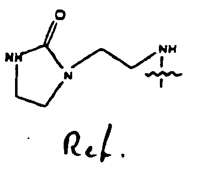 | 351.27 |
| 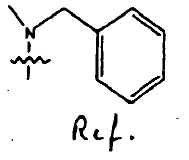 | 343.18 | 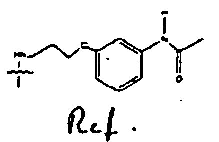 | 430.35 |
| 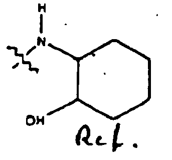 | 337.21 | 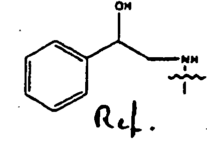 | 359.44 |
| 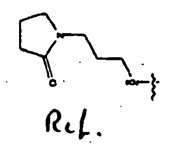 | 364.19 | 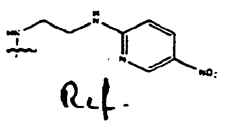 | 404.32 |
| 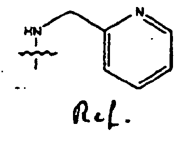 | 330.18 | 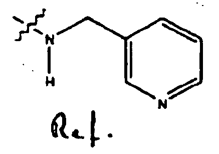 | 330.45 |
| 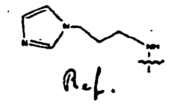 | 347.22 | 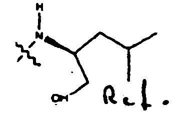 | 339.47 |
| 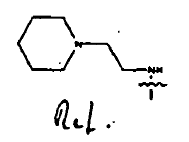 | 350.28 | 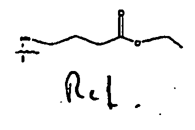 | 353.41 |
| 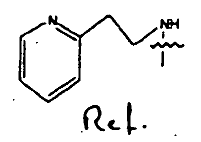 | 344.19 | 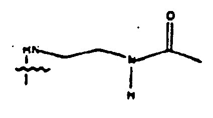 | 324.45 |
| 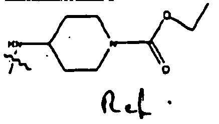 | 394.16 | 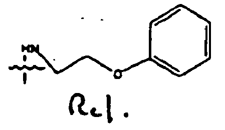 | 359.38 |
| 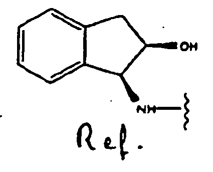 | 371.12 | 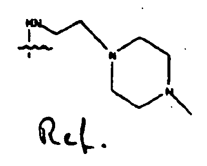 | 379.40 |
| 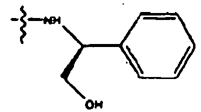 | 359.39 | 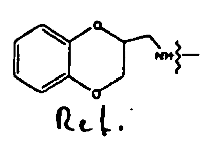 | 387.41 |
| 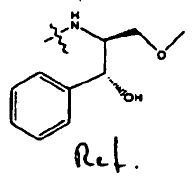 | 403.33 | 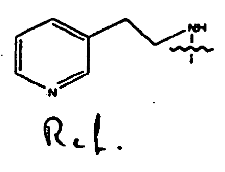 | 344.48 |
| 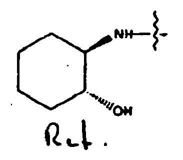 | 351.49 | 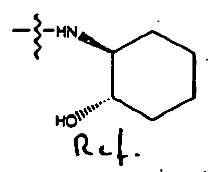 | 337.53 |
| 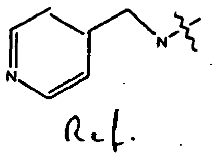 | 330.37 | 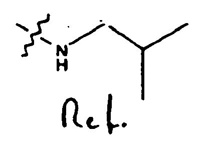 | 295.2 |
| 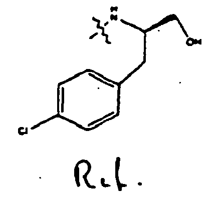 | 407.23 | 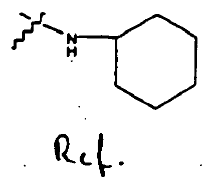 | 321.2 |
| 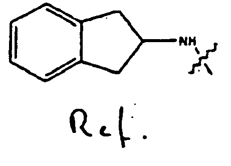 | 355.45 | 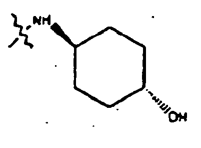 | 337.53 |
| 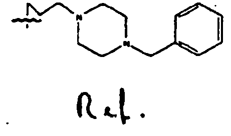 | 441.33 | 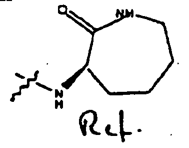 | 350.2 |
| 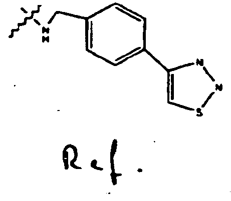 | 413.24 | 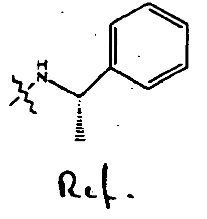 | 343.2 |
| 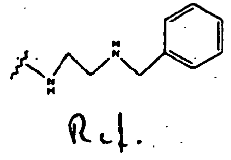 | 372.48 | 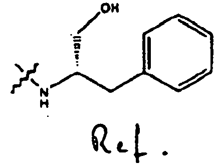 | 373.2 |
| | | 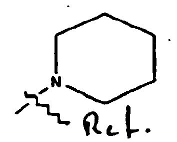 | 307.2 |

A general approach to prepare 6-substituted pyrroles is depicted in the following scheme (Scheme II). 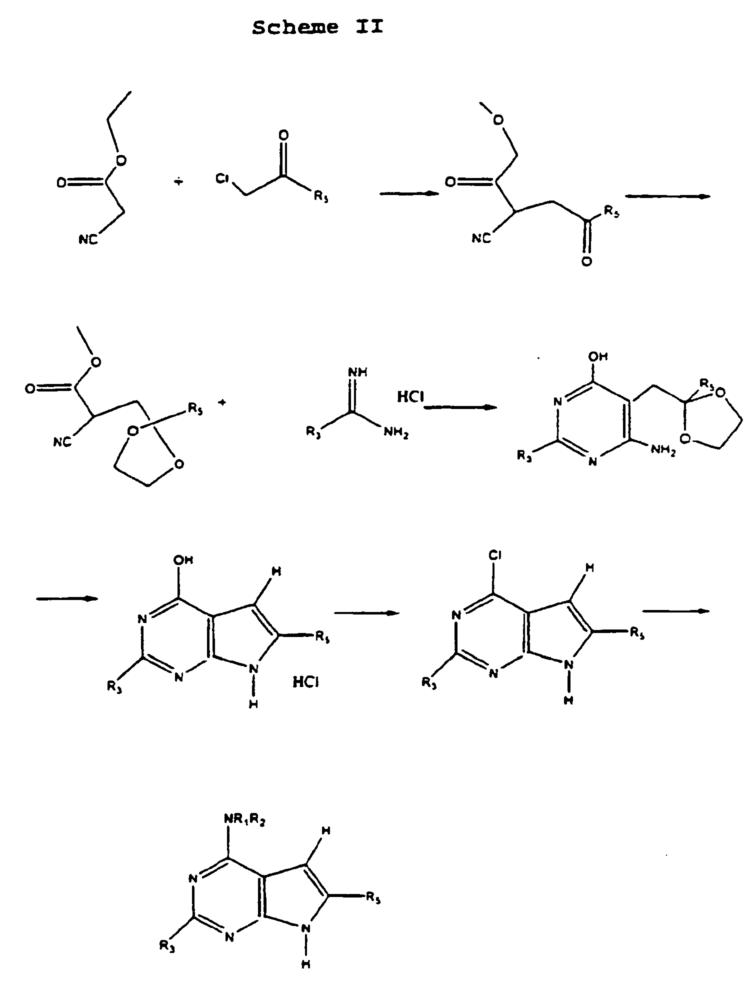 wherein R₁ through R₅ are as defined above.

Transesterification and alkylation of ethyl cyanoacetate with an α-haloketone affords a ketomethylester. Protection of the ketone followed by treatment with an amidine (*e.g.*, alkyl, aryl or alkylaryl) hydrochloride produced the resultant ketal protected pyrimidine. Removal of the protecting group, followed by cyclization and treatment with phosphorous oxychloride afforded the chloride intermediate which could be further treated with an amine to afford an amine 6-substituted pyrrole. Additionally, alkylation of the pyrrole nitrogen can be achieved under art recognized conditions.

A general approach to prepare 5-substituted pyrroles is depicted in the following scheme (Scheme III). 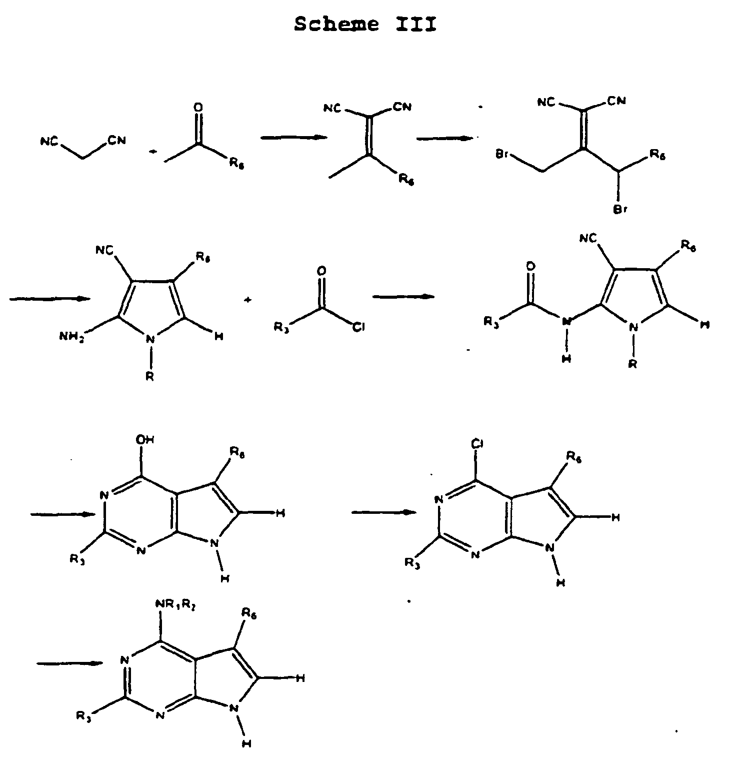 wherein R₁ through R₆ are defined as above and R is a removable protecting group.

Condensation of malononitrile and an excess of a ketone followed by bromination of the product afforded a mixture of starting material, monobrominated and dibrominated products which were treated with an alkylamine, arylamine or alkylarylamine. The resultant amine product was acylated with an acid chloride and the monacylated pyrrole was cyclized in the presence of acid to afford the corresponding pyrimidine. The pyrrole protecting group was removed with polyphosphoric acid and treated with phosphorous oxychloride to produce a chlorinated product. The chlorinated pyrrole could subsequently be treated with an amine to produce an amino 5-substituted pyrrole. Alkylation of the pyrrole nitrogen can be achieved under art recognized conditions.

Schemes IV and V depict methods according to the invention for preparing the deazapurines 1 and 2. 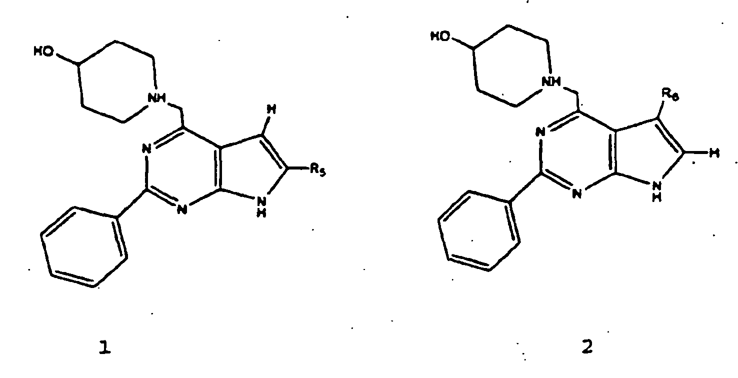 wherein R₅ and R₆ are as described above, *e.g*., CH₃.

### Specific Preparation of 6-methyl pyrrolopyrimidines

The key reaction toward 6-methylpyrrolopyrimidines (1) [R₅ = CH₃] was cyclization of a cyanoacetate with benzamidine to a pyrimidine. It was believed methyl cyanoacetate would cyclize more efficiently with benzamidine to a pyrimidine than the corresponding ethyl ester, Therefore, transesterification and alkylation of ethyl cyanoacetate in the presence of NaOMe and an excess of an α-haloacetyl moiety, *e.g*., chloroacetone, gave the desired methyl ester (3) in 79% yield (Scheme IV). The ketoester (3) was protected as the acetal (4) in 81% yield. A new cyclization method to the pyrimidine (5) was achieved with an amidine hydrochloride, *e.g.*, benzamidine hydrochloride, with 2 equivalents of DBU to afford the 5 in 54% isolated yield. This method improves the yield from 20% using the published conditions, which utilizes NaOMe during the cyclization with guanidine. Cyclization to the pyrrole-pyrimidine (6) was achieved via deprotection of the acetal in aqueous HCl in 78% yield. Reaction of (6) with phosphorous oxychloride at reflux gave the corresponding 4-chloro derivative (7). Coupling with trans-4-aminocyclohexanol in dimethyl sulfoxide at 135°C gave (1) in 57% from (7). One skilled in the art will appreciate that choice of reagents allows for great flexibility in choosing the desired substituent R₅.

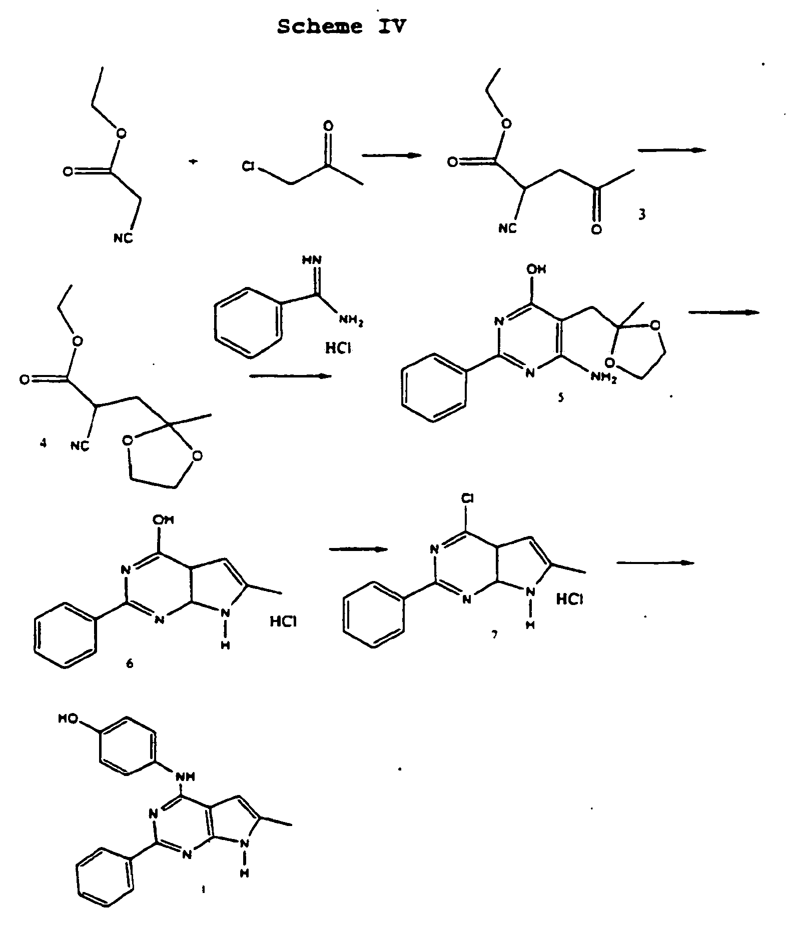

### Specific Preparation of 5-methylpyrrolopyrimidines

Knoevengel condensation of malononitrile and an excess ketone, *e.g*., acetone in refluxing benzene gave 8 in 50% yield after distillation. Bromination of 8 with *N-*bromosuccinimde in the presence of benzoyl peroxide in chloroform yielded a mixture of starting material, mono- (9), and di-brominated products (5/90/5) after distillation (70%). The mixture was reacted with an α-methylalkylamine or α-methylarylamine, *e.g*., α-methylbenzylamine, to deliver the aminopyrrole (10). After passing through a short silica gel column, the partially purified amine (31% yield) was acylated with an acid chloride, *e.g.,* benzoyl chloride to deliver mono- (11), and diacylated (12) pyrroles, which were separated by flash chromatography. Acid hydrolysis of the disubstituted pyrrole (12) generated a combined yield of 29% for the acylpyrrole (11). Cyclization in the presence of concentrated sulphuric acid and DMF yielded (13) (23%), which was deprotected with polyphosphoric acid to (14). Reaction of (14) with phosphorous oxychloride at reflux gave the corresponding 4-chloro derivative (15). Coupling with trans-4-aminocyclohexanol in dimethyl sulfoxide at 135°C gave (2) [R₆ = CH₃] in 30% from (14) (See Scheme V). One skilled in the art will appreciate that choice of reagents allows for great flexibility in choosing the desired substituent R₆.

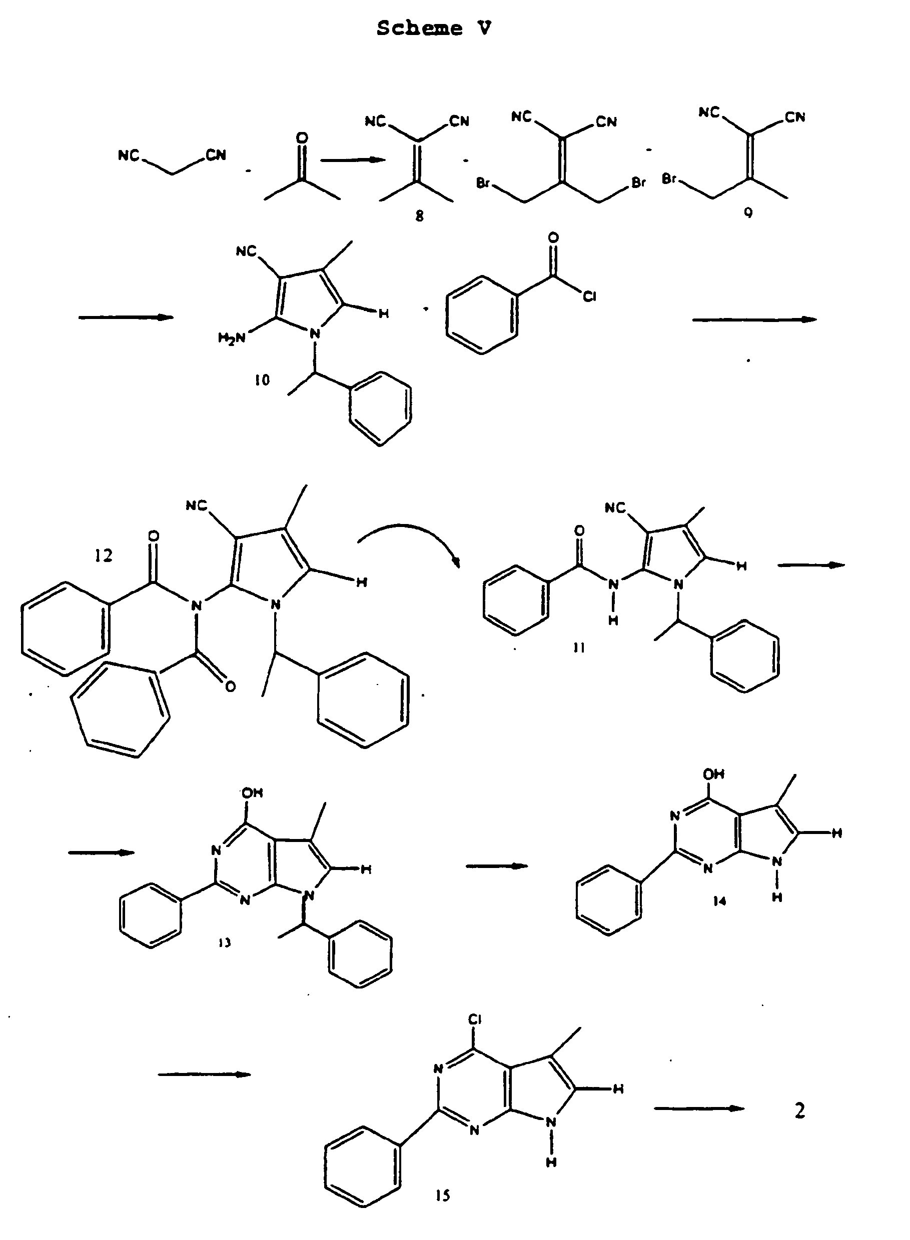

### Alternative Synthetic Route to R₆-Substituted Pyrroles, e.g., 5-methyl pyrrolopyrimidines:

This alternative route to R₆-substituted pyrroles, *e.g*., 5-methylpyrrolopyrimidines, involves transesterification and alkylation of ethyl cyanoacetate to (16) (Scheme VI). The condensation of (16) with benzamidine hydrochloride with 2 equivalents of DBU affords the pyrimidine (17). Cyclization to the pyrrole-pyrimidine (14) will be achieved via deprotection of the acetal in aqueous HCl. Reaction of (14) with phosphorous oxychloride at reflux gave the corresponding 4-chloro derivative (15). Coupling with *trans*-4-aminocyclohexanol in dimethyl sulfoxide at 135°C gives 2. This procedure reduces the number of synthetic reactions to the target compound (2) from 9 to 4 steps. Moreover, the yield is dramatically improved. Again, one skilled in the art will appreciate that choice of reagents allows for great flexibility in choosing the desired substituent R₆.

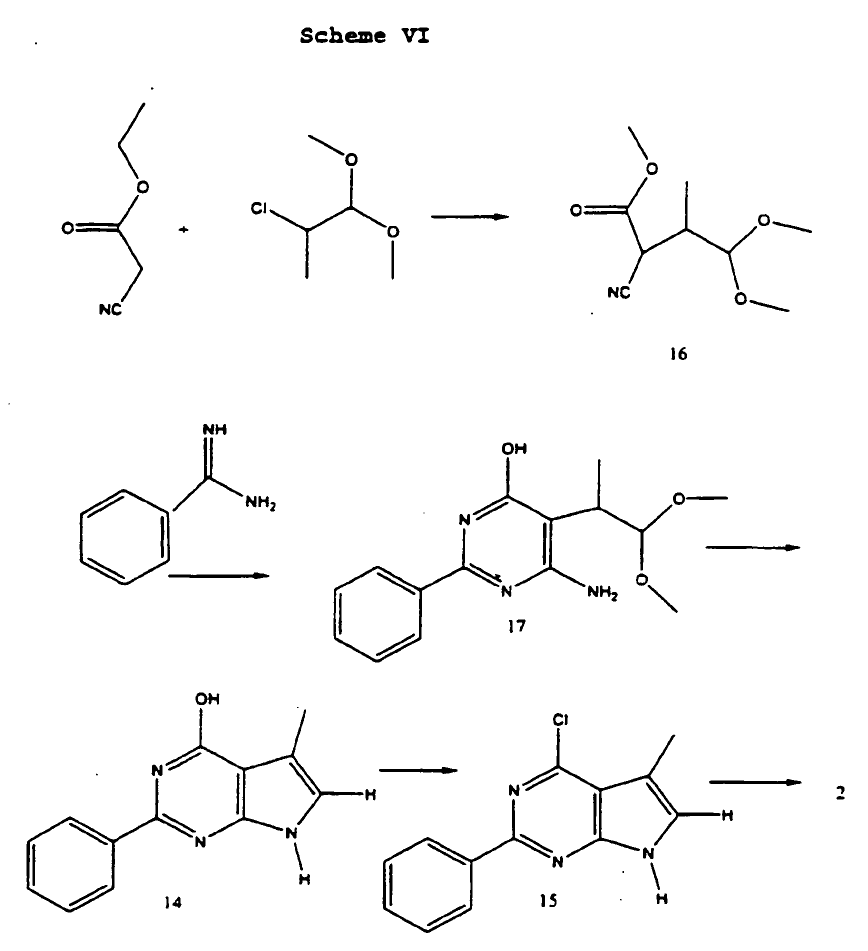

A general approach to prepare des-methyl pyrrole is depicted in the following scheme (Scheme VII) 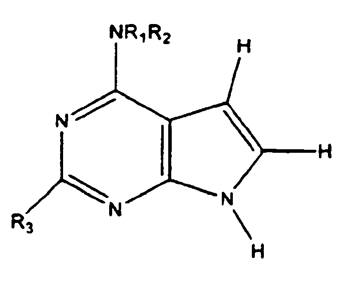 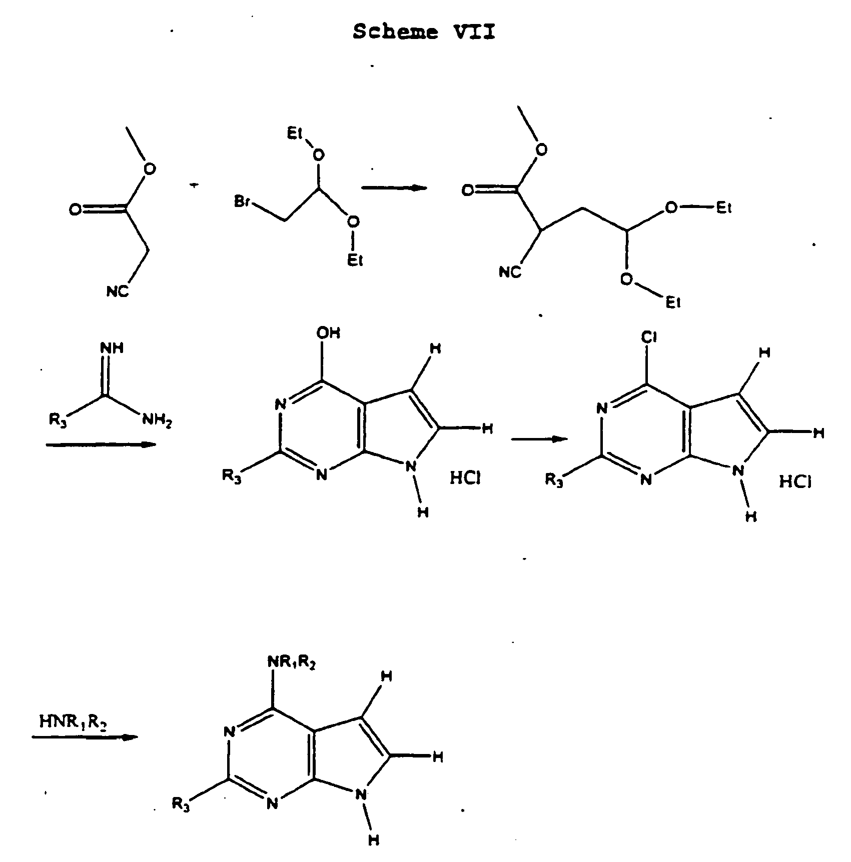 wherein R₁ through R₃ are defined as above.

Alkylation of an alkyl cyanoacetate with a diethyl acetal in the presence of a base afforded a cyano diethyl acetal which was treated with an amidine salt to produce a methyl pyrrolopyrimidine precursor. The precursor was chlorinated and treated with an amine to form the des-methyl pyrrolopyrimidine target as shown above.

For example, Scheme VIII depicts the synthesis of compound (18).

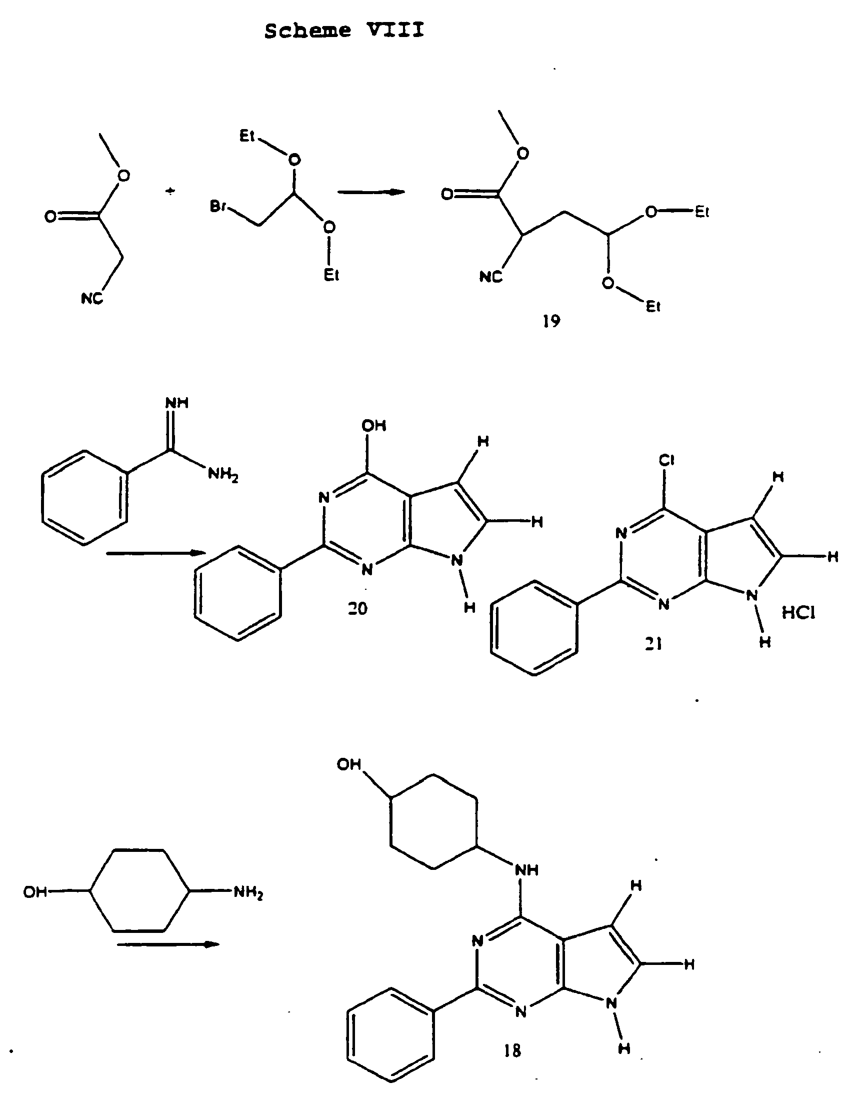

Commercially available methyl cyanoacetate was alkylated with bromoacetaldehyde diethyl acetal in the presence of potassium carbonate and NaI to yield (19). Cyclization to the pyrimidine (20) was achieved in two steps. Initially, the pyrimidine-acetal was formed via reaction of (19) with benzamidine hydrochloride with 2 equivalents of DBU. The resultant pyrimidine-acetal was deprotected without purification with aqueous 1 N HCl and the resultant aldehyde cyclized to the pyrrolo-pyrimidine (20), which was isolated by filtration. Reaction of (20) with phosphorous oxychloride at reflux afforded the corresponding 4-chloro derivative (21).. Coupling of the chloro derivative with crans-4-aminocyclohexanol in DMSO at 135°C gave compound (18) from compound (21).

Schemes II-VIII demonstrate that it is possible to functionalize the 5- and 6-position of the pyrrolopyrimidine ring. Through the use of different starting reagents and slight modifications of the above reaction schemes, various functional groups can be introduced at the 5- and 6-positions in formula (I). Table 2 illustrates some examples.

**Table 2. Selected list- of 5- and 6-substituted pyrrolopyrimidines.**

| Starting Reagent | R₅ | R₆ |
|---|---|---|
| 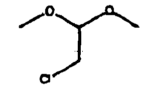 | H | |
| 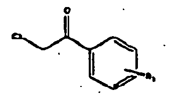 | H | Substituted Ar |
| 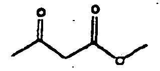 | H | CH₂C(O)OCH₃ |
| 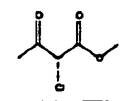 | C(O)OCH₃ | CH₃ |
| 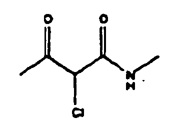 | C (O)NHCH₃ | CH₃ |

A skilled artisan will know that metabolism of the compounds disclosed herein in a subject.produces certain biologically active metabolites which can serve as drugs.

The invention is further illustrated by the following examples which in no way should be construed as being further limiting. It should be understood that the models used throughout the examples are accepted models and that the demonstration of efficacy in these models is predictive of efficacy in humans.

### Exemplification

### Preparation 1:

A modification of the alkylation method of Seela and Lüpke was used.¹ To an ice-cooled (0°C) solution of ethyl cyanoacetate (6.58 g, 58.1 mmol) in MeOH (20 mL) was slowly added a solution of NaOMe (25% w/v; 58.1 mmol). After 10 min, chloroacetone (5 mL; 62.8 mmol) was slowly added. After 4 h, the solvent was removed. The brown oil was diluted the EtOAc (100 mL) and washed with H₂O (100 mL). The organic fraction was dried, filtered, and concentrated to a brown oil (7.79 g; 79%). The oil (3) (Scheme IV) was a mixture of methyl/ethyl ester products (9/1), and was used without further purification. ¹H NMR (200 MHz, CDCl₃) δ_4.24 (q, *J* = 7.2 Hz, OCH₂), 3.91 (dd, 1H, *J* = 7.2, 7.0 Hz, CH), 3.62 (s, 3H, OCH₃), 3.42 (dd, 1H, *J* = 15.0, 7.1 Hz, 1 x CH₂); 3.02 (dd, 1H, *J* = 15.0, 7.0 Hz, 1 x CH₂); 2.44 (s, 3H, CH₃), 1.26 (t, *J* = 7.1 Hz, ester-CH₃).
¹Seela, F.; Lüpke, U. Chem. Ber. 1977, 110, 1462-1469.

### Preparation 2:

The procedure of Seela and Lüpke was used.¹ Thus, protection of the ketone (3) (Scheme IV; 5.0 g, 32.2 mmol) with ethylene glycol (4 mL, 64.4 mmol) in the presence of TsOH (100 mg) afforded (4) as an oil (Scheme IV; 5.2 g, 81.0) after flash chromatography (SiO₂; 3/7 EtOAc/Hex, *R*_{*f*} 0.35). Still contains -5% ethyl ester: ¹H NMR (200 MHz, CDCl₃) δ_4.24 (q, *J* = 7.2 Hz, OCH₂), 3.98 (s, 4H, 2 x acetal-CH₂), 3.79 (s, 3H, OCH₃), 3.62 (dd, 1H, *J* = 7.2, 7.0 Hz, CH), 2.48 (dd, 1H, *J* = 15.0, 7.1 Hz, 1 x CH₂), 2.32 (dd, 1H, *J* = 15.0, 7.0 Hz, 1 x CH₂) ; 1.35 (s, 3H, CH₃), 1.26 (t, *J* = 7.1 Hz, ester-CH₃); MS (ES): 200.1 (M⁺+1).
Seela, F.; Lupke, U. Chem. Ber. 1977, 110, 1462-1469.

### Preparation 3:

A solution of acetal (4) (Scheme IV, 1 g, 5.02 mmol), benzamidine (786 mg, 5.02 mmol), and DBU (1.5 mL, 10.04 mmol) in dry DMF (15 mL) was heated to 85°C for 15 h. The mixture was diluted with CHCl₃ (30 mL) and washed with 0.5 N NaOH (10 mL) and H₂O (20 mL). The organic fraction was dried, filtered and concentrated to a brown oil. Flash chromatography (SiO₂; 1/9 EtOAc/CH₂Cl₂, *R*_{f} 0.35) was attempted, but material crystallized on the column. The silica gel was washed with MeOH. Fractions containing the product (5) (Scheme IV) were concentrated and used without further purification (783 mg, 54.3%) : ¹H NMR (200 MHz, CDCl₃) δ 8.24 (m, 2H, Ar-H), 7-45 (m, 3H, Ar-H), 5.24 (br s, 2H, NH₂), 3.98 (s, 4H, 2 x acetal-CH₂), 3.60-3.15 (m, 2H, CH₂), 1.38 (s, 3H, CH₃); MS (ES): 288.1 (M⁺+1).

Preparation of compound (20) (Scheme VIII): A solution of acetal (19) (4.43 g, 20.6 mmol)¹, benzamine hydrochloride (3.22 g, 20.6 mmol), and DBU (6.15 mL, 41.2 mmol) in dry DMF (20 mL) was heated to 85°C for fifteen hours. The mixture was diluted with 100mL of CHCl₃, and washed with H₂O (2 x 50 mL). The organic fraction was dried, filtered, and concentrated to a dark brown oil. The dark brown oil was stirred in 1N HCl (100 mL) for 2 hours at room temperature. The resulting slurry was filtered yielding the HCl salt of (20) as a tan solid (3.60 g, 70.6%); ¹H NMR (200 MHz, DMSO-d6) 11.92 (s 1H), 8.05 (m, 2H, Ar-H), 7.45 (m, 3H, Ar-H), 7.05 (s, 1H, pyrrole-H); MS (ES) : 212.1 (M⁺+1) .

### Preparation 4:

A solution of acetal (5) (700 mg, 2.44 mmol) in 1 N HCl (40 mL) was stirred for 2 h at RT. The resultant slurry was filtered yielding the HCl salt of 2-phenyl-6-methyl-7*H-*pyrrolo[2,3d]pyrimidin-4 (3*H*)-one as a tan solid (498 mg, 78.0%): ^{**1**}H NMR (200 MHz, DMSO-d₆) δ 11.78 (s, 1H), 8.05 (m, 2H, Ar-H), 7.45 (m, 3H, Ar-H), 6.17 (s, 1H, pyrrole-H), 2.25 (s, 3H, CH₃); MS (ES): 226.1 (M⁺+1).

### Preparation 5:

A modification of the Chen *et al.* cyclization method was used.¹ To an ice-cooled (0°C) solution of bromide (9). (Scheme V; 20.0 g, 108 mmol; 90% pure) in isopropyl alcohol (60 mL) was slowly added a solution of α-methylbenzylamine (12.5 mL, 97.3 mmol). The black solution was allowed to warm to RT and stir for 15 h. The mixture was diluted with EtOAc (200 mL) and washed with 0.5 N NaOH (50 mL) . The organic fraction was dried, filtered, and concentrated to a black tar (19.2 g; 94%). The residue was partially purified by flash chromatography (SiO₂; 4/96 MeOH/CH₂Cl₂, *R*_{*f*} 0.35) to a black solid (6.38 g, 31%) as the compound *dl*-1-(1-phenylethyl)-2-amino-3-cyano-4-methylpyrrole: MS (ES): 226.1 (M⁻+1).
¹Chen, Y. L.; Mansbach, R. S.; Winter, S. M.; Brooks, E.; Collins, J.; Corman, M. L.; Dunaiskis, A. R.; Faraci, W. S.; Gallaschun, R. J.; Schmidt, A.; Schulz, D. W. *J. Med. Chem.* **1997**, 40, 1749-1754.

### Preparation 6:

To a solution of *dl*-1-(1-phenylethyl)-2-amino-3-cyano-4,5-dimethylpyrrole¹ (14.9 g, 62.5 mmol) and pyridine (10.0 mL) in dichloromethane (50.0 mL) was added benzoyl chloride (9.37 g, 66.7 mmol) at 0°C. After stirring at 0°C for 1 hr, hexane (10.0 mL) was added to help precipitation of product. Solvent was removed *in vacuo* and the solid was recrystallized from EtOH/H₂O to give 13.9 g (65%) of *dl*-1-(1-phenylethyl)-2-phenylcarbonylamino-3-cyano-4,5-dimethylpyrrole. mp 218-221°C; ¹H NMR (200 MHz, CDCl₃) δ_1.72 (s, 3H), 1.76 (d, *J* = 7.3 Hz, 3H), 1.98 (s, 3H), 5.52 (q, J = 7.3 Hz, 1H), 7.14-7.54 (m, 9H), 7.68-7.72 (dd, J = 1.4 Hz, 6.9 Hz , 2H), 10.73 (s, 1H); MS (ES) : 344.4 (M⁺+1).
Liebigs Ann. Chem. 1986, 1485-1505.

The following compounds were obtained in a similar manner.

### Preparation 6A:

*dl*-1-(1-phenylethyl)-2-(3-pyridyl)carbonylamino-3-cyano-4,5-dimethylpyrrole. ¹H NMR (200 MHz, CDCl₃) δ_1.83 (d, J = 6.8 Hz, 3H), 2.02 (s, 3H), 2.12 (s, 3H), 5.50 (q, J = 6.8 Hz, 1H), 7.14-7.42 (m, 5H), 8.08 (m, 2H), 8.75 (m, 3H); MS (ES): 345.2 (M⁻+1).
*dl*-1-(1-phenylethyl)-2-(2-furyl)carbonylamino-3-cyano-4,5-dimethylpyrrole. ¹H NMR (200 MHz, CDCl₃) δ 1.84 (d, J = 7.4 Hz, 3H), 1.92 (s, 3H), 2.09 (s, 3H), 5.49 (q, J = 7.4 Hz, 1H), 6.54 (dd, J = 1.8 Hz, 3.6 Hz, 1H), 7.12-7.47 (m, 7H); MS (ES): 334.2 (M⁻+1), 230.1.
*dl*-1-(1-ahenylethyl)-2-(3-furyl)carbonylamino-3-cyano-4,5-dimethylpyrrole. ¹H NMR (200 MHz, CDCl₃) δ 1.80 (d, J = 7 Hz 3H), 1.89 (s, 3H), 2.05 (s, 3H), 5.48 (q, J = 7 Hz, 1H), 6.59 (s, 1H), 7.12-7.40 (m, 6H), 7.93 (s, 1H); MS (ES): 334.1 (M⁻+1), 230.0.
*dl*-1-(1-phenylethyl)-2-cyclopentylcarbonylamino-3-cyano-4,5-dimethylpyrrole. ¹H NMR (200 MHz, CDCl₃) δ 1.82 (d, J = 7.4 Hz, 3H), 1,88 (s, 3H), 2.05 (s, 3H), 1.63-1.-85 (m, 8H), 2.63 (m, 1H), 5.43 (q, J = 7.4 Hz, 1H), 6.52 (s, 1H), 7.05-7.20 (m, 5H) ; MS (ES): 336.3 (M⁻+1).
*dl*-1-(1-phenylethyl)-2-(2-thieyl)carbonylamino-3-cyano-4,5-dimethylpyrrole, ¹H NMR (200 MHz, CDCl₃) δ 1.82 (d, J = 6.8 Hz, 3H), 1.96 (s, 3H), 2.09 (s, 3H), 5.49 (q, J= 6.8 Hz, 1H), 7.05-7.55 (m, 8H); MS (ES): 350.1 (M⁻+1), 246.0.
*dl*-1-(1-phenylethyl)-2-(3-thienyl)carbonylamino-3-cyano-4,5-dimethylpyrrole.
   ¹H NMR (200 MHz, CDCl₃) δ 1.83 (d, J = 7.0 Hz, 3H), 1.99 (s, 3H), 2. 12 (s, 3H), 5.49 (q, J = 7.0 Hz, 1H), 6.90 (m, 1H), 7.18-7.36 (m, 6H), 7.79 (m, 1H); MS (ES): 350.2 (M⁻+1), 246.1.
*dl*-1-(1-phenylethyl)-2-(4-fluorophenyl)carbonylamino-3-cyano-4,5-dimethylpyrrole.
   ¹H NMR (200 MHz, CDCl₃) δ 1.83 (d, J = 7.4 Hz, 3H), 1.96 (s, 3H), 2.08 (s, 3H), 5.51 (q, J = 7.4 Hz, 1H), 7.16-7.55 (m, 9H) ; MS (ES): 362.2 (M⁻+1), 258.1.
*dl*-1-(1-phenylethyl)-2-(3-fluorophenyl)carbonylamino-3-cyano-4,5-dimethylpyrrole.
   ¹H NMR (200 MHz, CDCl₃) δ 1.83 (d, J = 7.4 Hz 3H), 1.97 (s, 3H), 2.10 (s, 3H), 5.50 (q, J = 7.4 Hz, 1H), 7.05-7. 38 (m, 7 H), 7.67-7.74 (m, 2H) ; MS (ES): 362.2 (M⁻+1), 258.1.
*dl*-1-(1-phenylethyl)-2-(2-fluorophenyl)carbonylamino-3-cyano-4, 5-dimethylpyrrole. ¹H NMR (200 MHz, CDCl₃) δ 1.85 (d, J = 7.2 Hz, 3H), 1.94 (s, 3H), 2.11 (s, 3H), 5.50 (q, J = 7.2 hz, 1H), 7.12-7.35 (m, 6H), 7.53 (m, 1H), 7.77 (m, 1H), 8.13 (m, 1H); MS (ES): 362.2 (M⁻+1), 258.0.
*dl*-1-(1-phenylethyl)-2-isoproylcarbonylamino-3-cyano-4,5-dimethylpyrrole ¹H NMR (200 MHz, CDCl₃) δ 1.19 (d, J = 7.0 Hz, 6H), 1.82(d, J = 7.2 Hz, 3H), 1.88 (s, 3H), 2.06 (s, 3H), 2.46 (m, 1H), 5.39 (m, J = 7.2 Hz, 1H), 6.64 (s, 1H), 7.11-7.36 (m, 5H); MS (ES): 310.2 (M⁻+1), 206.1

In the case of acylation of *dl*-1-(1-phenylethyl)-2-amino-3-cyano-4-methylpyrrole, monoacylated *dl*-1-(1-phenylethyl)-2-benzoylamino-3-cyano-4-dimethylpyrrole and diacylated pyrrole *dl*-1-(1-phenylethyl)-2-dibenzoylamino-3-cyano-4-methylpyrrole were obtained. Monoacylated pyrrole: ¹H NMR (200 MHz, CDCl₃) δ_7.69 (d, 2H, *J* = 7.8 Hz, Ar-H), 7.58-7.12 (m, 8H, Ar-H), 6.18 (s, 1H, pyrrole-H), 5.52 (q, 1H, *J* = 7.2 Hz, CH-CH₃), 2.05 (s, 3H, pyrrole-CH₃), 1.85 (d, 3H, *J* = 7.2 Hz, CH-CH₃) ; MS (ES) : 330.2 (M⁻+1); Diacylated pyrrole: ¹H NMR (200 MHz, CDCl₃) δ_7.85 (d, 2H, *J* = 7.7 Hz, Ar-H), 7.74 (d, 2H, *J* = 7.8 Hz, Ar-H), 7.52-7.20 (m, 9H, Ar-H), 7.04 (m, 2H, Ar-H), 6.21 (s, 1H, pyrrole-H), 5.52 (q, 1H, *J* = 7.2 Hz, CH-CH₃), 1.77 (d, 3H, J = 7.2 Hz, CH-CH₃), 1.74 (s, 3H, pyrrole-CH₃); MS (ES) : 434.1 (M⁻+1).

### Preparation 7:

To a solution of *dl*-1-(1-phenylethyl)-2-phenylcarboxyamido-3-cyano-4,5-dimethylpyrrole (1.0 g, 2.92 mmol) in methanol (10.0 mL) was added concentrated sulfuric acid (1.0 mL) at 0°C. The resulted mixture was refluxed for 15 hr and cooled down to room temperature. The precipitate was filtered to give 0.48 g (48%) of *dl*-5,6-dimethyl-2-phenyl-7*H*-7-(1-phenylethyl)pyrrolo[2,3d]pyrimidin-4(3H)-one. ¹H NMR (200 MHz, CDCl₃) δ_2.02 (d, J = 7.4 Hz, 3H), 2.04 (s, 3H), 2.41 (s, 3H), 6.25 (q, J = 7.4 Hz, 1H), 7.22-7.50 (m, 9H), 8.07-8.12 (dd, J = 3.4 Hz, 6.8 Hz, 2H), 10.51 (s, 1H); MS (ES): 344.2 (M⁻+1). The following compounds were obtained in a similar manner as that of Preparation 7:
*dl*-5,6-dimethyl-2-(3-pyridyl)-7*H*-7-(1-phenylethyl) pyrrolo [2, 3d] pyrimidin-4 (3*H*) -one. ¹H NMR (200 MHz, CDCl₃) δ_2.03 (d, J = 7.2 Hz, 3H), 2.08 (s, 3H), 2.42 (s, 3H), 6.24 (q, J = 7.2 Hz, 1H), 7.09-7.42 (m, 5H), 8.48 (m, 2H), 8.70 (m, 3H); MS (ES) : 345.1 (M⁻+1).
*dl*-5,6-dimethyl-2-(2-furyl)-7*H*-7-(1-phenylethyl) pyrrolo [2,3d]pyrimidin-4 (3*H*)-one. ¹H NMR (200 MHz, CDCl₃) δ 1.98 (d, J = 7.8 Hz, 3H), 1.99 (s, 3H), 2.37 (s, 3H), 6.12 (q, J = 7.8 Hz, 1H), 6.48 (dd, J=1.8 Hz, 3.6 Hz, 1H), 7.17-7.55 (m, 7H), 9.6 (s, 1H); MS (ES) : 334.2 (M⁻+1).
*dl*-5,6-dimethyl-2-(3-furyl)-7*H*-7-(1-phenylethyl)pyrrolo [2,3d]pyrimidin-4 (3*H*)-one. ¹H NMR (200 MHz, CDCl₃) δ 1.99 (d, J = 7 Hz, 3H), 2.02 (s, 3H), 2.42 (s, 3H), 6.24 (q, J = 7 Hz, 1H), 7.09 (s, 1H), 7.18-7.32 (m, 5H), 7.48 (s, 1H), 8.51 (s, 1H); MS (ES) : 334.2 (M⁻+1) .
*dl*-5,6-dimethyl-2-cyclopentyl-7H-7-(1-phenylethyl) pyrrolo[2,3d]pyrimidin-4(3H)-one. ¹H NMR (200 MHz, CDCl₃) δ 1.95 (d, J = 7.4 Hz, 3H), 2.00 (s, 3H), 2.33 (s, 3H), 1.68-1.88 (m, 8H), 2.97 (m, 1H), 6.10 (q, J = 7.4 Hz, 1H), 7.16-7.30 (m, 5H), 9.29 (s, 1H) ; MS (ES) : 336.3 (M⁻+1).
*dl*-5,6-dimethyl-2-(2-thienyl)-*7H*-7-(1-phenylethyl) pyrrolo[2,3d]pyrimidin-4(3*H*)-one ¹H NMR (200 MHz, CDCl₃) δ 2.02(d, J = 7.2 Hz, 3H), 2.06 (s, 3H), 2.41 (s, 3H), 6.13 (q, J = 7.2 Hz, 1H), 7.12 (dd, J = 4.8, 2.8 Hz, 1H), 7.26-7.32 (m, 5H), 7.44 (d, J = 4.8 Hz, 1H), 8.01 (d, J = 2.8 Hz, 1H) 11.25 (s, 1H) ; MS (ES) : 350.2 (M⁻+1).
*dl*-5,6-dimethyl-2-(3-thienyl)-7*H*-7-(1-phenylethyl) pyrrolo [2, 3d] pyrimidin-4(3*H*)-one. ¹H NMR (200 MHz, CDCl₃) δ 2.00 (d, J = 7.4 Hz, 3H), 2.05 (s, 3H), 2.43 (s, 3H), 6.24(q, J = 7.4 Hz, 1H), 7.24-7.33 (m, 5H), 7.33-7.39 (m, 1H), 7.85 (m, 1H), 8.47 (m, 1H), 12.01 (s, 1H); MS (ES): 350.2 (M⁻+1).
*dl*-5,6-dimethyl-2-(4-fluorophenyl)-7*H*-7-(1-phenylethyl) pyrrolo [2, 3d] pyrimidin-4 (*3H*)-one ¹H NMR (200 MHz, CDCl₃) δ 2.01 (d, J = 6.8 Hz, 3H), 2.05 (s, 3H), 2.42 (s, 3H), 6.26 (q, J = 6.8 Hz, 1H), 7.12-7.36 (m, 7H), 8.23-8.30 (m, 2H), 11.82 (s, 1H) ; MS (ES): 362.3 (M⁻+1).
*dl-*5,6-dimethyl-2-(3-fluorophenyl)-7*H*-7-(1-phenylethyl) pyrrolo [2,3d] pyrimidin-4 (3*H*) -one. ¹H NMR (200 MHz, CDCl₃) *δ* 2.02 (d, J = 7.4 Hz, 3H), 2.06 (s, 3H), 2.44 (s, 3H), 6.29 (q, J = 7.4 Hz, 1H), 7.13-7.51(m, 7H), 8.00-8.04 (m, 2H), 11.72 (s, 1H) ; MS (ES) : 362.2 (M⁻+1) .
*dl*-5,6-dimethyl-2-(2-fluorophenyl)-7*H*-7-(1-phenylethyl) pyrrolo [2, 3d] pyrimidin-4 (3H) -one. ¹H NMR (200 MHz, CDCl₃) δ 2.00(d, J = 7.2 Hz, 3H), 2.05 (s, 3H), 2.38 (s, 3H), 6.24 (q, J = 7.2 Hz. 1H), 7.18 - 7.45 (m, 8 H), 8.21 (m, 1H), 9.54 (s, 1H); MS (ES): 362.2 (M⁻+1).
*dl*-5,6-dimethyl-2-isopropyl-7*H*-7-(1-phenylethyl)pyrrolo [2, 3d] pyrimidin-4 (3*H*) -one
   ¹H NMR (200 MHz, CDCl₃) δ 1.30 (d, J = 6.8 Hz, 3H), 1.32 (d, J = 7.0 Hz, 3H), 2.01 (s, 3H), 2.34 (s, 3H), 2.90 (m, 1H), 6.13 (m, 1H), 7.17-7.34 (m, 5H), 10.16 (s, 1H); MS (ES): 310.2 (M⁻+1).

### Preparation 8:

A solution of *dl*-1-(1-phenylethyl)-2-benzoylamino-3-cyano-4-dimethylpyrrole (785 mg, 2.38 mmol) with concentrated H₂SO₄ (1 mL) in DMF (13 mL) was stirred at 130°C for 48 h. The black solution was diluted with CHCl₃ (100 mL) and washed with 1 N NaOH (30 mL), and brine (30 mL). The organic fraction was dried, filtered, concentrated, and purified by flash chromatography (SiO₂; 8/2 EtOAc/Hex, *R*_{*f*} 0.35) to a brown solid (184 mg, 24%) as *dl*-5-methyl-2-phenyl-7*H*-7-(1-phenylethyl)pyrrolo[2,3d]pyrimidin-4(3*H*)-one. ¹H NMR (200 MHz, CDCl₃) δ_8.18 (m, 2H, Ar-H) 7.62-7.44 (m, 3H, Ar-H), 7.40-7.18 (m, 5H, Ar-H), 6.48 (s, 1H, pyrrole-H), 6.28 (q, 1H, *J* = 7.2 Hz, CH-CH₃), 2.18 (s, 3H, pyrrole-CH₃) , 2.07 (d, 3H, *J* = 7.2 Hz, CH-CH₃) ; MS (ES) : 330.2 (M⁺ + 1) .

### Preparation 9:

A mixture of *dl*-1-(1-phenylethyl)-2-amino-3-cyano-4,5-dimethylpyrrole (9.60 g, 40.0 mmol) and of formic acid (50.0 mL, 98%) was refluxed for 5 hr. After cooling down to room temperature and scratching the sides of flask, copious precipitate was formed and filtered. The material was washed with water until washings showed neutral pH to give *dl*-5,6-dimethyl-7*H*-7-(1-phenylethyl)pyrrolo[2,3d]pyrimidin-4(3H)-one. ¹H NMR (200 MHz, CDCl₃) δ 1.96 (d, J = 7.4 hz, 3H), 2.00 (s, 3H), 2.38 (s, 3H), 6.21 (q, J = 7.4 Hz, 1H), 7.11-7.35 (m, 5H), 7.81 (s, 1H). 11.71 (s, 1H) ; MS (ES): 268.2 (M⁻+1).

### Preparation 10:

*dl*-5,6-dimethyl-2-phenyl-7*H*-7-(1-phenylethyl) pyrrolo [2, 3d]pyrimidin-4(3*H*)-one (1.0 g, 2.91 mmol) was suspended in polyphosphoric acid (30.0 mL). The mixture was heated at 100°C for 4 hr. The hot suspension was poured onto ice water, stirred vigorously to disperse suspension, and basified to pH 6 with solid KOH. The resulting solid was filtered and collected to give 0.49 g (69%) of 5,6-dimethyl-2-phenyl-7*H-*pyrrolo [2, 3d] pyrimidin-4 (3*H*) -one. ¹H NMR (200 MHz, DMSO-d₆) δ_2.17 (s, 3H), 2.22 (s, 3H), 7.45 (br, 3H), 8.07 (br, 2H,), 11.49 (s, 1H), 11.82 (s, 1H); MS (ES) : 344.2 (M⁻+1) .

The following compounds were obtained in a similar manner as that of Preparation 10:
5-methyl-2-phenyl-7*H*-pyrrolo[2,3d]pyrimidin-4(3*H*)-one. MS (ES) : 226.0 (M⁻+1) .
5, 6-dimethyl-2- (3-pyridyl)-7*H*-pyrrolo [2, 3d] pyrimidin-4 (3*H*) - one. MS (ES): 241.1 (M⁻+1).
5, 6-dimethyl-2- (2-furyl) -7*H*-pyrrolo [2, 3d]pyrimidin-4 (3*H*) -one. ¹H NMR (200 MHz, DMSO-d₆) δ 2.13 (s, 3H), 2.18 (s, 3H), 6.39 (dd, J = 1.8, 3.6 Hz, 1H), 6.65 (dd, J = 1.8 Hz, 3.6 Hz, 1H), 7.85 (dd, J = 1.8, 3.6 Hz, 1H,), 11.45 (s, 1H), 11.60 (s, 1H) ; MS (ES) : 230.1 (M⁻+1) .
5, 6-dimethyl-2- (3-furyl)-7*H*-pyrrolo [2, 3d] pyrimidin-4 (3*H*) -one. ¹H NMR (200 MHz, DMSO-d₆) δ 2.14 (s, 3H), 2.19 (s, 3H), 6.66 (s, 1H), 7.78 (s, 1H), 8.35 (s, 1H), 11.3 (s, 1H), 11.4 (s, 1H) ; MS (ES) : 230.1 (M⁻+1) .
5,6-dimethyl-2-cyclopentyl-7*H*-pyrrolo[2,3d]pyrimidin-4(3*H*)-one. ¹H NMR (200 MHz, DMSO-d₆) δ 1.57-1.91 (m, 8 H), 2.12 (s, 3H), 2.16 (s, 3H), 2.99 (m, 1H), 11.24 (s, 1H), 11.38 (s, 1H) ; MS (ES): 232.2 (M⁻+1).
5,6-dimethyl-2-(2-thienyl)-7*H*-pyrrolo[2,3d]pyrimidin-4(3*H*)-one. ¹H NMR (200 MHz, DMSO-d₆) δ 2.14 (s, 3H), 2.19 (s, 3H). 7.14 (dd, J = 3.0, 5.2 Hz, 1H), 7.70 (d, J = 5.2 Hz 1H), 8.10 (d, J=3.0 Hz, 1H), 11.50 (s, 1H); MS (ES): 246.1 (M⁻+1).
5,6-dimethyl-2-(3-thienyl)-7*H*-pyrrolo[2,3d]pyrimidin-4(3*H*)-one. ¹H NMR (200 MHz, DMSO-d₆) δ 2.17 (s, 3H), 2.21 (s, 3H), 7.66(m, 1H), 7.75 (m, 1H), 8.43 (m, 1H), 11.47 (s, 1H), 11.69 (s, 1H) ; MS (ES): 246.1 (M⁻+1).
5,6-dimethyl-2-(4-fluorophenyl)-7*H*-pyrrolo[2,3d]pyrimidin-4 (3*H*) -one. ¹H NMR (200 MHz, DMSO-d₆) δ 2.17 (s, 3H), 2 .21 (s, 3H), 7.31 (m, 2H), 8.12 (m, 2H), 11.47 (s, 1H) ; MS (ES): 258.2 (M⁺+1) .
5,6-dimethyl-2-(3-fluorophenyl)-*7H*-pyrrolo[2, 3d]pyrimidin-4(*3H*)-one. ¹H NMR (200 MHz, DMSO-d₆) δ 2.18 (s, 3H), 2.21 (s, 3H), 7.33 (m, 1H), 7.52 (m, 1H), 7.85-7.95 (m, 2H), 11.56 (s, 1H), 11.80 (s, 1H); MS (ES) : 258.1 (M⁻+1) .
5,6-dimethyl-2-(2-fluorophenyl)-*7H*-pyrrolo[2,3d]pyrimidin-4 (3*H*) -one. ¹H NMR (200 MHz, DMSO-d₆) δ 2.18 (s, 3H), 2.22 (s, 3H), 7.27-7.37 (m, 2H), 7.53 (m 1H), 7.68 (m, 1H), 11.54 (s, 1H), 11.78 (s, 1H) ; MS (ES) : 258.1 (M⁻+1) .
5, 6-dimethyl-2-isopropyl-7*H*-pyrrolo[2,3d]pyrimidin-4(3*H*)-one. ¹H NMR (200 MHz, DMSO-d₆) δ 1.17 (d, J= 6.6 Hz, 6H), 2.11 (s, 3H), 2.15 (s, 3H), 2.81 (m, 1H), 11.20 (s, 1H), 11.39 (s, 1H) ; MS (ES): 206.1 (M⁻+1) .
5,6-dimethyl-7*H*-pyrrolo[2,3d]pyrimidin-4(3H)-one. ¹H NMR (200 MHz, DMSO-d₆) δ 2.13 (s, 3H), 2.17 (s, 3H), 7.65 (s, 1H); MS (ES): 164.0 (M⁻+1) .

### Preparation 11:

A solution of 5,6-dimethyl-2-phenyl-7*H*-pyrrolo[2,3d] pyrimidin-4(3*H*)-one (1.0 g, 4.2 mmol) in phosphorus oxychloride (25.0 mL) was refluxed for 6 hr and then concentrated in *vacuo* to dryness. Water was added to the residue to induce crystallization and the resulting solid was filtered and collected to give 0.90 g (83%) of 4-chloro-5,6-dimethyl-2-phenyl-7*H*-pyrrolo[2,3d]pyrimidine. ¹H NMF (200 MHz, DMSO-d₆) δ_2.33 (s, 3H), 2.33 (s, 3H), 7.46-7.49 (m, 3H), 8.30-8.35 (m, 2H), 12.20 (s, 1H); MS (ES) : 258.1 (M⁻+1). The following compounds were obtained in a similar manner as that of Preparation 11:
4-chloro-5-methyl-2-phenyl-7*H*-pyrrolo[2,3d]pyrimidine. MS (ES) : 244.0 (M⁺+1) .
4-chloro-6-methyl-2-phenyl-7*H*-pyrrolo[2,3d]pyrimidine. MS (ES) : 244.0 (M⁻+1) .
4-chloro-2-phenyl-7*H*-pyrrolo[2,3d]pyrimidine. ¹H NMR (200 MHz, DMSO-d6) 8.35 (2, 2H), 7.63 (br s, 1H), 7.45 (m, 3H), 6.47 (br s, 1H) ; MS (ES) : 230.0 (M⁻+1) .
4-chloro-5,6-dimethyl-2-(3-pyridyl)-7*H*-pyrrolo[2,3d] pyrimidine. MS (ES) : 259.0 (M⁺+1) .
4-chloro-5,6-dimethyl-2-(2-furyl)-7*H*-pyrrolo[2,3d]pyrimidine. ¹H NMR (200 MHz, DMSO-d₆) δ 2.35 (s, 3H), 2.35 (s, 3H), 6.68 (dd, J = 1.8, 3.6 Hz, 1H), 7.34 (dd, J = 1.8 Hz, 3.6 Hz, 1H), 7.89 (dd, J = 1.8, 3.6 Hz, 1H); MS (ES): 248.0 (M⁻+1).
4-chloro-5,6-dimethyl-2-(3-furyl)-7*H*-pyrrolo[2,3d]pyrimidine. ¹H NMR (200 MHz, DMSO-d₆) δ 2.31 (s, 3H), 2.31 (s, 3H), 6.62 (s, 1H), 7.78 (s, 1H), 8.18 (s, 1H), 12.02 (s, 1H) ; MS (ES) : 248.1 (M⁺+1).
4-chloro-5,6-dimethyl-2-cyclopentyl-7*H*-pyrrolo[2,3d] pyrimidine. ¹H NMR (200 MHz, DMSO-d₆) δ 1.61- 1.96 (m, 8H), 2.27 (s, 3H), 2.27 (s, 3H), 3.22 (m. 1H), 11.97 (s, 1H), MS (ES) : 250.1 (M⁻+1)
4-chloro-5,6-dimethyl-2-(2-thienyl)-7*H*-pyrrolo[2,3d] pyrimidine. ¹H NMR (200 MHz, DMSO-d₆) δ 2.29 (s, 3H), 2.31 (s, 3H), 7.14 (dd, J = 3.1 Hz, 4.0 Hz, 1H), 7.33 (d, J = 4.9 Hz, 1H), 7.82 (d, J = 3.1 Hz, 1H), 12.19 (s, 1H); MS (ES): 264.1 (M⁻+1) .
4-chloro-5,6-dimethyl-2-(3-thienyl)-7*H*-pyrrolo[2,3d] pyrimidine. ¹H NMR (200 MHz, DMSO-d₆) δ 2.32 (s, 3H), 2.32 (s, 3H), 7.62 (dd, J = 3.0, 5.2 Hz, 1H), 7.75 (d, J = 5.2 Hz, 1H), 8.20 (d, J = 3.0 Hz, 1H) ; MS (ES): 264.0 (M⁻+1) .
4-chloro-5,6-dimethyl-2-(4-fluorophenyl)-7*H*-pyrrolo[2,3d] pyrimidine. ¹H NMR (200 MHz, DMSO-d₆) δ 2.33(s, 3H), 2.33 (s, 3H), 7.30 (m, 2H), 8.34 (m, 2H), 12.11 (s, 1H); MS (ES): 276.1. (M⁻+1).
4-chloro-5,6-dimethyl-2-(3-fluorophenyl)-7*H*-pyrrolo[2,3d] pyrimidine. ¹H NMR (200 MHz, DMSO-d₆) δ 2.31(s, 3H), 2.33 (s, 3H), 7.29(m, 1H), 7.52 (m, 1H), 7.96 (m, 1H), 8.14(m, 1H), 11.57 (s, 1H) ; MS (ES): 276.1 (M⁻+1) .
4-chloro-5,6-dimethyl-2-(2-fluorophenyl)-7*H*-pyrrolo[2,3d] pyrimidine. ¹H NMR (200 MHz, DMSO-d₆) δ 2.34 (s, 3H), 2.34 (s, 3H), 7.33 (m, 2H), 7.44 (m, 1H), 7.99 (m, 1H), 12.23 (s, 1H); MS (ES): 276.1 (M⁻+1).
4-chloro-5,6-dimethyl-2-isopropyl-7*H*-pyrrolo[2,3d]pyrimidine. ¹H NMR (200 MHz, DMSO-d₆) δ 1.24 (d, J = 6.6 Hz, 6H), 2.28 (s, 3H), 2.28 (s, 3H), 3.08 (q, J = 6.6 Hz, 1H), 11.95 (s, 1H); MS (ES) : 224.0 (M⁻+1).
4-chloro-5,6-dimethyl-7*H*-pyrrolo[2,3d]pyrimidine. ¹H NMR (200 MHz, DMSO-d₆) δ 2.31 (s, 3H), 2.32 (s, 3H), 8.40 (s, 1H); MS (ES) : 182.0 (M⁻+1).
*dl*-4-chloro-5, 6-dimethyl-2-phenyl-7*H*-7-(1-phenylethyl)pyrrolo [2,3d]pyrimidine.

### Preparation 12:

To a solution of *dl*-1,2-diaminopropane (1.48 g, 20.0 mmol) and sodium carbonate (2.73 g, 22.0 mmol) in dioxane (100.0 mL) and water (100.0 mL) was added di-tert-dicarbonate (4.80 g, 22.0 mmol) at room temperature. The resulted mixture was stirred for 14 hr. Dioxane was removed *in vacuo.* The precipitate was filtered off and the filtrate was concentrated *in vacuo* to dryness. The residue was triturated with EtOAc and then filtered. The filtrate was concentrated *in vacuo* to dryness to give a mixture of *dl*-1-amino-2-(1,1-dimethylethoxy)carbonylamino-propane and *dl*-2-amino-1-(1,1-dimethylethoxy)carbonylamino-propane which were not separable by normal chromatography method. The mixture was used for the reaction in Example 8.

### Preparation 13:

To solution of Fmoc-β-Ala-OH (1.0 g, 3.212 mmol) and oxalyl chloride (0.428 g, 0.29 mL, 3.373 mmol) in dichloromethane (20.0 mL) was added a few drops of N,N-dimethylformamide at 0°C. The mixture was stirred at room temperature for 1 hr followed by addition of cyclopropylmethylamine (0.229 g, 0.28 mL, 3.212 mmol) and triethylamine (0.65 g, 0.90 mL, 6.424 mmol). After 10 min, the mixture was treated with 1 M hydrochloride (10.0 mL) and the aqueous mixture was extracted with dichloromethane (3 x 30.0 mL). The organic solution was concentrated *in vacuo* to dryness. The residue was treated with a solution of 20% piperidine in N,N-dimethylforamide (20.0 mL) for 0.5 hr. After removal of the solvent *in vacuo*, the residue was treated with 1 M hydrochloride (20.0 mL) and ethyl acetate (20.0 mL). The mixture was separated and the aqueous layer was basified with solid sodium hydroxide to pH = 8. The precipitate was removed by filtration and the aqueous solution was subjected to ion exchange column eluted with 20% pyridine to give 0.262 g (57%) of N-cyclopropylmethyl β-alanine amide. ¹H NMR (200 MHz, CD₃OD) δ_0.22 (m, 2H), 0.49 (m, 2H), 0.96 (m, 2H), 2.40 (t, 2H), 2.92 (t, 2H), 3.05 (d, 2H); MS (ES) : 143.1 (M⁻+1)

### Preparation 14:

### N-tert-butoxycarbonyl-trans-1,4-cyclohexyldiamine

*trans*-1,4-cyclohexyldiamine (6.08 g, 53.2 mmol) was dissolved in dichloromethane (100mL) . A solution of di-t-butyldicarbonate (2.32 g, 10.65 mmol in 40 mL dichloromethane) was added via cannula. After 20 hours, the reaction was partitioned between CHCl₃ and water. The layers were separated and the aqueous layer was extracted with CHCl₃ (3x). The combined organic layers were dried over MgSO₄, filtered and concentrated to yield 1.20 g of a white solid (53%) ¹H-NMR (200MHz, CDCl₃) : δ 1.0-1.3 (m, 4H), 1.44 (s, 9H), 1.8 -2.1 (m, 4H), 2.62 (brm, 1H), 3.40 (brs, 1H), 4.37 (brs, 1HO; MS (ES): 215.2 (M⁻+1).

### 4-(N-acetyl)-N-tert-butoxycarbonyl-trans-1,4-cyclohexyl diamine.

N-tert-butoxycarbonyl-*trans*-1,4-cyclohexyldiamine (530 mg, 2.47 mmol) was dissolved in dichloromethane (20 mL). Acetic anhydride (250 mg, 2.60 mmol) was added dropwise. After 16 hours, the reaction was diluted with water and CHCl₃. The layers were separated and the aqueous layer was extracted with CHCl₃ (3x). The combined organic layers were dried over MgSO₄, filtered and concentrated. Recrystallization (EtOH/H₂O) yielded 190 mg of white crystals (30%) . ¹H NMR (200 MHz, CDCl₃) : δ 0.9 - 1.30 (m, 4H), 1.43 (s, 9H), 1.96-2.10 (m, 7H), 3.40 (brs, 1H), 3.70 (brs, 1H), 4.40 (brs, 1H), 4.40 (brs, 1H) ; MS (ES): 257.2 (M⁻+1), 242.1 (M⁻ - 15), 201.1 (M⁻ - 56).

### 4-(4-trans-acetamidocyclohexyl)amino-5,6-dimethyl-2-phenyl-7H-(1-phenylethyl) pyrrolo[2,3d]pyrimidine.

4-(N-acetyl)-N-*tert*-butoxycarbonyl-*trans*-1,4-cyclohexyldiamine (190 mg, 0.74 mmol), was dissolved in dichloromethane (5 mL) and diluted with TFA (6 ml). After 16 hours, the reaction was concentrated. The crude solid, DMSC (2mL), NaHCO₃ (200 mg, 2.2 mmol) and 4-chloro-5,6-dimethyl-2-phenyl-7H-pyrrolo[2,3d]pyrimidine (35 mg, 0.14 mmol) were combined in a flask and heated to 130 °C. After 4.5 hours, the reaction was cooled to room temperature and diluted with EtOAc and water. The layers were separated and the aqueous layer was extracted with EtOAc (3x). The combined organic layers were dried over MgSO₄, filtered and concentrated Chromatography (silica preparatory plate; 20:1 CHCl₃:EtOH) yielded 0.3 mg of a tan solid (1% yield). MS (ES): 378.2 (M⁻+1).

### 4-(N-methanesulfonyl)-N-tert-butoxycarbonyl-trans-1,4-cyclohexyldiamine.

*trans*-1,4-cyclohexyldiamine (530 mg, 2.47 mmol) was dissolved in dichloromethane (20 ml) and diluted with pyridine (233 mg, 3.0 mmol). Methanesulfonyl chloride (300 mg, 2.60 mmol) was added dropwise. After 16 hours, the reaction was diluted with water and CHCl₃. The layers were separated and the aqueous layer was extracted with CHCl₃ (3x) The combined organic layers were dried over MgSO₄, filtered and concentrated recrystallization (EtOH/H₂O) yielded 206 mg of white crystals (29%) ¹H-NMR (200MHz, CDCl₃) : δ 1.10-1.40 (m, 4H), 1.45 (s, 9H), 2.00-2.20 (m, 4H), 2.98 (s, 3H), 3.20-3.50 (brs, 2H), 4.37 (brs, 1H) ; MS (ES) 293.1 (M⁻+1), 278.1 (M-15), 237.1 (M⁻-56) .

### 4-(4-trans-methanesulfamidocyclohexyl)amino-5,6-dimethyl-2-phenyl-7H-(1-phenylethyl)pyrrolo[2,3d]pyrimidine.

4- (N-sulfonyl)-N- tert-butoxycarbonyl- *trans*-1, 4-cyclohexyldiamine (206 mg, 0.71 mmol), was dissolved in dichloromethane (5ml) and diluted with TFA (6 ml). After 16 hours, the reaction was concentrated. The crude reaction mixture, DMSO (2 ml), NaHCO₃ (100 mg, 1.1 mmol) and 1-chloro-5,6-dimethyl-2-phenyl-7H-pyrrolo[2,3d]pyrimidine were combined in a flask and heated to 130 °C. After 15 hours, the reaction was cooled to room temperature, and diluted with EtOAc (3x). The combined organic layers were dried over MgSO₄, filtered and concentrated. Chromatography (silica preparatory plate, 20:1 CHCl₃/EtOH) yielded 2.6 mg of a tan solid (5% yield). MS (ES) : 414.2 (M⁻+1).

### Reference Example 1:

A solution of 4-chloro-5,6-dimethyl-2-phenyl-7*H*-pyrrolo[2,3d] pyrimidine (0.50 g, 1.94 mmol) and 4-trans-hydroxy cyclohexylamine (2.23 g, 19.4 mmol) in methyl sulfoxide (10.0 mL) was heated at 130°C for 5 hr. After cooling down to room temperature, water (10.0 mL) was added and the resulted aqueous solution was extracted with EtOAc (3 x10.0 mL). The combined EtOAc solution was dried (MgSO₄) and filtered, the filtrate was concentrated in *vacuo* to dryness, the residue was chromatographed on silica gel to give 0.49 g (75%) of 4-(4-*trans*-hydroxycyclohexyl)amino-5,6-dimethyl-2-phenyl-7*H-*pyrrolo[2,3d]pyrimidine. mp 197-199°C; ¹H NMR (200 MHz, CDCl₃) δ_1.25-1.59 (m, 8H), 2.08 (s, 3H), 2.29 (s, 3H), 3.68-3.79 (m, 1H), 4.32-4.38 (m, 1H), 4.88 (d, J = 8 Hz, 1H), 7.26-7.49 (m, 3H), 8.40-8.44 (dd, J = 2.2, 8 Hz, 2H), 10.60 (s, 1H); MS (ES) : 337.2 (M⁻+1).

The following compounds were obtained in a similar manner to that of Ref. Example 1:
*dl*-4-(3- *trans*-hydroxycyclopentyl) amino-5,6-dimethyl-2-phenyl-7*H*-pyrrolo[2,3d]pyrimidine.
   ¹H NMR (200 MHz, CDCl₃) δ_1.58-1.90 (br, 6 H,), 2.05 (s, 3H), 2.29 (s, 3H), 4.48-4.57 (m, 1H), 4.91-5.01 (m, 2H), 7.35-7.46 (m, 3H), 8.42-8.47 (m, 2H), 10.11 (s, 1H); MS (ES): 323.2 (M⁻+1).

For preparation of 3-*trans*-hydroxycyclopentylamine, see EP-A-322242.
*dl*-4-(3-*cis*-hydroxycyclopentyl)amino-5,6-dimethyl-2-phenyl-7*H*-pyrrolo[2,3d]pyrimidine.
   ¹H NMR (200 MHz, CDCl₃) δ_1.82-2.28 (br, 6H), 2.02 (s, 3H), 2.30 (s, 3H), 4.53-4.60 (m, 1H), 4.95-5.08 (m, 1H), 5.85-5.93 (d, 1H), 7.35-7.47 (m, 3H), 8.42-8.46 (m, 2H), 10.05 (s, 1H); MS (ES) : 323.2 (M⁻+1) .
   ¹ For preparation of 3-*cis*-hydroxycyclopentylamine, see EP-A-322242.

### Ref. Example 2:

To a stirred suspension of triphenylphosphine (0.047 g, 0.179 mmol) and benzoic acid (0.022 g, 0.179 mmol) in THF (1.0 mL) cooled to 0°C was added 4 - (4 - *trans*-hydroxycyclohexyl) amino-*5,6*-dimethyl-*2*-phenyl-*7H*-pyrrolo[2,3d]pyrimidine (0.05 g, 0.149 mmol) at 0°C. Diethyl azodicarboxylate (0.028 ml, 0.179 mmol) was then added dropwise over 10 minutes. The reaction was then allowed to warm to room temperature. After reaction was complete by TLC the reaction mixture was quenched with aqueous sodium bicarbonate (3.0 mL). The aqueous phase was separated and extracted with ether (2 X 5.0 mL). The organic extracts were combined, dried, and concentrated *in vacuo* to dryriess. To the residue was added ether (2.0 mL) and hexane (5.0 mL) whereupon the bulk of the triphenylphosphine oxide was filtered off. Concentration of the filtrate gave a viscous oil which was purified by column chromatography (hexane:ethyl acetate=4:1) to give 5.0 mg (7.6%) of 4-(4-*cis*-benzoyloxycyclohexyl)amino-5,6-dimethyl-2-phenyl-7*H-*pyrrolo[2,3d]pyrimidine. MS (ES): 441.3 (M⁻+1). The reaction also produced 50.0 mg (84%) of 4-(3-cyclohexenyl) amino- 5,6-dimethyl-2-phenyl-7*H*-pyrrolo[2,3d]pyrimidine. MS (ES): 319.2 (M+1)

### Ref. Example 3:

To a solution of 4-(4-cis-benzoyloxycyclohexyl)amino-5,6-dimethyl-2-phenyl-7*H*-pyrrolo[2,3d]pyrimidine (5.0 mg, 0.0114 mmol) in ethanol (1.0 mL) was added 10 drops of 2M sodium hydroxide. After 1 hr, the reaction mixture was extracted with ethyl acetate (3 x 5.0 mL) and the organic layer was dried, filtered and concentrated *in vacuo* to dryness. The residue was subjected to column chromatography (hexane:ethyl acetate=4:1) to give 3.6 mg (94%) of 4-(4-*cis-*hydroxycyclohexyl)amino-5,6-dimethyl-2-phenyl-7*H-*pyrrolo[2,3d]pyrimidine. MS (ES): 337.2 (M⁺+1).

### Ref. Example 4:

4 - (3-tert-bucyloxy-3-oxopropyl)amino- 5,6-dimethyl-2-phenyl - 7H-pyrrolo[2,3d]pyrimidine (70.0 mg, 0.191 mmol)) was dissolved in trifluoroacetic acid:dichloromechane (1:1, 5.0 mL). The resulting solution was stirred at room temperature for 1 hr. and then refluxed for 2 hr. After cooling down to room temperature, the mixture was concentrated in vacuo to dryness. The residue was subjected to preparative thin layer chromatography (EtOAc:hexane: AcOH=7:2.5:0.5) to give 40.0 mg (68%) of. 4-(3-hydroxy-3-oxopropyl)amino-5,6-dimethyl-2-phenyl-7*H*-pyrrolo[2,3d] pyrimidine. ¹H NMR (200 MHz, CD₂OD) δ 2.32 (s, 3H), 2.38 (s, 3H), 2.81 (t, 2H), 4.01 (t, 2H), 7.55 (m, 3H), 8.24 (m, 2H); MS (ES): 311.1 (M⁻+1).

### Ref. Example 5:

4-(3-hydroxy-3-oxopropyl)amino-5,6-dimethyl-2-phenyl-7*H-*pyrrolo[2,3d]pyrimidine (50.0 mg, 0.161 mmol) was dissolved in a mixture of N,N-dimethylformamide (0.50 mL), dioxane (0.50 mL) and water(0.25 mL) . To this solution was added methylamine (0.02 mL, 40% wt in water, 0.242 mmol), triethylamine (0.085 mL) and N,N,N'N'-tetramethyl uronium tetrafluoroborate (61.2 mg, 0.203 mmol). After stirring at room temperature for 10 min, the solution was concentrated and the residue was subjected to preparative thin layer chromatography (EtOAc) to give 35.0 mg (67%) of4-(3-N-methyl-3-oxopropyl)amino-5,6-dimethyl-2-phenyl-7*H-*pyrrolo[2,3d]pyrimidine. ¹H NMR (200 MHz, CDCl₃) δ 1.92 (s, 3H), 2.30 (s, 3H), 2.65 (t, 2H), 4.08 (t, 2H), 5.90 (t, 1H), 6.12 (m, 1H), 7.45 (m, 3H), 8.41 (m, 2H), 10.68 (s, 1H); MS

### Ref. Example 6:

A mixture of 4-chloro-5,6-dimethyl-2-phenyl-7*H*-pyrrolo[2,3d] pyrimidine (0.70 g, 2.72 mmol) and 1,2-diaminoethane (10.0 mL, 150 mmol) was refluxed under inert atmosphere for 6 hr. The excess amine was removed in vacuo, the residue was washed sequentially with ether and hexane to give 0.75 g (98%) of 4-(2-aminoethyl)amino-5,6-dimethyl-2-phenyl-7*H-*pyrrolo[2,3d]pyrimidine. MS (ES); 282.2 (M⁻+1) , 265.1 (M⁻-NH₃).

### Ref. Example 7:

To a solution of 4-(2-aminoethyl)amino-5,6-dimethyl-2-phenyl-7*H*-pyrrolo[2,3d]pyrimidine (70.0 mg, 0.249 mmol) and triethylamine (50.4 mg, 0.498 mmol) in dichloromethane (2.0 mL) was added propionyl chloride (25.6 mg, 0.024 mL. 0.274 mmol) at 0°C. After 1 hr, the mixture was concentrated *in vacuo* and the residue was subjected to preparative thin layer chromatography (EtOAc) to give 22.0 mg (26%) of 4-(2-propionylaminoethyl)amino-5,6-dimethyl-2-phenyl-7*H-*pyrrolo[2,3d]pyrimidine. MS (ES): 338.2 (M⁺+1).

The following compounds were obtained in a similar manner as that of Ref. Example 7:
4-(2-N'-methylureaethyl)amino-5,6-dimethyl-2-phenyl-7*H-*pyrrolo [2, 3d]pyrimidine. ¹H NMR (200 MHz, CDCl₃) δ 2.13 (s, 3H), 2.32 (s, 3H), 3.53 (d, 3H), 3.55 (m, 2H), 3.88 (m, 2H), 4.29 (m, 1H), 5.68 (t, 1H), 5.84 (m, 1H), 7.42 (m, 3H), 8.36 (dd, 2H), 9.52 (s, 1H); MS (ES): 339.3 (M⁻+1) .

### Ref. Example 8:

To a solution of 1-(3-dimethylaminopropyl)-3-ethylcarbodiimide hydrochloride (41.1 mg, 0.215 mmol), dimethylaminopyridine (2.4 mg, 0.020 mmol) and pyruvic acid (18.9 mg, 0.015 mL, 0.215 mmol) in dichloromethane (2.0 mL) was added 4-(2-aminoethyl)amino-5,6-dimethyl-2-phenyl-7*H*-pyrrolo[2,3d] pyrimidine (55.0 mg, 0.196 mmol). The mixture was stirred at room temperature for 4 hr. Usual workup and column chromatography (EtOAc) then gave 10.0 mg (15%) of 4-(2'-pyruvylamidoethyl)amino-S,6-dimethyl-2-phenyl-7*H-*pyrrolo[2,3d]pyrimidine.. MS (ES): 352.2 (M⁻+1).

### Ref. Example 9:

To a solution of 4-(2-aminoethyl)amino-5,6-dimethyl-2-phenyl-7*H*-pyrrolo[2,3d]pyrimidine (60.0 mg, 0.213 mmol) in dichloromethane (2.0 mL) was added N-trimethylsilyl isocyanate (43.3 mg, 0.051 mL, 0.320 mmol). The mixture was stirred at room temperature for 3 hr followed by addition of aqueous sodium bicarbonate. After filtration through small amount of silica gel, the filtrate was concentrated *in vacuo* to dryness to give 9.8 mg (14%) of 4-(2-ureaethyl)amino-5,6-dimethyl-2-phenyl-7*H*-pyrrolo(2,3d)pyrimidine. MS (ES): 325.2 (M⁻+1).

### Ref. Example 10:

Reaction of 4-chloro-5,6-dimethyl-2-phenyl-7H-pyrrolo[2.3d] pyrimidine with the mixture of *dl*-1-amino-2-(1,1-dimethyl ethoxy)carbonylamino-propane and *dl*-2-amino-1-(1,1-dimethyl ethoxy)carbonylamino-propane was run in a similar manner as that of Example 1. The reaction gave a mixture of *dl*-4-(1-methyl-2-(1,1-dimethylethoxy)carbonylamino)ethylamino-5,6--dimethyl-2-phenyl-7H-pyrrolo[2,3d]pyrimidine and *dl*-4-(2-methyl-2-(1,1-dimethylethoxy)carbonylamino)ethylamino-5,6-dimethyl-2-phenyl-7H-pyrrolo[2,3d]pyrimidine which were separated by column chromatography (EtOAc:hexanes=1:3). The first fraction was *dl*-4- (1-methyl-2- (1,1-dimethylethoxy) carbonylaminoethyl)amino-5,6-dimethyl-2-phenyl-7H-pyrrolo[2,3d]pyrimidine: ¹H NMR (200 MHz, CDCl₃) δ 1.29 - 1.38 (m, 12 H), 1.95 (s, 3H), 2.31 (s, 3H) 3.34-3.43 (m, 2H), 4.62-4.70 (m, 1H), 5.36-5.40 (d, J=8 Hz, 1H), 5.53 (br, 1H), 7.37-7.49(m, 3H), 8.37-8.44(m, 2H), 10.75 (s, 1H). MS 396.3 (M⁻+1) ; The second fraction was *dl*-4-(2- (1, 1-dimethylethoxy)carbonylaminopropyl)amino-5,6-dimethyl-2-phenyl-7H-pyrrolo[2,3d]pyrimidine: ¹H NMR (200 MHz, CDCl₃) 5 1.26-1.40 (m, 12 H), 2.00 (s, 3H), 2.31 (s, 3H) 3.60-3.90 (m, 2H), 3.95-4.10 (m, 1H), 5.41-5.44 (d, J=6.0 Hz, 1H), 5.65(br, 1H), 7.40-7.46(m, 3H), 8.37-8.44(m, 2H), 10.89 (s, 1H); MS (ES): 396.2 (M⁻+1) .

### Ref. Example 11:

*dl*-4-(1-methyl-2-(1,1-dimethylethoxy) carbonyl aminoethyl) amino-5,6-dimethyl-2-phenyl-7H-pyrrolo[2,3d]pyrimidine (60.6 mg, 0.153 mmol) was treated with trifluoroacetic acid (0.5 mL) in dichloromethane (2.0 mL) for 14 hr. The organic solvent was removed *in vacuo* to dryness. The residue was dissolved in N,N-dimethylformamide (2.0 mL) and triethylamine (2.0 mL). To the solution at 0°C was added acetic anhydride (17.2 mg, 0.016, 0.169 mmol). The resulted mixture was stirred at room temperature for 48 hr and then concentrated in *vacuo* to dryness. The residue was subjected to preparative thin layer chromatography (EtOAc) to give 27.0 mg (52%) of *dl*-4- (1-methyl-2-acetylaminoethyl)amino-5,6-dimethyl-2-phenyl-7H-pyrrolo[2,3d]pyrimidine. ¹H NMR (200 MHz, CDCl₃) δ 1.38-1.42 (d, J=8 Hz, 3 H), 1.69.(s, 3H), 2.01 (s, 3H), 2.32 (s, 3H) 3.38-3.60 (m, 2H), 4.65-4.80 (m, 1H), 5.23-5.26 (d, J=6 Hz, 1H), 7.40-7.51(m, 3H), 8.37-8.43(m, 2H), 10.44 (s, 1H); MS (ES): 338.2 (M⁻+1).

### Ref. Example 12 :

(R,R) -4- (2-aminocyclohexyl) amino -5,6 -dimethyl-2-phenyl-7H-pyrrolo[2,3d]pyrimidine, prepared in a similar manner as that of Ref. Example 1 from 4-chloro-5,6-dimethyl-2-phenyl-7*H-*pyrrolo[2,3d]pyrimidine (0.15 g, 0.583 mmol) and (1R, 2R)-(-)-1,2-diaminocyclohexane (0.63 g, 5.517 mmol), was treated with triethylamine (0.726 g, 7.175 mmol) and acetic anhydride (0.325 g, 3.18 mmol) in N,N-dimethylformamide (10.0 mL) at room temperature for 2 hr. After removal of solvent *in vacuo,* ethyl acetate (10.0 mL) and water (10.0 mL) were added to the residue. The mixture was separated and the aqueous layer was extracted with ethyl acetate (2 x 10.0 mL). The combined ethyl acetate solution was dried (MgSO₄) and filtered. The filtrate was concentrated in vacuo to dryness and the residue was subjected to column chromatography (EtOAc:Hexane=1:1) to give 57.0 mg (26%) of (R,R)-4-(2-acetylaminocyclohexyl)amino-5,6-dimethyl-2-phenyl-7H-pyrrolo(2,3d)pyrimidine. ¹H NMR (200 MHz, CDCl₃) δ_1.43 (m, 4 H), 1.60 (s, 3H), 1. 84 (m, 2 H), 2.22 (s, 3 H), 2.30 (m, 2 H); 2.33 (s, 3 H), 3.72 (br, 1H), 4.24 (br, 1H), 5.29 (d, 1H), 7.43-7.48 (m, 3H), 8.35-8.39 (m, 2H), 8.83 (s, 1H); MS (ES) : 378.3 (M⁻+1).

### Ref. Example 13:

To a solution of 4-(2-hydroxyethyl)amino-5,6-dimethyl-2-phenyl-7*H*-pyrrolo[2,3d]pyrimidine (40.0 mg, 0.141 mmol) in pyridine (1.0 mL) was added acetic anhydride (0.108 g, 1.06 mmol) at 0°C. The mixture was stirred at room temperature for 4 hr and the solvent was removed in vacuo. The residue was subjected to preparative thin layer chromatography (EtOAc:hexane=1:1) to give 32.3 mg (71%) of 4-(2-acetyloxyethyl) amino- 5, 6-dimethyl-2-phenyl-7*H*-pyrrolo [2, 3d] pyrimidine. ¹H NMR (200 MHz, CDCl₃) δ_1.90 (s, 3H), 2.08 (s. 3H), 2.31 (s, 3H); 4.05 (m, 2H), 4.45 (t, 2H), 5.42 (m, 1H). 7.41-7.49 (m, 3H)., 8.42(m, 2H), 11.23 (s, 1H).

### Ref. Example 14:

A solution of Fmoc-β-Ala-OH (97.4 mg, 0.313 mmol) and oxalyl chloride (39.7 mg, 27.3 µL, 0.313 mmol) in dichloromethane (4.0 mL) with 1 drop of N,N-dimethylformamide was stirred at 0°C for 1 hr followed by addition of 4-(2-aminoethyl)amino-5,6-dimethyl-2-phenyl-7*H*-pyrrolo[2,3d]pyrimidine (80.0 mg, 0.285 mmol) and triethylamine (57.6 mg, 79.4 µL, 0.570 mmol) at 0°C. After 3 hr, the mixture was concentrated in vacuo and the residue was treated with the solution of 20% piperidine in N,N-dimethylforamide (2.0 mL) for 0.5 hr. After removal of the solvent *in vacuo*, the residue was washed with diethyl ether:hexane (1:5) to give 3.0 mg (3%) of 4-(6-amino-3-aza-4-oxohexyl)amino-5,6-dimethyl-2-phenyl-7*H*-pyrrolo[2.3d] pyrimidine. MS (ES): 353.2 (M⁻+1).

### Ref. Example 15:

A solution of 4-(2-aminoethyl) amino-5,6-dimethyl-2-phenyl-7*H-*pyrrolo[2,3d]pyrimidine (70.0 mg, 0.249 mmol) and succinic anhydride (27.0 mg, 0.274 mmol) in dichloromethane (4.0 mL) with 1 drop of N,N-dimethylformamide was stirred at room temperature for 4 hr. The reaction mixture was extracted with 20% sodium hydroxide (3 x 5.0 mL). The aqueous solution was acidified with 3 M hydrochloride to pH = 7.0. The whole mixture was extracted with ethyl acetate (3 x 10 mL). The combined organic solution was dried (MgSO₄) and filtered. The filtrate was concentrated *in vacuo* to dryness to give 15.0 mg (16%) of 4-(7-hydroxy-3-aza-4,7-dioxoheptyl) amino-5,6-dimethyl-2-phenyl-7*H*-pyrrolo[2,3d]pyrimidine. MS (ES) : 382.2 (M⁺+1).

### Example 16:

To 10 mL of dimethylformamide (DMF) at room temperature were added 700 mg of 4-*cis*-3-hydroxycyclopentyl)amino-2-phenyl-5,6-dimethyl-7*H*-pyrrolo[2,3d]pyrimidine followed by 455 mg of N-Boc glycine, 20 mg of N,N-dimethylaminopyridine (DMAP), 293 mg of hydroxybenzotriazole (HOBT) and 622 mg of 1-(3-dimethylaminopropyl)-3-ethylcarboiimide hydrochloride (EDCl). The reaction mixture was left stirring overnight. DMF was then removed under reduced pressure and the reaction mixture was partitioned between 20mL of ethyl acetate and 50mL of water. The aqueous portion was extracted further with 2x20mL of ethyl acetate and the combined organic portions were washed with brine, dried over anhydrous sodium sulfate, filtered and concentrated. Purification on silica gel, eluting with ethyl acetate/hexane gave 410 mg of the desired product: 4-(*cis*-3-(N-t-butoxycarbonyl-2-aminoacetoxy) cyclopentyl) amino-2-phenyl-5,6,-dimethyl-7*H*-pyrrolo[2,3d] pyrimidine, MS (ES) (M⁻+1)=480.2. The ester was then treated with 5 mL of 20% trifluoroacetic acid in dichloromethane at room temperature, left over night and then concentrated Trituration with ethyl acetate gave 300 mg of an off white solid; 4-(*cis*-3-(2-aminoacetoxy)cyclopentyl)amino-5,6-dimethy 1-2-phenyl-7*H*-pyrrolo[2,3d]pyrimidine trifluoroacetic acid salt, MS (ES)(M⁻+1)=380.1.

One skilled in the art will appreciate that the following compounds can be synthesized by the methods disclosed above:
4- (*cis*-3-hydroxycyclopentyl) amino-5, 6-dimethyl- 2-phenyl -*7H-*pyrrolo[2,3d] pyrimidine MS (ES) (M⁻+1)= 323.1.
4-(*cis*-3-(2-aminoacetoxy)cyclopentyl)amino-5,6-dimethyl-2-phenyl-7H-pyrrolo[2,3d] pyrimidinetrifluoroacetic acid salt MS (ES) (M⁻+1)= 380.1.

### Ref Example 17

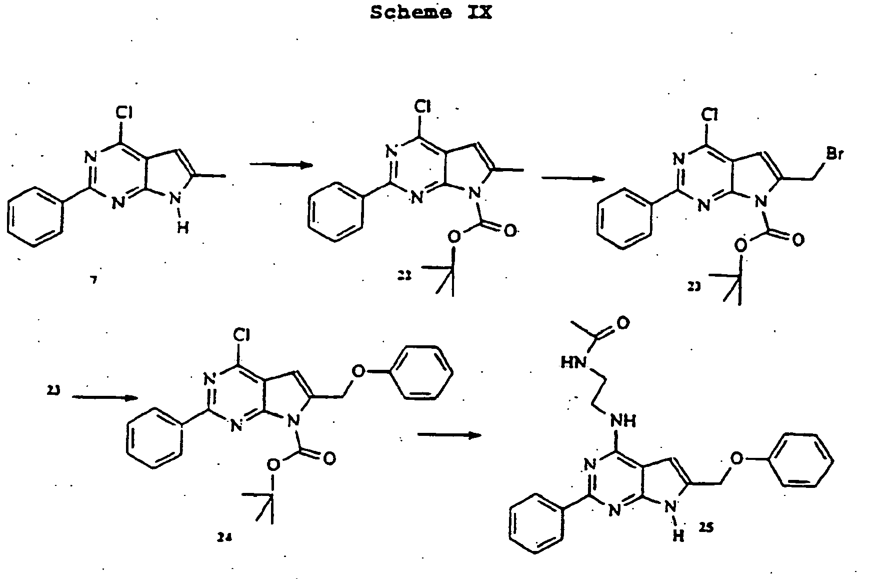

The pyrrole nitrogen of (7) (Scheme IX) was protected with di-*t*-butyldicarbonate under basic conditions to yield the corresponding carbamate (22). Radical bromination of (22) proceeded regioselectively to yield bromide (23). In general, compound (23) served as a key electrophilic intermediate for various nucleophilic coupling partners. Displacement of the alkyl bromide with sodium phenolate trihydrate yielded compound (24). Subsequent displacement of the aryl chloride and removal of the t-butyl carbamate protecting group occurred in one step yielding desired compound (25).

### Detailed Synthesis of Ref. Compounds (22)-(25) in Accordance with Scheme IX

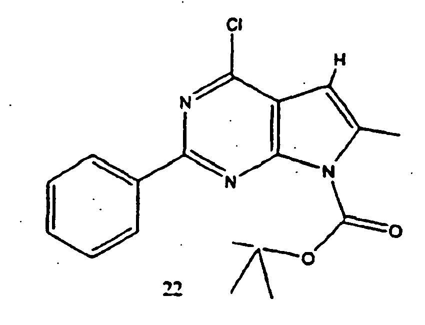

Di-*t*-butyl dicarbonate (5.37 g, 24.6 mmol) and dimethyl aminopyridine (1.13 g, 9.2 mmol) were added to a solution containing (7) (1.50 g, 6.15 mmol) and pyridine (30 mL). After 20 h the reaction was concentrated and the residue was partitioned between CH₂Cl₂ and water. The CH₂Cl₂ layer was separated, dried over MgSO₄, filtered and concentrated to yield a black solid. Flash chromatography (SiO₂; 1/9 EtOAc/Hexanes, *R*_{*f*} 0.40) yielded 1.70 g (80%) of a white solid (22). ¹H NMR (200 MHz, CDCl₃) δ_8.50 (m, 2H, Ar-H), 7.45 (m, 3H, Ar-H), 6.39 (s, 1H, pyrrole-H), 2.66 (s, 3H, pyrrole-*CH*₃). 1.76 (s, 9H, carbamate-*CH*_{*3*}); MS, M + 1 = 344.1; Mpt = 175-177°C.

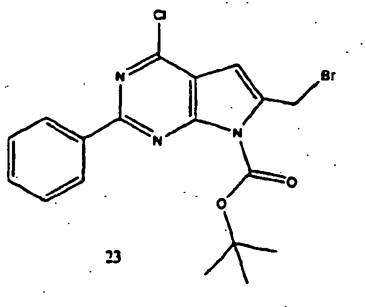

N-Bromosuccinimide (508 mg, 2.86 mmol) and AIBN (112 mg, 0.68 mmol) were added to a solution containing (22) (935 mg, 2.71 mmol) and CCl₄ (50 mL). The solution was heated to reflux. After 2 h the reaction was cooled to room temperature and concentrated *in vacuo* to yield a white solid. Flash chromatography (SiO₂; 1/1 CH₂Cl₂/Hexanes, *R*_{*f*} 0.30) yielded 960 mg (84%) of a white solid (23). ¹H NMR (200 MHz, CDCl₃) δ_8.52 (m, 2H, Ar-H), 7.48 (m, 3H, Ar-H), 6.76 (s, 1H, pyrole-H), 4.93 (s, 2H,pyrrole-*CH*₂Br), 1.79 (s, 9H, carbamate-*CH*₃) ; MS, M + 1 = 423.9; Mpt = 155-157°C.

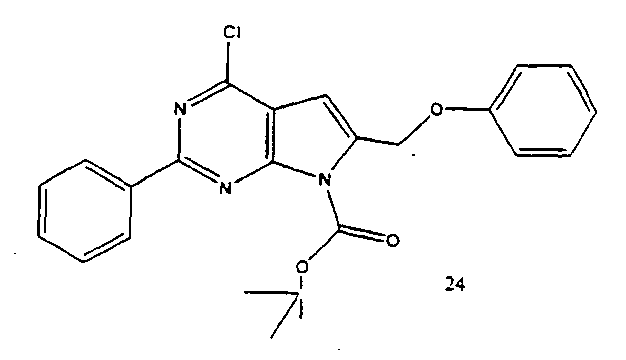

Sodium phenoxide trihydrate (173 mg, 1.02 mmol) was added in one portion to a solution of bromide (23) (410 mg, 0.97 mmol) dissolved in CH₂Cl₂ (5 mL) and DMF (10 mL). After 2 h the reaction solution was partitioned between CH₂Cl₂ and water. The water layer was extracted with CH₂Cl₂. The combined CH₂Cl₂ layers were washed with water, dried over MgSO₄, filtered and concentrated to yield a yellow solid. Flash chromatography (SiO₂; 1/6 EtOAc/Hexanes, R_{*f*} 0.30) yielded 210 mg (50%) of a white solid (24). ¹H NMR (200 MHz, CDCl₃) δ_8.53 (m, 2H, Ar-H), 7.48 (m, 3H, *Ar-H*), 7.34 (m, 2H, *Ar-H*), 7.03 (m, 3H, Ar-H), 6.83 (s, 1H, pyrrole-H), 5.45 (s, 2H, ArCH₂O), 1.76 (s, 9H, carbamate-*CH*_{*3*}); MS, M⁻ = 436.2.

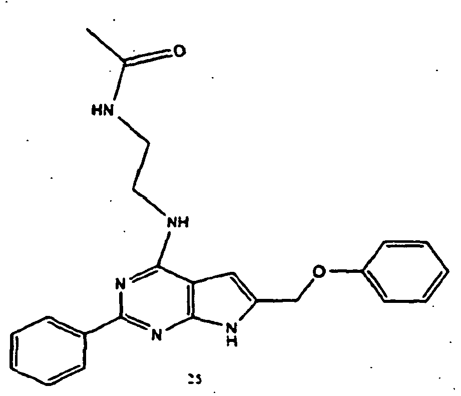

A solution containing (24) (85 mg, 0.20 mmol) N-acetylethylenediamine (201 mg, 1.95 mmol) and DMSO (3 mL) was heated to 100°C. After 1 h the temperature was raised to 130°C. After 3 h the reaction was cooled to room temperature and partitioned between EtOAc and water. The water layer was extracted with EtOAc (2x). The combined EtOAc layers are washed with water, dried over MgSO₄, filtered and concentrated. Flash chromatography (SiO₂; 1/10 EtOH/ CHCl₂, *R*_{*f*} 0.25) yielded 73 mg (93%) of a white foamy solid (25). ¹H NMR (200 MHz, DMSO-_{d6}) δ 11.81 (br s, 1H, N-H), 8.39 (m, 2H, Ar-H), 8.03 (br t, 1H, N-H), 7.57 (br t, 1H, N-H), 7.20 - 7.50 (m, 5H, Ar-H), 6.69 - 7.09 (m, 3H, Ar-H), 6.59 (s, 1H, pyrrole-H), 5.12 (s, 2H, *ArCH*_{*2*}O), 3.61 (m, 2H, NCH₂), 3.36 (m, 2H, NCH₂), 1.79 (s, 3H,COCH₂); MS, M+ 1 = 402.6

### Ref. Example 18: Synthesis of adenosine A₁ Antagonists.

Compound 1319 and Compound 1320 (Table 13 below) can be synthesized by the general procedures herein.

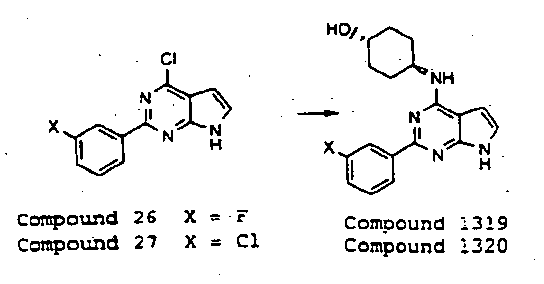

Compound 1319 (81%) ¹H-NMR (d₆-DMSO) d 1.37 (m, 4H), 1.93 (m, 2H), 2.01 (m, 2H), 4.11 (brs, 1H), 4.61 (d, 1H, J = 4.4 Hz), 6.59 (m, 1H), 7.09 (m, 1H), 7.21 (m, 2H), 7.49 (dd, 1H, J = 8Hz, 14Hz), 8.03 (m, 1H), 8.18 (d, 1H, J = 8 Hz), 11.55 (brs, 1H), MS (ES) : 327.0 (M⁻+1).

compound 1320 (31%) MS (ES) : 343.1 (M⁻+1).

### Ref. Example 19: Synthesis of adenosine A₁ Antagonist.

Compound 1321 (Table 13 below) can be synthesized by the general procedures given below.

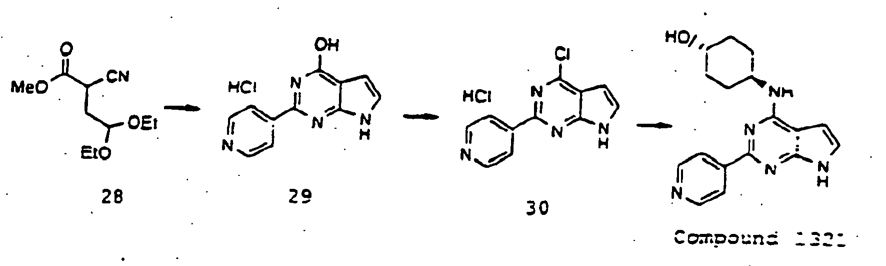

Compound 28 (10.93g, 50.76 mmol) was dissolved in DMF (67 mL). 4-Amidinopyridine hydrochloride (8.0g, 50.76 mmol) and DBU (15.4 g, 101.5 mmol) were added sequentially and the reaction was heated to 85°C. After 22 hours, the reaction was cooled to room temperature and the DMF was removed in vacuo. The dark oil was diluted with 2M HCl (80 mL). The reaction was allowed to stand. After 2 hours, the solution was cooled to 10°C and filtered. The solid was washed with cold water and dried to yield 7.40g of a yellow solid, Compound 29 (69%). ¹H-NMR (200MHz, d₆-DMSO) d 6.58 (s, 1H), 7.27 (s, 1H), 8.53 (d, 2H, J =5.6), 9.00 (d, 2H, J = 5.2Hz), 12.35 (brs, 1H). MS (ES) : 212.8 (M⁻+1).

Compound 29 (7.4 mmol, 29.8 mmol) was diluted with POCl₃ and heated to 105°C. After 18 hours, the reaction is cooled to room temperature and the POCl₃ is removed in vacuo. The chick dark oil is diluted with MeOH (75mL) followed by ether (120mL). The amorphous red solid is filtered and washed with ether to yield 3.82 g of a red solid. The crude solid is approximately 80% pure and used without further purification in the next reaction. MS (ES) : 230.7 (M⁻+1) .

Compound 1321 ¹H-NMR (15%) (200MH, d₆-DMSO) d 1.38 (m, 4H), 1.92 (brs, 2H), 2.02 (brs, 2H), 3.44 (brs, 1H), 4.14 (brs, 1H), 4.56 (d, 1H, J = 4 Hz), 6.63 (m. 1H), 7.15 (m, 1H). 7.32 (d, 1H, J = 6.2 Hz), 8.20 (d, 2H, J = 4.4 Hz) , 8.65 (d, 2H, J = 4.4Hz), 11.67 (brs, 1H) . MS (ES) : 310.2 (M⁻+1).

Compound 1501 (Table 15 below) ¹H-NMR (70%) (200MHz, CD₂OD) d 1.84 (s, 3H), 3.52 (t, 2H, J =6.0 Hz), 3.83, t, 2H, J = 6.0 Hz), 6.51 (d, 1H, J = 3.4Hz), 7.06 (d, 1H, J = 3.8 Hz), 7.42 (m, 3H), 8.36 (m, 2H). MS (ES): 296.0 (M⁻+1).

Compound 1502 (Table 15 below) MS (ES): 345.0 (M⁻+1).

Compound 1500 (Table 15 below) ¹H-NMR (200MHz, CDC1:) d 1.40 - 1.80 (m, 6H), 1.85 - 2.10 (m, 2H), 2.18 (s, 3H), 2.33 (s, 3H), 2.50 (d, 3H), 3.90 - 4.10 (m, 2H), 4.76 (m, 1H), 5.50 (d, 1H), 6.03 (m, 1H), 7.40 (m, 3H), 8.37 (m, 2H), 9.15 (brs, 1H) . MS (ES) : 393.3 3 (M'+1).

### Example 20: Synthesis of adenosine A₁ Antagonist.

Compound 1504 (Table 15 below) can be synthesized by the general procedures given below.

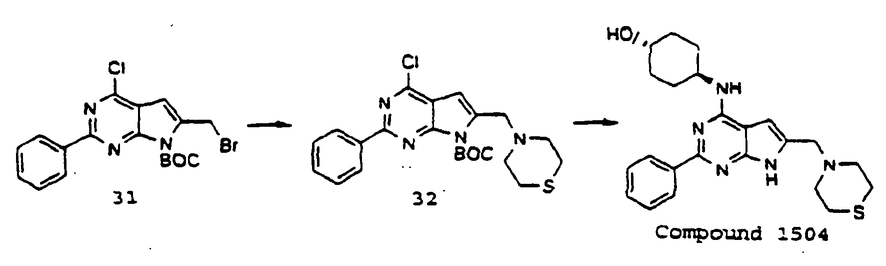

Compound 31 (200 mg, 0.47 mmol) was dissolved in DCM (4 mL). Triethylamine (51 mg, 0.5mmol) and thiomorpholine (52 mg, 0.5mmol) were added sequentially. The solution was mixed for several minutes and allowed to stand for 72 hours. The reaction was diluted with DCM and H₂O and the layers were separated. The aqueous layer was extracted with DCM. The combined DCM layers were dried over MgSO₄, filtered and concentrated. Ethyl ether was added to the crude sample and the resulting solid was filtered to yield 100mg of a white solid, 32 (62%) . ¹HNMR (200MHz, CDCl₃) d 1.76 (s, 9H), 2.66 (brs, 2H), 2.79 (brs, 2H), 3.86 (s, 2H), 7.46 (m, 3H), 8.50 (m, 2H).

Compound 32 was combined with DMSO (3mL) and trans-4-aminocyclohexanol (144mg, 1.25 mmol) and heated to 130°C for 4 hours. The reaction was cooled to room temperature, and diluted with EtoAc and H₂O. The layers were separated and the aqueous layer was extracted with EtOAc (2x). The combined organic layers were washed with H₂O and brine, dried over MgSO4, filtered and concentrated. Chromatography (silica, 8:1 CHCl3/EtOH) yields 32 mg of a tan oil. Ethyl ether was added and the resulting solid was filtered to yield 5 mg of a white solid (9%.).OSIC-148265: ¹H-NMR (200MHz, CD₂OD) : d 1.44 (brm, 4H), 2.03 (brm, 2H), 2.21 (brm, 2H), 2.70 (brm, 8H), 3.63 (m, 4H), 3.92 (m, 1H), 4.26 (brs, 1H), 6.42 (s, 1H), 7.42 (m, 3H), 8.33 (m, 2H).

### Example 21: Synthesis of adenosine A₁ Antagonist.

Compound 1503 (Table 15 below) can be synthesized by the general procedures given below.

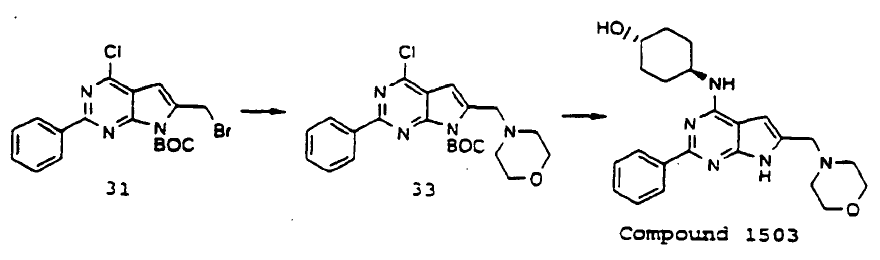

The bromide, compound 31 (220 mg, 0.47 mmol) was dissolved in 1:1 DMF: Dichloromethane (5 mL). To this was added K₂CO₃ (71 mg, 0.52 mmol) and morpholine (0.047 mL, 0.47 mmol). The mixture was allowed to stir at room temperature overnight. Solvents were removed in vacuo and the residue was partitioned between H₂O and dichloromethane. The organic layer was dried with MgSO₄, filtered, and concentrated to give an off white solid which upon trituration with ether/hexanes gave 175mg of a white solid, 33 (84%). ¹H-NMR (200MHz, CDCl₃) : ( 1.9 (9H, s) . 2.54 (4H, s) . 3.65 (4H, s), 3. 85 (1H, s), 6.59 (1H, s), 7.45 (3H, m),8.5 (2H, m).

Compound 33 (50 mg, 0.11 mmol) and trans-4-aminocyclohexanol (105 mg, 0.91 mmol) were taken up in - DMSO (2mL). The resultant solution was sparged with N₂ and then heated to 100°C in an oil bath and stirred overnight. The crude reaction mixture was poured into water and extracted twice with ethyl acetate (50mL). The combined organic layers were washed with H₂O. After drying with MgSO₄ and filtering, the organic layer was concentrated in vacuo to give an orange solid. Chromatography (silica, 10% CH₂OH in CH₂Cl₂) yielded 15mg (33%). ¹H-NMR (200 MHz, CDCl₃) : ( 1.24 - 1.62 (4H, m), 1.85 (2H, m), 2.10 (2H, m), 2.26 (4H, m), 3.53 (4H, m), 4.22 (1H, m), 4.73 (1H, m), 5.85 (1H, d), 6.15 (1H, s), 7.25 (3H, m), 8.42 (2H, M), 10.0 (1H, s). MS (ES) : 408 (M⁻ + 1).

Compounds 1500, 1501, and 1502 can be synthesized using similar preparation steps of Example 20 by treating compound 32 with an appropriately substituted amine.

**Yeast β-Galactosidase reporter gene assays for human adenosine A**_{**1**} **and A**_{**2**} **receptor:** Yeast strains (S. cerevisiae) were transformed with human adenosine A₁ (A₁R; CADUS strain CY12660) or human A₂ₐ (A₂ₐ; CADUS strain CY8362) and the addition of a lacZ(β-Galactosidase) reporter gene to utilize as a functional readout. A complete description of the transformations is listed below (see Yeast Strains). NECA (5'-N-ethylcarboxamidoadenosine), a potent adenosine receptor agonist with similar affinity for A₁ and A₂ₐ receptors, was used as a ligand for all assays. Test compounds were examined at 8 concentrations (0.1 - 10,000 nM) for ability to inhibit NECA-induced β-Galactosidase activity by CY12660 or CY8362.

Preparation of Yeast Stock Cultures: Each of the respective yeast strains, CY12660 and CY8362, were streaked onto an LT agar plate and incubated at 30°C until colonies were observed. Yeast from these colonies were added to LT liquid (pH 6.8) and grown overnight at 30°C. Each yeast strain was then diluted to an OD₆₀₀ = 1.0-2.0 (approximately 1-2 X 10⁶ cells/ml), as determined spectrophotometrically (Molecular Devices VMAX). For each 6 ml of yeast liquid culture, 4 ml of 40% glycerol (1:1.5 vol:vol) was added ("yeast/glycerol stock"). From this yeast/glycerol stock, ten 1 ml aliquots were prepared and stored at -80°C until required for assay.

Yeast A₁ R and A₂ₐR Assay: One vial each of CY8362 and CY12660 yeast/glycerol stock was thawed and used to inoculate Supplemented LT liquid media, pH 6.8 (92 ml LT liquid, to which is added: 5 ml of 40% glucose, 0.45 ml of 1M KOH and 2.5 ml of Pipes, pH 6.8). Liquid cultures were grown 16-18 hr (overnight) at 30°C. Aliquots from overnight cultures were then diluted in LT media, containing 4U/ml adenosine deaminase (Type VI or VII from calf intestinal mucosa, Sigma), to obtain OD₆₀₀ = 0.15 (1.5 X 10⁶ cells/ml) for CY8362 (A2aR) and OD₆₀₀ = 0.50 (5X10⁶ cells/ml) for CY12660 (A₁R).

Assays were conducted with a final volume of 100 ul in 96-well microtiter plates, such that a final concentration of 2% DMSO was achieved in all wells. For primary screening, 1-2 concentrations of test compounds were utilized (10 uM, 1µM). For compound profiling, 8 concentrations were tested (10000, 1000, 500, 100, 50, 10, 1 and 0.1 nM). To each microtiter plate, 10 ul of 20% DMSO was added to "Control" and "Total" wells while 10 ul of Test Compound (in 20% DMSO) was added to "Unknown" wells. Subsequently, 10 ul of NECA (5 uM for A₁R, 1 uM for A₂ₐR) were added to "Total" and "Unknown" wells; 10 ul of PBS was added to the "Control" wells. In the final addition, 80 ul of yeast strain, CY8362 or CY12660, were added to all wells. All plates were then agitated briefly (LabLine orbital shaker 2-3 min) and allowed to incubate for 4 hrs. at 30°C in a dry oven.

β-Galactosidase activity can be quantitated using either colorimetric (e.g., ONPG, CPRG), luminescent (e.g., Galacton-Star) or fluorometric substrates (e.g., FDG, Resorufin) substrates. Currently, fluorescence detection is preferred on the basis of superior signal:noise ratio, relative freedom from interference and low cost. Fluorescein digalactopyranoside (FDG, Molecular Probes or Marker Gene Technologies), a fluorescent β-Galactosidase substrate, was added to all wells at 20 ul/well (final concentration = 80 uM). Plates were shaken for 5-6 sec (LabLine orbital shaker) and then incubated at 37°C for 90 min (95% O₂/5% CO₂ incubator). At the end of the 90 min incubation period, β-Galactosidase activity was stopped using 20 ul/well of 1M Na₂CO₃ and all plates shaken for 5-6 sec. Plates were then agitated for 6 sec and relative fluorescence intensity determined using a fluorometer (Tecan Spectrafluor; excitation = 485 nm, emission = 535 nm).

Calculations: Relative fluorescence values for "Control" wells were interpreted as background and subtracted from "Total" and "Unknown" values. Compound profiles were analyzed via logarithmic transformation (x-axis: compound concentration) followed by one site competition curve fitting to calculate IC₅₀ values (GraphPad Prism).

Yeast strains: Saccharomyces cerevisiae strains CYl2660 [farl*1442 tbtl-l fusl-HIS3 canl ste14: :trpl: :LYS2 ste3*1156 gpal (41)-Gαi3 lys2 ura3 leu2 trp1: his3; LEU2 PGKp-Mfα1Leader-hA1R-PHO5term 2mu-orig REP3 Ampr] and CY8362 [gpa1p-rGαsE10K farl*1442 tbtl-l fusl-HIS3 can1 stel4::trpl: LYS2 ste3*1156 lys2 ura3 leu2 trpl his3; LEU2 PGKp-hA2aR 2mu-ori REP3 Ampr] were developed.

LT Media: LT (Leu-Trp supplemented) media is composed of 100g DIFCO yeast nitrogen base, supplemented with the following: 1.0g valine, 1.0g aspartic acid, 0.75g phenylalanine, 0.9g lysine, 0.45g tyrosine, 0.45g isoleucine, 0.3g methionine, 0.6g adenine, 0.4g uracil, 0.3g serine, 0.3g proline, 0.3g cysteine, 0.3g arginine, 0.9g histidine and 1.0g threonine.

### Construction of Yeast Strains Expressing Human A₁ Adenosine Receptor

In this example, the construction of yeast strains expressing a human A₁ adenosine receptor functionally integrated into the yeast pheromone system pathway is described.

### I. Expression Vector Construction

To construct a yeast expression vector for the human A₁ adenosine receptor, the A₁ adenosine receptor cDNA was obtained by reverse transcriptase PCR of human hippocampus mRNA using primers designed based on the published sequence of the human A₁ adenosine receptor and standard techniques. The PCR product was subcloned into the NcoI and XbaI sites of the yeast expression plasmid pMP15.

The pMP15 plasmid was created from pLPXt as follows: The XbaI site of YEP51 (Broach, J.R. et al. (1983) "Vectors for high-level, inducible expression of cloned genes in yeast" p. 83-117 in M. Inouye (ed.), Experimental Manipulation of Gene Expression. Academic Press, New York) was eliminated by digestion, end-fill and religation to create Yep51NccDXba. Another XbaI site was created at the BamHI site by digestion with BamHI, end-fill, linker (New England Biolabs, # 1081) ligation, XbaI digestion and re-ligation to generate YEP51NcoXt. This plasmid was digested with Esp31 and NccI and ligated to Leu2 and PGKp fragments generated by PCR. The 2 kb Leu2 PCR product was generated by amplification from YEP51Nco using primers containing Esp31 and BglII sites. The 660 base pair PGKp PCR product was generated by amplification from pPGKαs (Kang, Y.-S. et al. (1990) Mol. Cell. Biol. 10:2582-2590) with PCR primers containing BglII and NcoI sites. The resulting plasmid is called pLPXt. pLPXt was modified by inserting the coding region of the a-factor prepro leader into the NcoI site. The prepro leader was inserted so that the NcoI cloning site was maintained at the 3' end of the leader, but not regenerated at the 5' end. In this way receptors can be cloned by digestion of the plasmid with NcoI and XbaI. The resulting plasmid is called pMP15.

The pMP15 plasmid into which was inserted the human A₁ adenosine receptor cDNA was designated p5095. In this vector, the receptor cDNA is fused to the 3' end of the yeast a-factor prepro leader. During protein maturation the prepro peptide sequences are cleaved to generate mature full-length receptor. This occurs during processing of the receptor through the yeast secretory pathway. This plasmid is maintained by Leu selection (i.e., growth on medium lacking leucine). The sequence of the cloned coding region was determined and found to be equivalent to that in the published literature (GenBank accession numbers S45235 and S56143) .

### II. Yeast Strain Construction

To create a yeast strain expressing the human A₁ adenosine receptor, yeast strain CY7967 was used as the starting parental strain. The genotype of CY7967 is as follows:
MATα gpaD1163 gpal(41)Gαi3 far1D1442 tbt-1 FUS1-HIS3 can1 stel4::trpl::LYS2 ste3D1156 lys2 ura3 leu2 trpl his3

The genetic markers are reviewed below:

| | |
|---|---|
| MATa | Mating type a. |
| gpalDll63 | The endogenous yeast G-protein GPA1 has been deleted. |
| gpal (41) Gαi3 | gpa1 (41) -Gai3 was integrated into the yeast genome. This chimeric Ga protein is composed of the first 41 amino acids of the endogenous yeast Ga subunit GPA1 fused to the mammalian G-protein Gai3 in.which the cognate N-terminal amino acids have been deleted. |
| farlDl442 | FAR1 gene (responsible for cell cycle arrest) has been deleted (thereby preventing cell cycle arrest upon activation of the pheromone response pathway). |
| tbt-1 | strain with high transformation efficiency by electroporation. |
| FUS1-HIS3 | a fusion between the FUS1 promoter and the HIS3 coding region (thereby creating a pheromone inducible HIS3 gene). |
| can 1 | arginine/canavinine permease. |
| stel4::trpl::L | gene disruption of STE14, a C-farnesyl |
| YS2 | methyltransferase (thereby lowering basal signaling through the pheromone pathway). |
| ste3D1156 | endogenous yeast STR, the a factor pheromone receptor (STE3) was disrupted. |
| lys2 | defect in 2-aminoapidate reductase, yeast need lysine to grow. |
| ura3 | defect in orotidine-5'-phosphate decarboxylase, yeast need uracil to grow |
| leu2 | defect in b-isopropylmalate dehydrogenase, yeast need leucine to grow. |
| trp1 | defect in phosphoribosylanthranilate, yeast need tryptophan to grow. |
| his3 | defect in imidazoleglycerolphosphate dehydrogenase, yeast need histidine to grow. |

Two plasmids were transformed into strain CY7957 by electroporation: plasmid p5095 (encoding human A₁ adenosine receptor; described above) and plasmid p1584, which is a FUS1-β-galactosidase reporter gene plasmid. Plasmid p1584 was derived from plasmid pRS426 (Christianson, T.W. et al. (1992) Gene 110:119-1122). Plasmid pRS426 contains a polylinker site at nucleotides 2004-2016. A fusion between the FUS1 promoter and the β-galactosidase gene was inserted at the restriction sites EagI and XhoI to create plasmid p1584. The p1584 plasmid is maintained by Trp selection (i.e., growth on medium lacking leucine).

The resultant strain carrying p5095 and p1584, referred to as CY12660, expresses the human A₁ adenosine receptor. To grow this strain in liquid or on agar plates, minimal media lacking leucine and tryptophan was used. To perform a growth assay on plates (assaying FUS1-HIS3), the plates were at pH 6.8 and contained 0.5-2.5 mM 3-amino-1,2,4-triazole and lacked leucine, tryptophan and histidine. As a control for specificity, a comparison with one or more other yeast-based seven transmembrane receptor screens was included in all experiments.

### Construction of Yeast Strains Expressing Human A2a Adenosine Receptor

In this example, the construction of yeast strains expressing a human A2a adenosine receptor functionally integrated into the yeast pheromone system pathway is described.

### I. Expression Vector Construction

To construct a yeast expression vector for the human A2a adenosine receptor, the human A2a receptor cDNA was obtained from Dr. Phil Murphy (NIH). Upon receipt of this clone, the A2a receptor insert was sequenced and found to be identical to the published sequence (GenBank accession # S46950). The receptor cDNA was excised from the plasmid by PCR with VENT polymerase and cloned into the plasmid pLPBX, which drives receptor expression by a constitutive Phosphoglycerate Kinase (PGK) promoter in yeast. The sequence of the entire insert was once again sequenced and found to be identical with the published sequence. However, by virtue of the cloning strategy employed there were three amino acids appended to the carboxy-terminus of the receptor, GlySerVal.

### II. Yeast Strain Construction

To create a yeast strain expressing the human A2a adenosine receptor, yeast strain CY8342 was used as the starting parental strain. The genotype of CY8342 is as follows: MATa farlD1442 tbt1-1 lys2 ura3 leu2 trp1 his3 fus1-HIS3 can1 ste3D1156 gpaD1163 stel4::trpl::LYS2 gpa1p-rG_{αs}E10K (or gpalp-rG_{αs}D229S or gpalp-rG_{αs}E10K+D229S)

The genetic markers are as described in Example 1, except for the G-protein variation. For human A2a receptor-expression, yeast strains were utilized in which the endogenous yeast G protein GPAl had been deleted and replaced by a mammalian G_{αs}. Three rat G_{αs} mutants were utilized. These variants contain one or two point mutations which convert them into proteins which couple efficiently to yeast βγ. They are identified as G_{αS}E10K (in which the glutamic acid at position ten is replaced with lysine), GD_{αS}D229S (in which the aspartic acid at position 229 is replaced with serine) and G_{αS}E10K+D229S (which contains both point mutations).

Strain CY8342 (carrying one of the three mutant rat G_{αS} proteins) was transformed with either the parental vector pLPBX (Receptor⁻) or with pLPBX-A2a (Receptor⁻) . A plasmid with the FUS1 promoter fused to β-galactosidase coding sequences (described in above) was added to assess the magnitude of activation of the pheromone response pathway.

Functional Assay using Yeast Strains Expressing Human A₁ Adenosine Receptor In this example, the development of a functional screening assay in yeast for modulators of the human A₁ adenosine receptor is described.

### I. Ligands Used in Assay

Adenosine, a natural agonist for this receptor, as well as two other synthetic agonists were utilized for development of this assay. Adenosine, reported to have an EC₅₀ of approximately 75 nM, and (-)-N6-(2-phenylisopropyl)-adenosine (PIA) with a reported affinity of approximately 50 nM were used in a subset of experiments. 5'-N-ethylcarboxamidoadenosine (NECA) was used in all growth assays. To prevent signaling due to the presence of adenosine in the growth media, adenosine deaminase (4U/ml) was added to all assays.

### II. Biological Response in Yeast

The ability of the A₁ adenosine receptor to functionally couple in a heterologous yeast system was assessed by introducing the A₁ receptor expression vector (p5095, described above) into a series of yeast strains that expressed different G protein subunits. The majority of these transformants expressed G_{α} subunits of the G_{αi} or G_{αo} subtype. Additional G_{α} proteins were also tested for the possible identification of promiscuous receptor-Gα protein coupling. In various strains, a STE18 or a chimeric STE18-Gy2 construct was integrated into the genome cf the yeast. The yeast strains harbored a defective HIS3 gene and an integrated copy of FUS1-HIS3, thereby allowing for selection in selective media containing 3-amino-1,2,4-triazole (tested at 0.2, 0.5 and 1.0 mM) and lacking histidine. Transformants were isolated and monolayers were prepared on media containing 3-amino-1,2,4-triazole, 4 U/ml adenosine deaminase and lacking histidine. Five microliters of various concentrations of ligand (e.g., NECA at 0, 0.1, 1.0 and 10 mM) was applied. Growth was monitored for 2 days. Ligand-dependent growth responses were tested in this manner in the various yeast strains. The results are summarized in Table 1 below. The symbol (-) indicates that ligand-dependent receptor activation was not detected while (+) denotes ligand-dependent response. The term "LIRMA" indicates ligand independent receptor mediated activation.

**Table 3**

| Yeast strain | Gα subunit | Gγ subunit | Strain Variants | Result |
|---|---|---|---|---|
| | | | | |
| CY1316 | GPA₁ | STE18 | | |
| | GPA41-G_{αi1} | | | - |
| | GPA41-G_{αi2} | | | - |
| | GPA41-G_{αi3} | | | - |
| | GPA41-Gₐᵢ₂-G_{αOB} | | | LIRMA |
| | GPA41-G_{αSE10K} | | | - |
| | GPA41-G_{αSD22295} | | | - |
| | | | | |

| CY7967 | GPA41-G_{αi3}-integrated | STE18 | | +++ |
|---|---|---|---|---|
| | | | | |
| CY2120 | GPA₁ | STE18 | sst2Δ | |
| | GPA41-G_{αi1} | | | + |
| | GPA42-G_{αi2} | | | + |
| | GPA41-G_{αi3} | | | + |
| | GPA41-Gₐᵢ₂-G_{αOB} | | | LIRMA |
| | GPA41-G_{αSE10K} | | | - |
| | GPA41-G_{αSD2295} | | | - |
| | | | | |
| CY9438 | GPA₁ | STE18-Gγ2 | | - |
| | GPA41-G_{αi1} | | | + |
| | GPA41-G_{αi2} | | | + |
| | GPA41-G_{αi3} | | | + |
| | GPA41-Gₐᵢ₂-G_{αOB} | | | LIRMA |
| | GPA41-G_{αSE10K} | | | - |
| | GPA41-G_{αSD2295} | | | - |
| | | | | |
| CY10560 | GPA₁-intergrated | STE18-GV2 | sst2Δ | ++ |

As indicated in Table 3, the most robust signaling was found to occur in a yeast strain expressing the GPA₁(41)-G_{αi3} chimera.

### III. fus1-LacZ Assay

To characterize activation of the pheromone response pathway more fully, synthesis of β-galactosidase through fuslLacZ in response to agonist stimulation was measured. To perform the β-galactosidase assay, increasing concentrations of ligand were added to mid-log culture of human A₁ adenosine receptor expressed in a yeast strain co-expressing a Ste18-Gγ2 chimera and GPA₄₁-G_{αi3}. Transformants were isolated and grown overnight in the presence of histidine and 4 U/ml adenosine deaminase. After five hours of incubation with 4 U/ml adenosine deaminase and ligand, induction of β-galactosidase was measured using CPRG as the substrate for β-galactoside 5 x 10⁵ cells were used per assay.

The results obtained with NECA stimulation indicated that at a NECA concentration of 10⁻⁸ M approximately 2-fold stimulation of β-galactosidase activity was achieved. Moreover, a stimulation index of approximately 10-fold was observed at a NECA concentration of 10⁻⁵ M.

The utility of this assay was extended by validation of the activity of antagonists on this strain. Two known adenosine antagonist, XAC and DPCPX, were tested for their ability to compete against NECA (at 5 mM) for activity in the β-galactosidase assay. In these assays, β-galactosidase induction was measured using FDG as the substrate and 1.6 x 10⁵ cells per assay. The results indicated that both XAC and DPCPX served as potent antagonists of yeast-expressed A₁ adenosine receptor, with IC₅₀ values of 44 nM and 49 nM, respectively.

In order to determine if this inhibitory effect was specific to the A₁ subtype, a series of complementary experiments were performed with the yeast-based A₂ₐ receptor assay (described in Example 4). Results obtained with the A₂ₐ yeast-based assay indicated that XAC was a relatively effective A₂ₐ receptor antagonist, consistent with published reports. In contrast, DPCPX was relatively inert at this receptor, as expected from published reports.

### IV. Radioligand Binding

The A₁ adenosine receptor assay was further characterized by measurement of the receptor's radioligand binding parameters. Displacement binding of [³H]CPX by several adenosine receptor reference compounds. XAC, DPCPX, and CGS, was analyzed using membranes prepared from yeast expressing the human A adenosine receptor. The results with yeast membranes expressing the human A₁ adenosine receptor were compared to those from yeast membranes expressing the human A2a adenosine receptor or the human A3 receptor to examine the specificity of binding. To perform the assay, fifty mg of membranes were incubated with 0.4 nM [³H]CPX and increasing concentrations of adenosine receptor ligands. Incubation was in 50 mM Tris-HCl, pH 7.4, 1 mM EDTA, 10 mM MgCl₂, 0.25 % BSA and 2 U/ml adenosine deaminase in the presence of protease inhibitors for 60 minutes at room temperature. Binding was terminated by addition of ice-cold 50 mM Tris-HCl, pH 7.4 plus 10 mM MgCl₂, followed by rapid filtration over GF/B filters previously soaked with 0.5 % polyethyenimine, using a Packard 96-well harvester. Data were analyzed by nonlinear least square curve fitting procedure using Prism 2.01 software. The IC₅₀ values obtained in this experiment are summarized in Table 4, below:

**Table 4**

| | | IC₅₀ InMl | |
|---|---|---|---|
| Compound | hA1R | hA2aR | hA3R |
| XAC | 6.6 | 11.7 | 53.1 |
| DPCPX | 8.5 | 326.4 | 1307.0 |
| CGS-15943 | 13.1 | 15.8 | 55.5 |
| NECA | 215.5 | 294.9 | 34.9 |
| R-PIA | 67.6 | 678.1 | 23.6 |
| IB-MECA | 727.7 | 859.4 | 3.1 |
| Alloxozine | 1072.0 | 1934.0 | 8216.0 |

These data indicate that the reference compounds have affinities consistent with those reported in the literature. The data further indicate that the yeast-based assays are of sufficient sensitivity to discriminate receptor subtype specificity.

### Functional Assay using Yeast Strains Expressing Human Ala Adenosine Receptor

In this example, the development of a functional screening assay in yeast for modulators of the human A₁ adenosine receptor is described.

### I. Ligands Used in Assay

The natural ligand adenosine, as well as other thoroughly characterized and commercially available ligands were used for study of the human A2a receptor functionally expressed in yeast. Three ligands have been used in the establishment of this assay. They include:

| Ligand | Reported Kᵢ | Function |
|---|---|---|
| Adenosine | 500 nM | agonist |
| 5'-N-ethylcarboxamidoadenosine (NECA) | 10-15 nM | agonist |
| (-)-N6-(2-phenylisopropyl)-adenosine (PIA) | 100-125 nM | agonist |

To prevent signaling due to the presence of adenosine in the growth media, adenosine deaminase (4U/ml) was added to all assays.

### II. Biological Response in Yeast

A2a receptor agonists were tested for the capacity to stimulate the pheromone response pathway in yeast transformed with the A2a receptor expression plasmid and expressing either G_{αS}E10K, G_{αS}D229S or G_{αS}E10K+D229S. The ability of ligand to stimulate the pheromone response pathway in a receptor dependent manner was indicated by an alteration in the yeast phenotype. Receptor activation modified the phenotype from histidine auxotrophy to histidine prototrophy (activation of fusl-HIS3). Three independent transformants were isolated and grown overnight in the presence of histidine. Cells were washed to remove histidine and diluted to 2 x 10⁶ cells/ml. 5 *µ*l of each transformant was spotted onto nonselective media (including histidine) or selective media (1 mM AT) in the absence or presence of 4 U/ml adenosine deaminase. Plates were grown at 30 °C for 24 hours. In the presence of histidine both Receptor (R⁺) and Receptor⁻ (R⁻) strains were capable of growth. However, in the absence of histidine only R⁺ cells grew. Since no ligand had been added to these plates two explanations were possible for this result. One possible interpretation was that the receptor bearing yeast were at a growth advantage due to Ligand Independent Receptor Mediated Activation (LIRMA). Alternatively the yeast could have been synthesizing the ligand adenosine. To distinguish between these two possibilities, an enzyme which degrades the ligand, adenosine deaminase (ADA), was added to the growing yeast and plates. In the presence of adenosine deaminase R⁺ cells no longer grew in the absence of histidine, indicating that the yeast were indeed synthesizing ligand.

This interpretation was confirmed by an A2a growth assay in liquid. In this experiment R⁺ yeast (a G_{αs}E10K strain expressing the A2a receptor) were inoculated at three densities (1 x 10⁶ cell/ml; 3 x 10⁵ cells/ml; or 1 x 10⁵ cells/ml) in the presence or absence of adenosine deaminase (4 U/ml). The stringency of the assay was enhanced with increasing concentrations (0, 0.1, 0.2 or 0.4 mM)of 3-amino-1,2,4-triazole (AT), a competitive antagonist of imidazoleglycerol-P dehydratase, the protein product of the HIS3 gene. In the presence of adenosine deaminase and 3-amino-1,2,4-triazole yeast grew less vigorously. However in the absence of 3-amino-1,2,4-triazole, adenosine deaminase had little effect. Thus adenosine deaminase itself had no direct effect upon the pheromone response pathway.

An alternative approach to measuring growth and one that can be miniaturized for high throughput screening is an A2a receptor ligand spot assay. A G_{αs}E10K strain expressing the A2a receptor (A2aR+) or lacking the receptor (R-) was grown overnight in the presence of histidine and 4 U/ml adenosine deaminase. Cells were washed to remove histidine and diluted to 5 x 10⁶ cells/ml. 1 x 10 cells were spread once selective plates containing 4 U/ml adenosine deaminase and 0.5 or 1.0 mM 3-amino-1,2,4-triazole (AT) and allowed to cry for 1 hour. 5 *µl* of the following reagents were applied to the monolayer: 10 mM adenosine, 38.7 mM histidine, dimethylsulfoxide (DMSO), 10 mM PIA or 10 mM NECA. Cells were grown 24 hours at 30°C. The results showed that cells without receptor could only grow when histidine was added to the media. In contrast, R⁺ cells only grew in areas where the A2a receptor ligands PIA and NECA had been spotted. Since the plates contained adenosine deaminase, the lack of growth where adenosine had been spotted confirmed that adenosine deaminase was active.

### III. fusl LacZ Assay

To quantitate activation of the yeast mating pathway, synthesis of β-galactosidase through *fus1*LacZ was measured. Yeast strains expressing G_{αs}E10K, G_{αs}D229S or G_{αs}E10K+D229S were transformed with a plasmid encoding the human A2a receptor (R+) or with a plasmid lacking the receptor (R-). Transformants were isolated and grown overnight in the presence of histidine and 4 U/ml adenosine deaminase. 1 x 10⁷ cells were diluted to 1 x 10⁶ cells/ml and exposed to increasing concentrations of NECA for 4 hours, followed by determination of the β-galactosidase activity in the cells. The results demonstrated that essentially no β-galactosidase activity was detected in R- strains, whereas increasing amounts of β-galactosidase activity were detected in R+ strains expressing either G_{αs}E10K, G_{αs}D229S or G_{αs}E10K+D229S as the concentration of NECA increased, indicating a dose dependent increase in units of β-galactosidase detected in response to exposure to increased ligand concentration. This dose dependency was only observed in cells expressing the A2a receptor. Furthermore the most potent G_{αs} construct for the A2a receptor was G_{αs}E10K. The G_{αs}D229S construct was the second-most potent G_{αs}, construct for the A2a receptor, while the G_{αs}E10K+D229S construct was the least potent of the three G_{αs} constructs tested, although even the G_{αs}B10K-D2298 construct stimulated readily detectable amounts of 3-galactosidase activity.

For a further description of the assays identified, see U.S. Application Serial No. 09/088985, entitled "Functional Expression of Adenosine Receptors in Yeast", filed June 2, 1998 (Attorney Docket No. CPI-093), the entire contents of which are hereby incorporated herein by reference.

### Pharmacological Characterization of the Human Adenosine Receptor Subtypes

### Material and Methods

*Materials.* [³H]-DPCPX [Cyclopentyl-1,3-dipropylxantine, 8-[dipropyl-2,3-³H(N)] (120.0 Ci/mmol); [³H]-CGS 21680, [carboxyethyl-³H (N)] (30 Ci/mmol) and [¹²⁵I] -AB-MECA ([¹²⁵I]-4-Aminobenzyl-5'-N-Methylcarboxamideoadenosine) (2,200 Ci/mmol) were purchased from New England Nuclear (Boston, MA). XAC (Xantine amine congener); NECA (5'-N-Ethylcarboxamidoadenosine); and IB-MECA from Research Biochemicals International (RBI, Natick, MA). The Adenosine Deaminase and Complete protease inhibitor cocktail tablets were purchased from Boehringer Mannheim Corp. (Indianapolis, IN). Membranes from HEK-293 cells stably expressing the human Adenosine 2a [RB-HA2a]; Adenosine 2b [RB-HA2b] or Adenosine 3 [RB-HA3] receptor subtypes, respectively were purchased from Receptor Biology (Beltsville, MD). Cell culture reagents were from Life Technologies (Grand Island, NY) except for serum that was from Hyclone (Logan, UT).

*Yeast strains: Saccharomyces cerevisiae* strains CY12660 [farl*1442 tbtl-1 fus1-HIS3 can1 ste14::trp1::LYS2 ste3*1156 gpal (41)-Gαi3 lys2 ura3 leu2 trp1: his3; LEU2 PGKp-Mfα1Leader-hA1R-PHO5term 2mu-orig REP3 Ampr] and CY8362 [gpa1p-rGαsE10K far1*1442 tbt1-1 fusl-HIS3 can1 ste14::trp1: LYS2 ste3*1156 lys2 ura3 leu2 trp1 his3; LEU2 PGKp-hA2aR 2mu-ori REP3 Amprl were developed as described above.

*Yeast culture:* Transformed yeast were grown in Leu-Trp [LT] media (pH 5.4) supplemented with 2% glucose. For the preparation of membranes 250 ml of LT medium were inoculated with start titer of 1-2 x 10⁶ cells/ml from a 30 ml overnight culture and incubated at 30°C under permanent oxygenation by rotation. After 16 h growth the cells were harvested by centrifugation and membranes were prepared as described below.

*Mammalian Tissue Culture:* The HEK-293 cells stably expressed human Adenosine 2a receptor subtype (Cadus clone # 5) were grown in Dulbeco's minimal essential media (DMEM) supplemented with 10% fetal bovine serum and 1X penicillin/streptomycin under selective pressure using 500 mg/ml G418 antibiotic, at 37°C in a humidified 5% CO₂ atmosphere.

*Yeast Cell Membrane Preparations:* 250 ml cultures were harvested after overnight incubation by centrifugation at 2,000 x g in a Sorvall RT6000 centrifuge. Cells were washed in ice-cold water, centrifuged at 4°C and the pellet was resuspended in 10 ml ice-cold lysis buffer [5 mM Tris-HCl, pH 7.5 ; 5 mM EDTA; and 5 mM EGTA] supplemented with Protease inhibitor cocktail tablets (1 tablet per 25 ml buffer). Glass beads (17 g; Mesh 400-600; Sigma) were added to the suspension and the cells were broken by vigorous vortexing at 4°C for 5 min. The homogenate was diluted with additional 30 ml lysis buffer plus protease inhibitors and centrifuged at 3,000 x g for 5 min. Subsequently the membranes were peleted at 36,000 x g (Sorvall RC5B, type SS34 rotor) for 45 min. The resulting membrane pellet was resuspended in 5 ml membrane buffer [50 mM Tris-HCl, pH 7.5; 0.6 mM EDTA; and 5 mM MgCl₂] supplemented with Protease inhibitor cocktail tablets (1 tablet per 50 ml buffer) and stored at -80 °C for further experiments.

*Mammalian Cell Membrane Preparations:* HEK-293 cell membranes were prepared as described previously (*Duzic E et al.: J*. *Biol. Chem., 267, 9844-9851, 1992)* Briefly, cells were washed with PBS and harvested with a rubber policeman. Cells were pelted at 4°C 200 x g in a Sorvall RT6000 centrifuge. The pellet was resuspended in 5 ml/dish of lysis buffer at 4°C (5 mM Tris-HCl, pH 7.5; 5 mM EDTA; 5 mM EGTA; 0.1 mM Phenylmethylsulfonyl fluoride, 10 mg/ml pepstatin A; and 10 mg/ml aprotinin) and homogenized in a Dounce homogenizer. The cell lysate was then centrifuged at 36,000 x g (Sorvall RC5B, type SS34 rotor) for 45 min and the pellet resuspended in 5 ml membrane buffer [50 mM Tris-HCl, pH 7.5; 0.6 mM EDTA; 5 mM MgCl₂; 0.1 mM Phenylmethylsulfonyl fluoride, 10 mg/ml pepstatin A; and 10 mg/ml aprotinin) and stored at -80 °C for further experiments.

The Bio-Rad protein assay kits, based on the Bradford dye-binding procedure, (Bradford, M.: *Anal. Biochem.* 72:248 (1976)) were used to determine total protein concentration in yeast and mammalian membranes.

*Adenosine 1 receptor subtype saturation and competition radioligand binding:* Saturation and competition binding on membranes from yeast cell transformed with human A₁ receptor subtype were carried out using antagonist [³H] DPCPX as a radioactive ligand. Membranes was diluted in binding buffer (50 mM Tris-HCl, pH 7.4; containing 10 mM MgCl₂; 1.0 mM EDTA; 0.25% BSA; 2 U/ml adenosine deaminase and 1 protease inhibitor cocktail tablet/50 ml] at concentrations of 1.0 mg/ml.

In saturation binding membranes (50 *µ*g/well) were incubate with increasing concentrations of [³H] DPCPX (0.05 - 25 nM) in a final volume of 100 *µ*l of binding buffer at 25°C for 1 hr in the absence and presence of 10 *µ*M unlabeled XAC in a 96-well microtiter plate.

In competition binding membranes (50 *µ*g/well) were incubate with [³H] DPCPX (1.0 nM) in a final volume of 100 ml of binding buffer at 25°C for 1 hr in the absence and presence of 10 *µ*M unlabeled XAC or increasing concentrations of competing compounds in a 96-well microtiter plate.

*Adenosine 2a receptor subtype competition radicligand binding:* Competition binding on membranes from HEK293 cell stably expressing the human A2a receptor subtype were carried out using agonist [³H] CGS-21680 as a radioactive ligand. Membranes was diluted in binding buffer [50 mM Tris-HCl, pH 7.4; containing 10 mM MgCl₂; 1.0 mM EDTA; 0.25% BSA; 2 U/ml adenosine deaminase and 1 protease inhibitor cocktail tablet/50 ml] at concentrations of 0.2 mg/ml. Membranes (10 µg/well) were incubate with [³H] CGS-21680 (100 nM) in a final volume of 100 ml of binding buffer at 25°C for 1 hr in the absence and presence of 50 µM unlabeled NECA or increasing concentrations of competing compounds in a 96-well microtiter plate.

*Adenosine 3 receptor competition radioligand binding:* Competition binding on membranes from HEK293 cell stably expressing the human A3 receptor subtype were carried out using agonist [¹²⁵I] AB-MECA as a radioactive ligand. Membranes was diluted in binding buffer [50 mM Tris-HCl, pH 7.4; containing 10 mM MgCl₂; 1.0 mM EDTA; 0.25% BSA; 2 U/ml adenosine deaminase and 1 protease inhibitor cocktail tablet/50 ml] at concentrations of 0.2 mg/ml. Membranes (10 *µ*g/well) were incubate with [¹²⁵I] AB-MECA (0.75 nM) in a final volume of 100 *µ*l of binding buffer at 25°C for 1 hr in the absence and presence of 10 *µ*M unlabeled IB-MECA or increasing concentrations of competing compounds in a 96-well microtiter plate.

At the end of the incubation, the A₁, A₂ₐ and A₃ receptor subtypes radioligand binding assays was terminated by the addition of ice-cold 50 mM Tris-HCl (pH 7.4) buffer supplemented with 10 mM MgCl₂, followed by rapid filtration over glass fiber filters (96-well GF/B UniFilters, Packard) previously presoaked in 0.5% polyethylenimine in a Filtermate 196 cell harvester (Packard). The filter plates were dried coated with 50 *µ*l /well scintillation fluid (MicroScint-20, Packard) and counted in a TopCount (Packard). Assays were performed in triplicate. Non-specific binding was 5.6 : 0.5%, 10.8 ± 1.4% and 15.1 ± 2.6% of the total binding in a A1R. A2aR and A3R binding assay, respectively.

*Adenosine 2b receptor subtype competition radioligand* binding: Competition binding on membranes from HEK293 cell stably expressing the human A2b receptor subtype were carried out using A₁ receptor antagonist [³H] DPCPX as a radioactive ligand. Membranes was diluted in binding buffer [10 mM Hepes-KOH, pH 7.4; containing 1.0 mM EDTA; 0.1 mM Benzamidine and 2 U/ml adenosine deaminase] at concentrations of 0.3 mg/ml. Membranes (15 µg/well) were incubate with [³H] DPCPX (15 nM) in a final volume of 100 µl of binding buffer at 25°C for 1 hr in the absence and presence of 10 µM unlabeled XAC or increasing concentrations of competing compounds in a 96-well microtiter plate. At the end of the incubation, the assay was terminated by the addition of ice-cold 10 mM Hepes-KOH (pH 7.4) buffer followed by rapid filtration over glass fiber filters (96-well GF/C UniFilters, Packard) previously presoaked in 0.5% polyethylenimine in a Filtermate 196 cell harvester (Packard). The filter plates were dried coated with 50 *µ*l/well scintillation fluid (MicroScint-20, Packard) and counted in a TopCount (Packard). Assays were performed in triplicate. Non-specific binding was 14.3 ± 2.3% of the total binding.

Specific binding of [³H] DPCPX; [³H] CGS-21680 and [¹²⁵I] AB-MECA was defined as the difference between the total binding and non-specific binding. Percent inhibition of the compounds was calculated against total binding. Competition data were analyzed by iterative curve fitting to a one site model, and K_{I} values were calculated from IC₅₀ values (Cheng and Prusof, Biochem. Pharmacol. 22, 3099-3109, 1973) using the GraphPad *Prizm 2.01* software.

### Results

A primary function of certain cell surface receptors is to recognize appropriate ligands. Accordingly, we determined ligand binding affinities to establish the functional integrity of the Adenosine 1 receptor subtype expressed in yeast. Crude membranes prepared from *Saccharomyces cerevisiae* transformed with human Adenosine 1 receptor subtype construct exhibited specific saturable binding of [³H] DPCPX with a K_{D} of 4.0 ± 0.19 nM. The K_{D} and Bₘₐₓ value were calculated from the saturation isotherm and Scatchard transformation of the data indicated a single class of binding sites. The densities of adenosine binding sites in the yeast membrane preparations were estimated to 716.8 ± 43.4 fmol/mg membrane protein.

The pharmacological subtype characteristics of the recombinant yeast cells transformed with human A₁ receptor subtype were investigated with subtype selective adenosine ligands (XAC, DPCPX; CGS-15943; Compound 600; Compound 1002; NECA, (R)-PIA; IB-MECA and Alloxazine) that competed with [³H] DPCPX in the expected rank order. Displacement curves recorded with these compounds show the typical steepness with all the ligands, and the data for each of the ligands could be modeled by a one-site fit. The apparent dissociation constants estimated for the individual compound from the curves (Table 5) are consistent with value published for the receptor obtained from other sources.

### Table 5

Ki values for membranes from yeast cells transformed with human A₁ receptor subtype

| Ligands | K_{I}(nM) |
|---|---|
| XAC | 5.5 |
| DPCPX | 7.1 |
| CGS-1594 | 10.8 |
| NECA | 179.6 |
| (R)-PIA | 56.3 |
| IB-MECA | 606.5 |
| Alloxazine | 894.1 |
| | |
| Compound 600 | 13.9 |
| Compound 1002 | 9.8 |

Tables 6 through 12 demonstrate the efficacy and structure activity profiles of deazapurines of the invention. Tables 13 and 14 demonstrate selectivity can be achieved for human adenosine receptor sites by modulation of the functionality about the deazapurine structure. Table 14 also demonstrates the surprising discovery that the compounds set forth therein have subnanomolar activity and higher selectivity for the A_{2b} receptor as compared to the compounds in Table 13.

**TABLE 6**

| Effect of N₆-Substituent | | | |
|---|---|---|---|
| 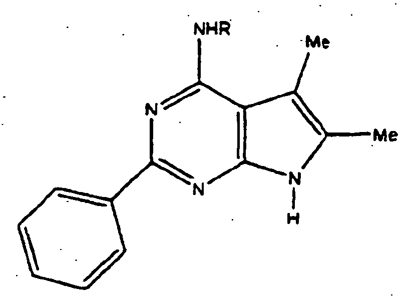 | | | |

| | | Al | |
|---|---|---|---|
| Compound | R | Binding Ki (nM) | Yeast IC50 (nM) |
| 600 Ref. | 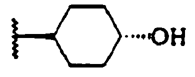 | 13.9 | 97.2 |
| 601 Ref. | 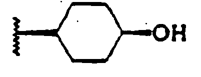 | 1423 | >10.000 |
| 602 Ref. | 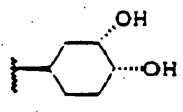 | 483.5 | >10.000 |
| 603 Ref. | 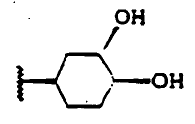 | 196.6 | 4442.0 |
| 604 Ref. | 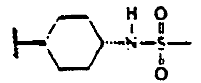 | >10.000 | >10000 |
| 605 Ref. | 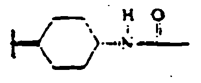 | >10000 | >10000 |
| 606 Ref. | 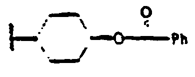 | 297.9 | >10000 |
| 607 Ref. | 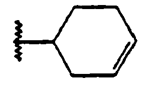 | 309.7 | > 10000 |
| 608 | 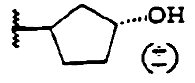 | 29.1 | |
| 609 | 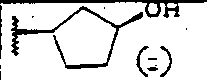 | 193.9 | |
| 610 Ref. | 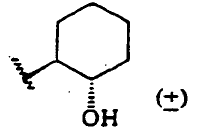 | 411.5 | |
| Ref. 611 | 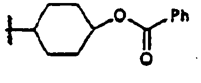 | 785.6 | >10000 |
| 612 Ref. | 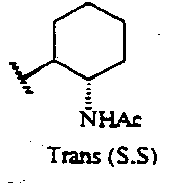 | 64.8 | |
| 613 Ref. | 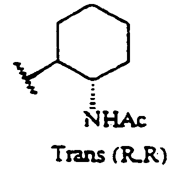 | 6726.0 | |
| 614 Ref. | 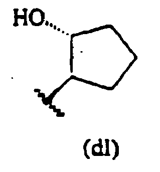 | 32.1 | |
| 615 Ref. | 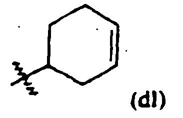 | 816.9 | 2577.0 |
| 616 Ref. | 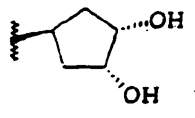 | 34.3 | |

**TABLE 7**

| Effect of C₂-Substituent | | | |
|---|---|---|---|
| 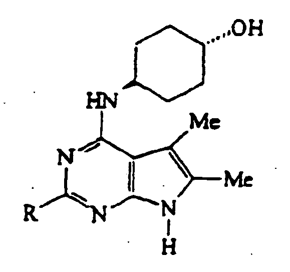 | | | |

| | | Al | |
|---|---|---|---|
| Ref. Compounds | R | Binding Ki (nM) | Yeast IC50 (nM) |
| 700 | 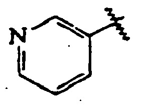 | 604.5 | >10000 |
| 701 | 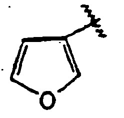 | 157.7 | 763.1 |
| 702 | 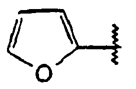 | 198.5 | 2782.5 |
| 703 | 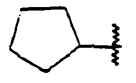 | 443.6 | >10000 |
| 704 | 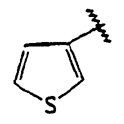 | 61.1 | 297.0 |
| 705 | 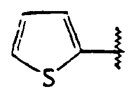 | 30.1 | 194.7 |
| 706 | 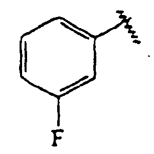 | 19.9 | |
| 707 | 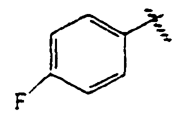 | 62.8 | |
| 703 | 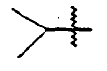 | 2145 | |
| 709 | 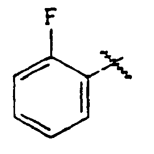 | 48.7 | |

**TABLE 8**

| Effect of Pyrrole Ring Substituent | | | | | | |
|---|---|---|---|---|---|---|
| 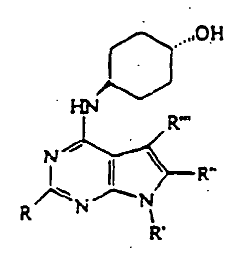 | | | | | | |

| | | | | | Al | |
|---|---|---|---|---|---|---|
| Reference Compounds | R | R' | R" | R"' | Binding Ki (nM) | Yeast IC50 (nM) |
| 800 | 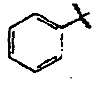 | Me | Me | Me | 3311 | > 10000 |
| 801 | 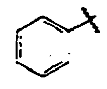 | H | Me | H | 22.3 | 148.3 |
| 802 | 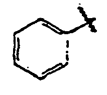 | H | H | Me | 8.9 | |
| 803 | 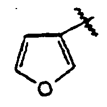 | 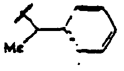 | Me | Me | 2210 | >10000 |
| 804 | 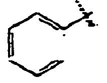 | 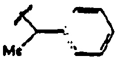 | Me | Me | 863.1 | |
| 805 | 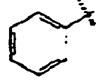 | 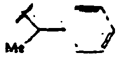 | Me | Me | 4512 | |
| 806 | 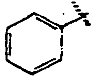 | 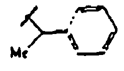 | Me | Me | 8451 | |
| 807 | 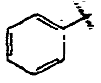 | 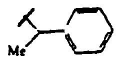 | Me | Me | 35.3 | |

**TABLE 9**

| | | | |
|---|---|---|---|
| 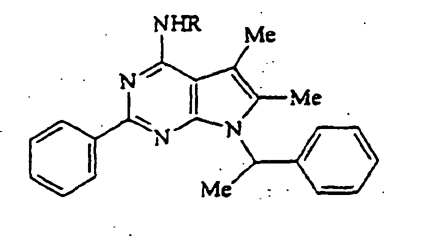 | | | |

| | | Al | |
|---|---|---|---|
| Reference Compounds | R | Binding Ki (nM) | Yeast IC50 (nM) |
| 900 | 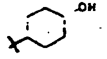 | 863.1 | |
| 901 | 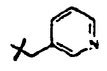 | 4512 | |
| 902 | 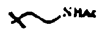 | 8451 | |
| 903 | 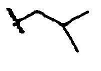 | 35.3 | |

**TABLE 10**

| Effect of N₆-Substituent | | | |
|---|---|---|---|
| 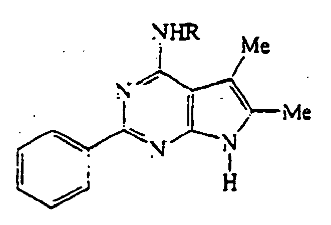 | | | |

| | | Al | |
|---|---|---|---|
| Reference Compounds | R | Binding Ki (nM) | Yeast IC50 (nM) |
| 1000 | 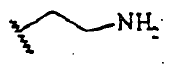 | 1789 | >10000 |
| 1001 | 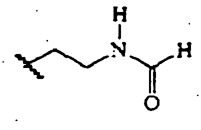 | 54.4 | 1865 |
| 1002 | 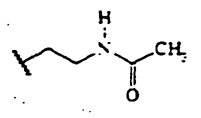 | 9.8 | 82.8 |
| 1003 | 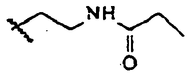 | 26.7 | 195.7 |
| 1004 | 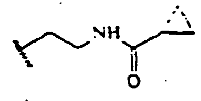 | 32.8 | 545.8 |
| 1005 | 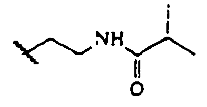 | 147.5 | 3972 |
| 1006 | 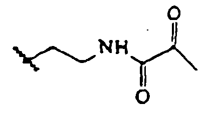 | 151.7 | 2918 |
| 1007 | 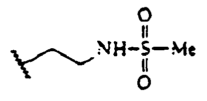 | 692.5 | >10000 |
| 1008 | 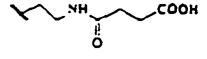 | 93.1 | 3217 |
| 1009 | 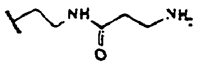 | 475.3 | >10000 |
| 1010 | 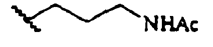 | 674.9 | 9376.0 |
| 1011 | 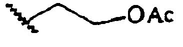 | 121.9 | 2067.5 |
| 1012 | 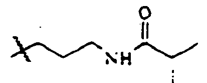 | 233.9 | 3462 |
| 1013 | 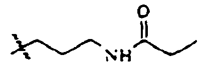 | 270.1 | 3009.5 |
| 1014 | 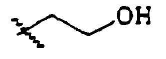 | 384.9 | 2005 |
| 1015 | 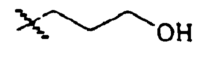 | 179.3 | 3712 |
| 1016 | 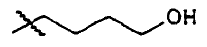 | 176.1 | 5054 |

**TABLE 11**

| Effect of N₆-Substituen: | | | |
|---|---|---|---|
| 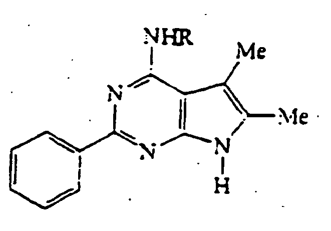 | | | |

| | | Al | |
|---|---|---|---|
| Compound | R | Binding Ki (nM) | Yeast IC50 (nM) |
| 1100 Ref. | 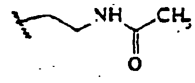 | 9.8 | 115.4 |
| 1101 Ref. | 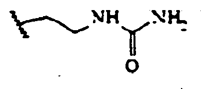 | 53.9 | 551.0 |
| 1102 | 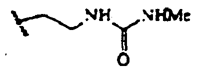 | 10.3 | 101.3 |
| 1103 Ref. | 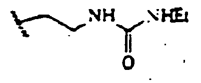 | 71.1 | 3217 |
| 1104 Ref. | 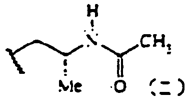 | 6.5 | 58.7 |
| 1105 Ref. | 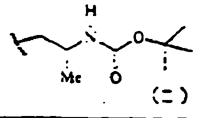 | 105.4 | 472.1 |
| 1106 Ref. | 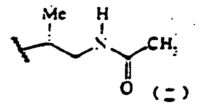 | 27.9 | 162.4 |
| 1107 Ref. | 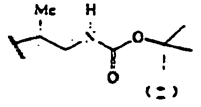 | 126.5 | 1297.0 |
| 1108 Ref: | 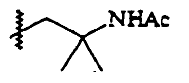 | 92.3 | |
| 1109 Ref. | 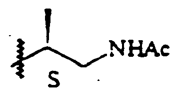 | 9.0 | |
| 1110 Ref. | 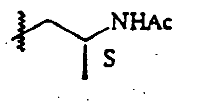 | 17.3 | |
| 1111 Ref. | 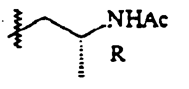 | 2.5 | |
| 1112 Ref. | 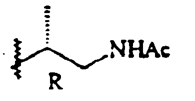 | 213 | |

**TABLE 12**

| "Retro-Amide" Analogues | | | |
|---|---|---|---|
| 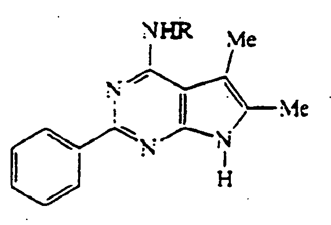 | | | |

| | | Al | |
|---|---|---|---|
| Reference Compounds | R | Binding Ki (nM) | Yeast IC50 (nM) |
| 1200. | 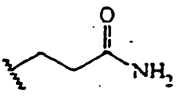 | 16.5 | 189.4 |
| 1201 | 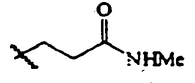 | 7.4 | 45.7 |
| 1202 | 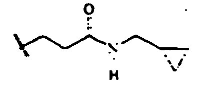 | 95.8 | 3345.0 |
| 1203 | 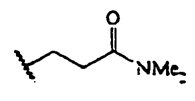 | 529.1 | 4040.0 |
| 1204 | 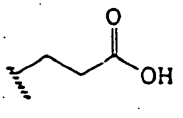 | 1060.0 | > 10000 |
| 1205 | 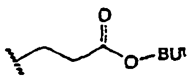 | 1272 | >10000 I |
| 1206 | 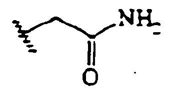 | 50.8 | 4028 |
| 1207 | 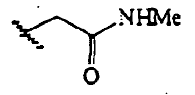 | 48.5 | 701.5 |

**TABLE 13**

| **Profile of Selective Adenosine Antagonists** | | | | | |
|---|---|---|---|---|---|
| 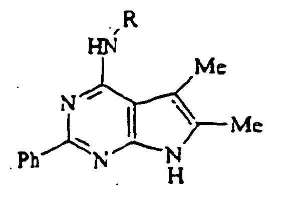 | | Binding Ki (nM) | | | |
| Compound | R | A1 | A2a | A2b | A3 |
| 1300 Ref. | 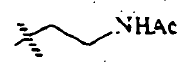 | 9.8 25.1 | 18.0 48.6 | 80.3 | 513.0 |
| 1301 Ref. | 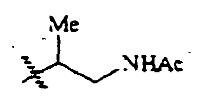 | 27.8 | 50.7 | 84.6 | 429.8 |
| 1302 | 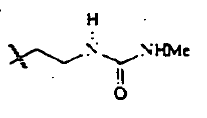 | 20.2 | 75.6 | 20.1 | 4.3 |
| 1303 Ref. | 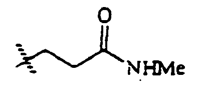 | 17.4 | 111.3 | 120.6 | 44.6 |
| 1304 Ref. | 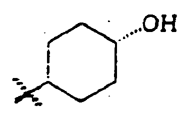 | 13.9 30.9 | 933.7 | 138.0 | 21.5 |
| 1305 Ref. | 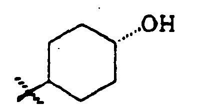 | 46.6 | 730.9 | 30% | 9.9 |
| 1306¹ Ref. | 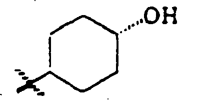 | 16.4 | 766.3 | 168.3 | 71.7 |
| 1307 | 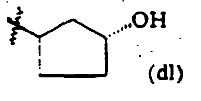 | 39.1 | 190.6 | 1143.0 | 3.1 |
| 1308 | 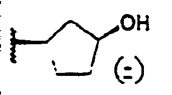 | 180 | 230 | 670 | 1.0 |
| 1309 Ref. | 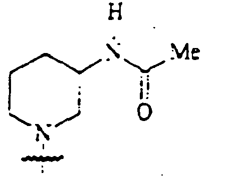 | 40 | 109 | 109 | 0.3 |
| 1310 Ref. | 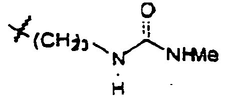 | 255 | 76% | 275 | ≤2.6 |
| 1311 Ref. | 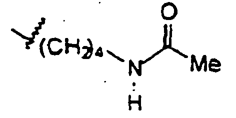 | 531 | 981 | 736 | 5.3 |
| 1312 Ref. | 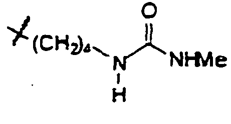 | 443 | 2965 | 375 | ≤6.2 |
| 1313 Ref. | 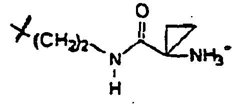 | 30% | 65% | 515 | 24 |
| 1314 Ref. | 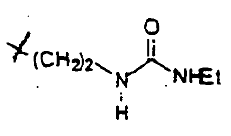 | 87 | 204 | 30 | 0.02 |
| 1315 Ref. | 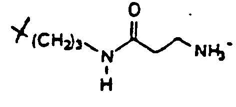 | 75.000 | 720.000 | 3.400 | 507 |
| 1316 Ref. | 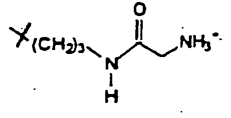 | 333 | 710.000 | 710.000 | 97 |
| 1317 Ref. | 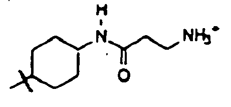 | 710.000 | 710.000 | 720.000 | 369 |
| 1318 Ref. | 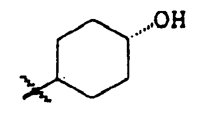 | 3.7±0.5 | 630± 56.4 | 2307± 926 | 630±76 |
| 1319 Ref. | 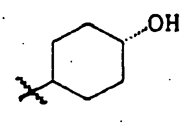 | 1.8 | 206 | 802 | 270 |
| 1320 Ref. | 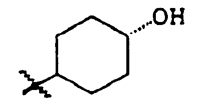 | 8.0 | 531 | 530 | 419 |
| 1321 Ref. | 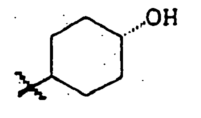 | 8.0 | 131 | 1031 | 54%⁸ |

| | | | | | |
|---|---|---|---|---|---|
| ¹2-thienyl-2-yl;²C₅-H; ³water soluble: ⁴R₅ and R₆ are hydrogen: ⁵R₃ is 3-fluorophenyl: | | | | | |
| ⁶R₃ is 3-chlorophenyl; ⁷R₃ is 4-pyridyl;⁸ % activity @ 10 µM | | | | | |

**Table 14:**

| Profile of Selective A_{2b} Antagonists | | | | | | |
|---|---|---|---|---|---|---|
| 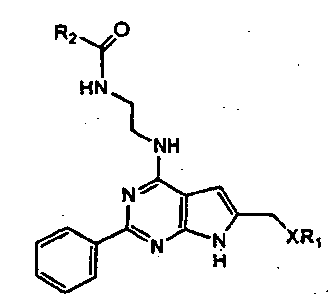 | | | | | | |

| Reference Compounds | XR₁ | R₂ | Binding Data K₁ (nM) | | | |
|---|---|---|---|---|---|---|
| | | | A₁ | A₂ₐ | A_{2B} | A₃ |
| 1400 | -O-Ph | Me | 41.7 | 21 | 10.3 | 14.6 |
| 1401 | -O-Ph (p) F | Me | 33 | 58 | 8.8 | 18 |
| 1402 | -O-Ph(p)Cl | Me | 825 | 591 | 22 | 60 |
| 1403 | -N-pyridin-2-one | Me | 60 | 41 | 18 | 48 |
| 1404 | -NH-Ph | Me | 49 | 31 | 4.6 | 57 |

**TABLE 15. Adenosine A₁ Receptor Selective Compounds * at least 10 times more selective than other three subtypes.**

| Compound | Structure | Ki-A₁ | Relative Ki -A₂ₐ | Relative Ki- A_{2b} | Relative Ki- A₃ |
|---|---|---|---|---|---|
| 706 Ref. | 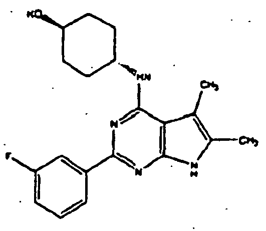 | * | | | |
| 1318 Ref. | 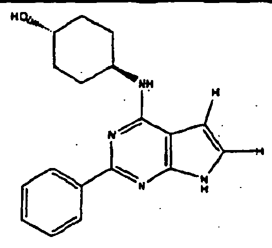 | * | | | |
| 1319 . Ref. | 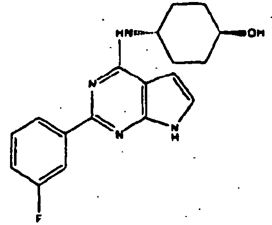 | ***** | | | |
| 1320 Ref. | 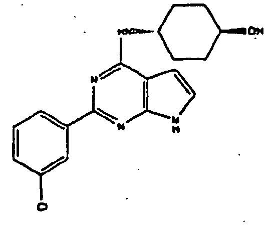 | * | | | |
| 1500 | 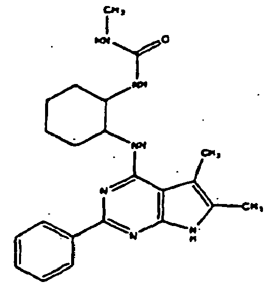 | * | | | |
| 1321 Ref. | 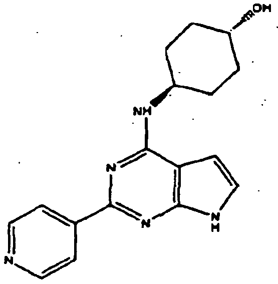 | * | | | |
| 1501 | 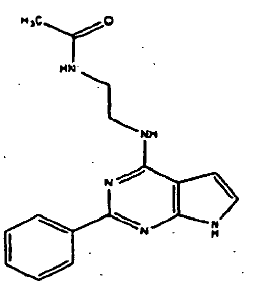 | * | | | |
| 1502 | 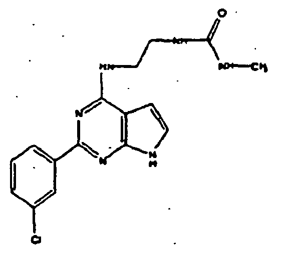 | * | | | |
| 1503 | 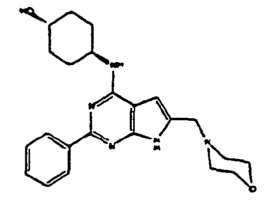 | * | | | |
| 1504 | 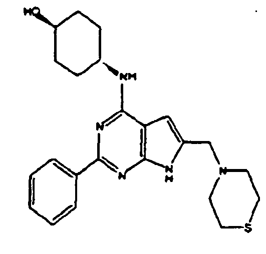 | * | | | |

The present invention is also based on compounds which selectively bind to adenosine A₂ₐ receptor, thereby treating a disease associated with A₂ₐ adenosine receptor in a subject by administering to the subject a therapeutically effective amount of such compounds. The disease to be treated are associated with, for example, a central nervous system disorder, a cardiovascular disorder, a renal disorder, an inflammatory disorder, a gastrointestinal disorder, an eye disorder, an allergic disorder or a respiratory disorder.

This invention also features a compound having the structure: 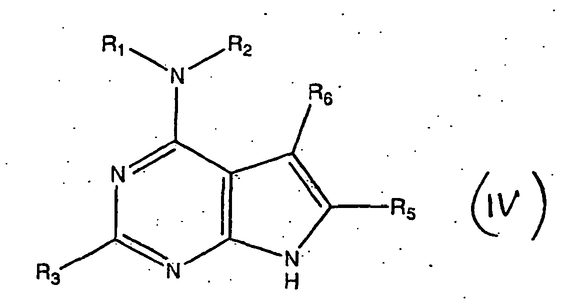 wherein NR₁R₂ is *(D)*-2-aminocarbonyl N-pyrrolidinyl, *(D)-*2-hydroxymethyl N-pyrrolidinyl, *(D)*-2-hydroxymethyl-*trans*-4-hydroxy N-pyrrolidinyl, or N-piperazinyl;
wherein R₃ is as defined above;
wherein R₅ is H, phenyl or 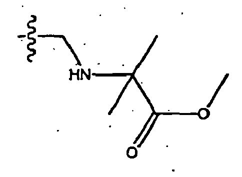 wherein R₆ is H, alkyl or cycloalkyl; and
a compound of the above structure,
wherein NR₁R₂ is 3-hydroxymethyl N-piperidinyl;
wherein R₃ is a 4-pyridyl;
wherein R₅ is H or alkyl; and
wherein R₆ is H or alkyl

This invention also features a method for inhibiting the activity of an A₂ₐ adenosine receptor in a cell in vitro, which comprises contacting said cell with the above-mentioned compounds.

In another embodiment of the compound, the compound has the structure:

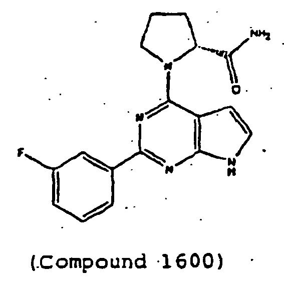

In another embodiment of the compound, the compound has the structure:

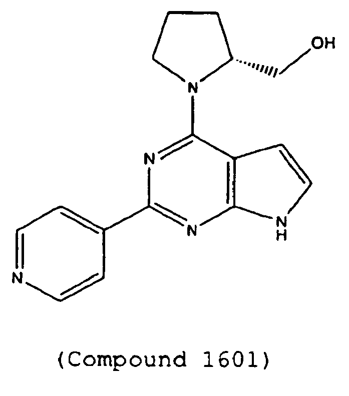

In another embodiment of the compound, the compound has the structure:

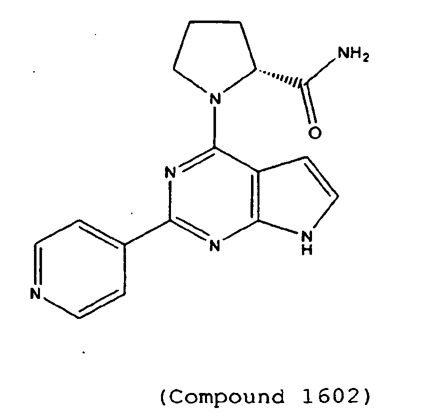

In another embodiment of the compound, the compound has the structure:

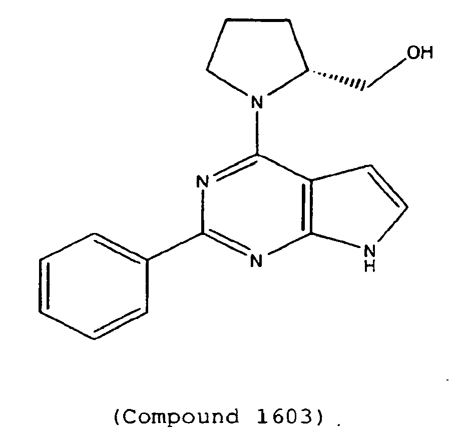

In another embodiment of the compound, the compound has the structure:

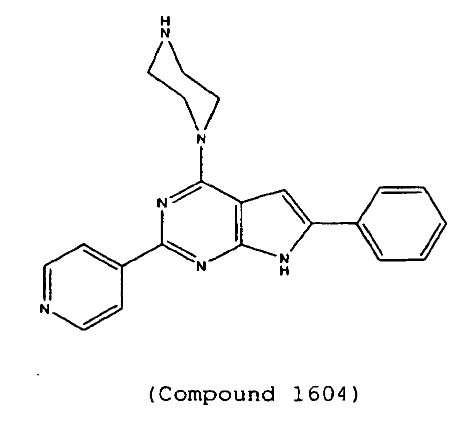

In another embodiment of the compound, the compound has the structure:

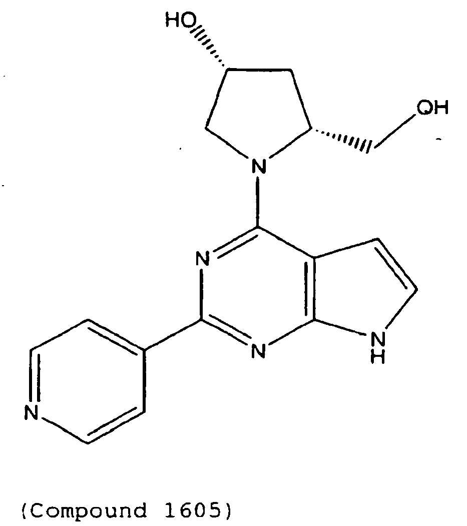

In another embodiment of the compound, the compound has the structure:

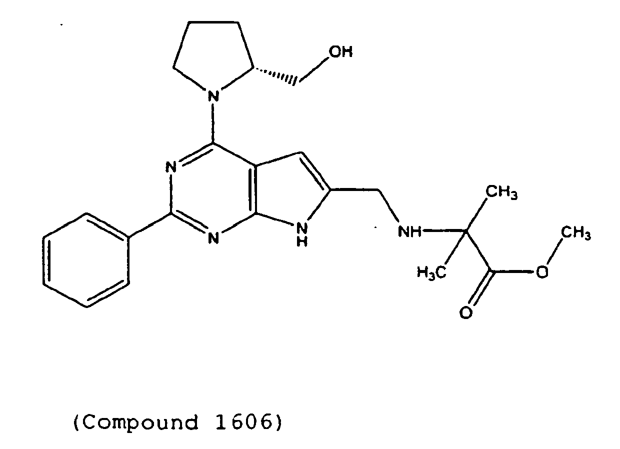

In another embodiment of the compound, the compound has the structure:

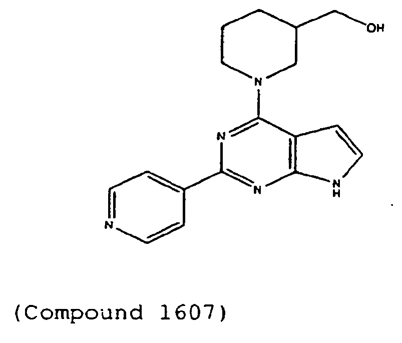

In yet another embodiment of the compound, the compound has the structure:

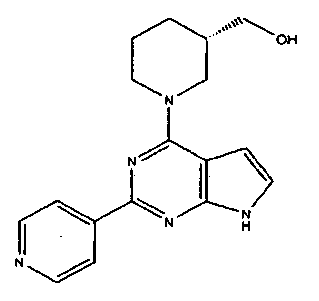

In a further embodiment of the compound, the compound has the structure:

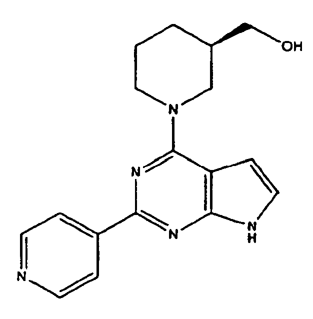

This invention further provides a compound having the structure (V) : 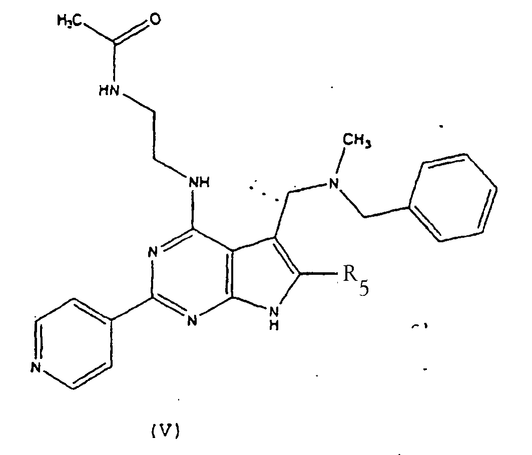 wherein R₅ is H, or methyl.

In one embodiment of the compound V, the compound has the structure:

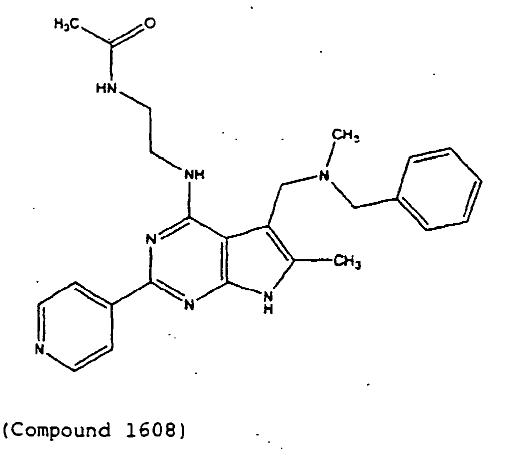

In another embodiment of the compound V, the compound has the structure:

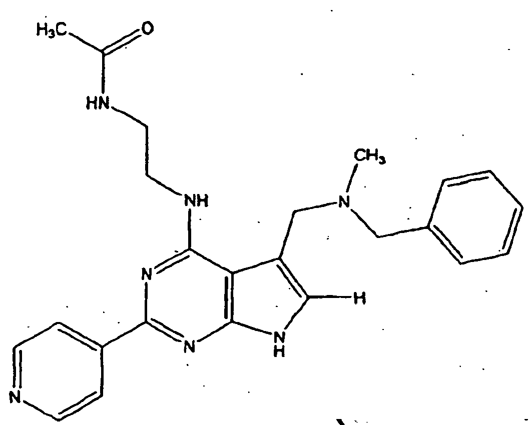

This invention also relates to the use of a therapeutically effective amount of compounds IV, or V for the manufacture of a medicament for treating a disease associated with A2a adenosine receptor in a subject.

In one embodiment, the compound treats said diseases by stimulating adenylate cyclase.

In another embodiment, the subject is a mammal.

In another embodiment, the mammal is a human.

In another embodiment of the method, said A2a adenosine receptor is associated with Parkinson's disease and diseases associated with locomotor activity, vasodilation, platelet inhibition, neutrophil superoxide generation, cognitive disorder, or senile dementia.

Diseases associated with adenosine A1, A2a, A2b and A3 receptors are disclosed in WO 99/06053 and WO-09822465, WO-09705138, WO-09511681, WO-09733879, JP-09291089, PCT/US98/16053 and U.S. Patent No. 5,516,894, the entire content of which are fully incorporate herein by reference.

This invention also provides a water-soluble prodrug of compounds IV, or V; wherein said water-soluble prodrug that is metabolized *in vivo* to produce an active drug which selectively inhibit A2a adenosine receptor.

In one embodiment of the prodrug, said prodrug is metabolized *in vivo* by esterase catalyzed hydrolysis.

This invention also provides a pharmaceutical composition comprising the prodrug and a pharmaceutically acceptable carrier.

This invention also provides a method for inhibiting the activity of an A2a adenosine receptor in a cell in vitro, which comprises contacting said cell with compounds IV, or V.

In one embodiment of the method, the compound is an antagonist of said A2a adenosine receptor.

In another embodiment of the pharmaceutical composition, said pharmaceutical composition is an ophthalmic formulation.

In another embodiment of the pharmaceutical composition, said pharmaceutical composition is an periocular, retrobulbar or intraocular injection formulation.

In another embodiment of the pharmaceutical composition, said pharmaceutical composition is a systemic formulation.

This invention also relates to the use of an effective amount of compounds IV, or V for the manufacture of a medicament for treating a gastrointestinal disorder in an subject.

In one embodiment, said disorder is diarrhea.

In another embodiment, the subject is a human.

In another embodiment, the compound is an antagonist of A2a adenosine receptors.

This invention further relates to the use of an effective amount of compounds IV, or V for the manufacture of a medicament for treating respiratory disorder in a subject.

In one embodiment, said disorder is asthma, chronic obstructive pulmonary disease, allergic rhinitis, or an upper respiratory disorder.

In another embodiment, the subject is a human.

In another embodiment, said compound is an antagonist of A2a adenosine receptors.

This invention also relates to the use of an effective amount of compounds IV, or V for the manufacture of a medicament for treating damage to the eye of a subject.

In one embodiment, said damage comprises retinal or optic nerve head damage.

In another embodiment, said damage is acute or chronic.

In another embodiment, said damage is the result of glaucoma, edema, ischemia, hypoxia or trauma.

In another embodiment, the subject is a human.

In another embodiment, the compound is an antagonist of A2a adenosine receptors.

This invention also provide a pharmaceutical composition comprising a therapeutically effective amount of compounds IV, or V and a pharmaceutically acceptable carrier.

In one embodiment of the pharmaceutical composition, said therapeutically effective amount is effective to treat Parkinson's disease and diseases associated with locomotor activity, vasodilation, platelet inhibition, neutrophil superoxide generation, cognitive disorder, or senile dementia.

In another embodiment of the pharmaceutical composition, said pharmaceutical composition is an ophthalmic formulation.

In another embodiment of the pharmaceutical composition, said pharmaceutical composition is an periocular, retrobulbar or intraocular injection formulation.

In another embodiment of the pharmaceutical composition, said pharmaceutical composition is a systemic formulation.

In another embodiment of the pharmaceutical composition, said pharmaceutical composition is a surgical irrigating solution.

This invention also provides a combination therapy for Parkinson's disease comprising compounds IV and V, and any of the dopamine enhancers.

This invention further provides a combinational therapy, for cancer comprising compounds IV and V, and any of the cytotoxic agents.

This invention further provides a combinational therapy for glaucoma, comprising compounds IV or V, and a prostaglandin agonist, a muscrinic agonist, or a β-2 antagonist.

This invention also provides a packaged pharmaceutical composition for treating a disease associated with A2a adenosine receptor in a subject, comprising: (a) a container holding a therapeutically effective amount of compounds IV, or V; and (b) instructions for using said compound for treating said disease in a subject.

This invention also provide a method of preparing compound IV, comprising the steps of
a) reacting 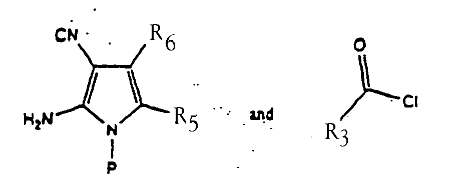 to provide 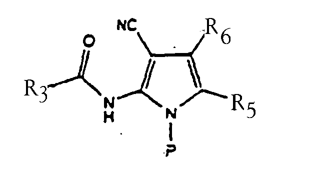 wherein P is a removable protecting group;
b) treating the product of step a) under cyetization conditions to provide 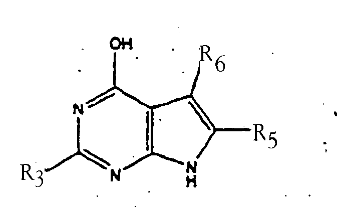
c) treating the product of step b) under suitable conditions to provide 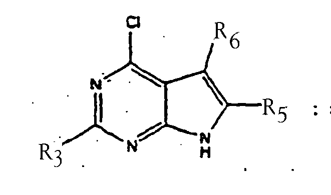 and
d) treating the chlorinated product of step c) with NHR₁R₂ to provide 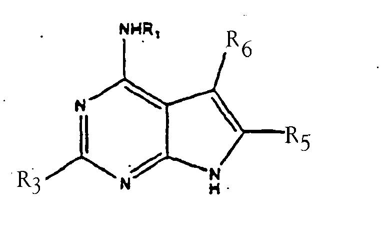 wherein R₁, R₂, R₃, R₅ and R₆ are as defined above.

This invention further provides a method of preparing compound V, comprising the steps of
a) reacting 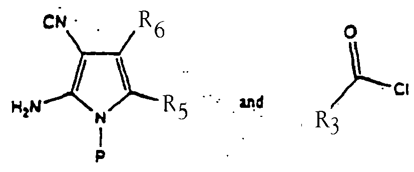 to provide 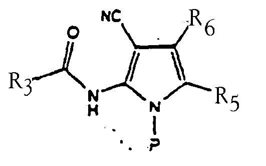 wherein P is a removable protecting group;
b) treating the product of step a) under cyclization conditions to provide 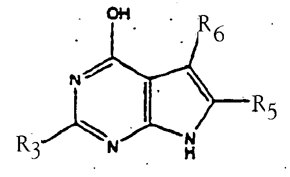
c) treating the product of step b) under suitable conditions to provide 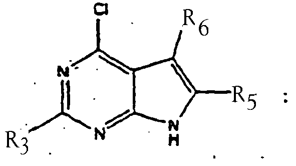 and
d) treating the chlorinated product of step c) first with dimethylamine and formaldehyde, then with N-methyl benzylamine and finally with NH₂R₁ to provide 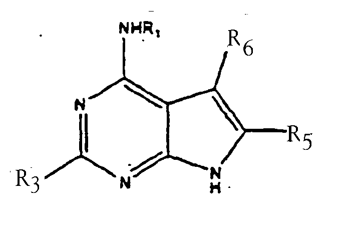 wherein R₁ is acetamido ethyl; wherein R₃ is 4-pyridyl; wherein R₅ is H, or methyl; wherein R₆ is N-methyl-N-benzyl aminomethyl.

As used herein, "A compound is A₂ₐ selective." means that a compound has a binding constant to adenosine A₂ₐ receptor of at least five time higher then that to adenosine A₁, A_{2b}, or A₃.

The invention is further illustrated by the following examples which in no way should be construed as being further limiting. The contents of all references, pending patent applications and published patent applications, cited throughout this application, including those referenced in the background section, are hereby incorporated by reference. It should be understood that the models used throughout the examples are accepted models and that the demonstration of efficacy in these models is predictive of efficacy in humans.

This invention will be better understood from the Experimental Details which follow. However, one skilled in the art will readily appreciate that the specific methods and results discussed are merely illustrative of the invention as described more fully in the claims which follow thereafter.

### Example 22: Synthesis of Adenosine A₂ₐ Antagonists, compounds 1601, 1602, and 1603.

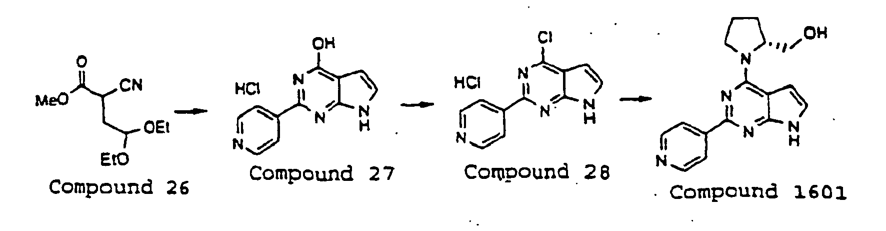

Compound 26 (10.93g, 50.76 mmol) was dissolved in DMF (67 mL). 4-Amidinopyridine hydrochloride (8.0g, 50.76 mmol) and DBU (15.4 g, 101.5 mmol) were added sequentially and the reaction was heated to 85°C. After 22 hours, the reaction was cooled to room temperature and the DMF was removed in vacuo. The dark oil was diluted with 2M HCl (80 mL). The reaction was allowed to stand. After 2 hours, the solution was cooled to 10°C and filtered. The solid was washed with cold water and dried to yield 7.40g of a yellow solid, compound 27 (69%). ¹H-NMR (200MHz, d₆-DMSO) d 6.58 (s, 1H), 7.27 (s, 1H), 8.53 (d, 2H, J = 5.6), 9.00 (d, 2H, J = 5.2Hz), 12.35 (brs, 1H). MS (ES): 212.8 (M⁻+1).

Compound 27 (7.4 mmol, 29.8 mmol) was diluted with POCl₃ and heated to 105°C. After 18 hours, the reaction is cooled to room temperature and the POCl₃ is removed in vacuo. The thick dark oil is diluted with MeOH (75mL) followed by ether (120mL) . The amorphous red solid is filtered and washed with ether to yield 3.82 g of a red solid. The crude solid, compound 28, is approximately 80% pure and used without further purification in the next reaction. ¹H-NMR (200MHz, d₆-DMSO) d 6.58 (s, 1H), 7.27 (s, 1H), 8.53 (d, 2H, J = 5.6), 9.00 (d, 2H, J = 5.2Hz), 12.35 (brs, 1H) . MS (ES) : 212.8 (M⁺+1) .

**Compound 1601:** DMSO (5 mL) and D-prolinol (500mg, 4.94 mmol) were added to compound 28 (500mg, 2.17 mmol) was added. The reaction was heated to 120°C. After 18 hours, The reaction was cooled to room temperature and diluted with EtOAc and H₂O. The layers were separated and the aqueous layer was extracted with EtOAc (2x). The combined organic layers were washed with H₂O (2x), brine, dried over MgSO₄, filtered and concentrated to yield 200mg of a tan solid. The solid was recrystallized from EtOAc to yield 82 mg of a tan solid (13%). ¹H-NMR (200 MHz, d₆-DMSO) d 2.05 (m, 4H), 3.43 (m, 1H), 3.70 - 4.00 (m, 3H), 9.50 (brs, 1H), 4.92 (brs, 1H), 6.62 (m, 1H), 7.22 (m, 1H), 8.22 (d, 2H, J = 6.0 Hz), 6.69 (d, 2H, J = 6.2 Hz), MS (ES): 296.0 (M⁺+1), mp = 210 - 220°C (decomp.).

**Compound 1602**: Chromatography (silica, 9:1 CHCl3/MeOH) yielded 10 mg of a tan solid (2%) ¹H-NMR (d₆-DMSO) d 2.00 - 2.50 (m, 4H), 4.05 (m, 1H), 4.21 (m, 1H), 6.71 (d, 1H, J = 3.2 Hz), 7.18 (d, 1H, J = 3.2 Hz), 8.37 (d, 2H, J = 4.8 Hz), 8.56 (d, 2H, J = 5 .0 Hz). MS (ES): 309.1 (M⁺+1).

**Compound 1603.** Chromatography (silica, 20:1 Hexanes /EtOAc) yielded 135 mg of a tan solid (53%). ¹H-NMR (d₆-DMSO) d 2.00 (m, 4H), 3.43 (brs, 1H), 3.74 (brs, 2H), 3.87 (brs, 1H), 4.49 (brs, 1H), 4.93 (m, 1H), 6.56 (m, 1H), 7.12 (m, 1H), 7.40 (m, 3H), 8.34 (m, 2H), 11.62 (brs, 1H). MS (ES): 295.1 (M⁺+1).

**Compound 1605.** Into a 50mL RBF 60mg of 2-(4'-pyridyl)-4-Chloropyrimidinopyrrole HCl salt was dissolved in 2mL anhydrous DMSO. 3-(R)-Hydroy-(D)-prolinol TFA salt (380mg) and 500mg sodium bicarbonate were added thereto. The mixture was then flashed with nitrogen gas for 5min and heated to 130°C. After 2 hours, the reaction was cooled to room temperature and the DMSO was removed in vacuo. The residue was partitioned between EtOAc (15mL) and saturated sodium bicarbonate aqueous solution (15mL). The organic layer was separated and washed with brine (15mL) and dried over Na₂SO₄. After removal of solvent, the crude product was purified by preparative TLC (CH₂Cl₂/MeOH = 95/5) to yield 35 mg (50%). ¹H-NMR (200MHz, CDCl₃) (2.3-2.5 (1H), 3.4-3.8 (3H), 4.4-4.6 (2H), 6.4 (1H); 7.1 (1H); 8.2 (d, 2H); 8.7 (d, 2H) ; 11.0 (1H) . MS (ES) : 312 (M⁺+1) .

### Example 23: Synthesis of Adenosine A₂ₐ Antagonist, compound 1606.

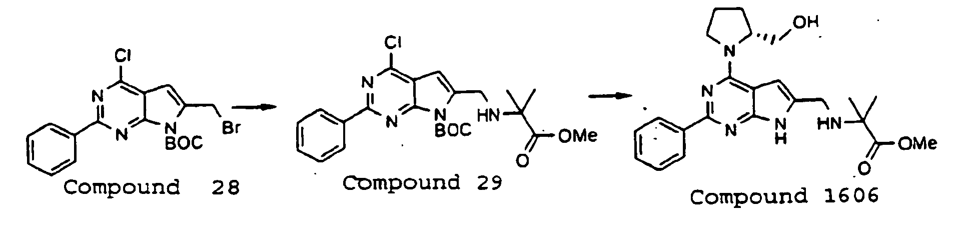

Compound 28 (200mg) was treated with DMF (30mL), (, (-dimethylglycine methyl ester (73mg HCl salt in 2mL water) and 500mg sodium bicarbonate. After 18 hours, the DMF was removed in vacuo. The residue was partitioned between EtOAc (30mL) and saturated sodium bicarbonate aqueous solution (15mL). The organic layer was washed with brine (15mL), dried over sodium sulfate, filtered and concentrated. Chromatography (silica, 10:4 hexanes/EtOAc) yielded 150mg of pure product, compound 29 (69%) . ¹H-NMR (200MHz, CDCl₃), ( 1.4 (s, 6H), 3.8 (s, 3H) ; 3.9 (s, 2H); 6.4 (s, 1H); 7.4-7.5 (m, 3H); 8.4 (m, 2H); 9.8 (s, 1H).

### Compound 1606:

Procedure is the same as Compound 1605 (72%). ¹H-NMR (200MHz, CDCl₃), (1.3 (s, 6H), 1.7-1.9 (m, 2H); 2.05-2.30 (m, 2H); 3.6-4.1 (m, 11H); 4.80-4.95 (m, 1H); 6.4 (s, 1H); 7.4-7.6 (m, 3H); 8.3-8.4 (d, J = 8.5 Hz, 2H), 10 (s, 1H). MS (ES): 424.0 (M⁺+1).

The following compounds can be synthesized in the same manner. **Compound 1600**: (51%). MS (ES): 326.0 (M⁺+1).

**Compound 1607:** ¹H-NMR (200MHz, CDCl₃), ( 1.40 - 1.80 (m, 5H), 2.80 - 3.50 (m, 3H), 4.60 - 4.80 (m, 3H), 6.66 (d, 1H, J = 6.2Hz), 7.26 (m, 1H), 8.21 (d, 2H, J = 6.3Hz), 8.65 (d, 2H, J = 5.8Hz), 11.90 (s, 1H) . MS (ES) : 310.1 (M⁺+1) .

**Compound 1608:** (64%). ¹H-NMR (200MHz, d₆-DMSO), (1.75 (s, 3H), 2.11 (s, 3H), 2.29 (s, 3H), 3.56 (m, 6H), 7.23 - 7.41 (m, 5H), 8.00 (brs, 1H), 8.23 (d, 2H, J = 6.0Hz), 8.63 (d, 2H, J = 5.4 Hz), 8.82 (brs, 1H), 11.56 (brs, 1H). MS (ES) : 444.0 (M⁻+1) .

**Compound 1604:** ¹H-NMR (200MHz, CD₃OD) ( 3.40 (m, 4H), 4.29 (m, 4H), 6.99 (s, 1H), 7.5 - 7.2 (m, 3H), 7.90 (d, 2H), 8.39 (d, 2H), 8.61 (d, 2H). MS (ES) : 357.0 (M⁺ +1) .

**TABLE 16. Adenosine A₂ₐ Receptor Selective Compounds**

| Compound | Structure | Relative Ki-Al | Ki-A2a | Relative Ki-A2b | Relative Ki-A3 |
|---|---|---|---|---|---|
| 1600 | 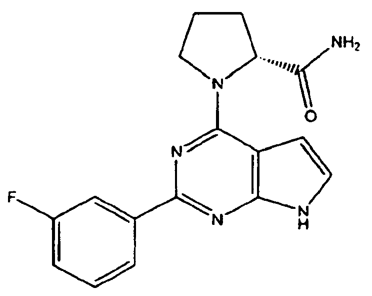 | | * | | |
| 1601 | 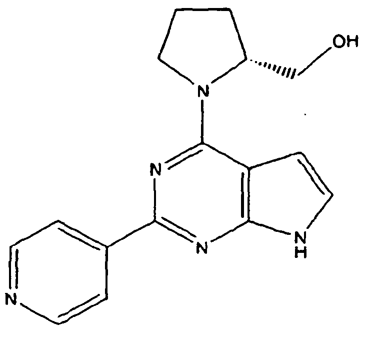 | | * | | |
| 1602 | 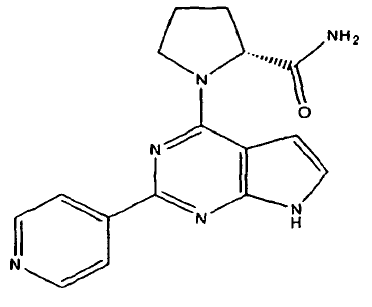 | | * | | |
| 1603 | 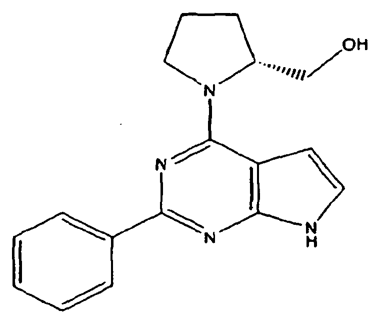 | | * | | |
| 1604 | 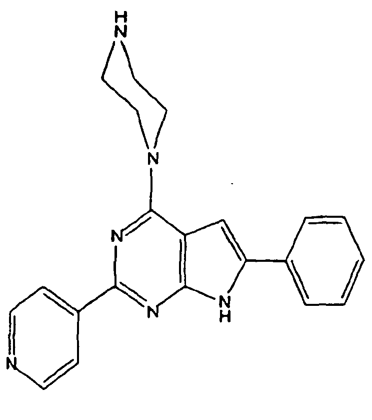 | | * | | |
| 1605 | 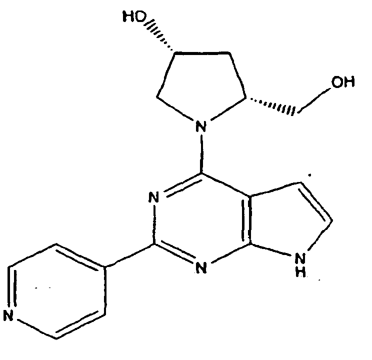 | | * | | |
| 1606 | 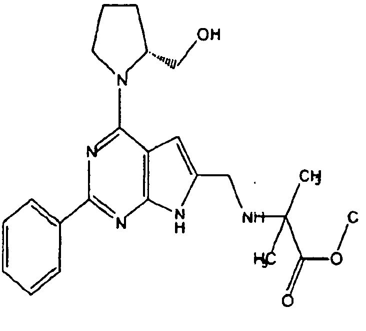 | | * | | |
| 1607 | 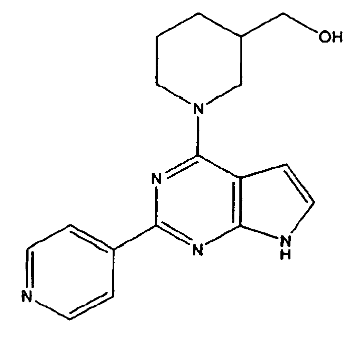 | | * | | |
| 1608 | 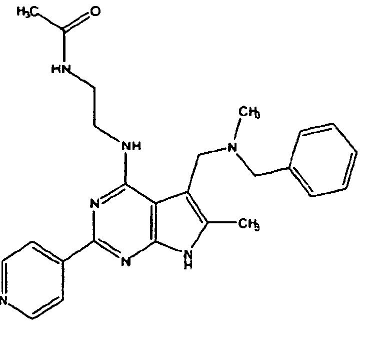 | | * | | |

| | | | | | |
|---|---|---|---|---|---|
| * at least 5 times more selective than other three subtypes. | | | | | |

The present invention is also based on compounds which selectively bind to adenosine A₃ receptor, thereby treating a disease associated with A₃ adenosine receptor in a subject by administering to the subject a therapeutically effective amount of such compounds. The disease to be treated are associated with, for example, asthma, hypersensicivity, rhinitis, hay fever, serum sickness, elleroicvasculitis, atopic dermatitis, dermatitis, psoriasis, eczema, idiopathic pulmonary fibrosis, eosinophilic chlorecysitis, chronic airway inflammation, hypereosinophilic syndromes, eosinophilic gastroenteritis, edema, urticaria, eosinophilic myocardial disease, episodic angioedema with eosinophilia, inflammatory bowel disease, ulcerative colitis, allergic granulomatosis, carcinomatosis, eosinophilic granuloma, familial histiocytosis, hypertension, mast cell degranulation, tumor, cardiac hypoxia, cerebral ischemia, diuresis, renal failure, neurological disorder, mental disorder, cognitive disorder, myocardial ischemia, bronchoconstriction, arthritis, autoimmune disease, Crohn's disease, Graye's disease, diabetes, multiple sclerosis, anaemia, psoriasis, fertility disorders, lupus erthyematosus, reperfusion injury, brain arteriole diameter, the release of allergic mediators, scleroderma, stroke, global ischemia, central nervous system disorder, cardiovascular disorder, renal disorder, inflammatory disorder, gastrointestinal disorder, eye disorder, allergic disorder, respiratory disorder, or immunological disorder.

This invention also features a compound having the structure:
wherein R₁ is 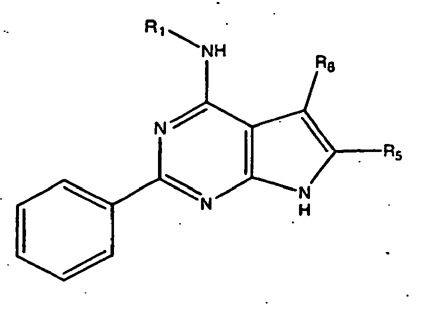 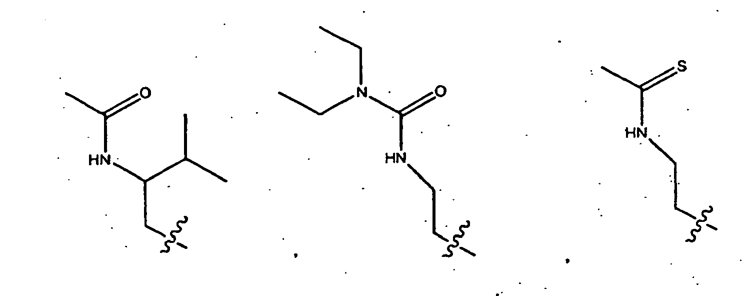 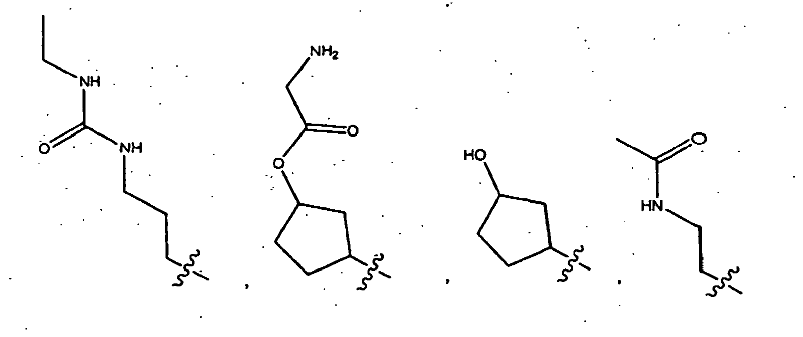 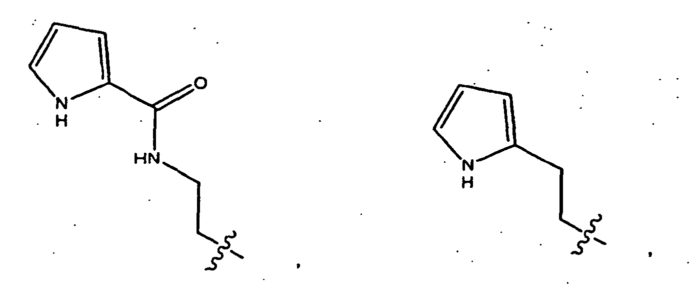 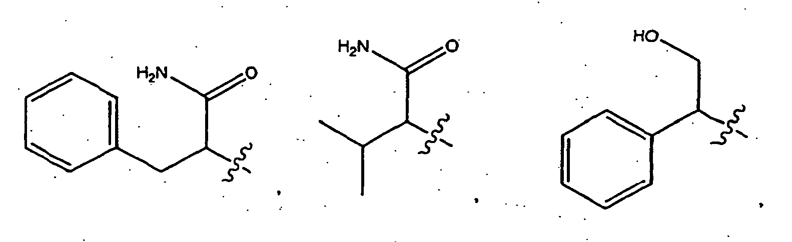 or

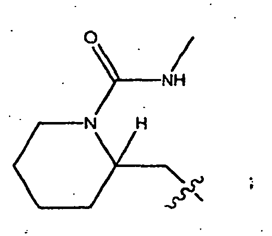

R₅ and R₆ are independently H, substituted or unsubstituted alkyl, or aryl,
wherein the substituent, when present, is as defined above,
wherein when R1 is 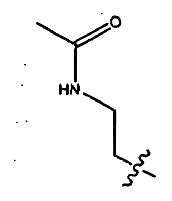 R5 is 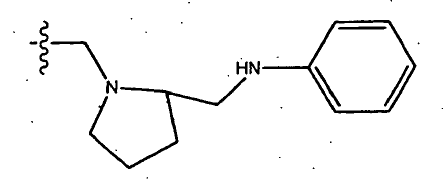 and R6 is H; and a compound having the structure: 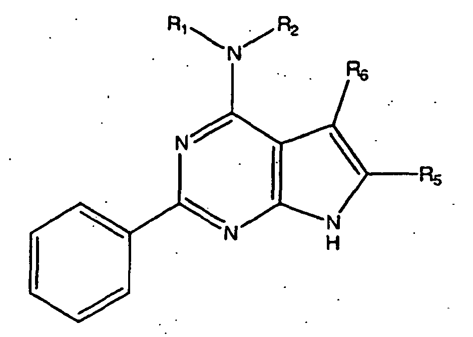 wherein R₁, R₂ and the nitrogen together are 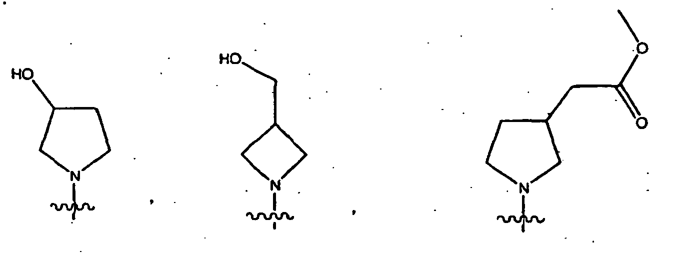 ; and 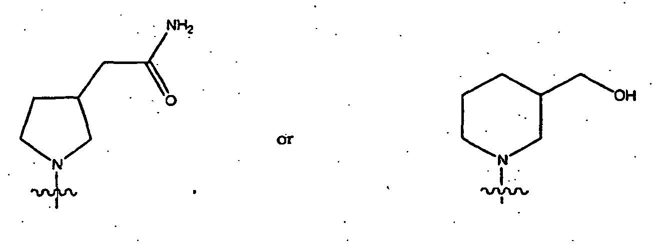 wherein R₅ and R₆ are independently H, substituted or unsubstituted alkyl, or aryl,
wherein the substituent, when present, is as defined above,
wherein when R1, R2 and the nitrogen together are 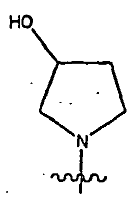 R5 is phenyl and R6 is H;
wherein when R1, R2 and the nitrogen together are 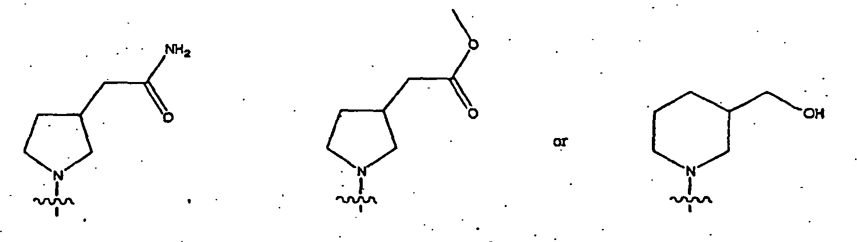 R5 is 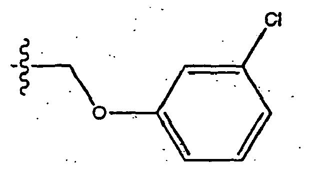 and R6 is H; and
wherein when R1, R2 and the nitrogen together are 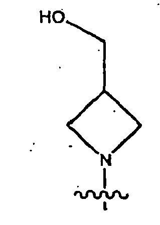 R5 and R6 are both H.

This invention also features a method for inhabiting the activity of an A₃ adenosine receptor in a cell in vitro, which comprises contacting said cell with the above-mentioned compounds.

In one embodiment of the compound, the compound has the structure:

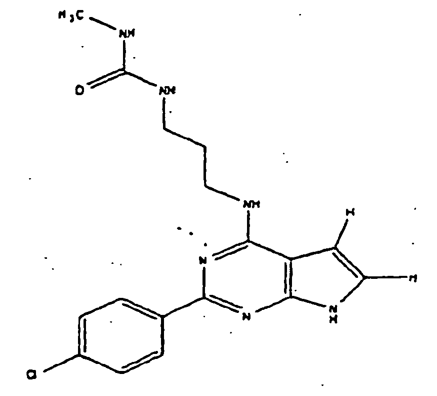

In one embodiment of the compound, the compound has the structure:

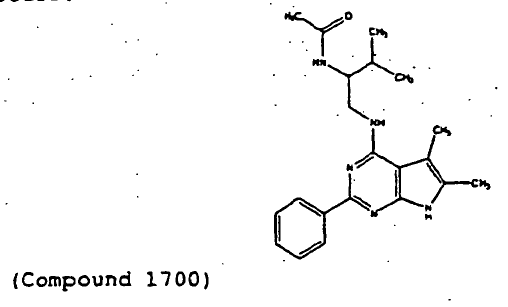

In another embodiment of the compound, the compound has the structure:

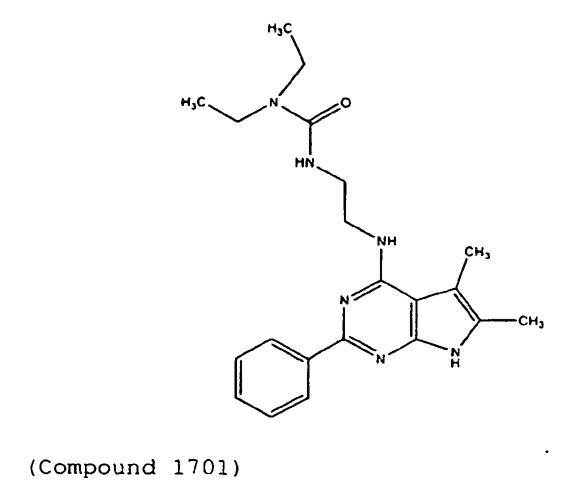

In another embodiment of the compound, the compound has the structure:

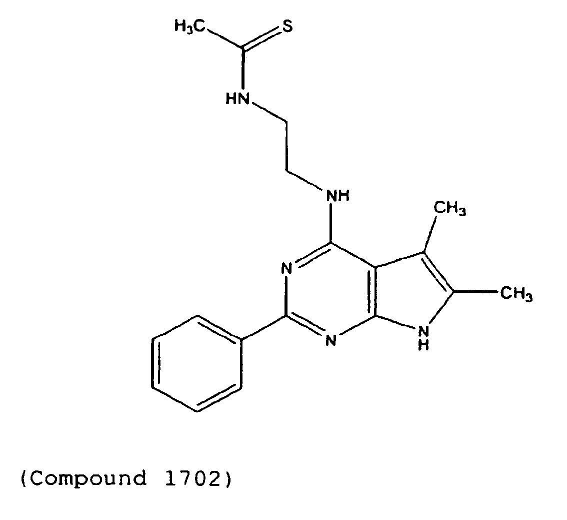

In another embodiment of the compound, the compound has the structure:

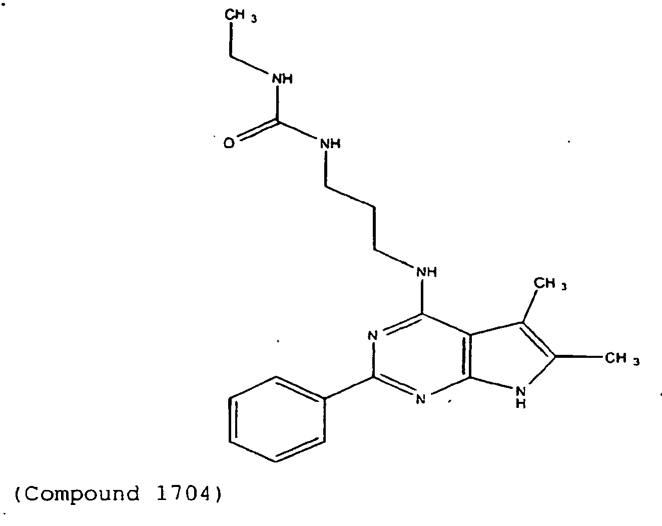

In another embodiment of the compound, the compound has the structure:

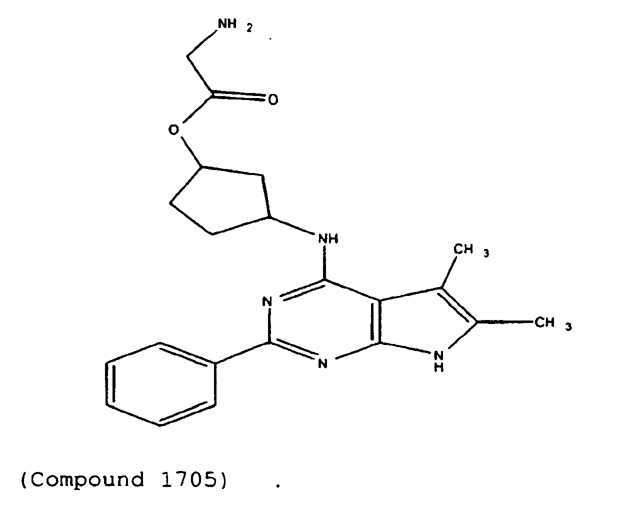

In another embodiment of the compound, the compound has the structure:

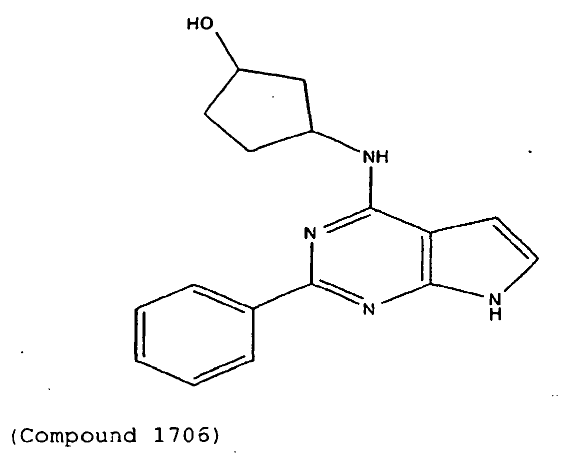

In another embodiment of the compound, the compound has the structure:

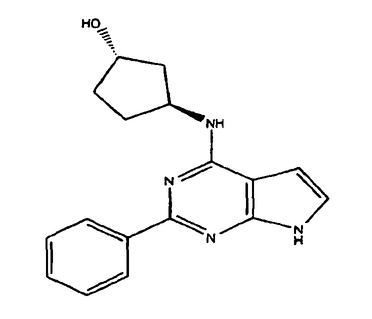

In another embodiment of the compound, the compound has the structure:

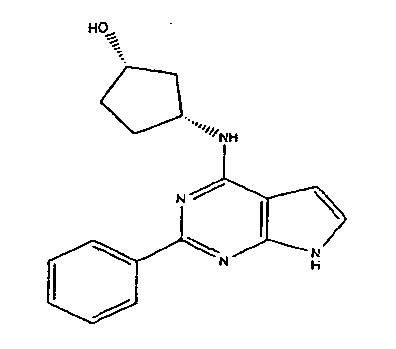

In another embodiment of the compound, the compound has the structure:

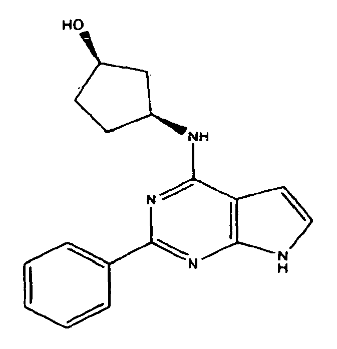

In another embodiment of the compound, the compound has the structure:

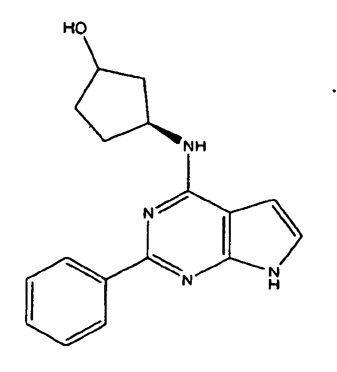

In another embodiment of the compound, the compound has the structure:

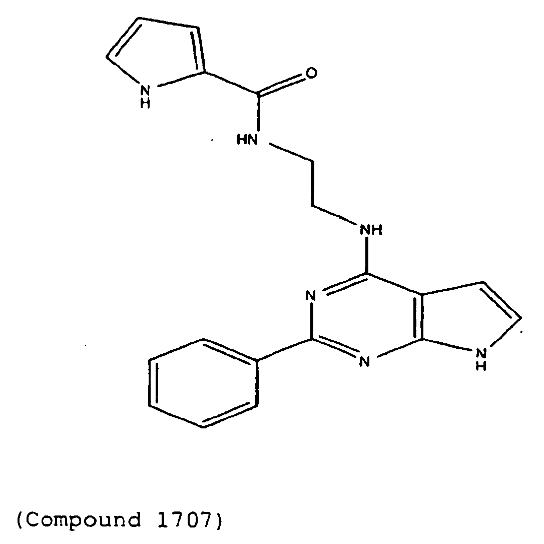

In another embodiment of the compound, the compound has the structure:

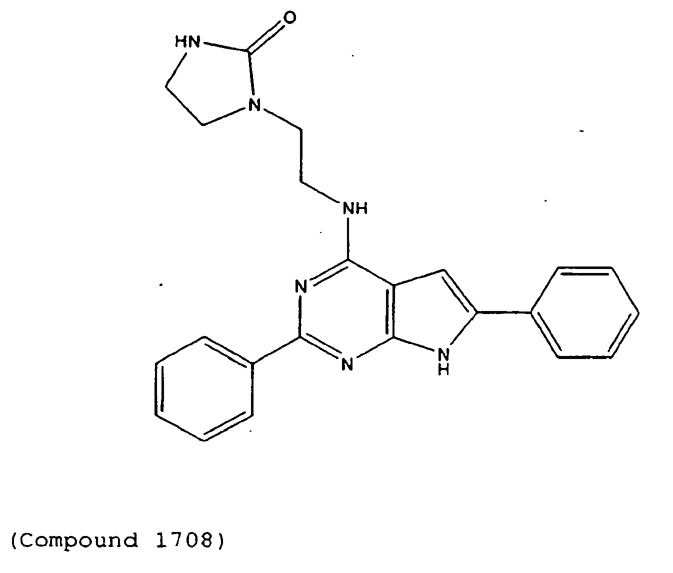

In another embodiment of the compound, the compound has the structure:

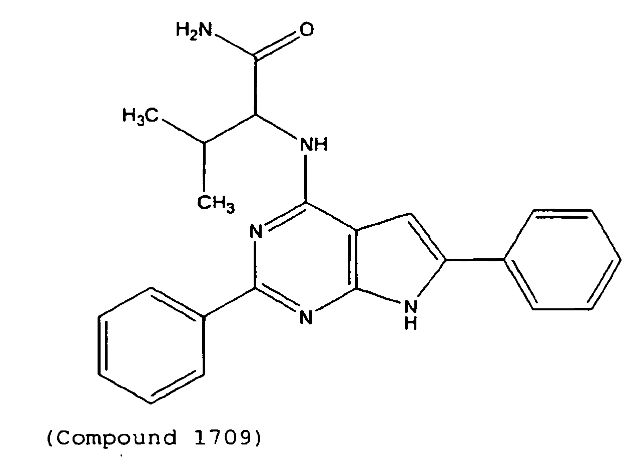

In another embodiment of the compound, the compound has the structure:

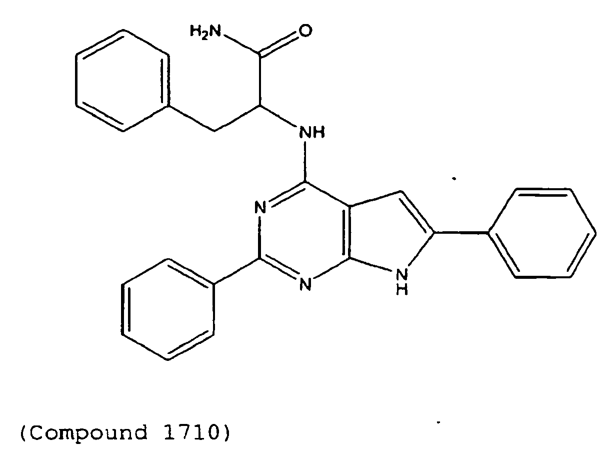

In another embodiment of the compound, the compound has the structure:

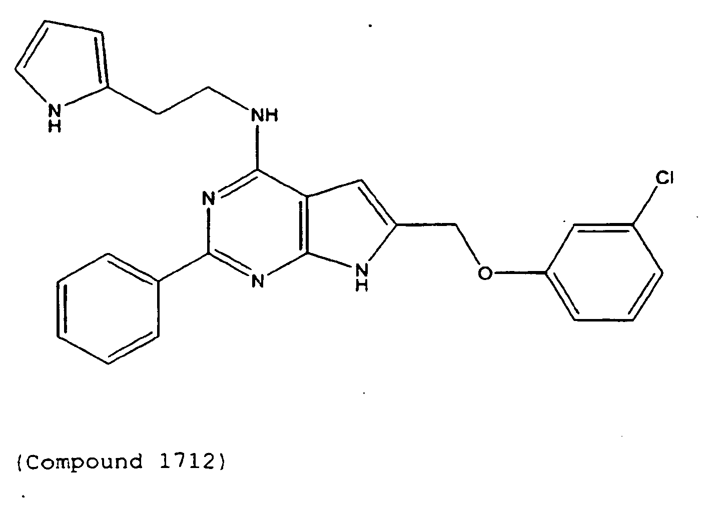

In another embodiment of the compound, the compound has the structure:

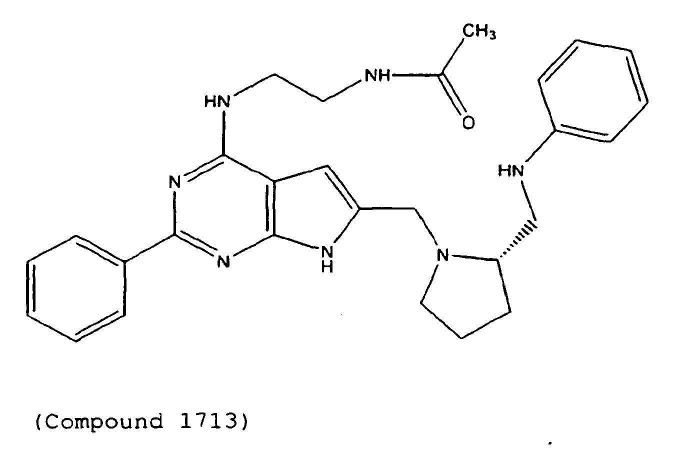

In another embodiment of the compound, the compound has the structure:

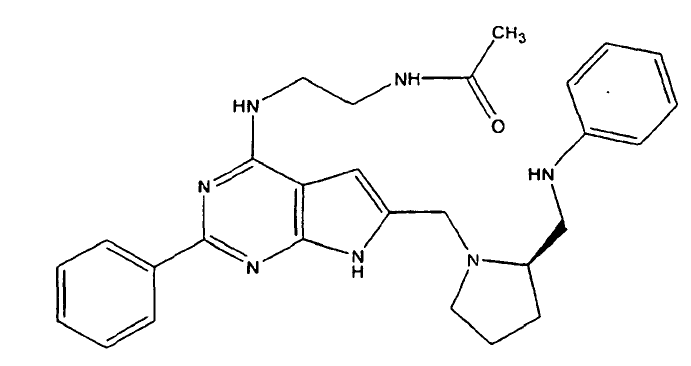

In another embodiment of the compound, the compound has the structure:

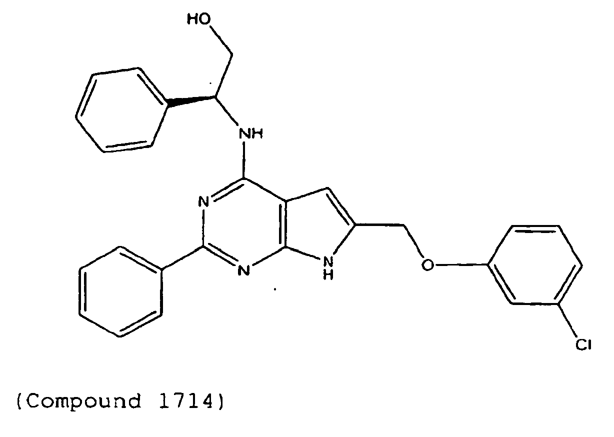

In another embodiment of the compound, the compound has the structure:

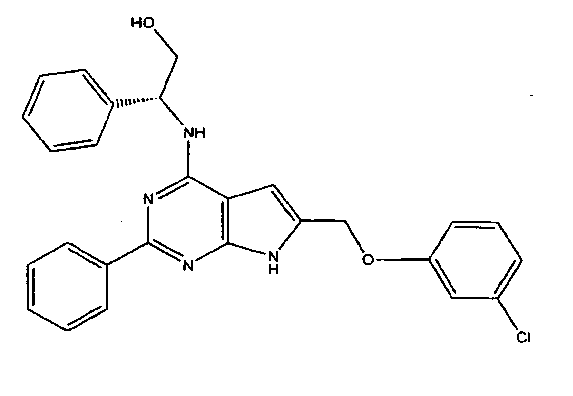

In another embodiment of the compound, the compound has the structure:

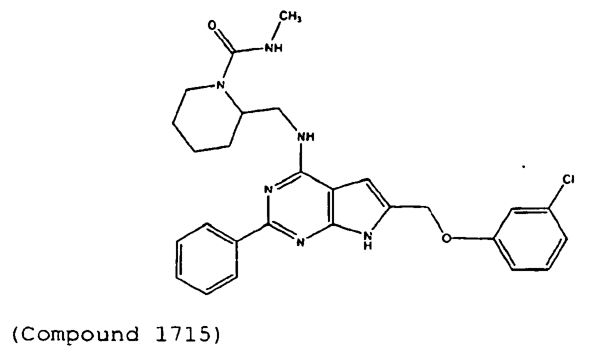

In another embodiment of the compound, the compound has the structure:

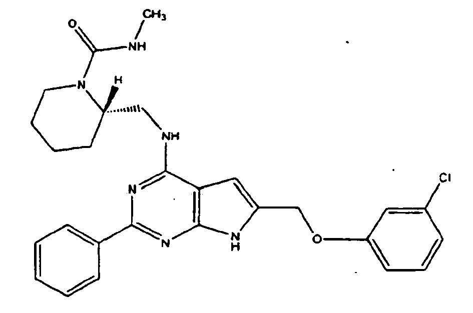

In another embodiment of the compound, the compound has the structure:

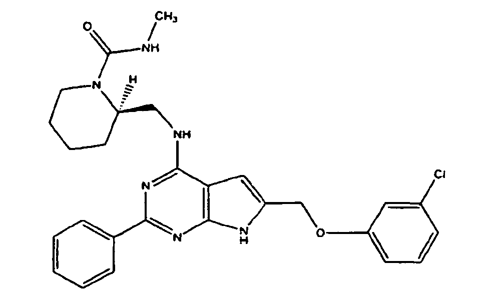

In one embodiment of the compound, the compound has the structure:

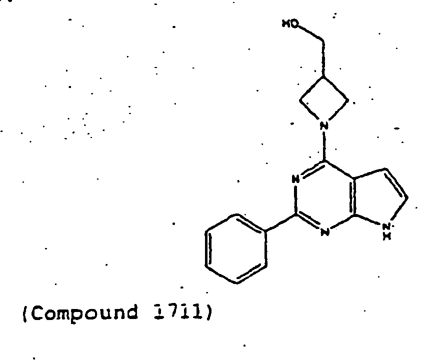

In another embodiment of the compound, the compound has the structure:

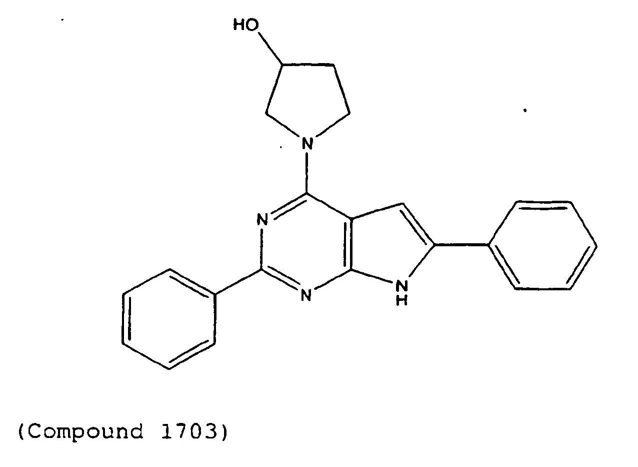

In another embodiment of the compound, the compound has the structure:

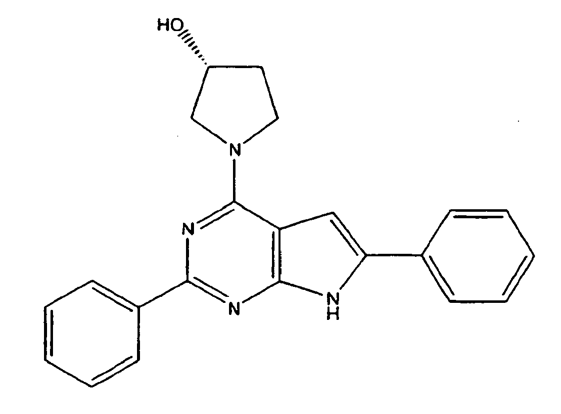

In another embodiment of the compound, the compound has the structure:

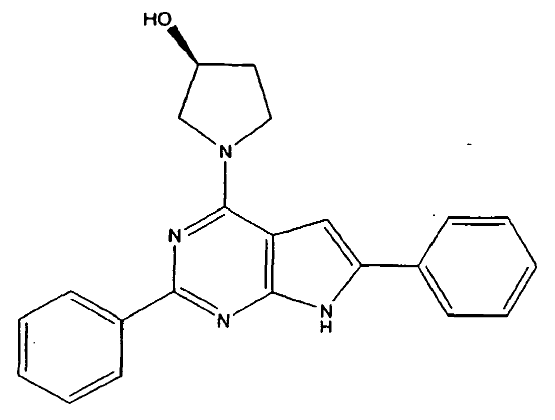

In another embodiment of the compound, the compound has the structure:

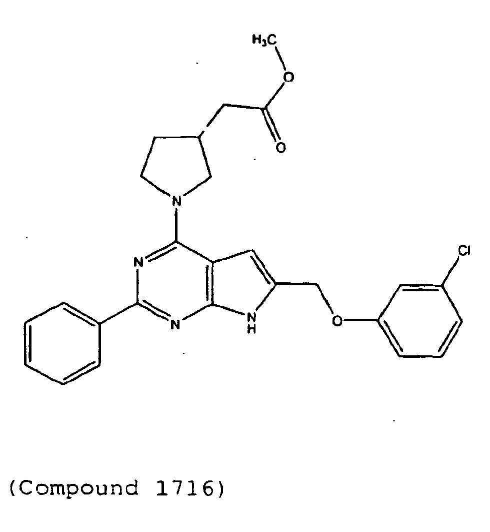

In another embodiment of the compound, the compound has the structure:

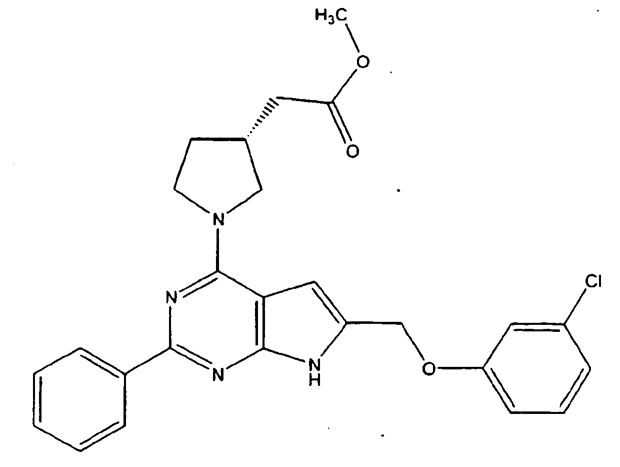

In another embodiment of the compound, the compound has the structure:

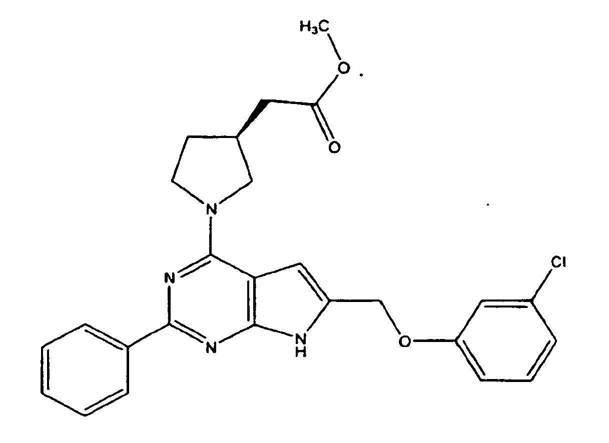

In another embodiment of the compound, the compound has the structure:

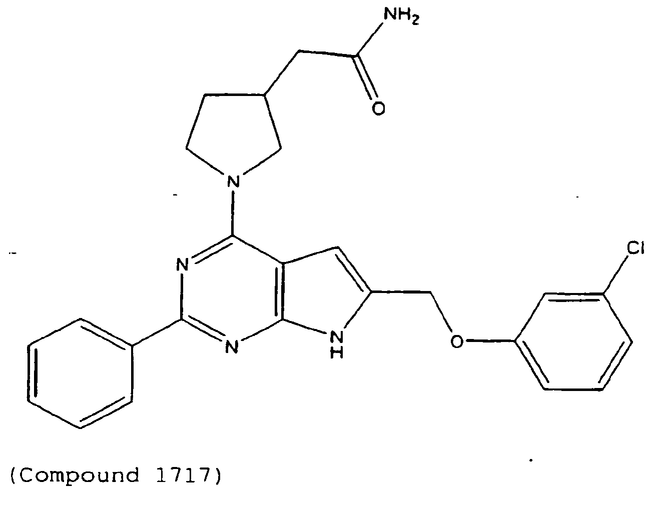

In another embodiment of the compound, the compound has the structure:

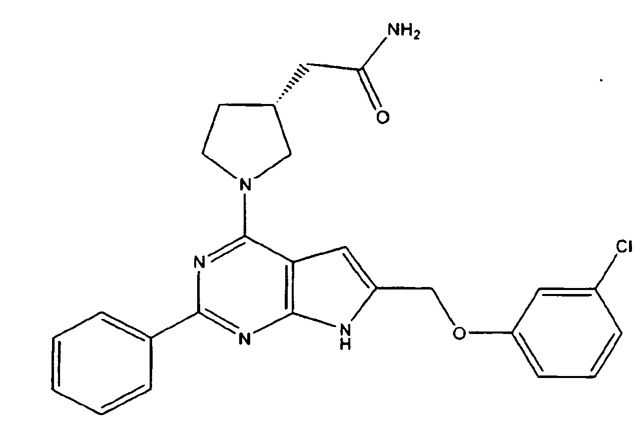

In another embodiment of the compound, the compound has the structure:

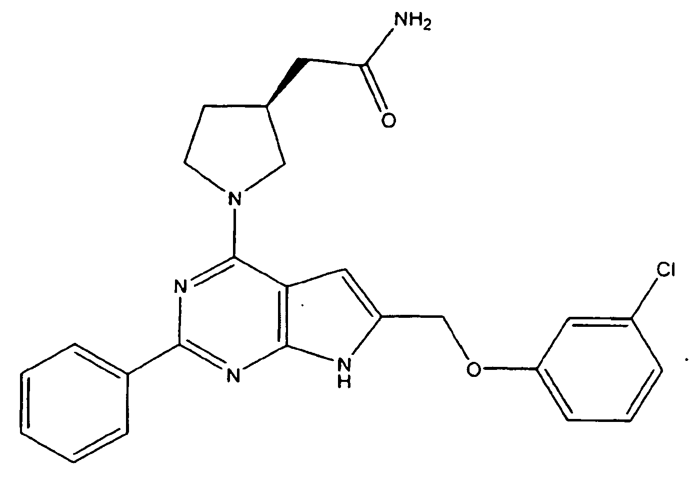

In another embodiment of the compound, the compound has the structure:

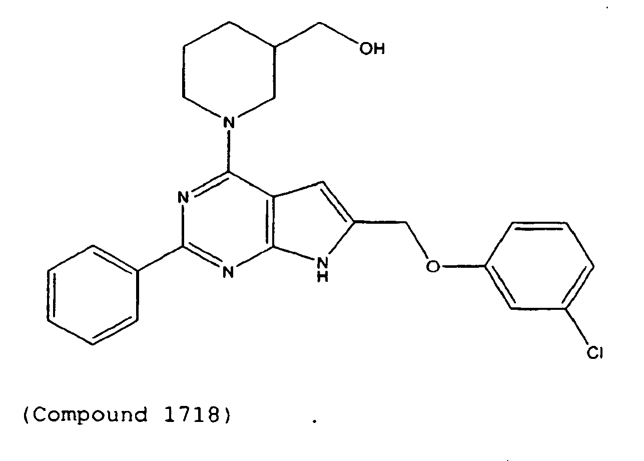

In another embodiment of the compound, the compound has the structure:

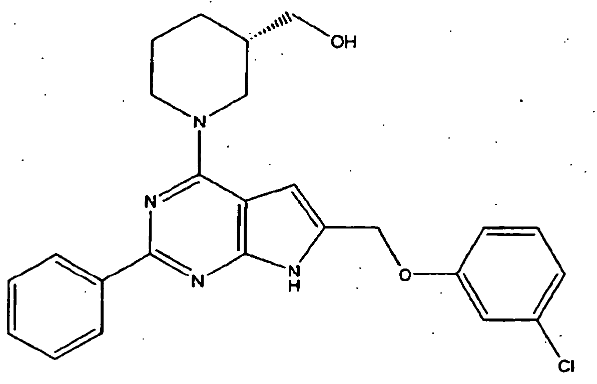

In another embodiment of the compound, the compound has the structure:

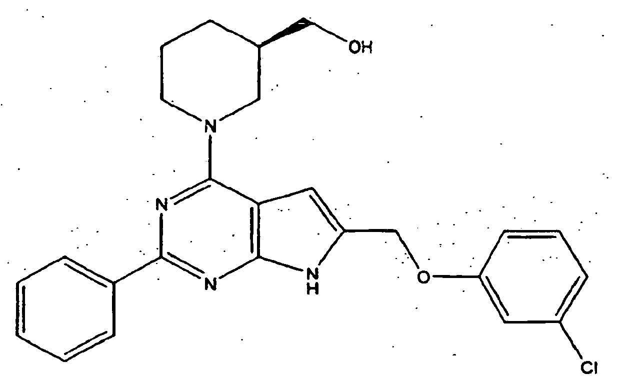

This invention also relates to the use of a therapeutically effective amount of any of the compounds mentioned above for the manufacture of a medicament for treating a disease associated with A3 adenosine receptor in a subject.

In one embodiment, the subject is a mammal.

In another embodiment, the mammal is a human.

In another embodiment, said A3 adenosine receptor is associated with a central nervous system disorder, a cardiovascular disorder, asthma, hypersensitivity, rhinitis, hay fever, serum sickness, allergic vasculitis, atopic dermatitis, dermatitis, psoriasis, eczema, idiopathic pulmonary fibrosis, eosinophilic chlorecystitis, chronic airway inflammation, hypereosinophilic syndromes, eosinophilic gastroenteritis, edema, urticaria, eosinophilic myocardial disease, episodic angioedema with eosinophilia, inflammatory bowel disease, ulcerative colitis, allergic granulomacosis, carcinomatosis, eosinophilic granuloma, familial histiocytosis, hypertension, mast cell degranulation, tumor, cardiac hypoxia, cerebral ischemia, diuresis, renal failure, neurological disorder, mental disorder, cognitive disorder, myocardial ischemia, bronchoconstriction, arthritis, autoimmune disease, Crohn's disease, Grave's disease, diabetes, multiple sclerosis, anaemia, psoriasis, fertility disorders, lupus erthyematosus, reperfusion injury, brain arteriole diameter, the release of allergic mediators, scleroderma, stroke, global ischemia, central nervous system disorder, cardiovascular disorder, renal disorder, inflammatory disorder, gastrointestinal disorder, eye disorder, allergic disorder, respiratory disorder, or immunological disorder.

Diseases associated with adenosine A1, A2a, A2b and A3 receptors are disclosed in WO 99/06053 and WO-09822465, WO-09705138, WO-09511681, WO-09733879, JP-09291089, PCT/US98/16053 and U.S. Patent No. 5,516, 894, the entire content of which are fully incorporate herein by reference.

This invention also provides a water-soluble prodrug of any of the compounds mentioned above; wherein said water-soluble prodrug that is metabolized *in vivo* to an active drug which selectively inhibit A3 adenosine receptor.

In one embodiment of the prodrug, said prodrug is metabolized *in vivo* by esterase catalyzed hydrolysis.

This invention also provides a pharmaceutical composition comprising the prodrug and a pharmaceutically acceptable carrier.

This invention also provides a method for inhibiting the activity of an A3 adenosine receptor in a cell in vitro, which comprises contacting said cell with any of the compounds mentioned above.

In one embodiment of the method, the compound is an antagonist of said A3 adenosine receptor.

In another embodiment of the pharmaceutical composition, said pharmaceutical composition is an ophthalmic formulation.

In another embodiment of the pharmaceutical composition, said pharmaceutical composition is an periocular, retrobulbar or intraocular injection formulation.

In another embodiment of the pharmaceutical composition, said pharmaceutical composition is a systemic formulation.

This invention also relates to the use of an effective amount of any of the compounds mentioned above for the manufacture of a medicament for treating a gastrointestinal disorder in an subject.

In one embodiment, said disorder is diarrhea.

In another embodiment, the subject is a human.

In another embodiment, the compound is an antagonist of A3 adenosine receptors.

This invention further relates to the use of an effective amount of any of the compounds mentioned above for the manufacture of a medicament for treating respiratory disorder in a subject.

In one embodiment, said disorder is asthma, chronic obstructive pulmonary disease, allergic rhinitis, or an upper respiratory disorder.

In another embodiment, the subject is a human.

In another embodiment, said compound is an antagonist of A3 adenosine receptors.

This invention also relates to the use of an effective amount of any of the compounds mentioned above for the manufacture of a medicament for treating damage to the eye of a subject.

In one embodiment, said damage comprises retinal or optic nerve head damage.

In another embodiment, said damage is acute or chronic.

In another embodiment, said damage is the result of glaucoma, edema, ischemia, hypoxia or trauma.

In another embodiment , the subject is a human.

In another embodiment, the compound is an antagonist of A3 adenosine receptors.

This invention also provide a pharmaceutical composition comprising a therapeutically effective amount of any of the compounds mentioned above and a pharmaceutically acceptable carrier.

In one embodiment of the pharmaceutical composition, said therapeutically effective amount is effective to treat a respiratory disorder or a gastrointestinal disorder.

In another embodiment of the pharmaceutical composition, said gastrointestinal disorder is diarrhea..

In another embodiment of the pharmaceutical composition, said respiratory disorder is asthma, allergic rhinitis, or chronic obstructive pulmonary disease.

In another embodiment of the pharmaceutical composition, said pharmaceutical composition is an ophthalmic formulation.

In another embodiment of the pharmaceutical composition, said pharmaceutical composition is an periocular, retrobulbar or intraocular injection formulation.

In another embodiment of the pharmaceutical composition, said pharmaceutical composition is a systemic formulation.

In another embodiment of the pharmaceutical composition, said pharmaceutical composition is a surgical irrigating solution.

This inventio also provides a packaged pharmaceutical composition for treating a disease associated with A3 adenosine receptor in a subject, comprising: (a) a container holding a therapeutically effective amount of any of the compounds mentioned above; and (b) instructions for using said compound for treating said disease in a subject.

The compounds of this invention can be synthesized by the Schemes I-IX.

As used herein, "A compound is A₃ selective." means that a compound has a binding constant to adenosine A3 receptor of at least ten time higher then that to adenosine A₁, A₂ₐ, or A_{2b}.

The invention is further illustrated by the following examples which in no way should be construed as being further limiting. The contents of all references, pending patent applications and published patent applications, cited throughout this application, including those referenced in the background section, are hereby incorporated by reference. It should be understood that the models used throughout the examples are accepted models and that the demonstration of efficacy in these models is predictive of efficacy in humans.

A skilled artisan will know that metabolism of the compounds disclosed herein in a subject produces certain biologically active metabolites which can serve as drugs.

This invention will be better understood from the Experimental Details which follow. However, one skilled in the art will readily appreciate that the specific methods and results discussed are merely illustrative of the invention as described more fully in the claims which follow thereafter.

### Example 24: Adenosine A₃ Antagonist Experimentals

**Compound 1700 (Table 17 below)**: MS (ES): 366.1 (M⁺+1).

**Compound 1710 (Table 17 below):** MS (ES): 381.1 (M⁺+1) .

**Compound 1316 (Table 17 below):** MS (ES): 353.2 (M⁺+1).

**Compound 1703 (Table 17 below):** MS (ES): 357.1 (M⁺+1).

**Compound 1719 (Table 17 below) :** ¹H-NMR (200MHz, d₆-DMSO) ( 1.75 (m, 2H), 3.11 (m, 2H), 3.35 (s, 3H), 3.59 (m, 2H), 5.72 (m, 1H), 5.96 (m, 1H), 6.55 (s, 1H), 7.15 (s, 1H), 7.49 (m, 2H), 8.32 (m, 2H).

**Compound 1704 (Table 17 below):** MS (ES): 367.0 (M⁺+1).

**Compound 1706 (Table 17 below):** ¹H-NMR (200MHz, CDCl₃) d 1.22 (m, 2H), 1.60-2.40 (m, 4H), 4.53 (m, 1H), 4.94 (m, 1H), 5.70 (d, 1H, J = 8.2 Hz), 6.35 (d, 1H, J = 2.8 Hz), 6.97 (d, 1H, J = 2.0 Hz), 7.50 (m, 3H), 8.40 (m, 2H), 10.83 (brs, 1H).

**Compound 1707 (Table 17 below)**': MS(ES) : 347.0 (M⁺+1) .

**Compound 1708 (Table 17 below):** MS (ES) 399.0 (M⁺+1).

**Compound 1709 (Table 17 below):** MS (ES) 385.9 (M⁺+1).

**Compound 1710 (Table 17 below):** MS (ES) 434.0 (M⁻+1).

**Compound 1711 (Table 17 below):** ¹H-NMR (200MHz, CD₃OD) d 3.95 (d, 2H, J = 5.8Hz), 4.23 - 4.31 (m, 2H), 4.53 (t, 2H, J = 8.8Hz), 6.30 (d, 1H, J = 3.0Hz), 6.98 (d, 1H, J = 3.0Hz), 7.45 - 7.48 (m, 3H), 7.83 - 8.42 (m, 2H), 9.70 (brs, 1H), MS (ES) : 281.1 (M⁻+1).

**Compound 1712 (Table 17 below):** ¹H-NMR (200MHz, CD₃OD) d 3.02 (m, 2H), 3.92 (m, 2H), 5.09 (2, 2H), 6.53 (s, 1H), 6.90-7.04 (br s, 1H), 6.92 (m, 2H), 7-02 (m, 1H), 7.21 (dd, 1H, J = 8.2Hz), 7.40 (m, 3H), 7.50-7.80 (br s, 1H), 8.33 (m, 2H). MS (ES): 445.1 (M'+1) .

**Compound 1713 (Table 17 below):** ¹H-NMR (200MHz, CDCl₃) d 1.65-1.80(m, 7H), 1.88-2.00(m, 1H), 2.10 - 2.40 (m, 1H), 2.70-3.05 (m, 3H), 3.09-3.14 (m, 2H), 3.16-3.38 (m, 1H), 3.45 (d, 1H, J = 14Hz), 3.53-3.60 (m, 2H), 3.84-3.92 (m, 2H), 3.97 (d, 1H, J = 14Hz), 5.55 (t, 1H, J = 5.8Hz), 6.17 (s, 1H), 6.55=6.59 (m, 2H), 6.69-6.71 (m, 1H), 7.11-7.19 (m, 2H), 7.43-7.46 (m, 3H), 8.38-8.42 (m, 2H), MS (ES): 484.0 (M⁺+1).

**Compound 1714 (Table 17 below):** MS (ES): 471.0 (M⁺+1).

**Compound 1715 (Table 17 below):** MS (ES): 505.0 (M⁺+1).

**Compound 1716 (Table 17 below):** ¹H-NMR (200MHz, CD₃OD) d 1.65 (m, 1H), 2.18 (m, 1H), 2.49 (br d, 2H, J = 6.2Hz), 2.64 (m, 1H), 3.38 (m, 1H), 3.69 (s, 3H), 3.72 (m, 1H), 3.93 (m, 1H), 4.10 (m, 1H), 5. 06 (2, 2H), 6.58 (s, 1H), 6. 92 (m, 2H), 7.02 (m, 1H), 7.23 (dd, 1H, J = 8.1Hz), 7.39 (m, 3H), 8.32 (m, 2H). MS (ES): 477.1 (M⁺+1).

**Compound 1717 (Table 17 below):** ¹H-NMR (20OMHz, CO₂OD) d 1.69 (m, 1H), 2.26 (m, 1H), 2.42 (d, 2H, J = 7.4Hz), 2.72 (m, 1H), 3.53 (m, 1H), 3.83 (m, 1H), 4.02 (m, 1H), 4.14 (dd, 1H, J = 10.6, 7.0Hz), 5.14 (2, 2H), 6.69 (s, 1H), 6.96 (m, 2H), 7.06 (m, 1H), 7.25 (dd, 1H, J = 8.0Hz), 7.39 (m, 3H), 8.35 (m, 2H). MS (ES): 462.2 (M⁺+1).

**Compound 1718 (Table 17 below):** ¹H-NMR (200MHz, CD₃OD) d 1.40 - 2.00 (m, 5H), 3.52 (d, 2H, 7.6Hz), 3.80 - 4.00 (m, 1H), 4.00 - 4.20 (m, 3H), 4.50 (m, 2H), 6.36 - 6.50 (m, 2H), 6.54 (s, 1H), 6.84 - 6.92 (m, 1H), 7.05 (t, 1H, J = 8.2Hz), 7.30 - 7.45 (m, 3H), 8.24 (d, 2H, J = 9.8Hz). MS (ES): 449.0 (M⁺+1).

**TABLE 17. Adenosine A₃ Receptor Selective Compounds**

| Compound | Structure | Ki-Al | Ki-A2a | Ki-A2b | Ki-A3 |
|---|---|---|---|---|---|
| 1202 Reference | 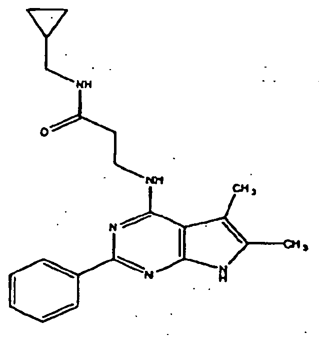 | | | | * |
| 1700 | 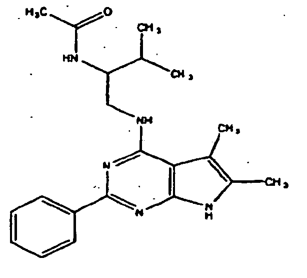 | | | | * |
| 1309. Ref | 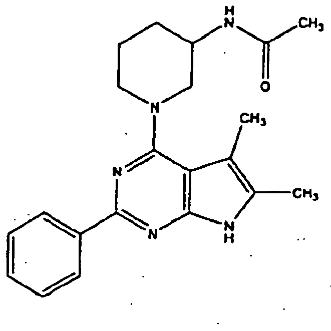 | | | | * |
| 1701 | 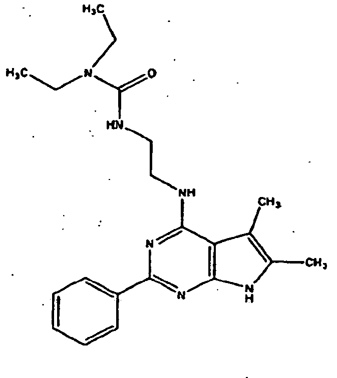 | | | | * |
| 1311 Ref. | 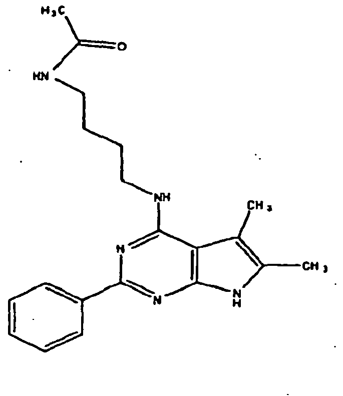 | | | | * |
| 1312 Ref. | 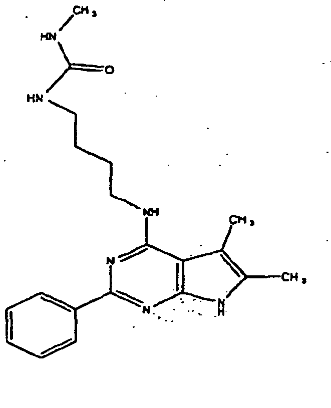 | | | | * |
| 1310 Ref. | 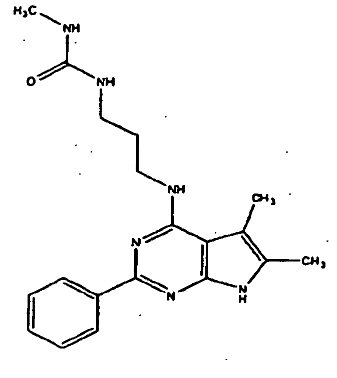 | | | | * |
| 1316 Ref. | 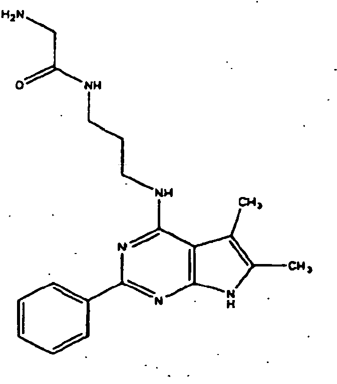 | | | | * |
| 1702 | 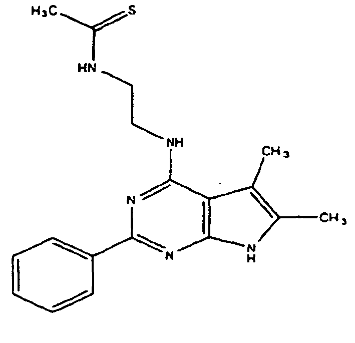 | | | | * |
| 1703 | 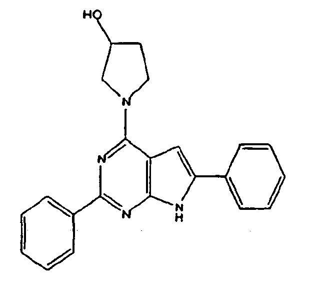 | | | | * |
| 1704 | 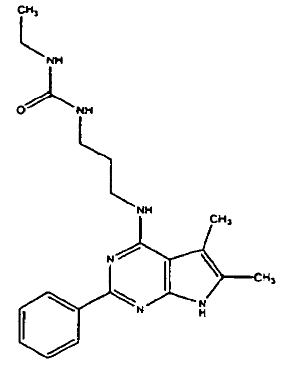 | | | | * |
| 1705 | 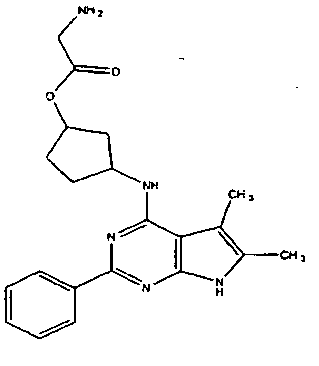 | | | | * |
| 1706 | 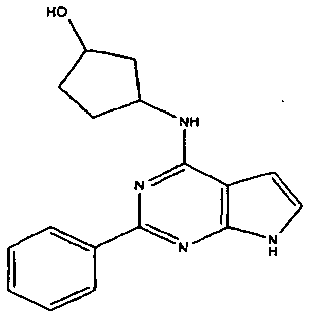 | | | | * |
| 1707 | 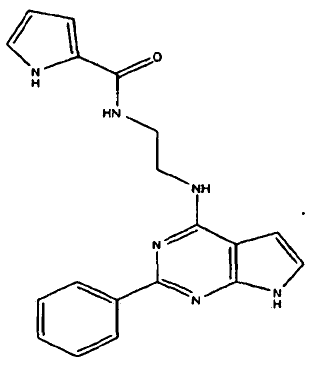 | | | | * |
| 1708 | 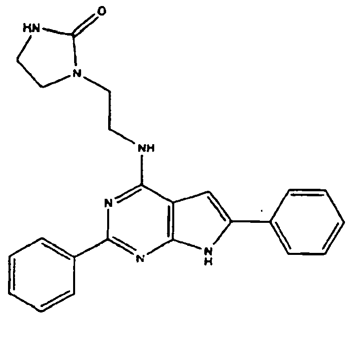 | | | | * |
| 1709 | 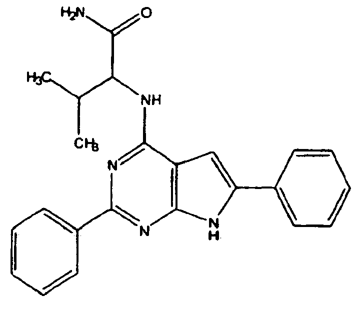 | | | | * |
| 1710 | 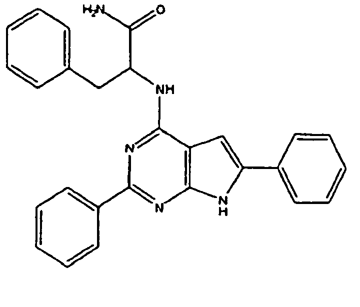 | | | | |
| 1711 | 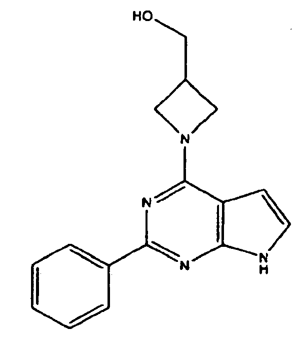 | | | | * |
| 1712 | 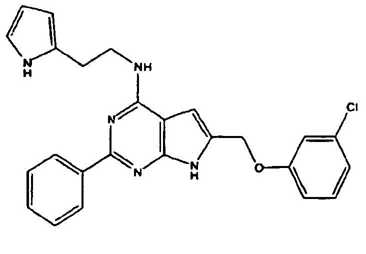 | | | | * |
| 1713 | 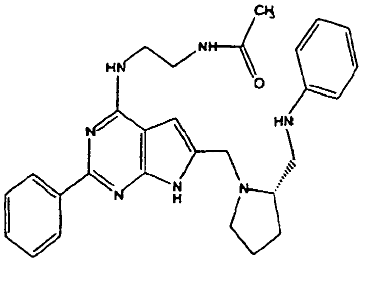 | | | | * |
| 1714 | 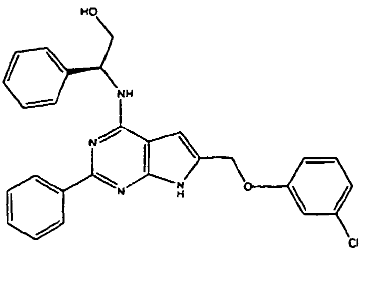 | | | | * |
| 1715 | 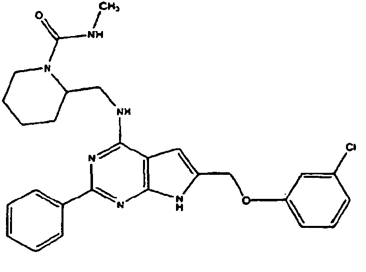 | | | | * |
| 1716 | 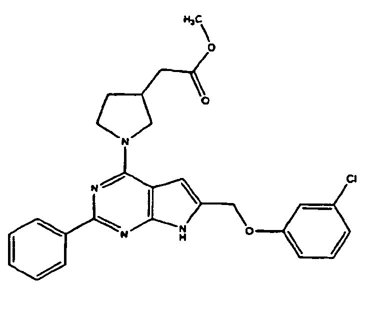 | | | | * |
| 1717 | 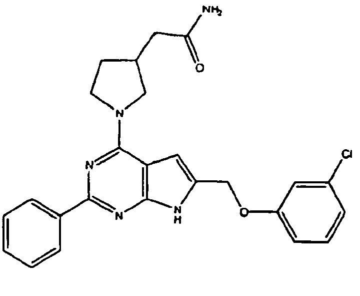 | | | | * |
| 1718 | 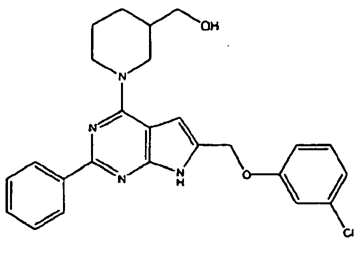 | | | | * |
| 1719 | 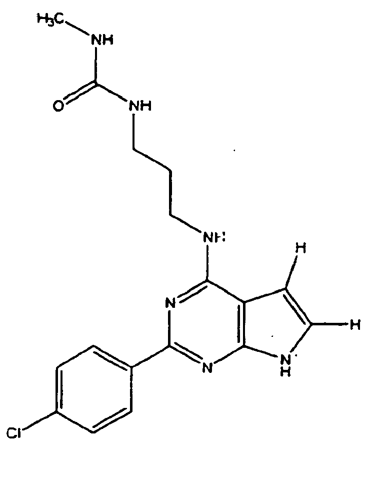 | | | | * |

| | | | | | |
|---|---|---|---|---|---|
| * at least 10 times more selective than other three subtypes. | | | | | |

This invention also provides a compound having the structure:

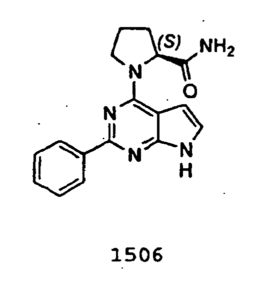

This invention further provides a compound having the structure:

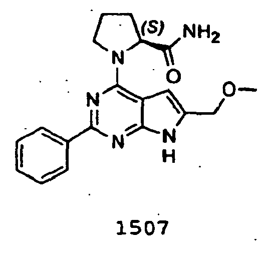

This invention further provides a compound having the structure:

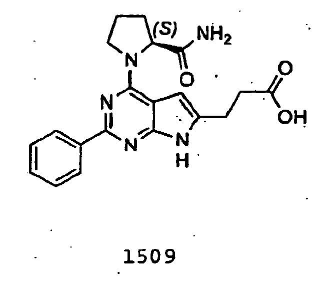

This invention further provides a compound having the structure:

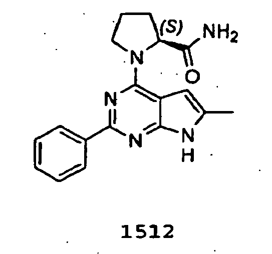

In a further embodiment the invention to the use of a therapeutically effective amount of compounds 1506, 1507, 1509, or 1512, for the manufacture of a medicament for treating a disease associated with A₁ adenosine receptor in a subject.

In a further embodiment the invention provides the above use, wherein the subject is a mammal.

In a further embodiment the invention provides the above use , wherein the mammal is a human.

In a further embodiment the invention provides the above use, wherein said A₁ adenosine receptor is associated with cognitive disease, renal failure, cardiac arrhythmias, respiratory epithelia, transmitter release, sedation, vasoconstriction, bradycardia, negative cardiac inotropy and dromotropy, branchoconstriction, neutropil chemotaxis, reflux condition, or ulcerative condition.

In a further embodiment the invention provides a water-soluble prodrug of compound 1506, 1507, 1509, or 1512, wherein the water-soluble prodrug is metabolized in vivo to produce an active drug which selectively inhibits A₁ adenosine receptor.

In a further embodiment the invention provides, wherein said prodrug is metabolized in vivo by esterase catalyzed hydrolysis.

In a further embodiment the invention provides a pharmaceutical composition comprising the above prodrug and a pharmaceutically acceptable carrier.

In a further embodiment the invention provides a method for inhibiting the activity of an A₁ adenosine receptor in a cell in vitro, which comprises contacting the cell with compounds 1506, 1507, 1509, or 1512.

In a further embodiment the invention provides the above method for inhibiting the activity of an A₁ adenosine receptor in a cell in vitro, wherein the compound is an antagonist of the A₁ adenosine receptor.

In a further embodiment the invention provides the above method for inhibiting the activity of an A₁ adenosine receptor in a cell in vitro, wherein the cell is human cell.

In a further embodiment the invention provides the above method for inhibiting the activity of an A₁ adenosine receptor in a human cell in vitro, wherein the compound is an antagonist of A₁ adenosine receptors.

In a further embodiment the invention relates to the use of an antagonist of A₁ adenosine receptors for the manufacture of a medicament for treating a disease associated with A₁ adenosine receptor in a subject, wherein said disease is asthma, chronic obstructive pulmonary disease, allergic rhinitis, or an upper respiratory disorder.

In a further embodiment, the subject is a human.

In a further embodiment the invention provides a combination therapy for asthma, comprising the compound 1506, 1507, 1509, or 1512, and a steroid, β2 agonist, glucocorticoid, lucotriene antagonist, or anticolinergic agonist.

In a further embodiment the invention provides a pharmaceutical composition comprising a therapeutically effective amount of the compound 1506, 1507, 1509, or 1512, and a pharmaceutically acceptable carrier.

In a further embodiment the invention provides a method for treating a respiratory disorder with the compound 1506, 1507, 1509, or 1512, wherein said respiratory disorder is asthma, allergic rhinitis, or chronic obstructive pulmonary disease.

In a further embodiment the invention provides the above pharmaceutical composition(s), wherein said pharmaceutical composition is an periocular, retrobulbar or intraocular injection formulation.

In a further embodiment the invention provides the above pharmaceutical composition(s), wherein said pharmaceutical composition is a systemic formulation.

In a further embodiment the invention provides the above pharmaceutical composition(s), wherein said pharmaceutical composition is a surgical irrigating solution.

In a further embodiment the invention provides a packaged pharmaceutical composition for treating a disease associated with A₁ adenosine receptor in a subject, comprising:
(a) a container holding a therapeutically effective amount of the compounds 1506, 1507, 1509, or 1512 ; and
(b) instructions for using said compound for treating said disease in a subject.

In a further embodiment the invention provides a pharmaceutically acceptable salt of the compound 1506, 1507, 1509, or 1512.

In a further embodiment the invention provides the above pharmaceutically acceptable salt, wherein the pharmaceutically acceptable salt of the compound 1509 contains a cation selected from the group consisting of sodium, calcium and ammonium.

In yet a further embodiment the invention relates to the use of aforementioned compounds for the manufacture of a medicament for treating a disease associated with A₁ adenosine receptor in a subject, wherein the A₁ adenosine receptor is associated with congestive heart failure.

### Exemplification

### Ref. Example 21: Synthesis of 1-[6-(4-Hydroxy-4-phenyl-piperidin-1-yl-methyl)-2-phenyl-7H-pyrrolo[2,3-d]pyrimidin-4-yl]-pyrrolidine-2-carboxylic acid amide (1505).

Compound 1505 was synthesized in a manner similar to that of Example 17 using synthesis scheme IX with L-prolineamide and 4-phenyl-piperidin-4-ol to obtain:

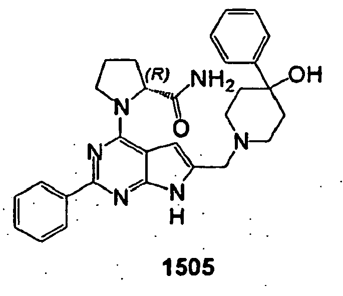

¹H-NMR (d₆-DMSO) d 1.53 (s, 1H), 1.60 (s, 1H), 1.84-2.30 (m, 6H), 2.66 (m, 2H), 3.60 (s, 2H), 3.88 (m, 1H), 4.02 (m, 1H), 4.66 (d, 1H, J = 6.BHz), 4.73 (s, 1H), 6.44 (s, 1H), 6.94 (s, 1H), 7.12 - 7.50 (m, 10H), 8.35 (m, 2H), 11.6 (brs, 1H); MS (ES) : 305.1 (M⁺+1); mp = 234-235°C.

### Example 22: Synthesis of [N-(2-Phenyl-7H-pyrrolo [2,3-d]pyrimidin-4-yl)(L)-prolinamide (1506)

Compound **1506** was synthesized using synthesis scheme VII with L-prolineamide to obtain:

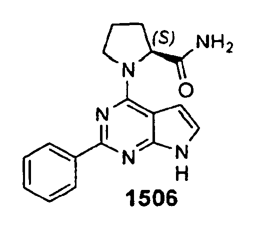

¹H-NMR (DMSO-*d*_{*6*}) d 2.05 (m, 4H), 3.85 (m, 1H), 4.05 (m, 1H), 4.70 (d, 1H, J=8.0Hz), 6.58 (brs, 1H), 6.95 (brs, 1H), 7.15 (d, 1H, J=3.4Hz), 7.40 (m, 3H), 7.50 (brs, 1H), 8.40 (m, 2H), 11.6 (brs, 1H) ; MS (ES) : 308.3 (M⁺+1). mp= 236-238°C.

### Example 23: Synthesis of [N-(2-phenyl-6-methoxymethyl-7H-pyrrolo[2,3-d]pyrimidin-4-yl)-(L)-prolinamide (1507)

Compound **1507** was synthesized using precursor compound 23 of synthesis scheme IX to obtain:

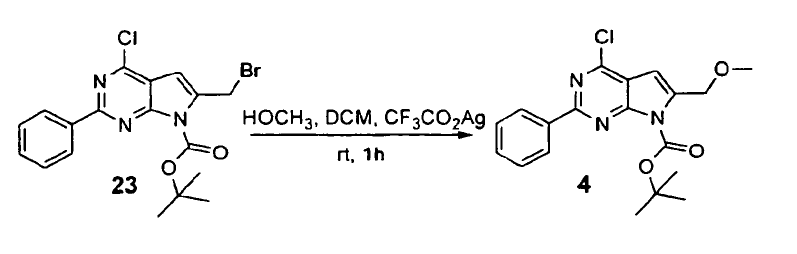

Bromide **23** (4.23g, 10mmol) is dissolved in anhydrous methanol (60mL) and DCM (120mL) and treated with AgO₂CCF₃ under N₂ at rt for 1h. The solid is removed by filtration and washed with DCM (2x20mL). The filtrate is concentrated *in vacuo.* The residue is redissolved in DCM (80mL). The resulted solution is then washed with saturated NaHCO₃ solution and brine, dried over MgSO₄, filtered and concentrated to give 3.71g (**4**, 99%) off white solid. ¹H-NMR (CDCl₃) d 1.75 (s, 9H), 3.51 (s, 3H), 4.83 (s, 2H), 6.70 (s, 1H), 7.47 (m, 3H), 8.52 (m, 2H).

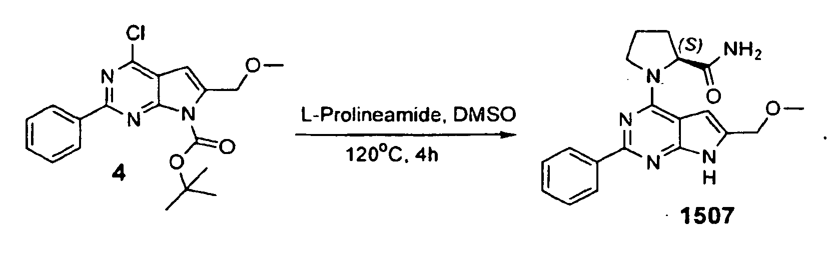

Aryl chloride **4** (2.448g, 6.55mmol), DMSO (15mL), L-prolineamide (4.0g, 35.0mmol) and NaHCO₃ (2.9g) are combined and heated to 120°C under nitrogen. After 4h, the reaction is cooled to room temperature and diluted with water (60ml). The resulted slurry is extracted with DCM (10x). The combined organic layers are washed with saturated NaHCO₃ solution and brine, dried over MgSO₄, filtered and concentrated to give 2.48g brown solid. Pure product (1.86g, 81%) is obtained after flash column as white solid. White crystals are gotten from THF/hexane. M.p. = 213-215°C. ¹H-NMR (CDCl₃) d 2.15 (m, 3H), 2.52 (m, 1H), 3.26 (s, 3H), 3.92 (m, 1H), 4.10 (m, 1H), 9.92 (s, 2H), 5.08 (d, 1H, J=8.2Hz), 5.49 (brs, 1H), 6.48 (s, 1H), 7.08 (brs, 1H), 7.42 (m, 3H), 8.38 (m, 2H), 9.78 (brs, 1H); MS (ES): 352.2 (M⁺+1).

### Ref. Example 24: Synthesis of 4-Hydroxy-1-(2-phenyl-7H-pyrrolo[2,3-d]pyrimidin-4-yl]-pyrrolidine-2-carboxylic acid amide (1508)

Compound **1508** was obtained with synthesis scheme VII using *cis-*hydroxy prolineamide to obtain:

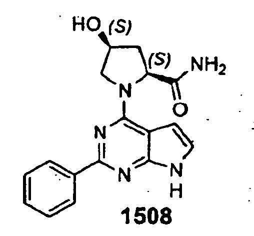

¹H-NMR (d₆-DMSO) d 1.90 (m, 1H), 3.85 (d, 1H, J = 9.2Hz), 4.08 (m, 1H), 4.37 (s, 1H), 4.67 (dd, 1H, J = 8.8, 4.0Hz), 5.30 (s, 1H), 6.55 (s, 1H), 7.15 (s, 2H), 7.37 (m, 3H), 7.64 (s, 1H), 8.37 (m, 2H), 11.65 (brs, 1H); MS (ES): 324.2 (M⁺+1); mp = 268-271°C.

### Example 25: Synthesis of 3-[4-((S)-2-Carbamoyl-pyrrolodin-1-yl)-2-phenyl-7H-pyrrolo[2,3-d]pyrimidin-6-yl]-propionic acid (1509)

Compound **1509** was obtained using precursor compound 23 of synthesis scheme IX to obtain:

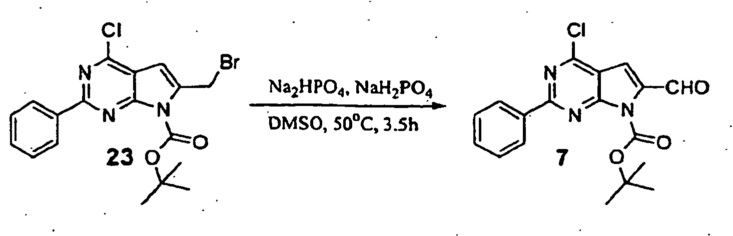

The tert-butoxycarbonyl protected aryl bromide **23** (4.0g, 9.5mmol), dry DMSO (25ml), NaH₂PO₄ (454mg, 3.79mmol) and Na₂HPO₄ (1.62g, 11.4mmol) were combined and heated to 50°C under argon for approximately 3.5h. The mixture was then poured into water (200ml) and extracted with three 100ml portions of EtOAc. The combined organic layers were thoroughly washed with water, brine, dried over MgSO₄, filtered and concentrated to give a yellow solid which was purified by triturating with ethanol to give 1.55g of a pale yellow solid (7). The mother liquor was purified by flash chromatography (10% EtOAc in hexane) to give an additional 454mg (60%). ¹H-NMR (CDCl₃) d 1.77 (s, 9H), 7.25 (s, 1H), 7.48 (m, 3H), 8.52 (m, 2H) 10.39 (s, 1H); m.p.= 156°C (dec).

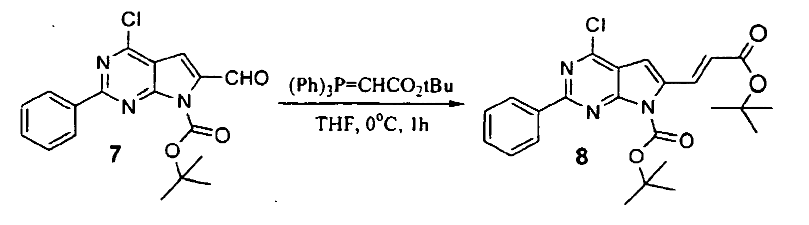

Aldehyde **7** (600mg, 1.7mmol) was dissolved in dry THF (20ml) and cooled to 0°C under argon. To this was added a 0°C solution of (tert-butoxycarbonylmethylene)-triphenylphosphorane (694mg, 1.8mmol) in 10ml of dry THF dropwise through a cannula. After 3h the mixture was concentrated and purified by triturating with ethanol to give 565mg (73%) of a white solid (**8**) . ¹HNMR (CDCl₃) d 1.58 (s, 9H), 1.79 (s, 9H), 0.46 (d, 1H), 6.95 (s, 1H), 7.48 (m, 3H), 8.09 (d, 1H), 8.56 (m, 2H).

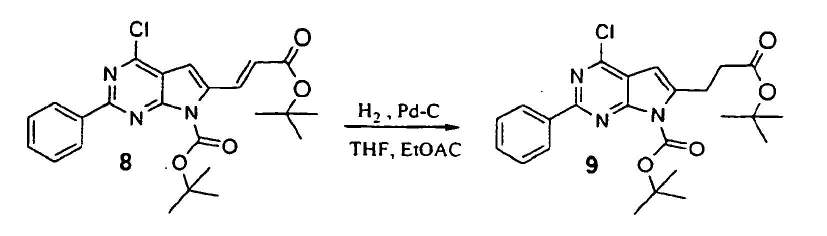

A solution of compound **8** (565mg 1.2mmol) in 5ml THF was diluted to 100ml with EtOAc. After adding 600mg of catalyst (5% wt Pd, 50% H₂O) and purging with argon, the mixture was hydrogenated under atmospheric pressure. After 8h the mixture was filtered, concentrated and purified with flash chromatography (10% EtOAc in hexane) to isolate 200mg (35%) of **9** as a clear oil that crystallized upon standing. ¹HNMR (CDCl₃) d 1.42 (s, 9H), 1.75 (s, 9H), 2.65 (t, 2H), 3.32 (t, 2H), 6.41 (s, 1H) 7.45 (m, 3H), 8.51 (m, 2H).

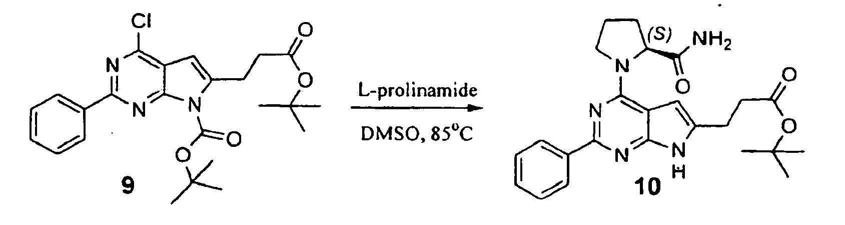

Aryl chloride **9** (200mg, 0.44mmol), DMSO (10ml) and L-prolinamide (440mg, 4.4mmol) were combined and heated to 85°C under argon. After 14 hours the mixture is cooled to room temperature and partitioned between water and ethyl acetate. The layers were separated and the aqueous layer washed with EtOAc (3x). The combined organic layers were thoroughly washed with water (3x), brine, dried over MgSO₄, filtered and concentrated to give **10** as a yellow film which was purified by flash chromatography (2.5% MeOH in CH₂Cl₂). 185mg (97%). MS (ES): 435.8 (M'+1).

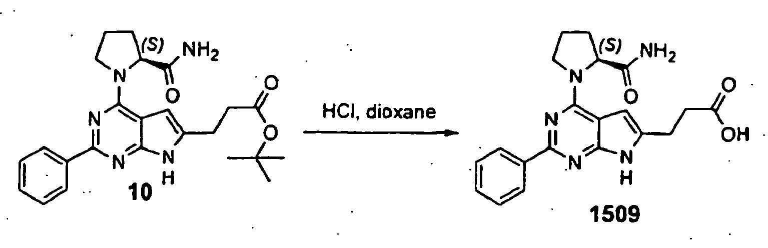

Ester **10** (30mg, mmol) in 5ml dioxane was hydrolyzed by adding 0.5ml concentrated HCl. After 3 hours the mixture was concentrated in vacuo and recrystalized in EtOH/ EtOAc to obtain **1509** as a white solid (20mg, 61%). MS (ES) : 380 (M⁻+1).

### Ref. Example 26: Synthesis of [N-(2-phenyl-6-aminocarbonyl 15 methoxymethyl-7H-pyrrolo[2,3-d]pyrimidin-4-yl)-(L)-prolinamide (1510)

Compound **1510** was obtained using precursor compound 23 of synthesis scheme IX to obtain:

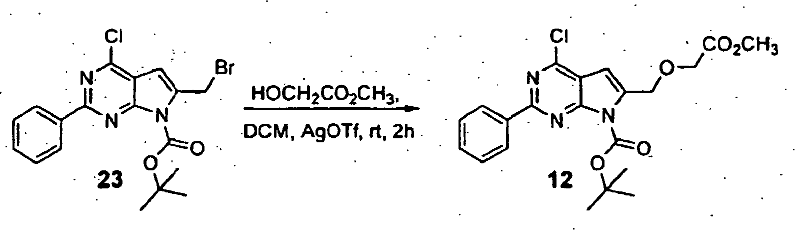

Bromide **23** (1.27g, 3mmol) and molecular sieve (5g) are stirred in anhydrous methyl glycolate (5.8g, 60mmol) and DCM (40mL). The solution is treated with AgOTf under N₂ and allowed to stir for 3h. The solid is removed by filtration and washed with DCM (2x20mL). The filtrate is concentrated *in vacuo*. The residue is redissolved in DCM (80mL). The resulted solution is then washed with water, saturated NaHCO₃ solution and brine, dried over MgSO₄, filtered and concentrated to give 1.35g (99%) off white solid (**12**). ¹H-NMR (CDCl₃) d 1.75 (s, 9H), 3.80 (s, 3H), 5.0 (s, 2H), 6.78 (s, 1H), 7.97 (m, 3H), 8.52 (m, 2H).

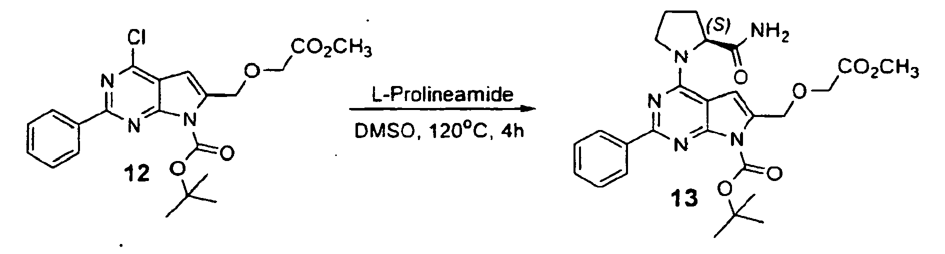

Aryl chloride **12** (177mg, 0.41mmol), DMSO (10mL), L-prolinamide (466mg, 4mmol) and NaHCO₃ (500mg) are combined and heated to 120°C under nitrogen. After 4h, the reaction is cooled to room temperature and diluted with water (60ml). The resulted slurry is extracted with DCM (5x30mL). The combined organic layers are washed with saturated NaHCO₃ solution and brine, dried over MgSO₄, filtered and concentrated to give brown solid. Pure product (154mg, 92%) is obtained after flash column as white solid **(13).** ¹H-NMR (CDCl₃) d 2.15 (m, 3H), 2.52 (m, 1H), 3.55 (s, 3H), 4.58 (s, 2H), 5.08 (s, 1H,), 5.85 (brs, 1H), 6.48 (s, 1H), 7.08 (brs, 1H), 7.42 (m, 3H), 8.40 (m, 2H), 10.58 (brs, 1H); MS (ES): 410.1 (M⁺+1).

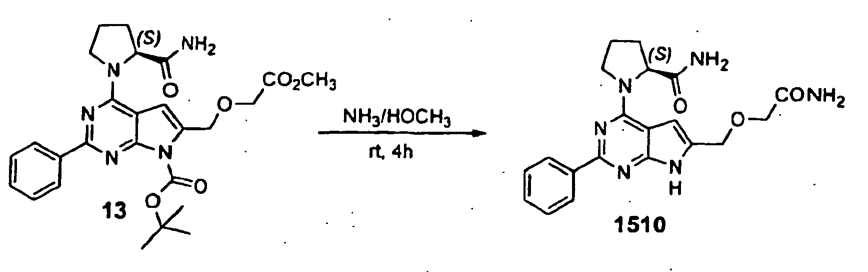

Methyl ester **13** (124mg, 0.3mmol) is dissolved in HOCH₃ (15mL). Ammonia is bubbled through the solution for 0.5h. The reaction mixture is then stirred for another 3h at rt. After removal of solvent 111mg of a white solid (**1510**, 93%) is obtained. ¹H-NMR (CDCl₃) d 1.82 (m, 3H), 2.20 (m, 1H), 2.80 (m, 1H), 3.10 (m, 1H), 3.63 (dd, 2H, J₁=13.8Hz, J₂=19.4Hz), 3.87 (m, 1H), 4.07 (m, 1H), 4.97 (m, 1H), 5.96 (m, 2H), 6.35 (s, 1H), 6.86 (brs, 1H), 7.11 (brs, 1H), 7.37 (m, 3H), 8.28 (m, 2H), 11.46 (brs, 1H); MS (ES) : 394.8 (M⁺+1) .

### Ref. Example 27: Synthesis of [4-(2-Carbamoylpyrrolidin-1-yl)-2-phenyl-7H-pyrrolo[2,3-d]pyrimidine-6-carboxylic acid] (1511)

Compound **1511** was synthesized using precursor compound 15 of synthesis scheme VII to obtain:

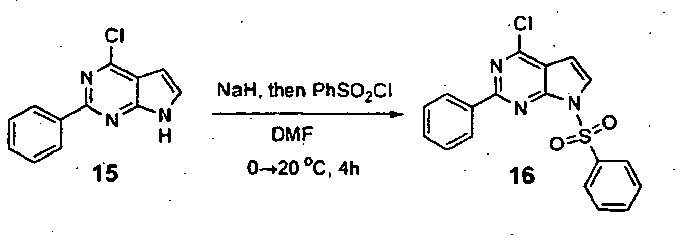

To a suspension of sodium hydride (780mg of a 60% oil suspension, 19.5mmol) in dry DMF (20mL), cooled by an ice/water bath, under nitrogen, is added a solution of the pyrrolopyrimidine **15** (2.00g, 7.52mmol) in DMF (10mL) over 5 min. After 15 min, benzenesulfonyl chloride (1.2mL, 9.40mmol) is added, then the cooling bath is removed. After 4h, the reaction mixture is poured into a mixture of ice and sat. NaHCO₃ sol., the precipitated solid is filtered off and triturated with acetone (3) and methanol (2), yielding 2.37g of a beige solid. This solid (**16**) contains approx. 10mol-% DMF (based on that 83% yield) and can be used in the next step; a pure sample can be obtained by chromatography on silica gel using acetone as eluent. ¹H-NMR (CDCl₃): d 6.70 (d, *J* = 4.2Hz, 1H), 7.47-7.68 (m, 6H), 7.76 (d, *J* = 4.2Hz, 1H), 8.24-8.32 (m, 2H), 8.48-8.56 (m, 2H); IR (solid): n = 3146 cm⁻¹, 1585, 1539, 1506, 1450, 1417, 1386, 1370, 1186, 1176, 1154, 1111, 1015, 919, 726, 683, 616, 607; MS (ES): 372/370 (MH⁺); mp = 226-227 °C.

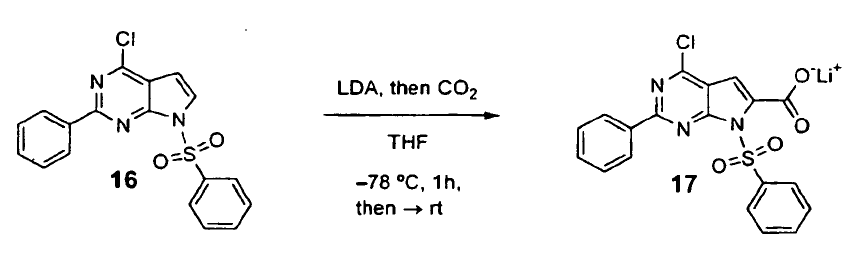

To a solution of the *N*-sulfonyl compound **16** (337mg, 0.911mmol) in dry THF (34mL), cooled by dry ice/acetone, is added LDA·THF (1.0mL, 1.5M solution in cyclohexane, 1.5mmol). After 45min, carbon dioxide is bubbled into the solution for 5min, then the cooling bath is removed. When the solution has reached ambient temp., the solvents are evaporated, yielding 398mg of the salt **17**, containing 0.5 equiv. of (.*i*Pr)₂NCO₂Li, as yellow solid. The salt is used without purification in the next step. ¹H-NMR (D₆₋DMSO): d = 6.44 (s, 1H), 7.50-7.75 (m, 6H), 8.33-8.40 (m, 2H), 8.53 (dd, *J* = 8.0, 1. 6Hz, 2H).

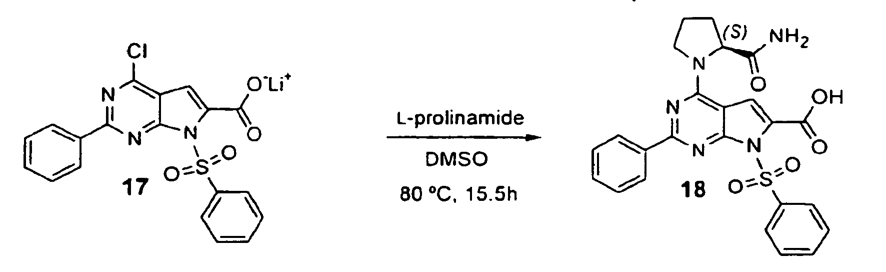

A solution of the lithium salt **17** (50mg) and L-prolinamide (122mg, 1.07mmol) in DMSO (1.5mL) is heated under nitrogen to 80 °C for 15.5h. 4% aq. acetic acid (10mL) is added to the cooled solution, and the mixture is extracted with EtOAc (5'10mL). The combined organic layers are washed with 4% aq. acetic acid (10mL), water (10mL) and brine (10mL) and are dried over MgSO4. Filtration and concentration gives 40mg of **18** as a yellowish solid, which is used without purification in the next step. ¹H-NMR (CD₃OD) : d = 1.95-2.36 (m, 4H), 3.85-3.95 (m, 1H), 3.95-4.17 (m, 1H), 4.72 (brs, 1H), 7.14 (s, 1H), 7.35-7.45 (m, 3H), 7.45-7.70 (m, 3H), 8.33-8.50 (m, 4H).

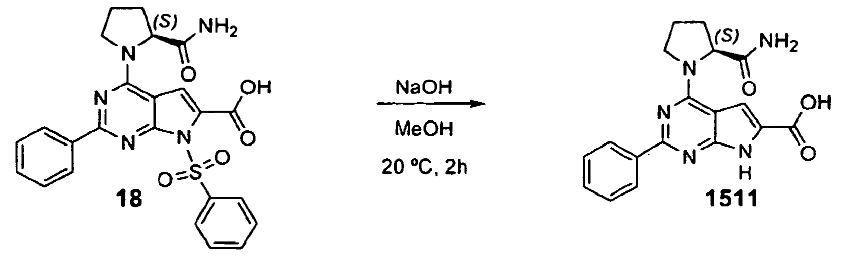

A solution of sodium hydroxide in methanol (1.5mL, 5M, 7.5mmol) is added to a solution of the pyrrolopyrimidine **18** (40mg, 0.081mmol) in methanol (2mL). After 2h, the pH is adjusted to 5, most of the methanol is evaporated, the mixture is extracted with EtOAc (5 10mL), the combined organic layers are washed with brine and dried over MgSO₄. Filtration and concentration yields 24mg of a pale yellow solid, which is triturated with toluene/EtOAc/MeOH to yield 15.6mg (55%) of the acid **1511** as slightly yellowish solid. ¹H-NMR (CD₃OD) : d = 2.05-2.20 (m, 4H), 3.95-4.10 (m, 1H), 4.15-4.25 (m, 1H), 4.85 (brs, 1H), 7.14 (s, 1H), 7.35-7.42 (m, 3H), 8.38-8.45 (m, 2H); IR (solid): n = 3192 cm⁻¹, 2964, 2923, 2877, 1682, 1614, 1567, 1531, 1454, 1374, 1352, 1295, 1262, 1190, 974, 754, 700; MS (ES): 352 (M'+1); m.p. = 220 °C (decomp.).

### Example 28: Synthesis of 1-(6-methyl-2-phenyl-7H-pyrrolo[2,3-d]pyrimidine-4-yl)-(S)-pyrrolidine-2 -carboxylic acid amide (1512)

Compound 1512 was synthesized by the following steps:

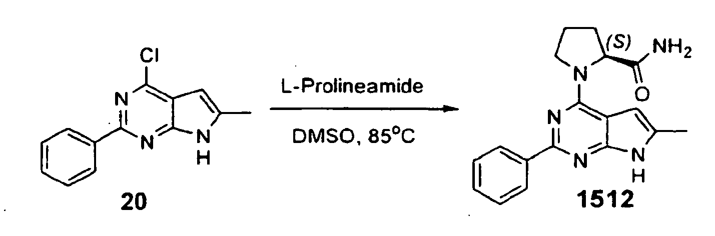

Aryl chloride **20** (3g, 10.7 mmol), DMSO (50ml) and (S)-prolinamide were combined and heated to 85°C under argon. After stirring overnight (14hrs), the mixture was cooled to room temperature and poured into 800ml of water. This was extracted with three 200ml portions of EtOAc. The combined organic layers were thoroughly washed with water (3 x 300 ml), brine, dried over MgSO₄, filtered and concentrated to give a dark brown solid. The solid was recrystallized twice from EtOAc to yield 1.95g (57%) of a tan solid (1512). ¹HNMR (DMSO-d₆) d 1.8-2.2 (m, 4H), 2.3 (s, 3H), 3.8 (m, 1H), 4.0 (m, 1H), 4.6 (d, 1H) 6.2 (s, 1H), 6.9 (s, 1H), 7.2 (m, 3H), 7.3 (s, 1H), 8.4 (m, 2H), 11.5 (s, 1H); MS (ES): 322 (M⁺+1)

### Ref. Example 29: Synthesis of 1-[6-(2-Hydroxy-ethoxymethyl) -2-phenyl-7H-pyrrolo[2,3-d]pyrimidin-4-yl]-pyrrolidine-2-carb oxylic acid amide(1513)

Compound **1513** was synthesized in a manner similar to that of Example 17 using synthesis scheme IX with L-prolineamide and ethane-1,2-diol to obtain: 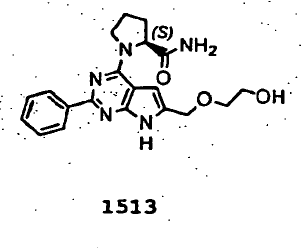 MS (ES): 382 (M⁺+1).

### Ref. Example 30: Synthesis of 4-(6-Imidazol-1-ylmethyl-2-phenyl-7H-pyrrolo[2,3-d]pyrimidin-4-ylamino)-cyclohexanol (1514).

Compound **1514** was synthesized in a manner similar to that of Example 17 using synthesis scheme IX with N-6 amino cyclohexanol and imidazole to obtain: 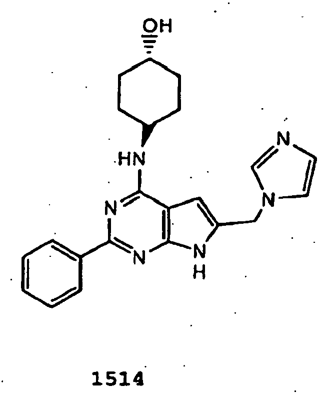 MS (ES) : 389. (M⁺+1)

### Ref. Example 31: Synthesis of 4-(4-Hydroxy-cyclohexylamino) -2-phenyl-7H-pyrrolo[2, 3-d]pyrimidine-6-carboxylic acid (1515)

Compound **1515** was synthesized in a manner similar to that of Example 27 using synthesis scheme IX with N-6 amino cyclohexanol to obtain: 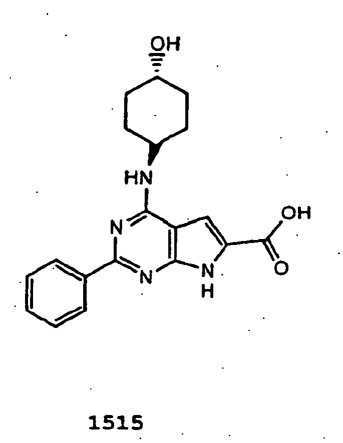 MS (ES): 353 (M⁺+1)

### Ref. Example 32: Synthesis of 9-[6-(2-Hydroxy-ethoxymethyl) -2-phenyl-7H-pyrrolo[2,3-d]pyrimidin-9-ylamino]-cyclohexanol (1516)

Compound **1516** was synthesized in a manner similar to that of Compound **1513** using synthesis scheme IX with N-6 amino cyclohexanol to obtain: 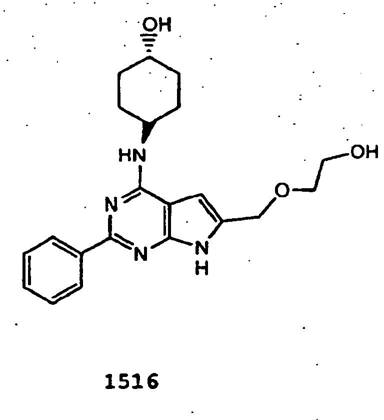 MS (ES) : 383 (M⁺+1)

### Ref. Example 33: Synthesis of 4-(4-Hydroxy-cyclohexylamino). -2-phenyl-7H-pyrrolo[2,3-d]pyrimidine-6-carboxylic acid methyl ester (1517)

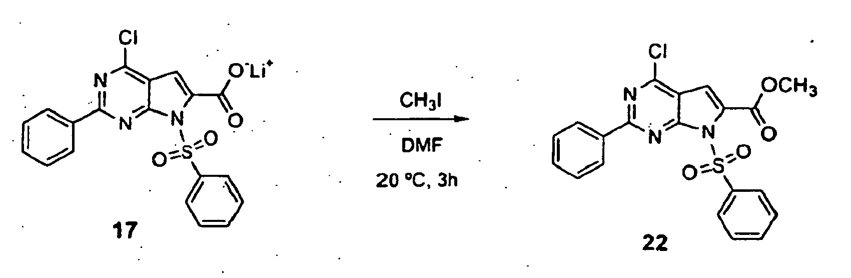

A solution of the lithium salt **17** (0.13mmol) in dry DMF (4mL) is stirred with methyl iodide (0.1mL, 1.6mmol) at 20 °C under argon for 3h. DMF is evaporated, and aqueous ammonium chloride solution is added (15mL). The mixture is extracted with EtOAc (3' 15mL), the combined organic layers are washed with water (2' 10mL) and brine (10mL) and are dried over MgSO₄. Filtration and concentration gives 21mg (38%) of the methyl ester **22**.

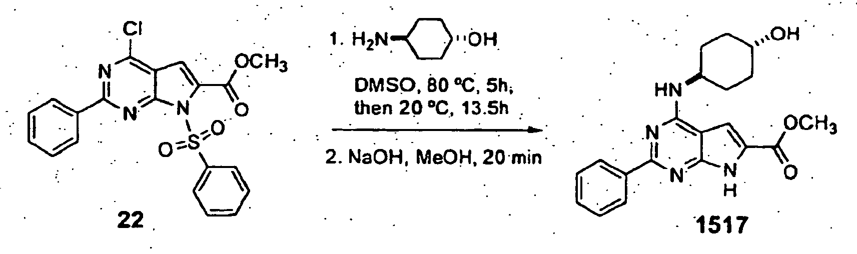

A solution of the methyl ester **22** (24 . 5mg, 0.057mmol) and 4-trans-aminocyclohexanol (66mg, 0.57mmol) in DMSO (1.5mL) is heated under nitrogen to 80 °C for 5h, then the heating is stopped, and stirring at 20°C is continued for 13.5h. 4% aq. acetic acid (10mL) is added to the cooled solution, and the mixture is extracted with EtOAc (3'10mL). The combined organic layers are washed with 4% aq. acetic acid (10mL), water (10mL) 2N NaOH (10mL), water (10mL), and brine (10mL) and are dried over MgSO₄. To a solution of the crude material obtained after filtration and concentration (1H NMR indicates about 50% removal of the benzenesulfonyl group) in THF (2mL) is added a solution of NaOH in MeOH (0.5mL of 5M solution, 2.5mmol) at ambient temperature. After 20min, water and sat. NaHCO₃ solution (5mL each) are added, and the mixture is extracted with EtOAc (4'15mL). The combined organic layers are washed with 2N NaOH (10mL), water (10mL), and brine (10mL) and are dried over MgSO₄. Chromatography of the crude material obtained after filtration and concentration on silica gel, eluting with hexanes/EtOAc 1:1 ® 1:2 yields 8.6mg (41%) of **1517** as a white solid, mp. 225-227 °C. ¹H-NMR (CD₃OD): d = 1.38-1.62 (m, 4H), 1.95-2.10 (m, 2H), 2.10-2.25 (m, 2H), 3.55-3.70 (m, 1H), 3.91 (s, 3H), 4.20-4.35 (m, 1H), 7.32 (s, 1H), 7.35-7.47 (m, 3H), 8.35-8.42 (m, 2H); IR (solid): n = 3352 cm⁻¹, 3064, 2935, 2860, 1701, 1605, 1588, 1574, 1534, 1447, 1386, 1333, 1263, 1206, 1164, 1074, 938, 756, 705; MS (ES) : 367 (MH⁺) .

### Ref. Example 34: Synthesis of [4-(2-Carbamoyl-pyrrolidin-1-yl) -2-phenyl-7H-pyrrolo[2,3-d]pyrimidin-6-ylmethoxy]-acetic acid methyl ester (1518) Compound 1518 was synthesized in a manner similar to example 26 using precursor compound 12 to obtain:

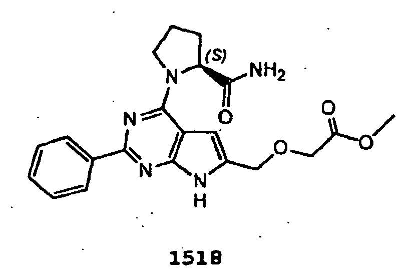

MS (ES): 410 (M⁺+1)

### Ref. Example 35: Synthesis of [4-(2-Carbamoyl-pyrrolidin-1-yl) -2-phenyl-7H-pyrrolo[2,3-d]pyrimidin-6-ylmethoxy]-acetic acid (1519)

Compound **1519** was synthesized in a manner similar to compound **1518** wherein the methyl ester group was hydrolized with a base to obtain: 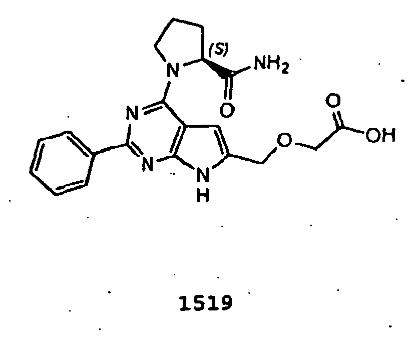 MS (ES): 396 (M⁺+1)

### Ref. Example 40: Synthesis of 4-(4-Hydroxy-cyclohexylamino)-2-phenyl-7H-pyrrolo(2,3-d)pyrimidine-6-carboxylic acid amide (1520)

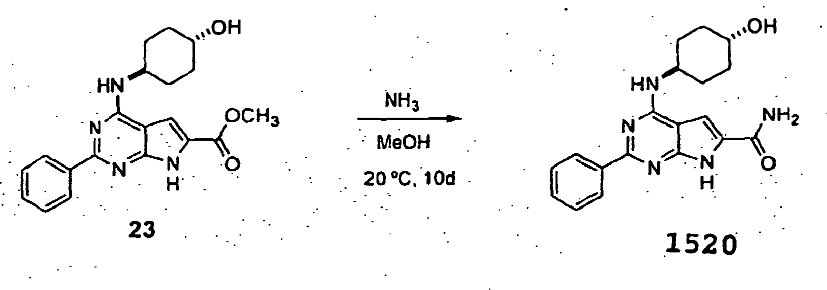

Gaseous ammonia is condensed into a solution of the pyrrolopyrimidine **23** (7.8mg, 0.021mmol) in methanol (6mL), cooled by dry ice/acetone, until a total volume of 12mL is reached. After stirring for 10d at 20 °C, the solvents are evaporated, and the residue is purified by preparative TLC on silica gel, eluting with 5% MeOH in CH₂Cl₂. The material thus obtained is triturated with ether to yield 6.5mg (88%) of the amide **1520** as white solid, mp. 210-220 °C (decomp.) . ¹H-NMR (CD₃OD) : d = 1.40-1.60 (m, 4H), 2.00-2.15 (m, 2H), 2.15-2.25 (m, 2H), 3.55-3.70 (m, 1H), 4.20-4.35 (m, 1H), 7.16 (s, 1H), 7.35-7.47 (m, 3H), 8.34-8.40 (m, 2H); IR (solid) : n = 3358 cm⁻¹, 3064, 3025, 2964, 2924, 2853, 1652, 1593, 1539, 1493, 1452, 1374, 1326, 1251, 1197, 1113, 1074, 1028, 751, 699; MS (ES): 352 (MH⁺).

### Activity of Compounds

Adenosine 1 (A₁) receptor subtype saturation and competition radio ligand binding were carried out for compounds 1505, 1506, 1507, 1508, 1509, 1510, 1511, 1512, 1513, 1514, 1516, 1517, 1518, 1519, and 1520 as described herein and *inter alia,* on pages 152-153 of this specification. All of the above-referenced compounds equaled or surpassed the A₁ receptor binding affinity of reference compounds 1318 or 1319 as described herein and, *inter alia,* in Table 13, on page 171 of the specification.

The water solubilities of the above compounds listed in Table 18 are expected to be better than reference compounds 1318 or 1319 due to their cLogP values, which were calculated using the computer program CS ChemDraw, ChemDraw Ultra ver. 6.0 ©1999 as provided by CambridgeSoft Corporation, 100 Cambridge Park Drive, Cambridge, MA 02140.

The compounds specific to the A₁ receptor listed in Table 18 had lower cLogP values, between about 1.5 to about 3.4, as compared to reference compounds 1318 or 1319 with a cLogP value about 3.8. It was not predicted that the more polar A₁ receptor compounds listed in Table 18 having lower cLogP values than the reference compounds 1318 or 1319 would still retain the potency and A₁ receptor binding selectivity as compared to those reference compounds.

**Table 18**

| **Compound** | **cLogP** |
|---|---|
| Ref. 1505 | 4.1 |
| 1506 | 3.0 |
| 1507 | 2.88 |
| Ref. 1508 | 2.1 |
| 1509 | 2.9 |
| Ref. 1510 | 1.5 |
| Ref. 1511 | 2.7 |
| 1512 | 3.37 |
| Ref. 1513 | 2.4 |
| Ref. 1514 | 2.8 |
| Ref. 1515 | 3.1 |
| Ref. 1516 | 2.8 |
| Ref. 1517 | 3.4 |
| Ref. 1518 | 2.4 |
| Ref. 1519 | 2.2 |
| Ref. 1520 | 2.4 |

### Incorporation by Reference

All patents, published patent applications and other references disclosed herein are hereby expressly incorporated herein by reference.

### Equivalents

Those skilled in the art will recognize, or be able to ascertain, using no more than routine experimentation, many equivalents to specific embodiments of the invention described specifically herein. Such equivalents are intended to be encompassed in the scope of the following claims.

## Claims

1. A compound having the structure: 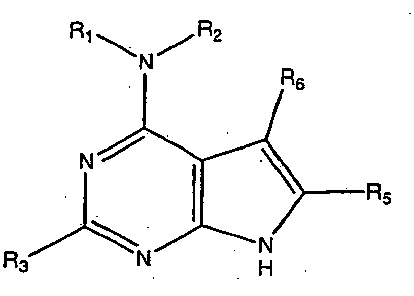 wherein R₁ is 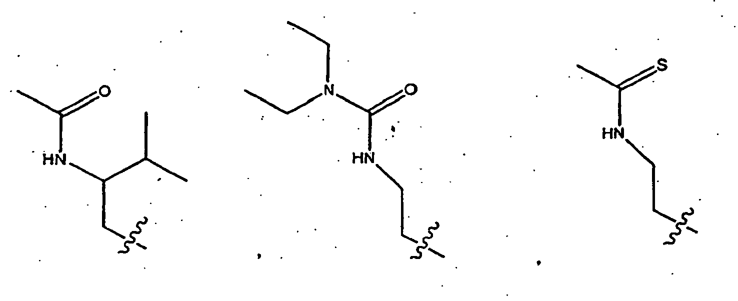 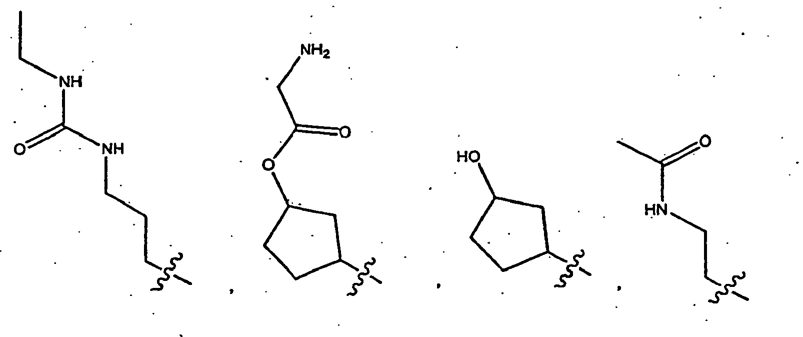 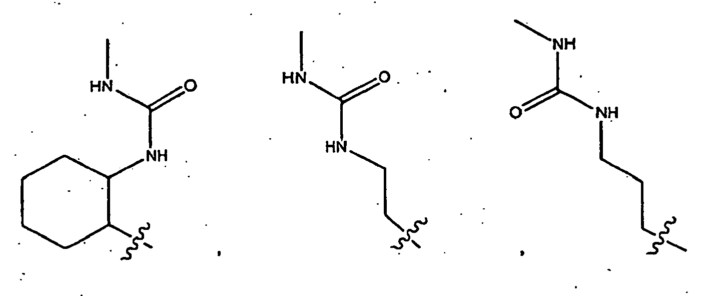 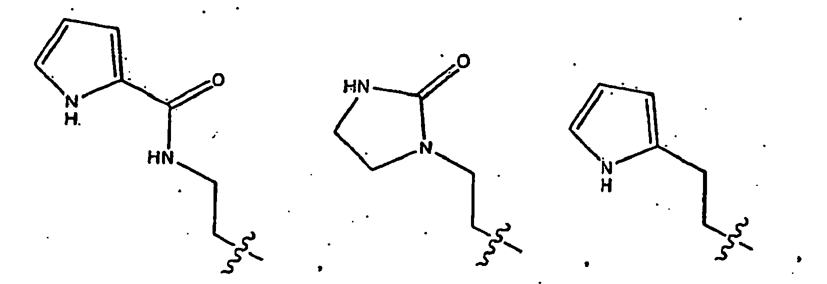 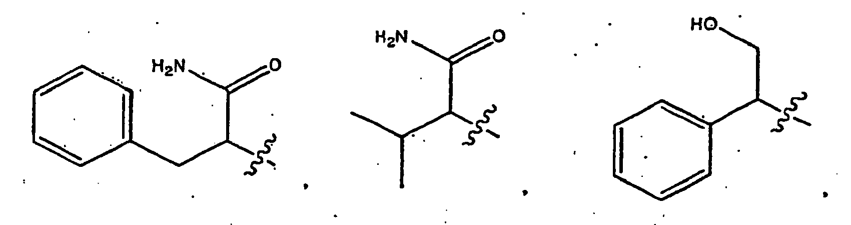 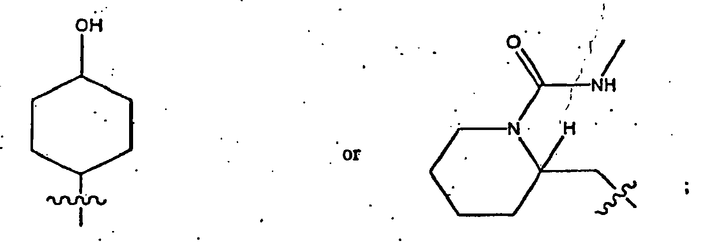 and
R₂ is H; or
NR₁R₂ together are 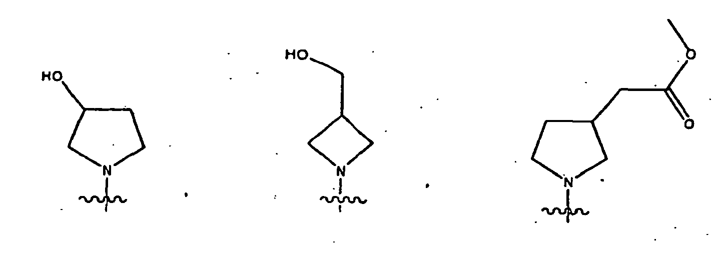 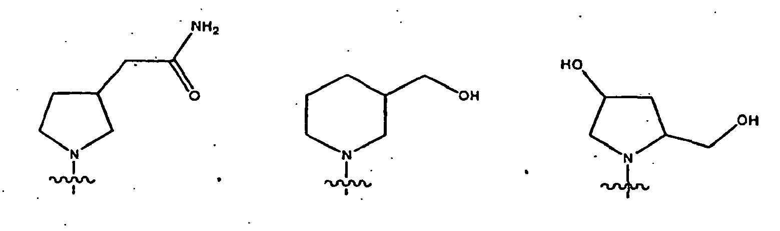 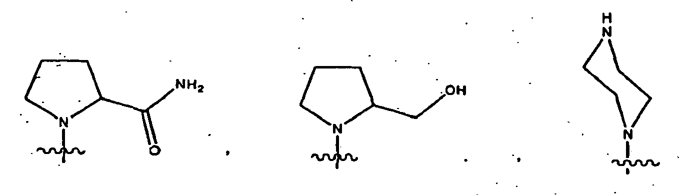 wherein R₃ is a substituted or unsubstituted phenyl, pyrrole, thiophene, furan, thiazole, imidazole, triazole, pyrazole, pyridine, 2(1H)-pyridone, 4 (1H)-pyridone, pyrazine, pyrimidine, pyridazine, isothiazole, isoxazole, oxazole, tetrazole, naphthyl, tetralin, benzofuran, benzothiophene, 2,3-dihydroindole, 1H-indole, indolyl, benzopyrazole, 1,3-benzodioxole, benzoxazole, purine, coumarin, chromone, quinolyl, tetrahydroquinoline, isoquinoline, benzimidazole, quinazoline, pteridine, 2(1H)-quinolone, 1 (2H)-isoquinolone, 1,4-benzisoxazine, benzothiazole, quinoxaline, quinoline-N-oxide, isoquinoline-N-oxide, quinoxaline-N-oxide, quinazoline-N-oxide, benzoxazine, phthalazine, or cinnoline;
wherein R₅ is H, CH₃, substituted or unsubstituted alkyl, aryl or phenyl,
or 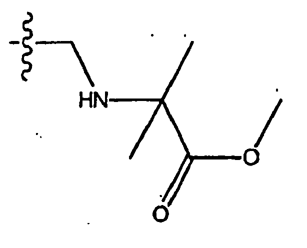 and
wherein R₆ is H, CH₃, substituted or unsubstituted alkyl, cycloalkyl,
wherein the substituent, when present, is halogen, hydroxyl, alkoxy, alkylcarbonyloxy, arylcarbonyloxy, alkoxycarboloxy, arylokycarbonyloxy, carboxylate, alkylcarbonyl, alkoxycarbonyl, aminocarbonyl, alkylthiocarbonyl, phosphate, phosphonato, phospinato, cyano, amino, acylamino, amidino, imino, sulfhydryl, alkylthio, arylthio, thiocarboxylate, sulfates, sulfonato, sulfamoyl, sulfonamido, nitro, trifluoromethyl, azido, heterocyclyl, or alkylaryl,
or a pharmaceutically acceptable salt thereof,
wherein when R₁ is 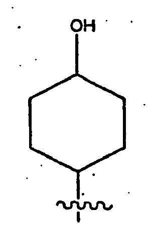 R3 is phenyl.;
R₅ is 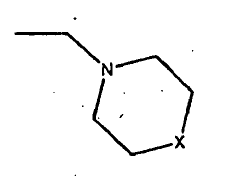 and R₆ is H
wherein X is O or S; and
wherein when R₁ is 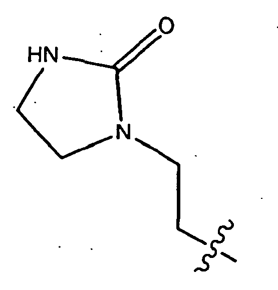 R₃ is phenyl;
R₅ is phenyl and
R₆ is H;
wherein when R₁ is 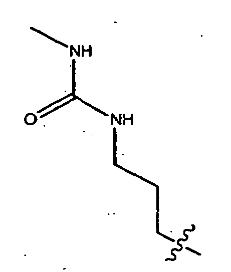 R₃ is 4-chlorophenyl;
R₅ and R₆ are each H;
wherein when R₁ is 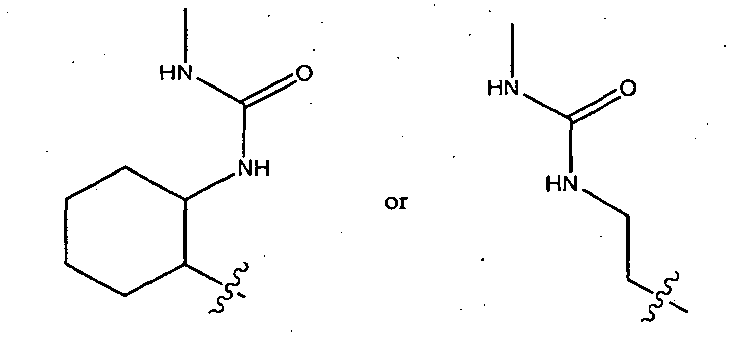 R₅ and R₆ are each independently H or alkyl;
wherein when R₁ is 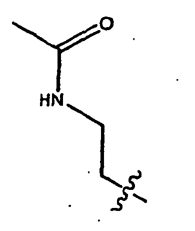 R₃ is phenyl; and
R₅ and R₆ are both H, or
R₃ is phenyl;
R₅ is 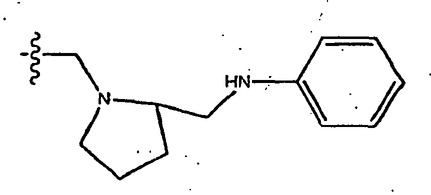 and R₆ is H, or
R₃ is 4-pyridyl;
R₅ is CH₃ and R₆ is

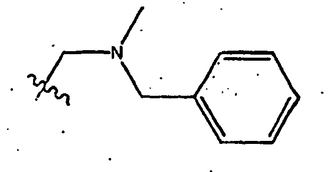

2. The compound of claim 1 having the structure: 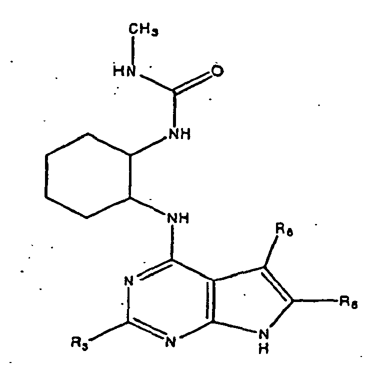 wherein R₃ is as defined in claim 1; wherein R₅ and R₆ are independently H, or alkyl.

3. The compound of claim 2, having the structure:

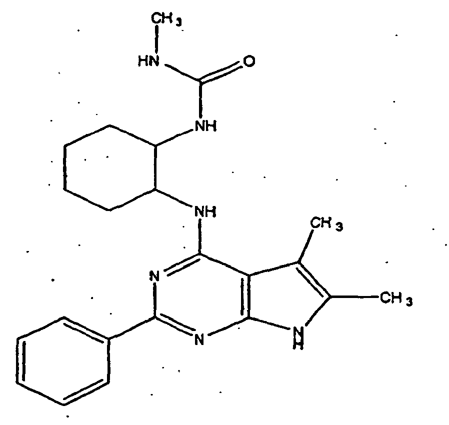

4. The compound of claim 3, having the structure:

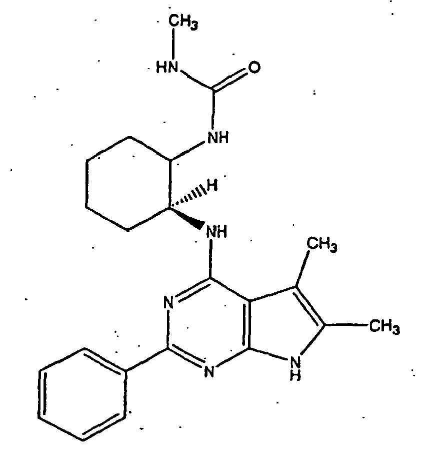

5. The compound of claim 3, having the structure:

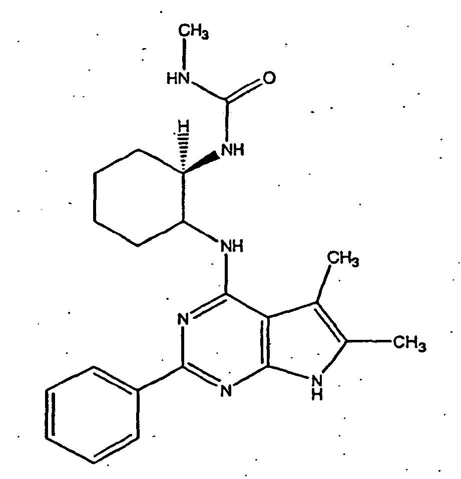

6. The compound of claim 3, having the structure:

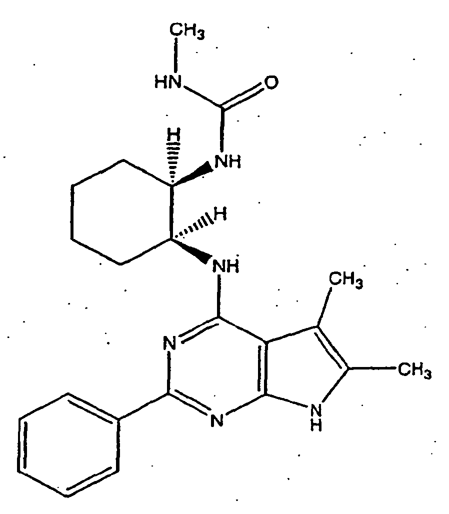

7. The compound of claim 3, having the structure:

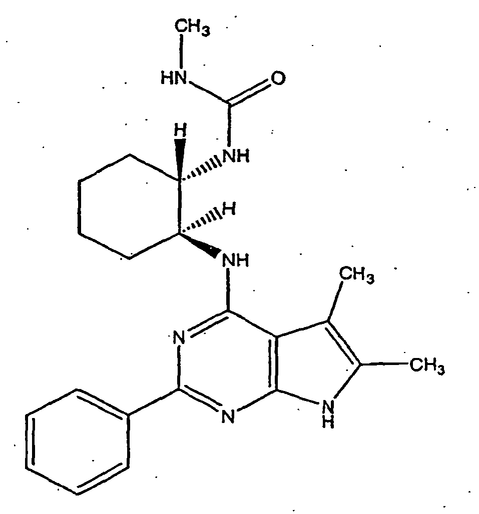

8. The compound of claim 1, having the structure:

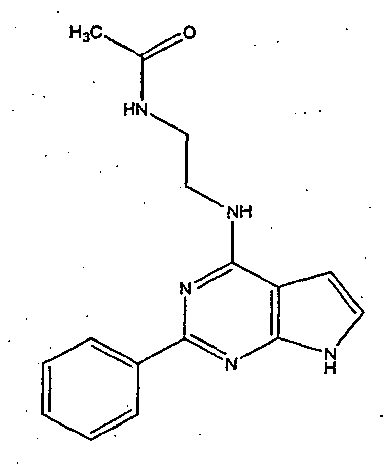

9. The compound of claim 1 having the structure: 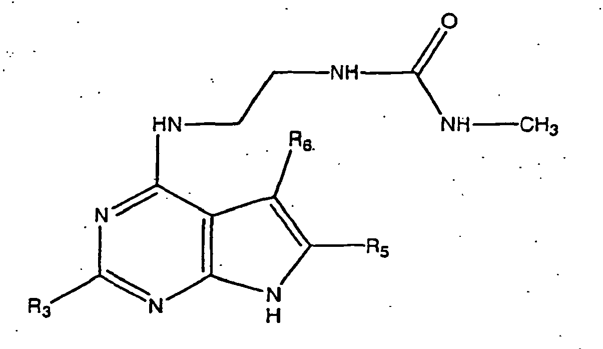 wherein R₃ is as defined in claim 1; wherein R₅ and R₆ are independently H, or alkyl.

10. The compound of claim 9, having the structure:

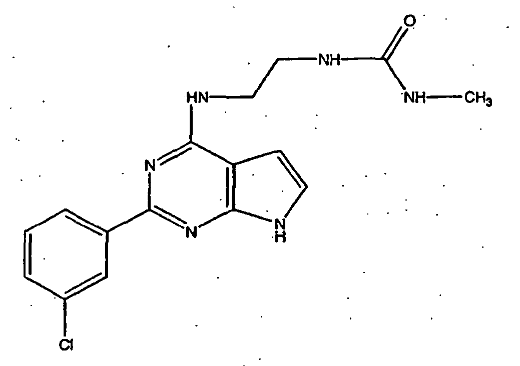

11. The compound of claim 1, having the structure: 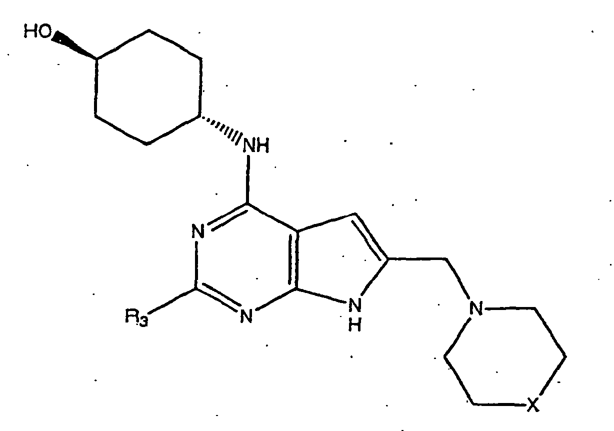 wherein R₃ is as defined in claim 1; wherein X is oxygen, or sulfur.

12. The compound of claim 11, having the structure:

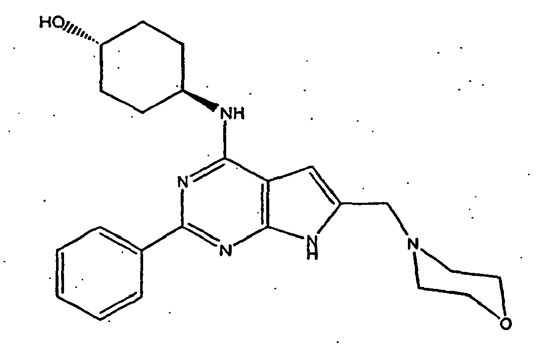

13. The compound of claim 11, having the structure:

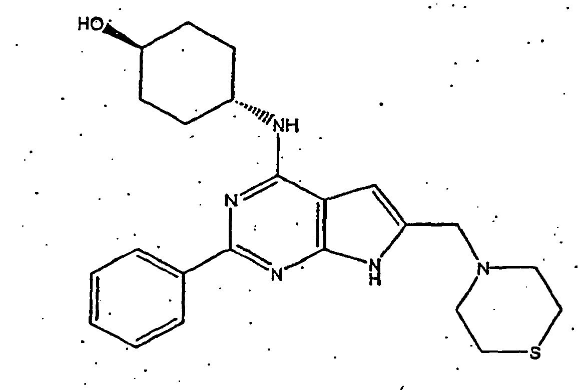

14. Use of a compound of claim 2, 8, 9 or 11 for manufacturing a medicament useful for treating a disease associated with an A₁ adenosine receptor in a subject in need of such treatment.

15. The use of claim 14, wherein said A₁ adenosine receptor is associated with cognitive disease, renal failure, cardiac arrhythmias, respiratory epithelia, transmitter release, sedation, vasoconstriction, bradycardia, negative cardiac inotropy and dromotropy, bronchoconstriction, neutrophil chemotaxis, reflux condition, or ulcerative condition.

16. A method for inhibiting the activity of an A₁ adenosine receptor in a cell in vitro, which comprises contacting said cell with a compound of claims 2, 8, 9 or 11.

17. The use of claim 14, wherein said disease is asthma, chronic obstructive pulmonary disease, allergic rhinitis, or an upper respiratory disorder.

18. A pharmaceutical composition comprising the compound of any one of claims 2, 8, 9 or 11, a pharmaceutically acceptable carrier and at least one of a steroid, β2 agonist, glucocorticoid, leukotriene antagonist, or anticholinergic agonist.

19. A pharmaceutical composition comprising a therapeutically effective amount of the compound of claims 2, 8, 9 or 11 and a pharmaceutically acceptable carrier.

20. The pharmaceutical composition of claim 19, wherein said therapeutically effective amount is effective to treat asthma, allergic rhinitis, or chronic obstructive pulmonary disease.

21. The compound of claim 1 having the structure: 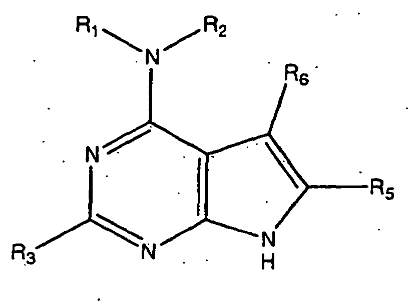 wherein NR₁R₂ is (*D*)-2-aminocarbonyl N-pyrrolidinyl, (*D*)-2-hydroxymethyl N-pyrrolidinyl, (*D*)-2-hydroxymethyl-trans-4-hydroxy N-pyrrolidinyl, or N-piperazinyl;
wherein R₃ is as defined in claim 1;
wherein R₅ is H, phenyl or 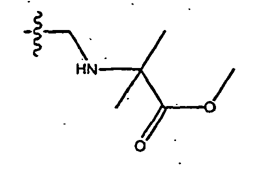 wherein R₆ is H, alkyl or cycloalkyl.

22. The compound of claim 1 having the structure: 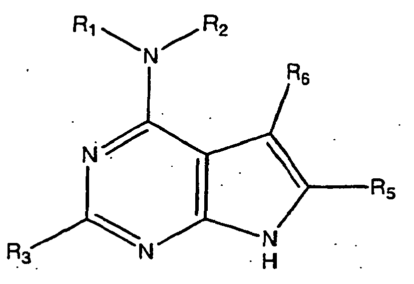 wherein NR₁R₂ is 3-hydroxymethyl N-piperidinyl;
wherein R₃ is a 4-pyridyl;
wherein R₅ is H or alkyl; and
wherein R₆ is H or alkyl.

23. The compound of claim 21, having the structure:

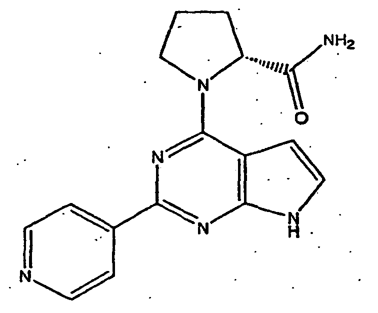

24. The compound of claim 21, having the structure:

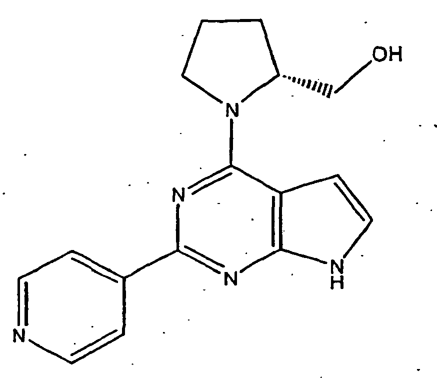

25. The compound of claim 21, having the structure:

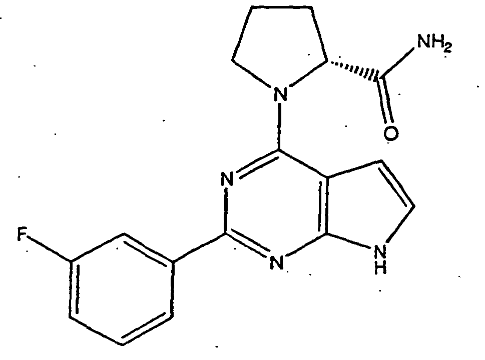

26. The compound of claim 21, having the structure:

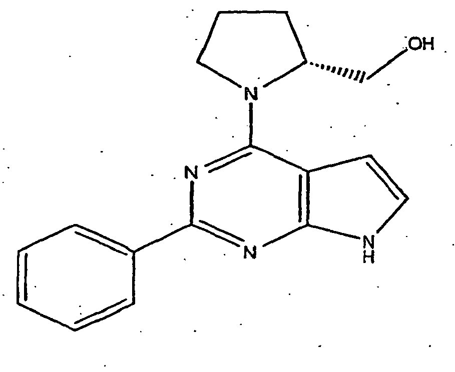

27. The compound of claim 21, having the structure:

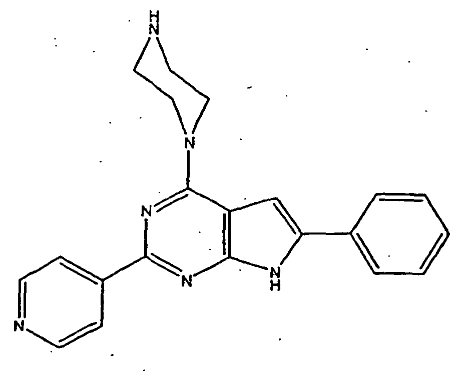

28. The compound of claim 21, having the structure:

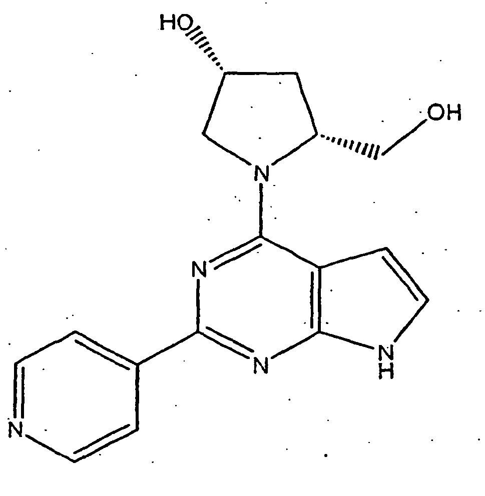

29. The compound of claim 1, having the structure:

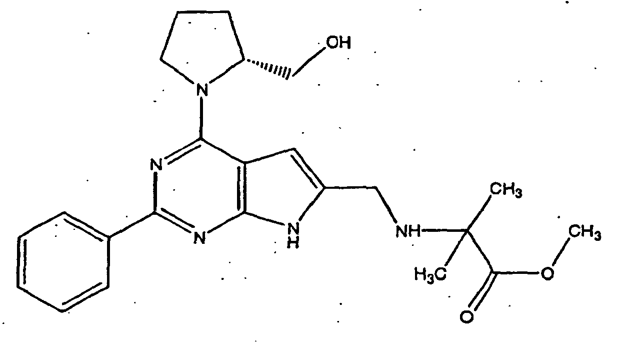

30. The compound of claim 22, having the structure:

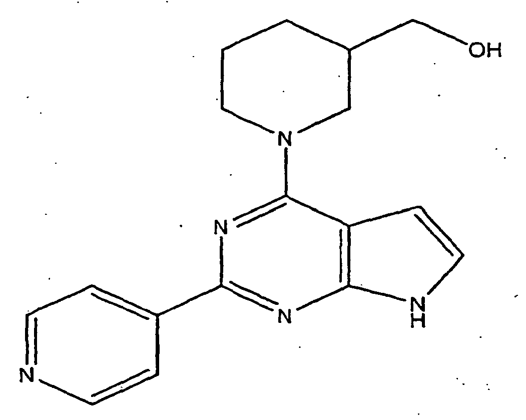

31. The compound of claim 30, having the structure:

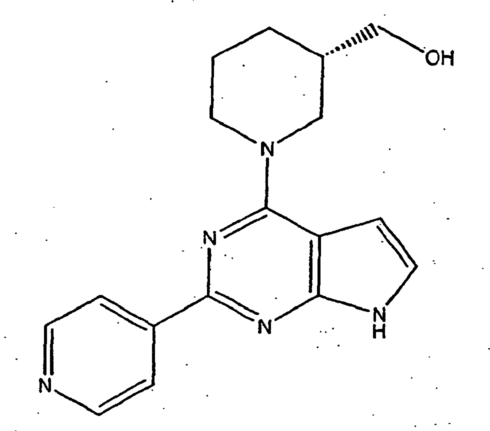

32. The compound of claim 30, having the structure:

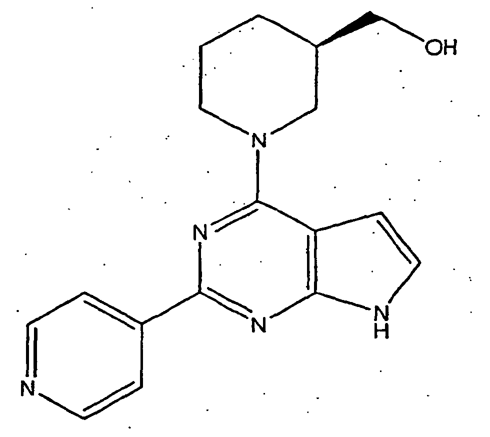

33. The compound of claim 1 having the structure: 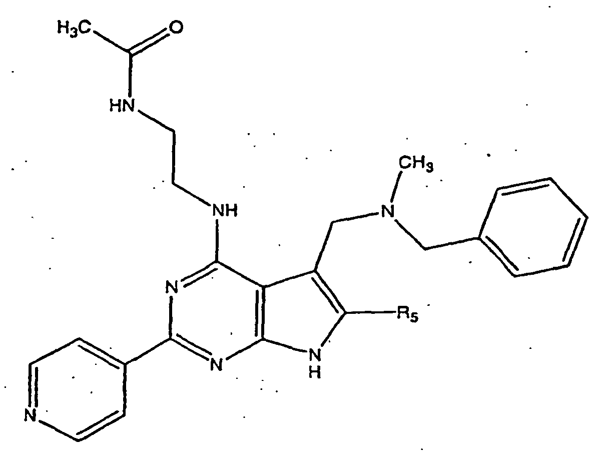 wherein R₅ is H, or methyl.

34. The compound of claim 33, having the structure:

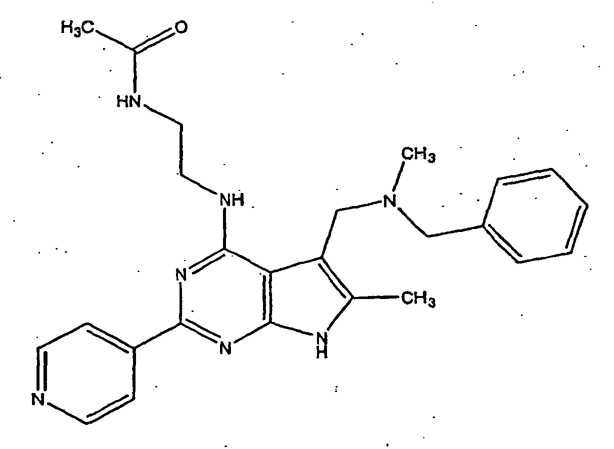

35. The compound of claim 33, having the structure:

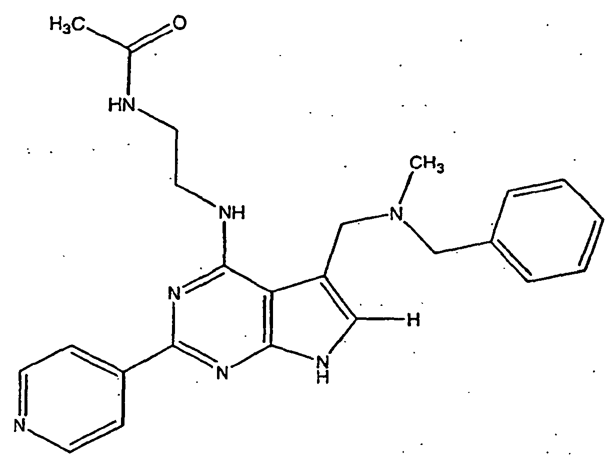

36. Use of a compound of claim 21, 22 or 33 for manufacturing a medicament useful for treating a disease associated with an A2a adenosine receptor in a subject in need of such treatment.

37. The use of claim 36, wherein said A₂ₐ adenosine receptor is associated with locomotor activity, vasodilation, platelet inhibition, neutrophil superoxide generation, cognitive disorder, senile dementia, or Parkinson's disease.

38. The use of claim 36, wherein the compound treats said diseases by stimulating adenylate cyclase.

39. A method for inhibiting the activity of an A2a adenosine receptor in a cell in vitro, which comprises contacting said cell with a compound of claims 21, 22 or 33.

40. A pharmaceutical composition comprising a therapeutically effective amount of the compound of claims 21, 22 or 33 and a pharmaceutically acceptable carrier.

41. The pharmaceutical composition of claim 40, wherein said therapeutically effective amount is effective to treat Parkinson's disease and diseases associated with locomotor activity, vasodilation, platelet inhibition, neutrophil superoxide generation, cognitive disorder, or senile dementia.

42. A pharmaceutical composition comprising any of the compounds of claims 21, 22 or 33, a pharmaceutically acceptable carrier and at least one dopamine enhancer.

43. A pharmaceutical composition comprising any of the compounds of claims 21, 22 or 33, a pharmaceutically acceptable carrier and at least one cytotoxic agent.

44. A pharmaceutical composition comprising any of the compounds of claims 21, 22 or 33, a pharmaceutically acceptable carrier and at least one of a prostaglandin agonist, a muscarinic agonist, or a β-2 antagonist.

45. A method of preparing the compound of claim 33, comprising the steps of
a) reacting 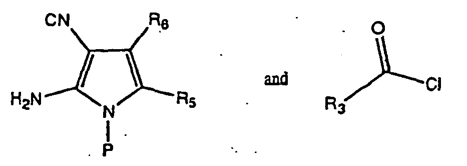 to provide 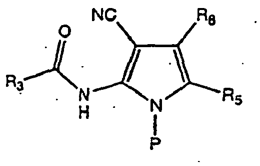 wherein P is a removable protecting group;
b) treating the product of step a) under cyclization conditions to provide 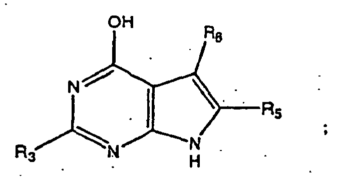
c) treating the product of step b) under suitable conditions to provide 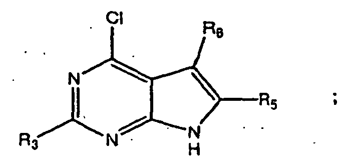 and
d) treating the chlorinated product of step c) first with dimethylamine and formaldehyde, then with N-methyl benzylamine and finally with NH₂R₁ to provide 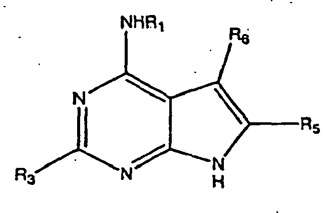
wherein R₁ is acetamido ethyl; wherein R₃ is 4-pyridyl;
wherein R₅ is H or methyl; wherein R₆ is N-methyl-N-benzyl aminomethyl.

46. The compound of claim 1 having the structure:
wherein R₁ is 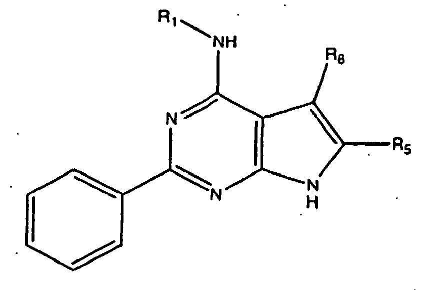 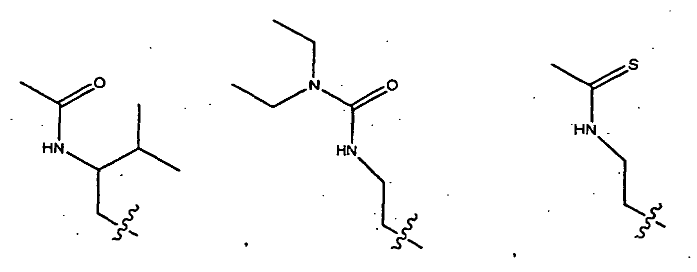 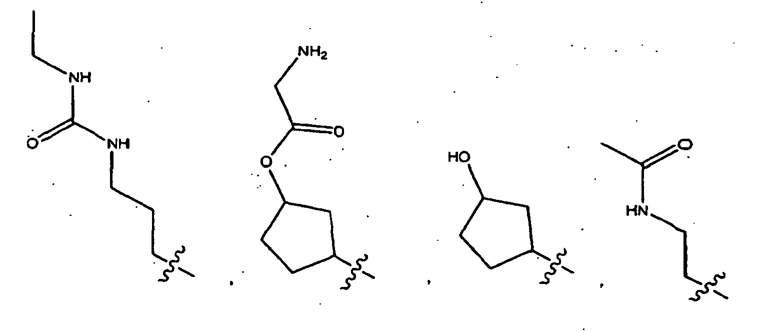 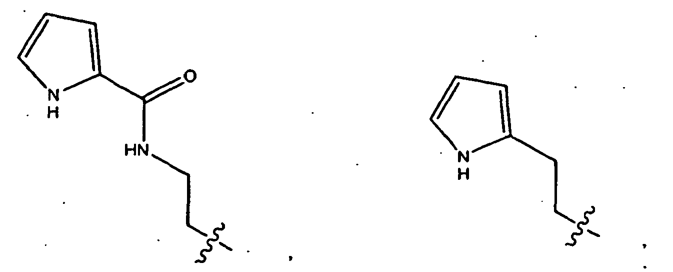 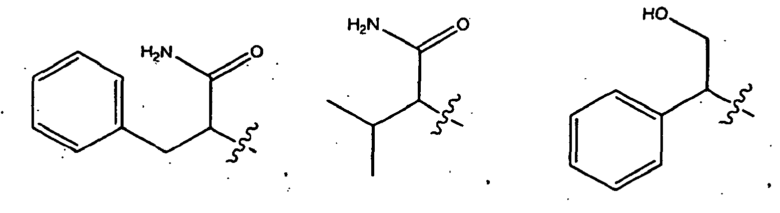 or 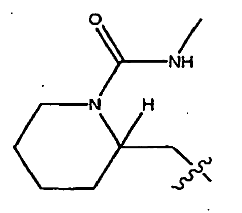 R₅ and R₆ are independently H, substituted or unsubstituted alkyl, or aryl,
wherein the substituent, when present, is as defined in claim 1.
wherein when R₁ is 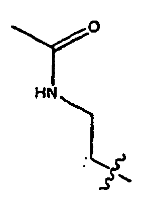 R₅ is 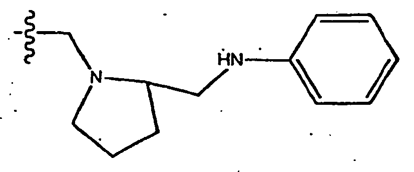 and R₆ is H.

47. The compound of claim 46, having the structure:

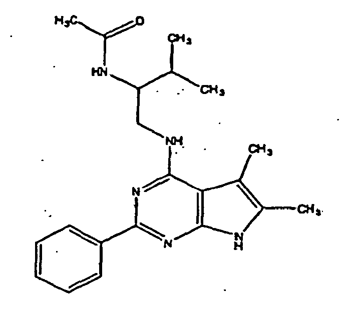

48. The compound of claim 46, having the structure:

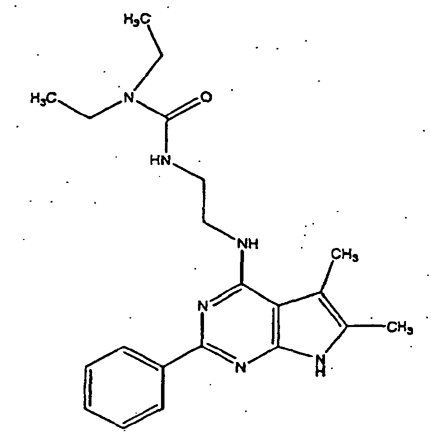

49. The compound of claim 46, having the structure:

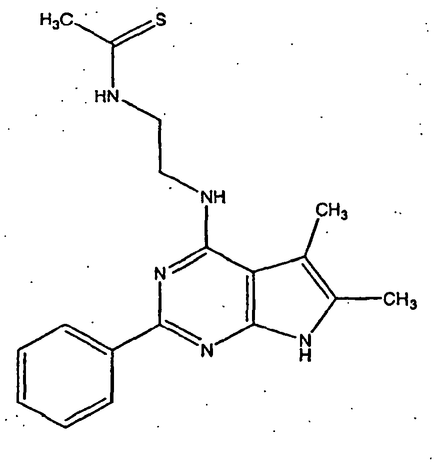

50. The compound of claim 46, having the structure:

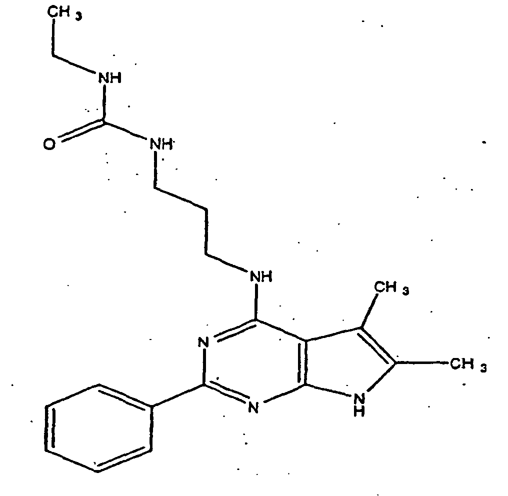

51. The compound of claim 46, having the structure:

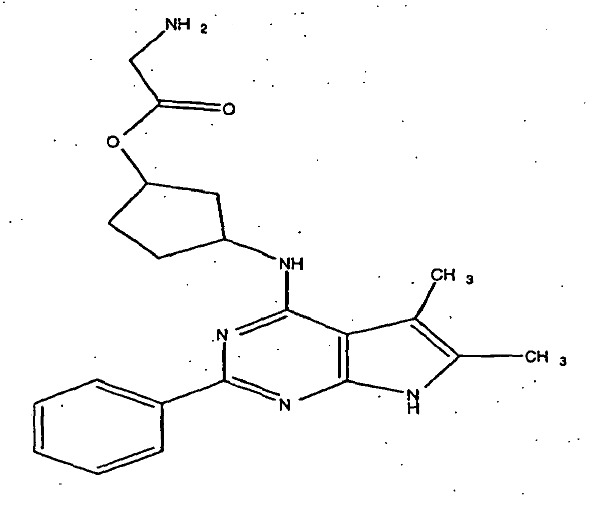

52. The compound of claim 46, having the structure:

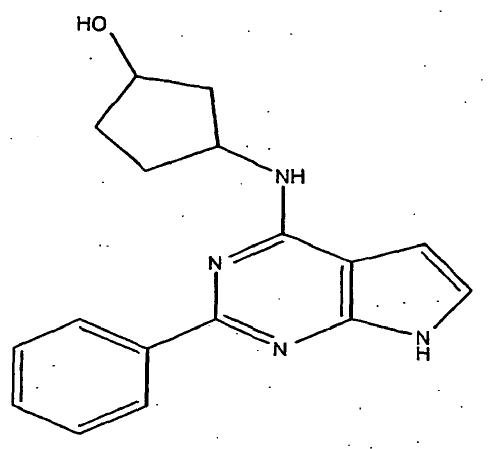

53. The compound of claim 52, having the structure:

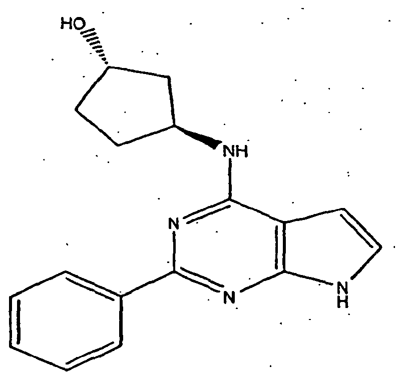

54. The compound of claim 52, having the structure:

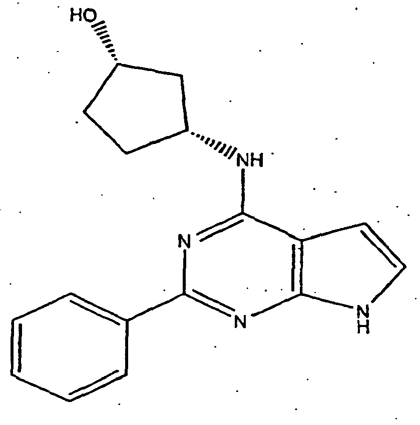

55. The compound of claim 52, having the structure:

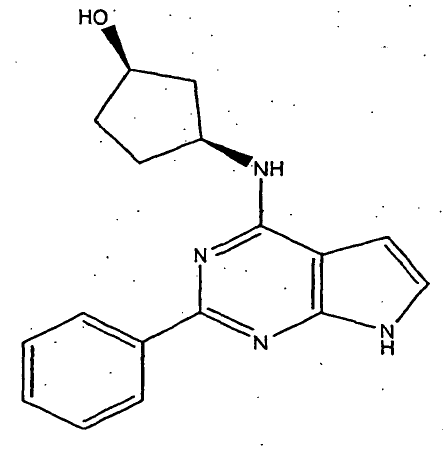

56. The compound of claim 52, having the structure:

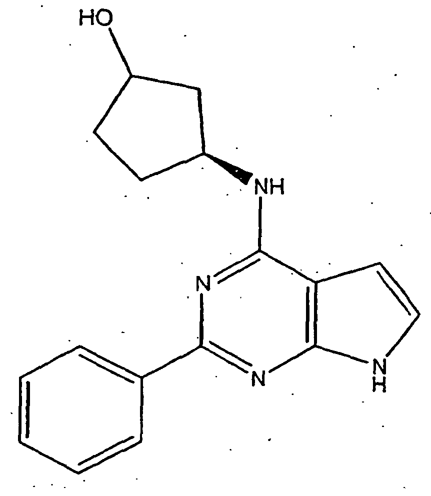

57. The compound of claim 46, having the structure:

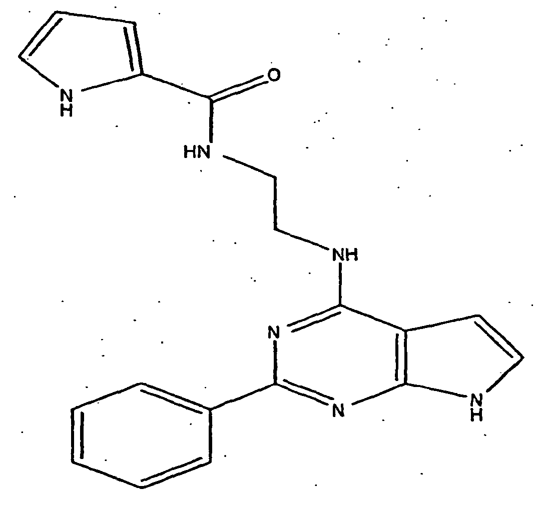

58. The compound of claim 1, having the structure:

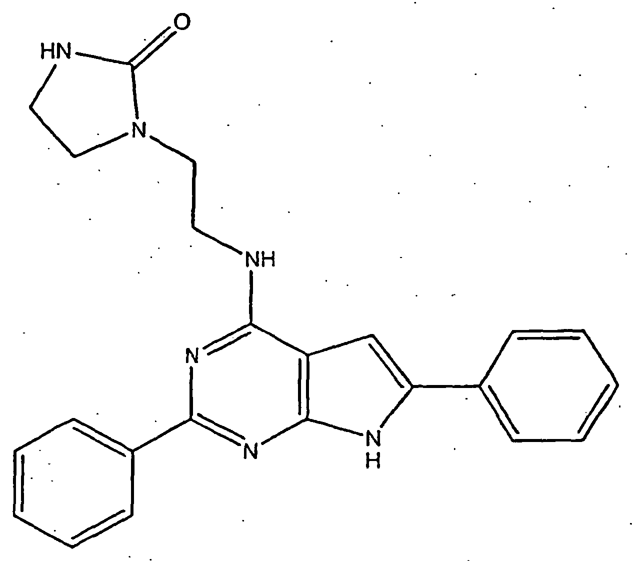

59. The compound of claim 46, having the structure:

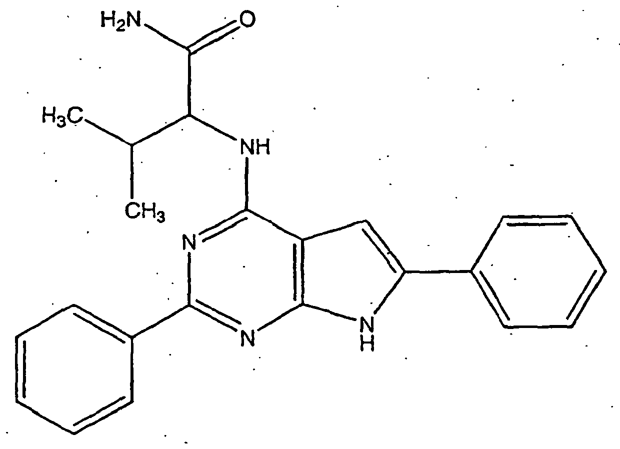

60. The compound of claim 46, having the structure:

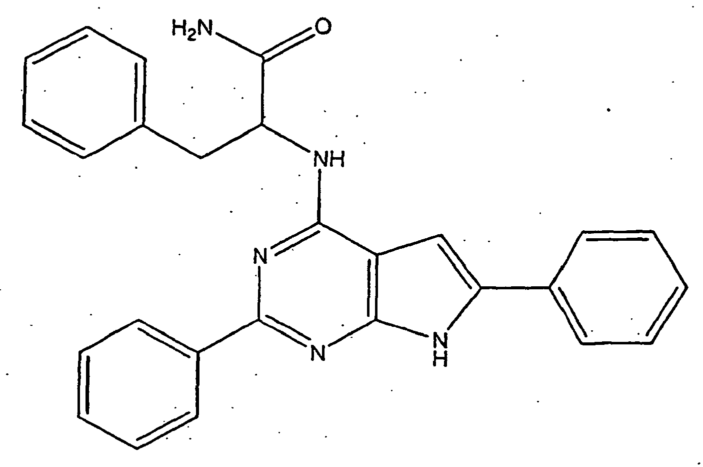

61. The compound of claim 46, having the structure:

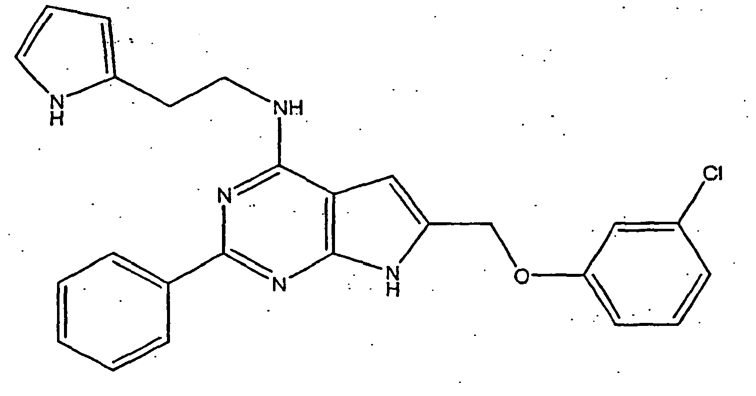

62. The compound of claim 46, having the structure:

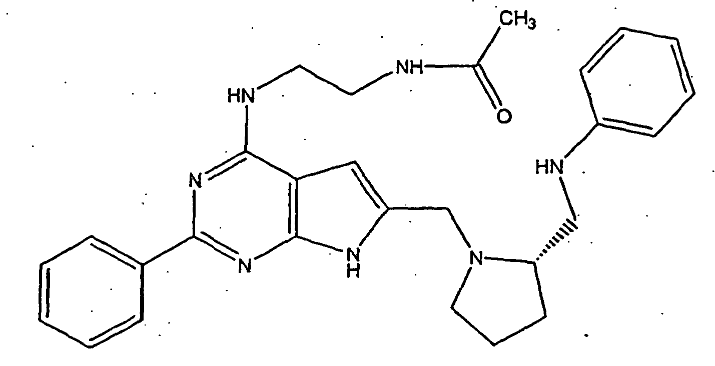

63. The compound of claim 62, having the structure:

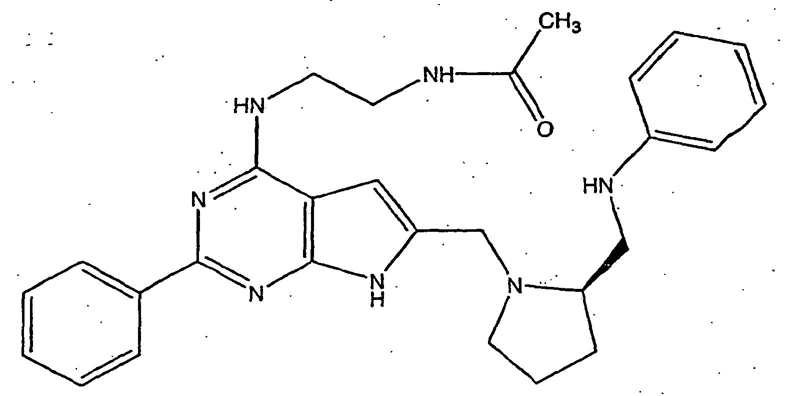

64. The compound of claim 46, having the structure:

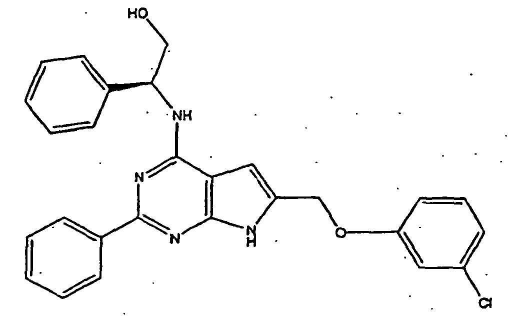

65. The compound of claim 64, having the structure:

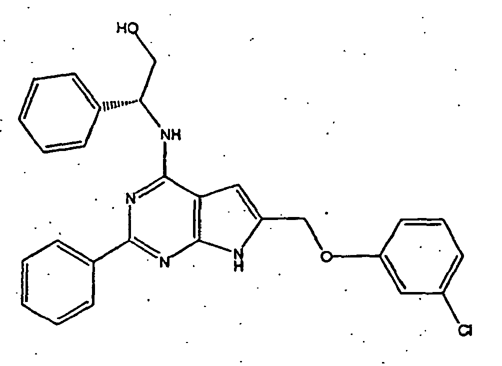

66. The compound of claim 46, having the structure:

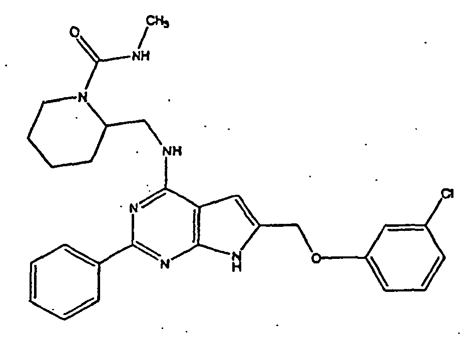

67. The compound of claim 66, having the structure:

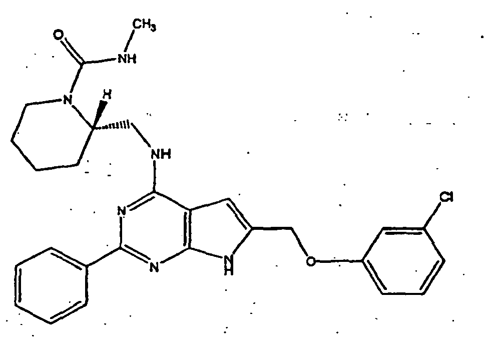

68. The compound of claim 66, having the structure:

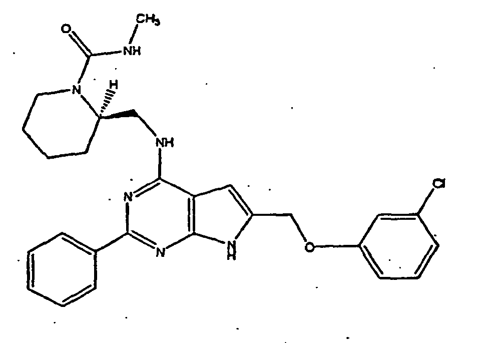

69. The compound of claim 1 having the structure: 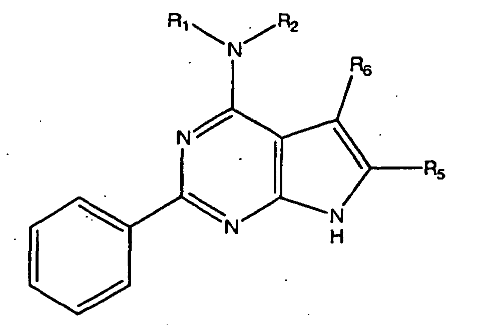 wherein R₁, R₂ and the nitrogen together are 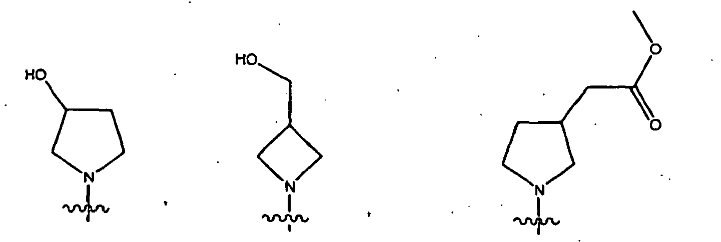 ; and 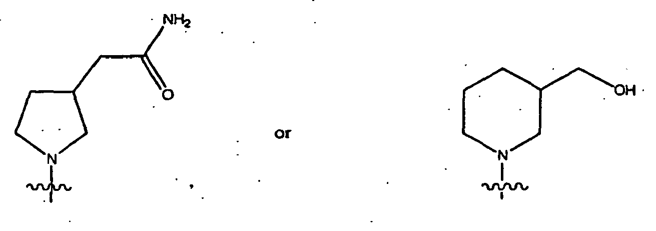 wherein R₅ and R₆ are independently H, substituted or unsubstituted alkyl, or aryl,
wherein the substituent, when present, is as defined in claim 1,
wherein when R₁, R₂ and the nitrogen together are 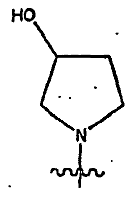 R₅ is phenyl and R₆ is H;
wherein when R₁, R₂, and the nitrogen together are 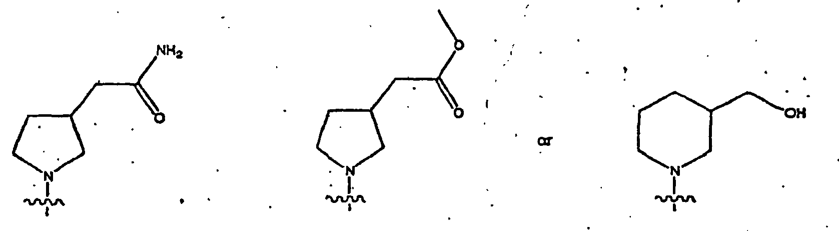 R₅ is 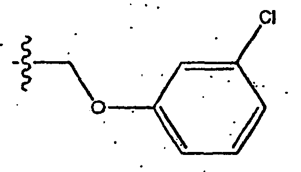 and R₆ is H; and
wherein when R₁, R₂ and the nitrogen together are 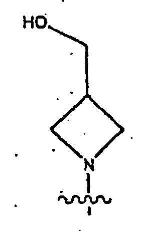 R₅ and R₆ are both H.

70. The compound of claim 69, having the structure:

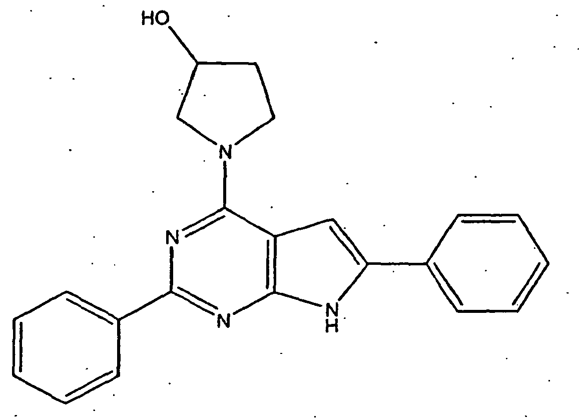

71. The compound of claim 70, having the structure:

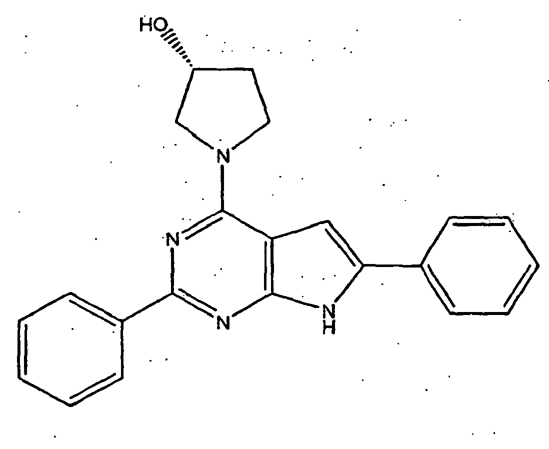

72. The compound of claim 70, having the structure:

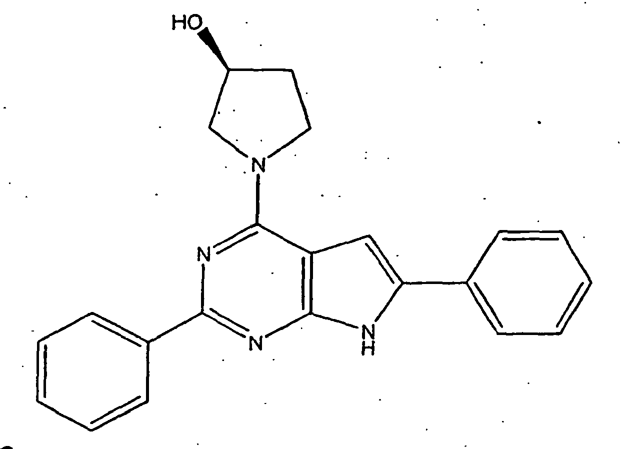

73. The compound of claim 69, having the structure:

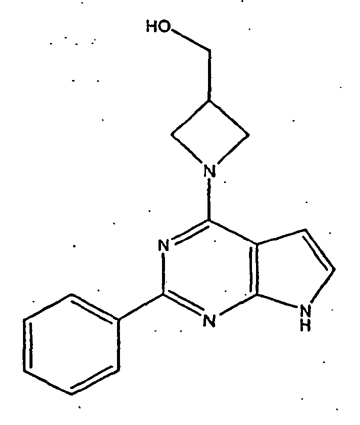

74. The compound of claim 69, having the structure:

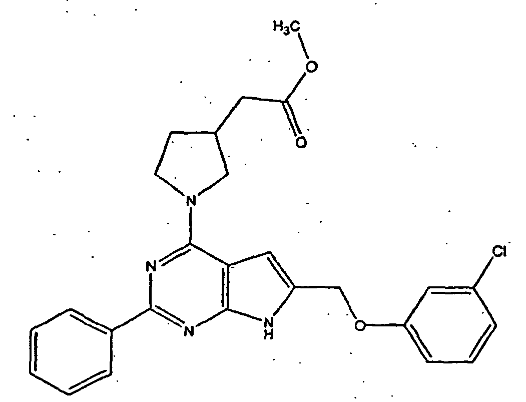

75. The compound of claim 74, having the structure:

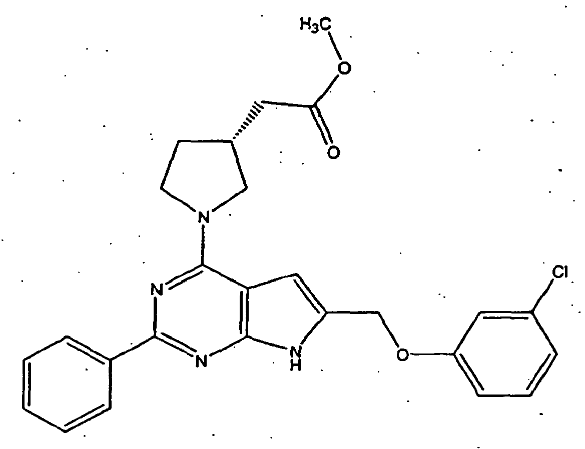

76. The compound of claim 74, having the structure:

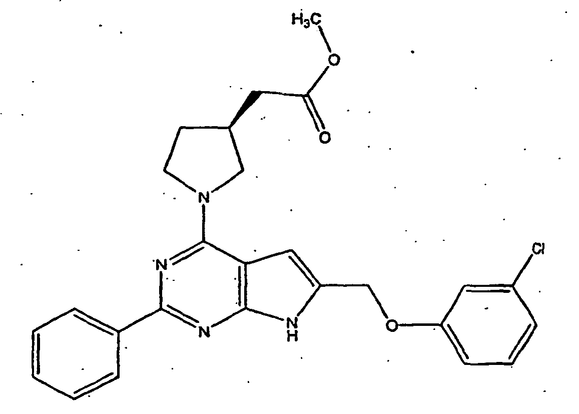

77. The compound of claim 69, having the structure:

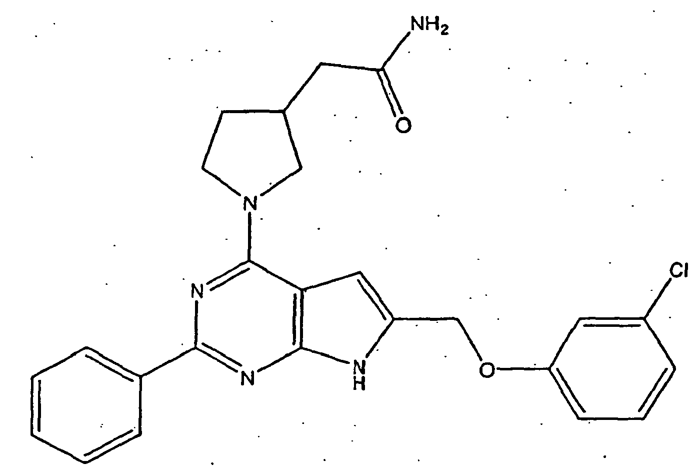

78. The compound of claim 77, having the structure:

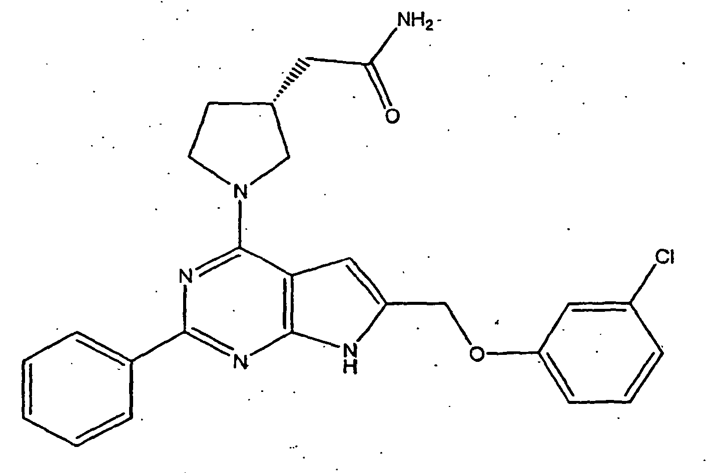

79. The compound of claim 77, having the structure:

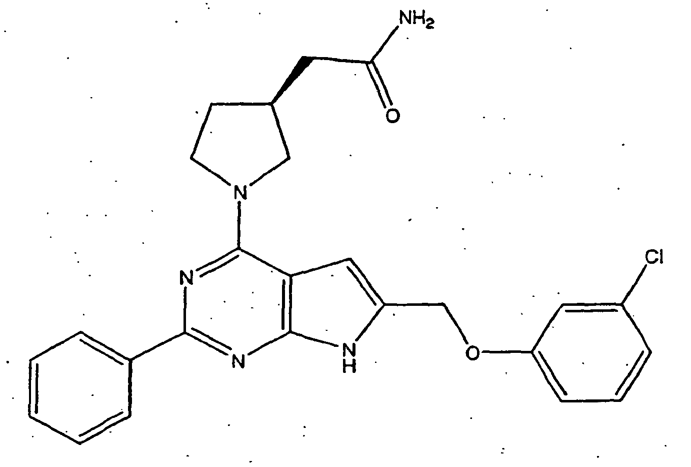

80. The compound of claim 69, having the structure:

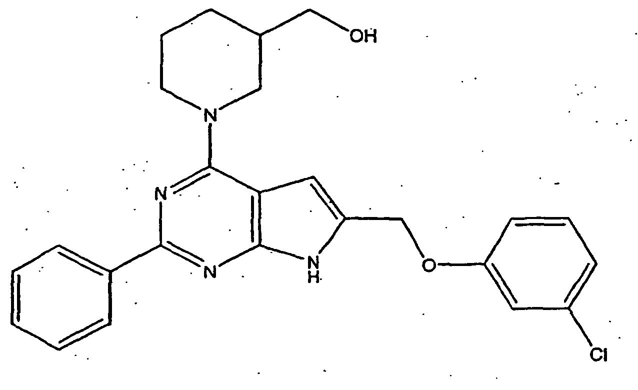

81. The compound of claim 80, having the structure:

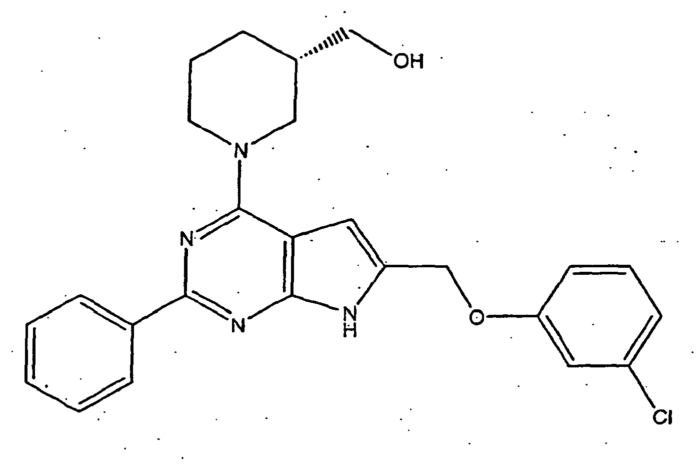

82. The compound of claim 80, having the structure:

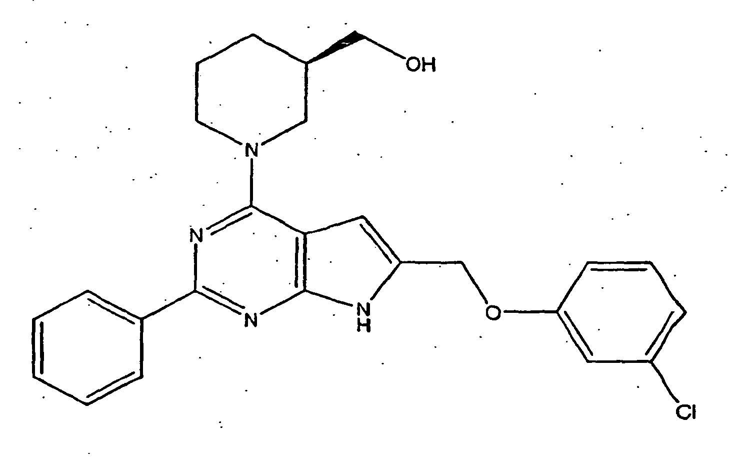

83. The compound of claim 1 having the structure:

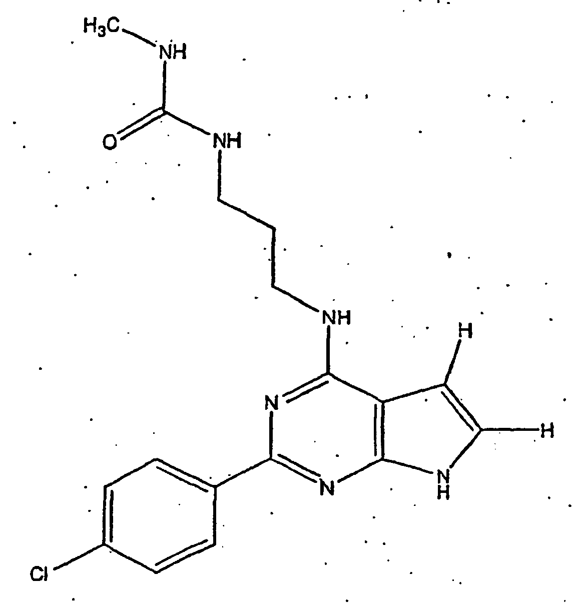

84. Use of a compound of claim 46, 58, 69 or 83 for manufacturing a medicament useful for treating a disease associated with an A3 adenosine receptor in a subject in need of such treatment.

85. The use of claim 84, wherein said A3 adenosine receptor is associated with asthma, hypersensitivity, rhinitis, hay fever, serum sickness, allergic vasculitis, atopic dermatitis, dermatitis, psoriasis, eczema, idiopathic pulmonary fibrosis, eosinophilic cholecystitis, chronic airway inflammation, hypereosinophilic syndromes, eosinophilic gastroenteritis, edema, urticaria, eosinophilic myocardial disease, episodic angioedema with eosinophilia, inflammatory bowel disease, ulcerative colitis, allergic granulomatosis, carcinomatosis, eosinophilic granuloma, familial histiocytosis, hypertension, mast cell degranulation, tumor, cardiac hypoxia, cerebral ischemia, diuresis, renal failure, neurological disorder, mental disorder, cognitive disorder, myocardial ischemia, bronchoconstriction, arthritis, autoimmune disease, Crohn's disease, Gravels disease, diabetes, multiple sclerosis, anaemia, fertility disorders, lupus erythematosus, reperfusion injury, brain arteriole diameter, the release of allergic mediators, sclerodernza, stroke, global ischemia, central nervous system disorder, cardiovascular disorder, renal disorder, inflammatory disorder, gastrointestinal disorder, eye disorder, allergic disorder, respiratory disorder, or immunological disorder.

86. A water-soluble prodrug, wherein said water-soluble prodrug is metabolized *in vivo* to produce the compound of claim 2, 8, 9, 11, 21, 22, 33, 46, 58, 69 or 83.

87. The prodrug of claim 86, wherein said prodrug is metabolized *in vivo* by esterase catalyzed hydrolysis.

88. A pharmaceutical composition comprising the prodrug of claim 86 and a pharmacetitically acceptable carrier.

89. The pharmaceutical composition of claim 88, wherein said pharmaceutical composition is an ophthalmic formulation.

90. The pharmaceutical composition of claim 88, wherein said pharmaceutical composition is an periocular, retrobulbar or intraocular injection formulation.

91. The pharmaceutical composition of claim 88, wherein said pharmaceutical composition is a systemic formulation.

92. A method for inhibiting the activity of an A3 adenosine receptor in a cell *in vitro,* which comprises contacting said cell with a compound of claim 46, 58,69 or 83.

93. The method of claim 16, 39 or 92 wherein the cell is a human cell.

94. Use of a compound of claim 46, 58, 69 or 83 for manufacturing a medicament useful for treating a gastrointestinal disorder in a subject.

95. The use of claim 94, wherein said disorder is diarrhea.

96. Use of a compound of claim 46, 58, 69 or 83 for manufacturing a medicament useful for treating respiratory disorder in a subject.

97. The use of claim 96, wherein said disorder is asthma, chronic obstructive pulmonary disease, allergic rhinitis, or an upper respiratory disorder.

98. Use of a compound of claim 46, 58, 69 or 83 for manufacturing a medicament useful for treating damage to the eye of a subject.

99. The use of claim 98, wherein said damage comprises retinal or optic nerve head damage.

100. The use of claim 98, wherein said damage is acute or chronic.

101. The use of claim 98, wherein said damage is the result of glaucoma, edema, ischemia, hypoxia or trauma.

102. The use of claim 14, 17, 36, 84, 94, 96 or 98 wherein the subject is human.

103. A pharmaceutical composition comprising any of the compounds of claims 46, 58, 69 or 83, a pharmaceutically acceptable carrier and at least one of a prostaglandin agonist, a muscarinic agonist, or a β-2 antagonist.

104. A pharmaceutical composition comprising a therapeutically effective amount of the compound of claims 46, 58, '69 or 83 and a pharmaceutically acceptable carrier.

105. The pharmaceutical composition of claim 104, wherein said therapeutically effective amount is effective to treat a respiratory disorder or a gastrointestinal disorder.

106. The pharmaceutical composition of claim 105, wherein said gastrointestinal disorder is diarrhea.

107. The pharmaceutical composition of claim 105, wherein said respiratory disorder is asthma, allergic rhinitis, or chronic obstructive pulmonary disease.

108. The pharmaceutical, composition of claim 40 or 104, wherein said pharmaceutical composition is an ophthalmic formulation.

109. The pharmaceutical composition of claim 19, 40 or 104, wherein said pharmaceutical composition is an periocular retrobulbar or intraocular injection formulation

110. The pharmaceutical composition of claim 19, 40 or 104; wherein said pharmaceutical composition is a systemic formulation.

111. The pharmaceutical composition of claim 19, 40 or 104, wherein said pharmaceutical composition is a surgical irrigating solution.

112. A method of preparing the compound of claim 1, comprising the steps of
a) reacting 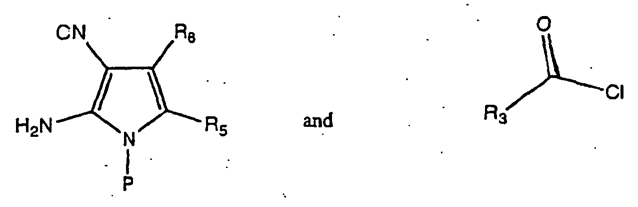 to provide 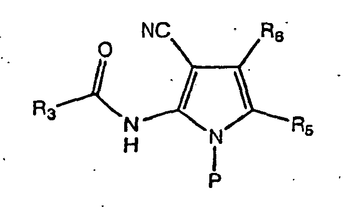 wherein P is a removable protecting group;
b) treating the product of step a) under cyclization conditions to provide 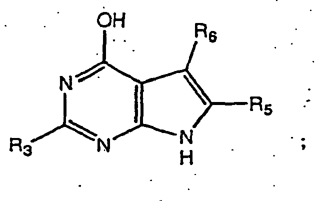
c) treating the product of step b) under suitable conditions to provide 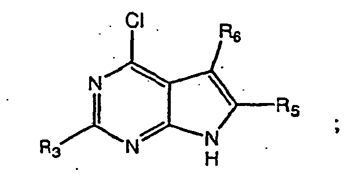 and
d) treating the chlorinated product of step c) with NHR₁R₂ to provide 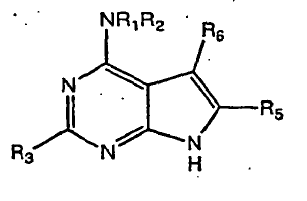 wherein
R₁ is 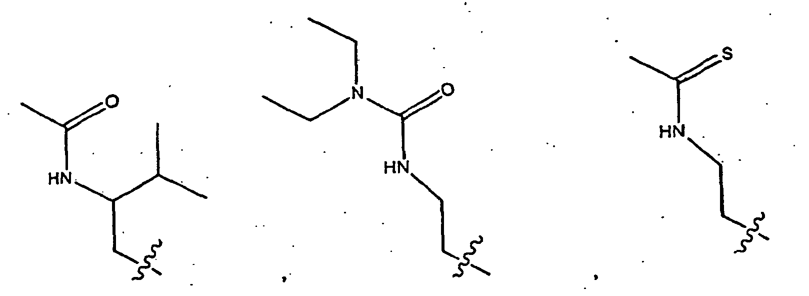 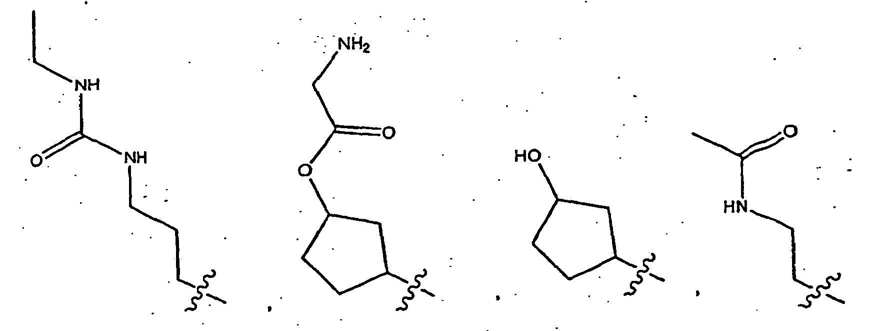 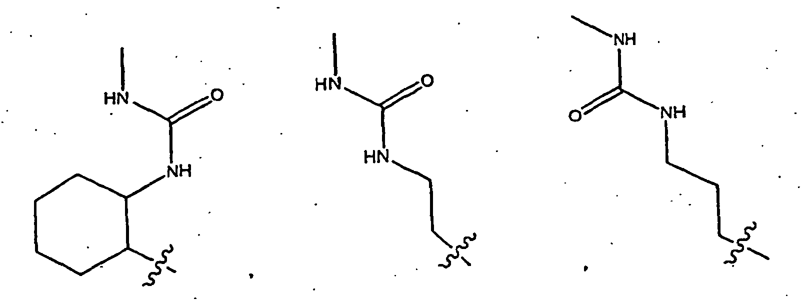 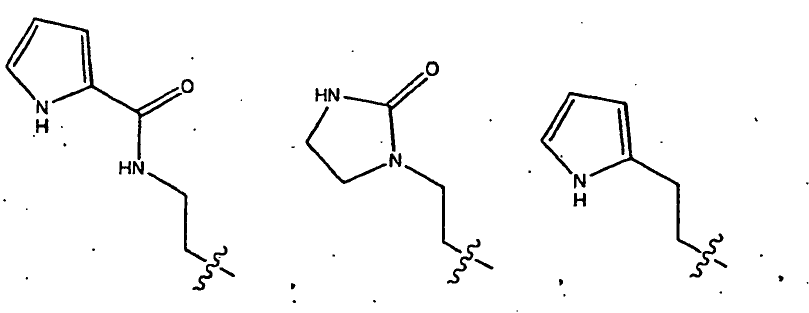 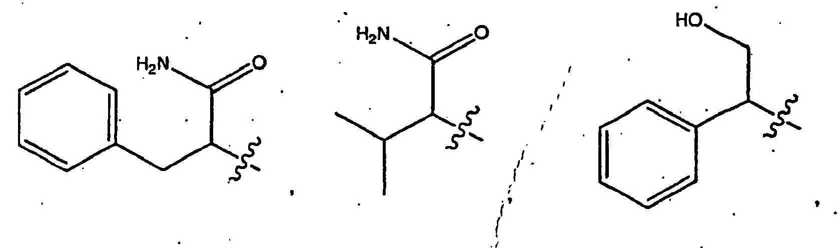 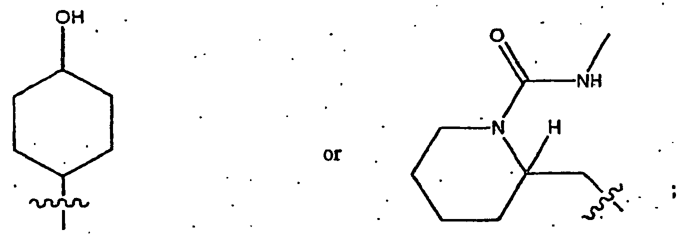 and
R₂ is H; or
NR₁R₂ together are 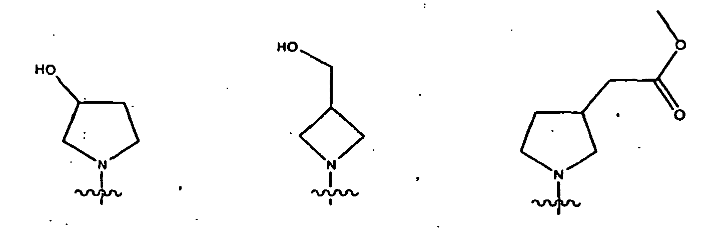 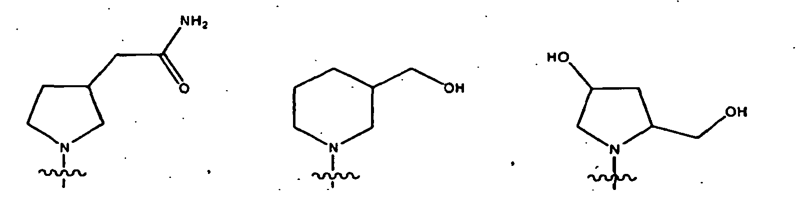 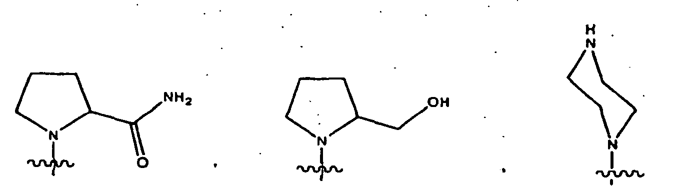 wherein R₃ is as defined in claim 1;
wherein R₅ is H, CH₃, substituted or unsubstituted alkyl, aryl or phenyl,
wherein the substituent, when present, is as defined in claim 1. 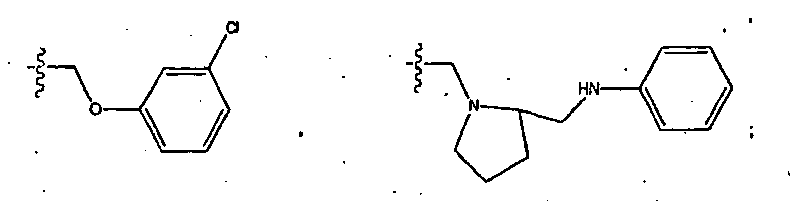 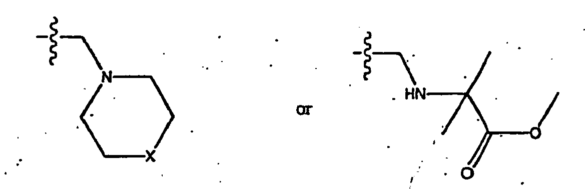
wherein X is O or S;
and
wherein R₆ is H, CH₃, substituted or unsubstituted alkyl, cycloalkyl or 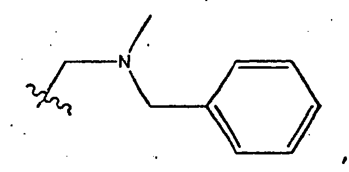 wherein when R₁ is 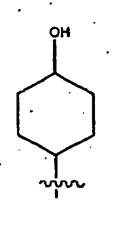 R₃ is phenyl,
R₅ is 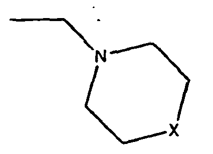 and R₆ is H;
wherein X is O or S;
wherein when R₁ is 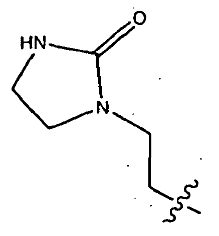 R₃ is phenyl;
R₅ is phenyl and R₆ is H;
wherein when R₁ is 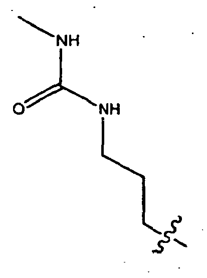 R₃ is 4-chlorophenyl;
R₅ and R₆ are each H;
wherein when R₁ is 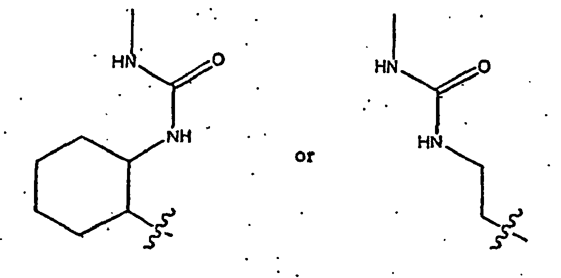 R₅ and R₆ are each independently H or alkyl;
wherein when R₁ is 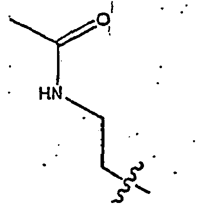 R₃ is phenyl; and
R₅ and R₆ are both H, or
R₃ is phenyl;
R₅ is 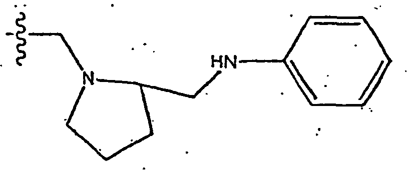 and R₆ is H, or
R₃ is 4-pyridyl;
R₅ is CH₃ and R₆ is

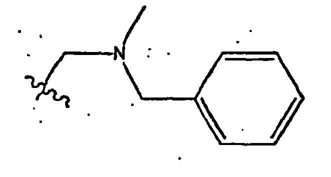

113. Use of the compound of any of claims 2, 8, 9 or 11 for manufacturing a medicament useful for treating a disease associated with an A1 adenosine receptor in a subject in need of such treatment, wherein the disease associated with the Al adenosine receptor is antidiuresis, bradycardia, bronchitis, bronchoconstriction, Alzheimer's disease, cardiac arrythmias, cardiac hypoxia, hypertension, inflammation, negative cardiac inotropy and dromotropy, renal failure, sedation or is associated with transmitter release, respiratory epithelia, contraction of smooth muscle underlying respiratory epithelia, vasoconstriction or mast cell degranulation.

114. Use of the compound of any of claims 2, 8, 9 or 11 for manufacturing a medicament useful for enhancing the memory of a subject.

115. Use of the compound of any of claims 21, 22 or 33 for manufacturing a medicament useful for treating a disease associated with an A2a adenosine receptor in a subject in need of such treatment, wherein the disease associated with the A2a adenosine receptor is Parkinson's disease or glaucoma.

116. The compound of claim 21, wherein when NR₁R₂ together form a 2-aminocarbonyl-pyrrolidine ring, R₃ is substituted phenyl or unsubstituted pyridine,
wherein the substituent is halogen, hydroxyl, alkoxy, alkylcarbonyloxy, arylcarbonyloxy, alkoxycarbonyloxy, aryloxycarbonyloxy, carboxylate, alkylcarbonyl, alkoxycarbonyl, aminocarbonyl, alkylthiocarbonyl, phosphate, phosphonato, phospinato, cyano amino, alkylamino, dialkylamino, arylamino, diarylamino, alkylarylamino, acylamino, alkylcarbonylamino, arylcarbonylamino, carbamoyl, ureido, amidino, inino, sulfhydryl, alkylthio, arylthio, thiocarboxylate, sulfates, sulfonato, sulfamoyl, sulfonamido, nitro, trifluoromethyl, azido, heterocyclyl, or alkylaryl.

117. Use of the compound of any of claims 46, 58, 69 or 83 for manufacturing a medicament useful for treating a disease associated with an A₃ adenosine receptor in a subject in need of such treatment, wherein the disease associated with the A3 adenosine receptor is myocardial ischemia, bronchitis, or bronchoconstriction.

118. Use of the compound of any of claims 46, 58, 69 or 83 for manufacturing a medicament useful for treating inflammation of the eye associated with the A3 adenosine receptor in a subject.

119. Use of the compound of any of claims 46, 58, 69 or 83 for manufacturing a medicament useful for treating a disease associated with an A3 adenosine receptor in a subject in need of such treatment, wherein the disease associated with the A3 adenosine receptor is associated with mast cell degranulation.

120. Use of the compound of any of claims 46, 58, 69 or 83 for manufacturing a medicament useful for treating a disease associated with an A3 adenosine receptor in a subject in need of such treatment, wherein the disease associated with the A3 adenosine receptor is asthma, glaucoma, retinopathy, ocular ischemia, or macular degeneration.

## Patentansprüche

1. Verbindung mit der Struktur: 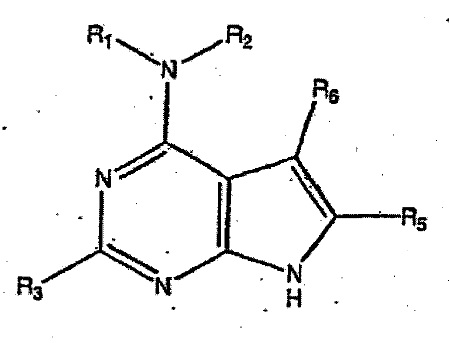 wobei R₁ 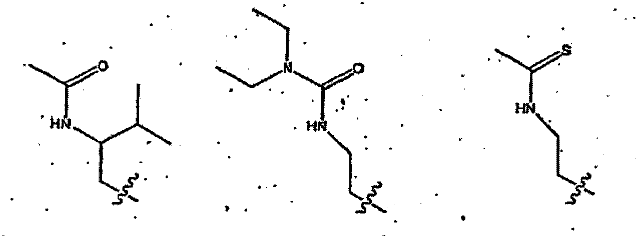 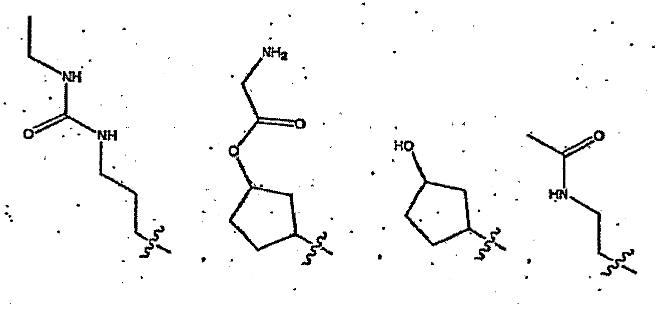 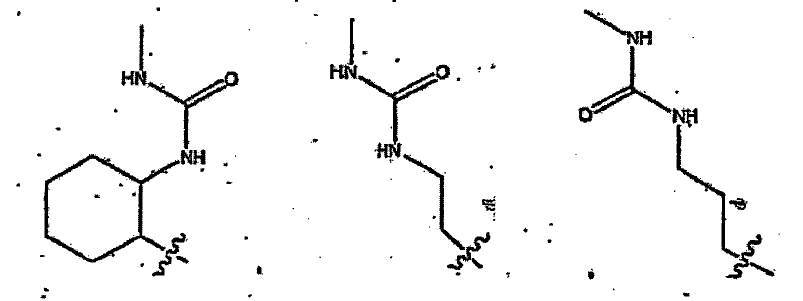 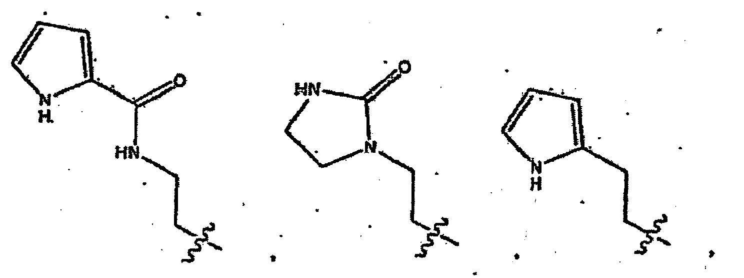 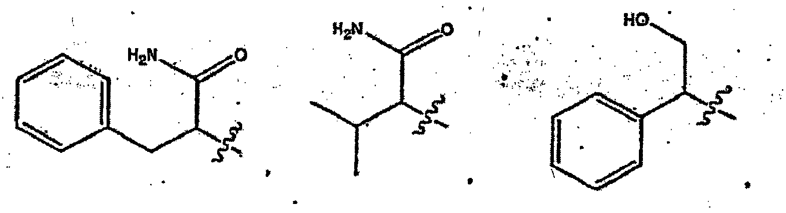 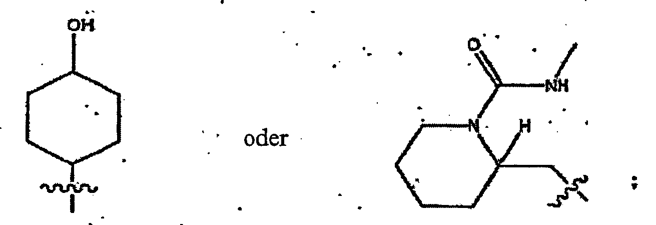 ist; und
R₂ für H steht; oder
NR₁R₂ zusammen 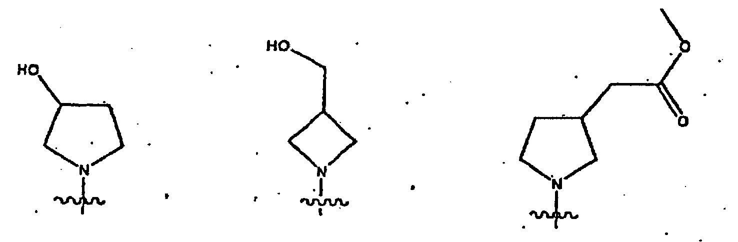 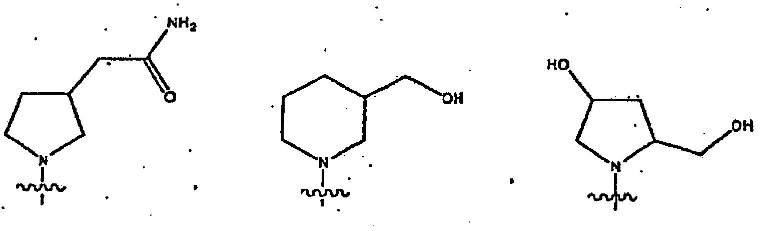 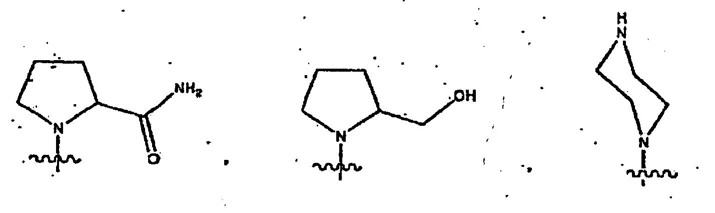 sind,
wobei R₃ ein substituiertes oder unsubstituiertes Phenyl, Pyrrol, Thiophen, Furan, Thiazol, Imidazol, Triazol, Pyrazol, Pyridin, 2(1H)-Pyridon, 4(1H)-Pyridon, Pyrazin, Pyrimidin, Pyridazin, Isothiazol, Isoxazol, Oxazol, Tetrazol, Naphthyl, Tetralin, Benzofuran, Benzothiophen, 2,3-Dihydroindol, 1H-Indol, Indolyl, Benzpyrazol, 1,3-Benzdioxol, Benzoxazol, Purin, Cumarin, Chromon, Chinolyl, Tetrahydrochinolin, Isochinolin, Benzimidazol, Chinazolin, Pteridin, 2(1H)-Chinolon, 1(2H)-Isochinolon, 1,4-Benzisoxazin, Benzothiazol, Chinoxalin, Chinolin-N-oxid, Isochinolin-N-oxid, Chinoxalin-N-oxid, Chinazolin-N-oxid, Benzoxazin, Phthalazin oder Cinnolin ist;
wobei R₅ für H, CH₃, substituiertes oder unsubstituiertes Alkyl, Aryl oder Phenyl, oder 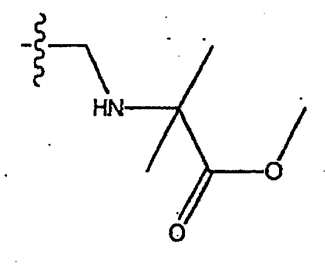 steht; und
wobei R₆ für H, CH₃, substituiertes oder unsubstituiertes Alkyl, Cycloalkyl steht,
wobei der Substituent, wenn vorhanden, Halogen, Hydroxyl, Alkoxy, Alkylcarbonyloxy, Arylcarbonyloxy, Alkoxycarbonyloxy, Aryloxycarbonyloxy, Carboxylat, Alkylcarbonyl, Alkoxycarbonyl, Aminocarbonyl, Alkylthiocarbonyl, Phosphat, Phosphonat, Phosphinat, Cyano, Amino, Acylamino, Amidino, Imino, Sulfhydryl, Alkylthio, Arylthio, Thiocarboxylat, Sulfate, Sulfonat, Sulfamoyl, Sulfonamido, Nitro, Trifluormethyl, Azido, Heterocyclyl oder Alkylaryl ist,
oder ein pharmazeutisch verträgliches Salz davon,
wobei, wenn R₁ 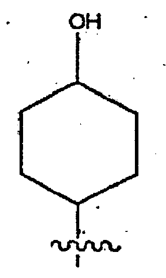 ist,
R₃ Phenyl ist;
R₅ 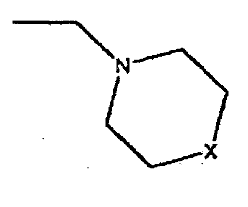 ist, und R₆ für H steht;
wobei X für O oder S steht; und
wobei, wenn R₁ 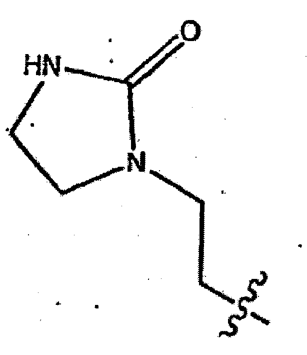 ist,
R₃ Phenyl ist;
R₅ Phenyl ist, und
R₆ für H steht;
wobei, wenn R₁ 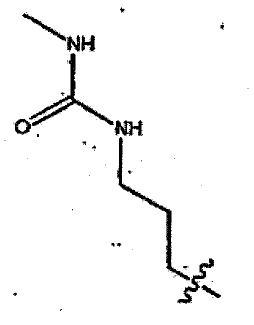 ist,
R₃ 4-Chlorphenyl ist;
R₅ und R₆ jeweils H darstellen;
wobei, wenn R₁ 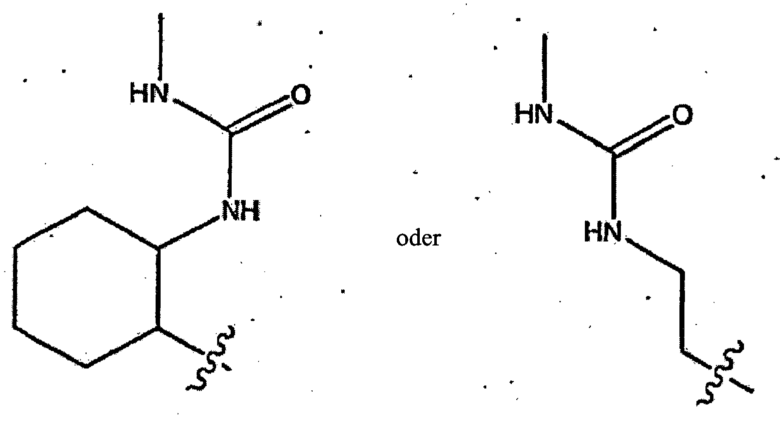 ist,
R₅ und R₆ jeweils unabhängig H oder Alkyl darstellen;
wobei, wenn R₁ 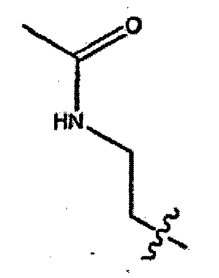 ist,
R₃ Phenyl ist; und
R₅ und R₆ beide H darstellen, oder
R₃ Phenyl ist;
R₅ 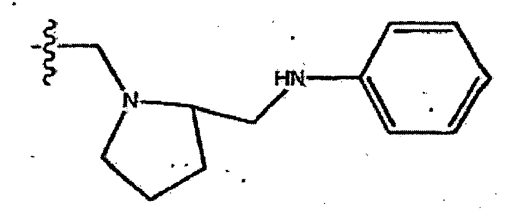 ist,
und R₆ für H steht, oder
R₃ 4-Pyridyl ist;
R₅ für CH₃ steht und R₆ 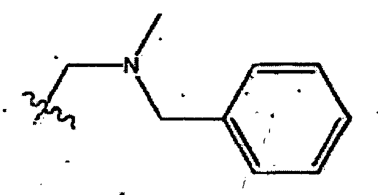 ist.

2. Verbindung gemäß Anspruch 1 mit der Struktur: 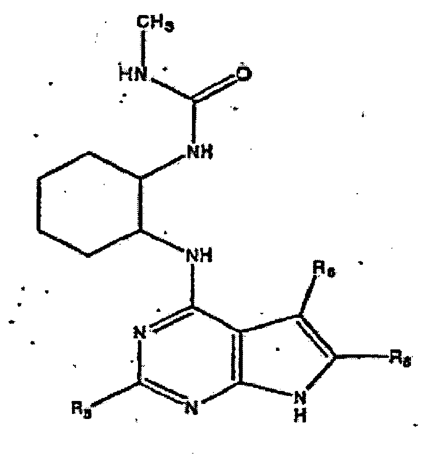 wobei R₃ wie in Anspruch 1 definiert ist; wobei R₅ und R₆ unabhängig H oder Alkyl darstellen.

3. Verbindung gemäß Anspruch 2 mit der Struktur:

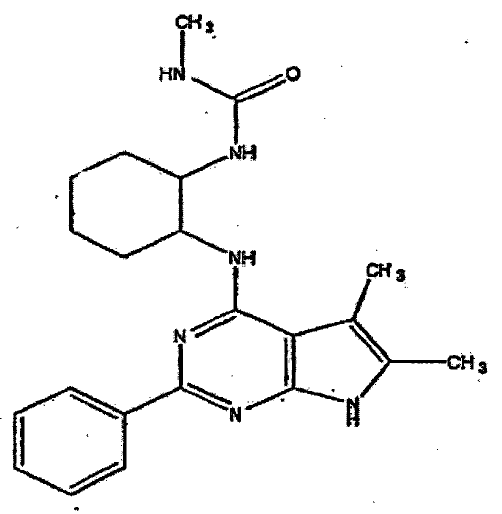

4. Verbindung gemäß Anspruch 3 mit der Struktur:

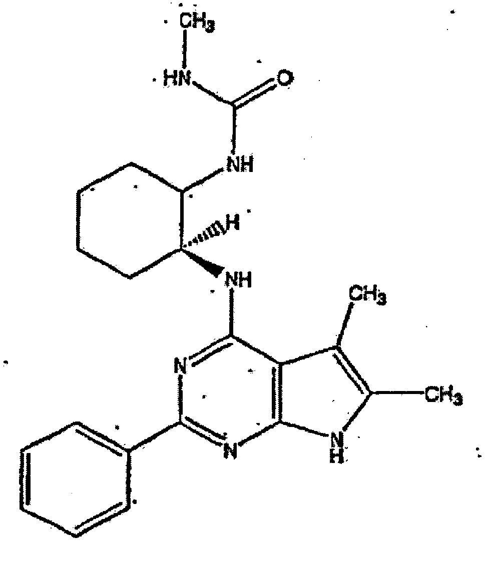

5. Verbindung gemäß Anspruch 3 mit der Struktur:

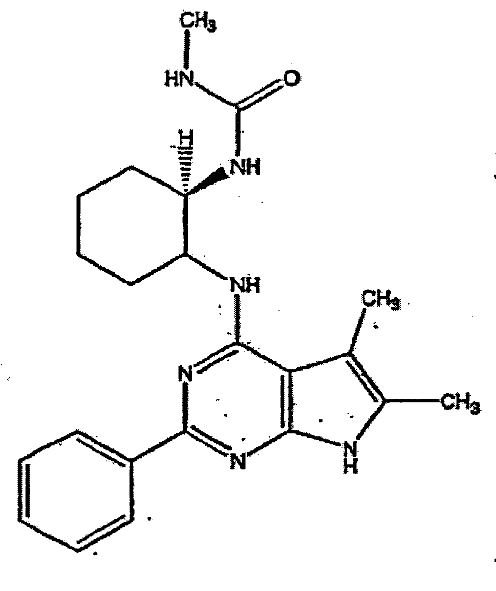

6. Verbindung gemäß Anspruch 3 mit der Struktur:

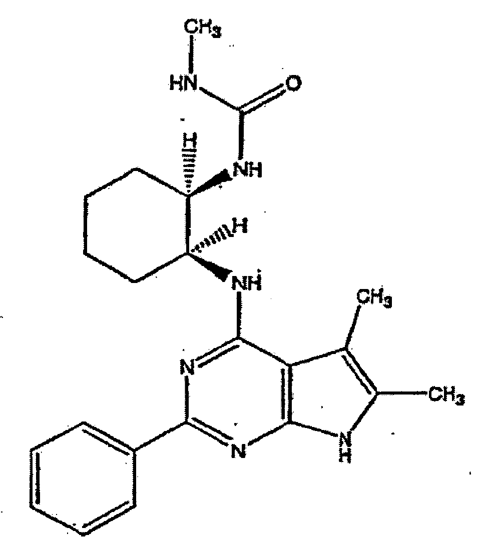

7. Verbindung gemäß Anspruch 3 mit der Struktur:

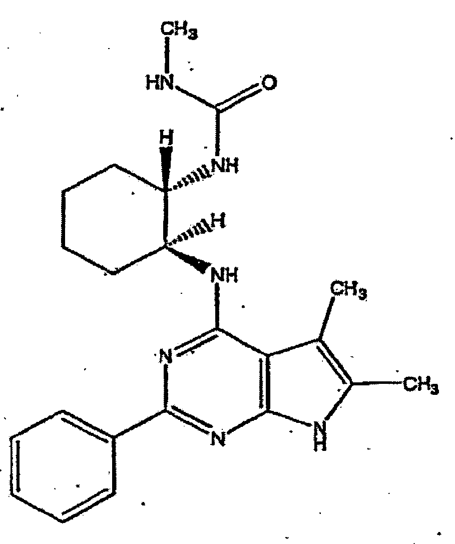

8. Verbindung gemäß Anspruch 1 mit der Struktur:

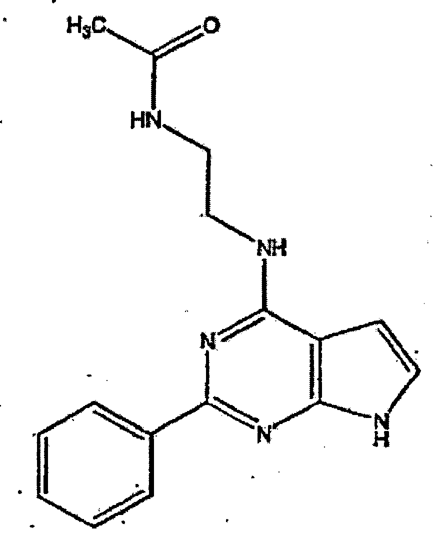

9. Verbindung gemäß Anspruch 1 mit der Struktur: 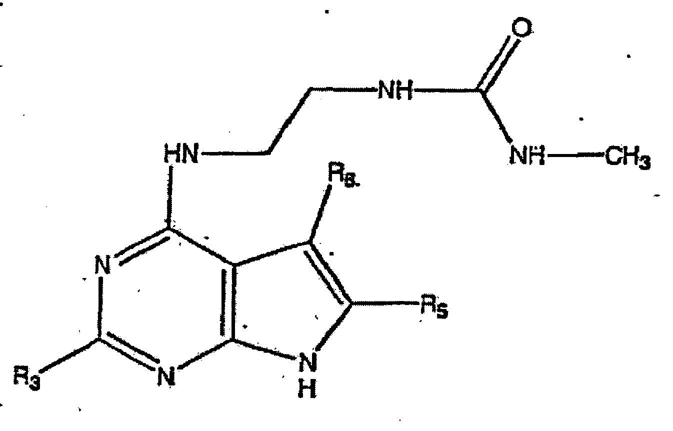 wobei R₃ wie in Anspruch 1 definiert ist;
wobei R₅ und R₆ unabhängig H oder Alkyl darstellen.

10. Verbindung gemäß Anspruch 9 mit der Struktur:

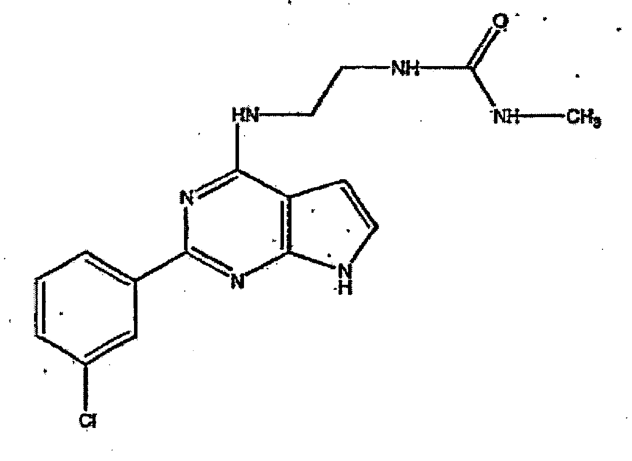

11. Verbindung gemäß Anspruch 1 mit der Struktur: 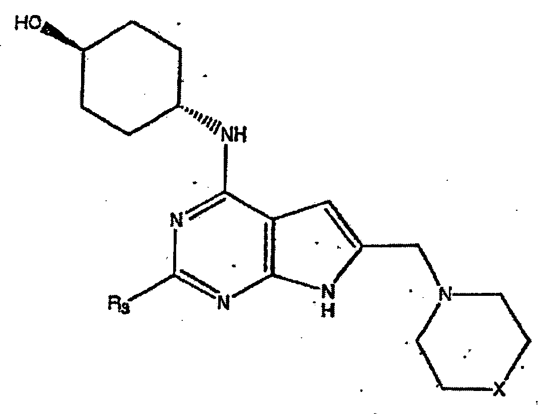 wobei R₃ wie in Anspruch 1 definiert ist; wobei X Sauerstoff oder Schwefel ist.

12. Verbindung gemäß Anspruch 11 mit der Struktur:

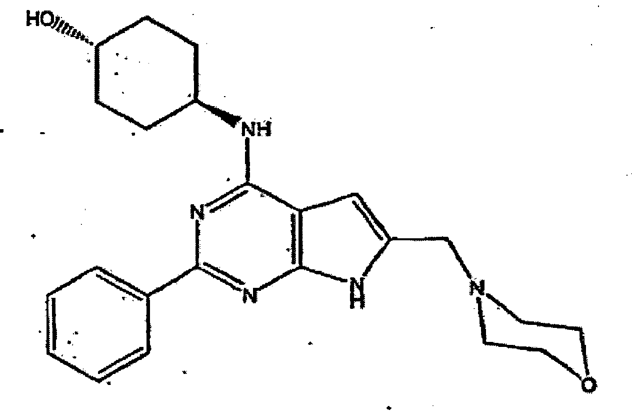

13. Verbindung gemäß Anspruch 11 mit der Struktur:

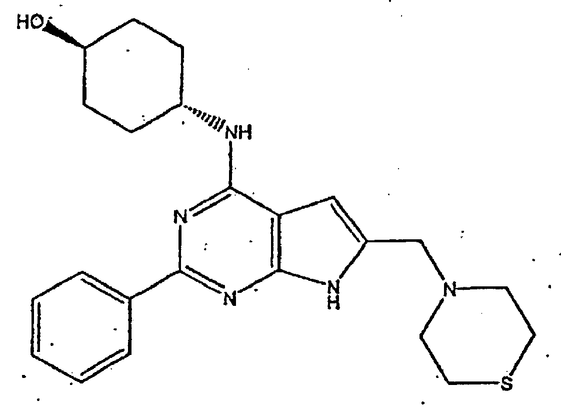

14. Verwendung einer Verbindung gemäß Anspruch 2, 8, 9 oder 11 für die Herstellung eines Medikaments, welches zur Behandlung einer Erkrankung, die in Zusammenhang mit einem A₁-Adenosinrezeptor steht, bei einem Patienten, welcher dieser Behandlung bedarf, nützlich ist.

15. Verwendung gemäß Anspruch 14, wobei der A₁-Adenosinrezeptor mit kognitiver Erkrankung, Nierenversagen, kardialen Arrhythmien, respiratorischen Epithelien, Transmitterabgabe, Sedation, Vasokonstriktion, Bradykardie, negativer kardialer Inotropie und Dromotropie, Bronchokonstriktion, Neutrophil-Chemotaxis, Refluxzustand oder ulzerösem Zustand in Verbindung steht.

16. Verfahren zur Inhibierung der Aktivität eines A₁-Adenosinrezeptors in einer Zelle *in vitro,* welches das Inkontaktbringen der Zelle mit einer Verbindung der Ansprüche 2, 8, 9 oder 11 umfaßt.

17. Verwendung gemäß Anspruch 14, wobei die Erkrankung Asthma, chronisch obstruktive Lungenerkrankung, allergische Rhinitis oder eine Störung der oberen Atemwege ist.

18. Arzneimittel, umfassend die Verbindung gemäß einem der Ansprüche 2, 8, 9 oder 11, einen pharmazeutisch verträglicher Träger und mindestens einem aus der Gruppe Steroid, β2-Agonist, Glucocorticoid, Leukotrien-Antagonist oder anticholinerger Agonist.

19. Arzneimittel, umfassend eine therapeutisch wirksame Menge der Verbindung gemäß den Ansprüchen 2, 8, 9 oder 11 und einen pharmazeutisch verträglichen Träger.

20. Arzneimittel gemäß Anspruch 19, wobei die therapeutisch wirksame Menge bei der Behandlung von Asthma, allergischer Rhinitis öder chronisch obstruktiver Lungenerkrankung wirksam ist.

21. Verbindung gemäß Anspruch 1 mit der Struktur: 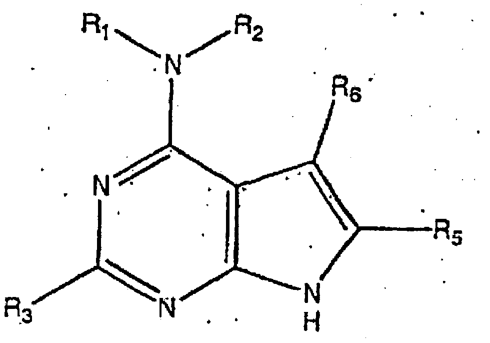 wobei NR₁R₂ (*D*)-2-Aminocarbonyl-N-pyrrolidinyl, (*D*)-2-Hydroxymethyl-N-pyrrolidinyl, *(D)*-2-Hydroxymethyl-trans-4-hydroxy-N-pyrrolidinyl oder N-Piperazinyl ist;
wobei R₃ wie in Anspruch 1 definiert ist;
wobei R₅ für H, Phenyl oder 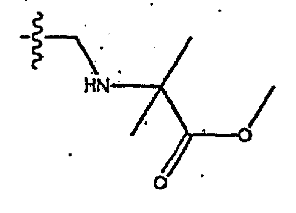 steht;
wobei R₆ für H, Alkyl oder Cycloalkyl steht.

22. Verbindung gemäß Anspruch 1 mit der Struktur: 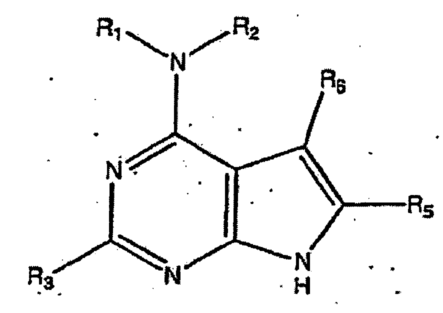 wobei NR₁R₂ für 3-Hydroxymethyl-N-piperidinyl steht;
wobei R₃ 4-Pyridyl ist;
wobei R₅ für H oder Alkyl steht; und
wobei R₆ für H oder Alkyl steht.

23. Verbindung gemäß Anspruch 21 mit der Struktur:

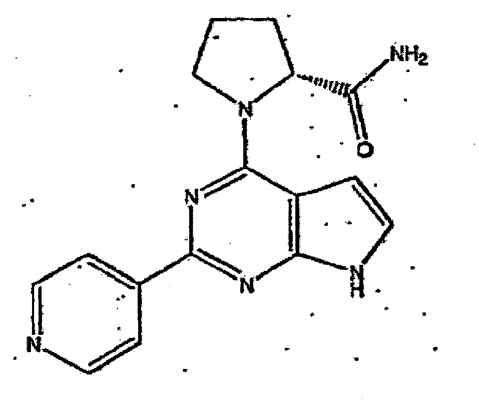

24. Verbindung gemäß Anspruch 21 mit der Struktur:

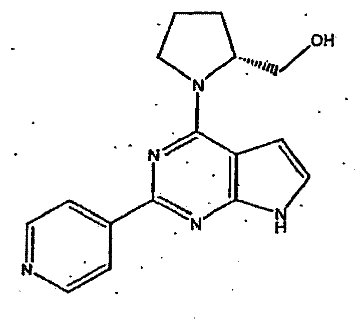

25. Verbindung gemäß Anspruch 21 mit der Struktur:

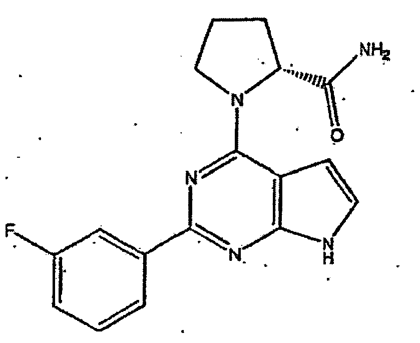

26. Verbindung gemäß Anspruch 21 mit der Struktur:

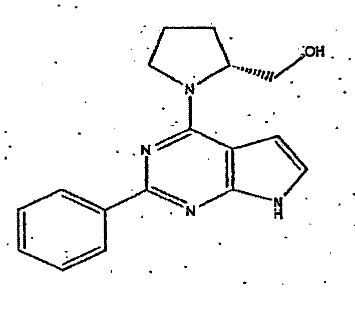

27. Verbindung gemäß Anspruch 21 mit der Struktur:

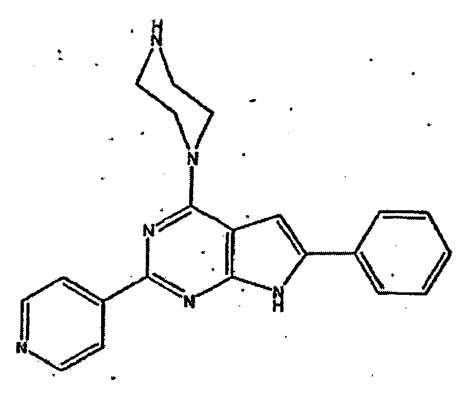

28. Verbindung gemäß Anspruch 21 mit der Struktur:

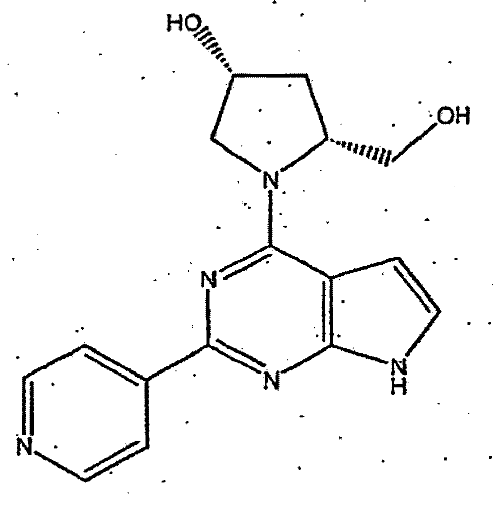

29. Verbindung gemäß Anspruch 1 mit der Struktur:

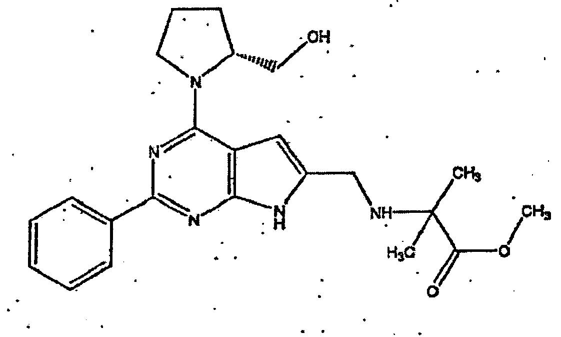

30. Verbindung gemäß Anspruch 22 mit der Struktur:

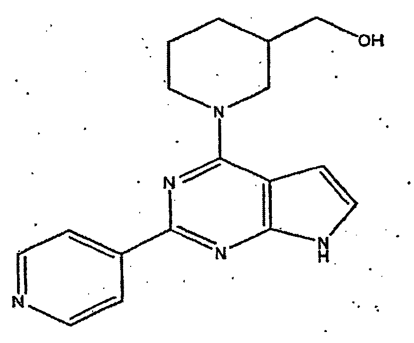

31. Verbindung gemäß Anspruch 30 mit der Struktur:

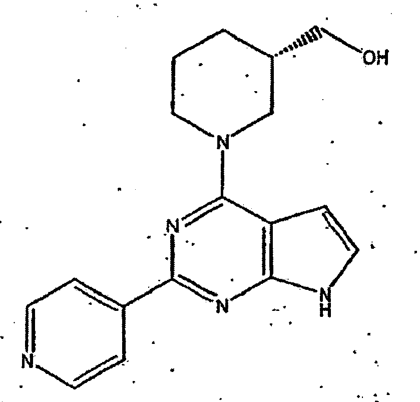

32. Verbindung gemäß Anspruch 30 mit der Struktur:

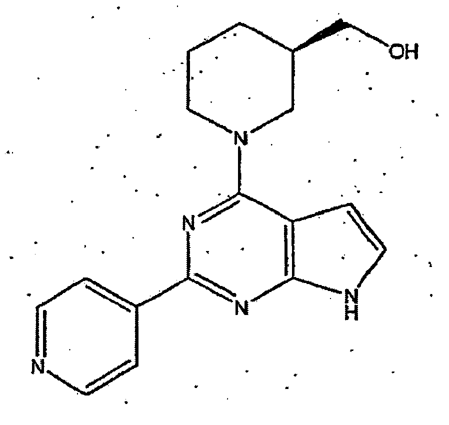

33. Verbindung gemäß Anspruch 1 mit der Struktur: 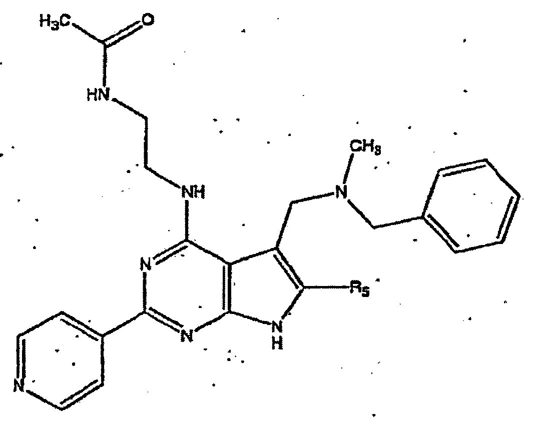 wobei R₅ für H oder Methyl steht.

34. Verbindung gemäß Anspruch 33 mit der Struktur:

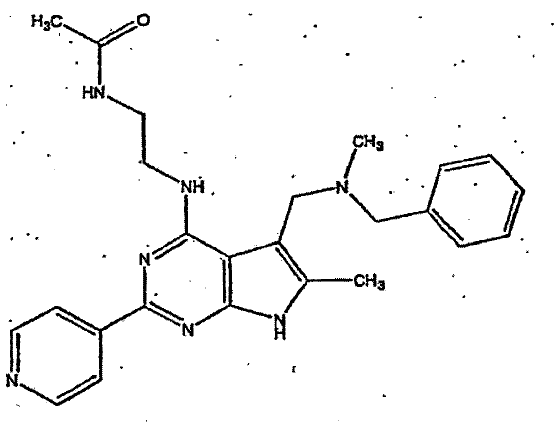

35. Verbindung gemäß Anspruch 33 mit der Struktur:

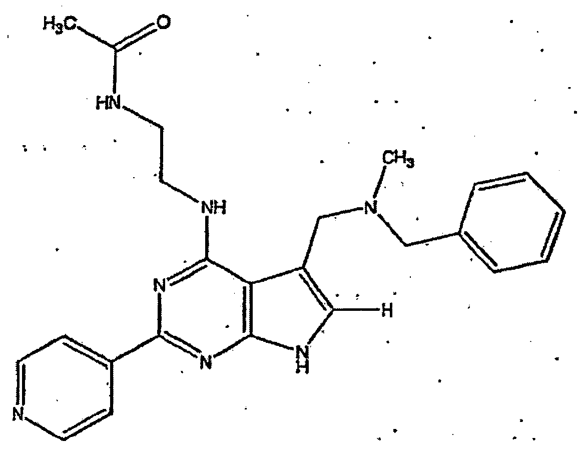

36. Verwendung einer Verbindung gemäß Anspruch 21, 22 oder 33 für die Herstellung eines Medikaments, welches zur Behandlung einer Erkrankung, die in Verbindung mit einem A2a-Adenosinrezeptor steht, bei einem Patienten, der einer solchen Behandlung bedarf, nützlich ist.

37. Verwendung gemäß Anspruch 36, wobei der A2a-Adenosinrezeptor mit lokomotorischer Aktivität, Vasodilatation, Thrombozytenhemmung, Superoxidbildung von Neutrophilen, kognitiver Störung, Altersdemenz oder Parkinsonscher Krankheit in Verbindung steht.

38. Verwendung gemäß Anspruch 36, wobei die Verbindung die Erkrankungen durch Stimulierung der Adenylatcyclase behandelt.

39. Verfahren zur Inhibierung der Aktivität eines A2a-Adenosinrezeptors in einer Zelle *in vitro,* welches das Inkontaktbringen der Zelle mit einer Verbindung der Ansprüche 21, 22 oder 33 umfaßt.

40. Arzneimittel, umfassend eine therapeutisch wirksame Menge der Verbindung gemäß den Ansprüchen 21, 22 oder 33 und einen pharmazeutisch verträglichen Träger.

41. Arzneimittel gemäß Anspruch 40, wobei die therapeutisch wirksame Menge bei der Behandlung von Parkinsonscher Krankheit und Erkrankungen, die in Verbindung mit lokomotorischer Aktivität, Vasodilatation, Thrombozytenhemmung, Superoxidbildung von Neutrophilen, kognitiver Störung oder Altersdemenz stehen, wirksam ist.

42. Arzneimittel, umfassend eine der Verbindungen gemäß den Ansprüchen 21, 22 oder 33, einen pharmazeutisch verträglichen Träger und mindestens einen Dopaminenhancer.

43. Arzneimittel, umfassend eine der Verbindungen gemäß den Ansprüchen 21, 22 oder 33, einen pharmazeutisch verträglichen Träger und mindestens ein cytotoxisches Mittel.

44. Arzneimittel, umfassend eine der Verbindungen gemäß den Ansprüchen 21, 22 oder 33, einen pharmazeutisch verträglichen Träger und mindestens einem aus der Gruppe Prostaglandin-Agonist, Muskarin-Agonist oder β-2-Agonist.

45. Verfahren zur Herstellung der Verbindung gemäß Anspruch 33, umfassend die Schritte
a) Umsetzen von 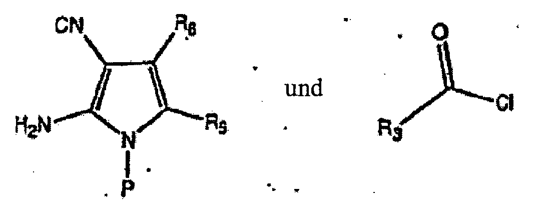 um 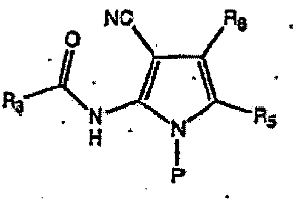 bereitzustellen,
wobei P eine entfernbare Schutzgruppe darstellt;
b) Behandeln des Produktes aus Schritt a) unter Cyclisierungsbedingungen, um 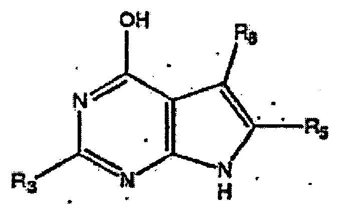 bereitzustellen;
c) Behandeln des Produktes aus Schritt b) unter geeigneten Bedingungen, um 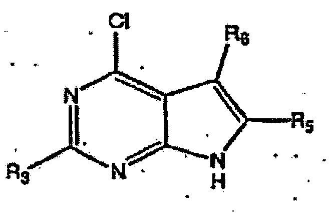 bereitzustellen; und
d) Behandeln des chlorierten Produkts aus Schritt c) zuerst mit Dimethylamin und Formaldehyd, dann mit N-Methylbenzylamin und schließlich mit NH₂R₁, um 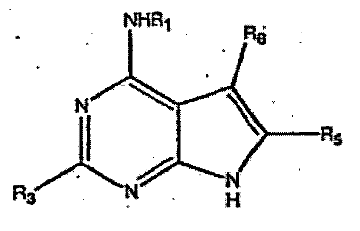 bereitzustellen,
wobei R₁ Acetamidoethyl ist; wobei R₃ 4-Pyridyl ist; wobei R₅ für H oder Methyl steht; wobei R₆ N-Methyl-N-benzyl-aminomethyl ist.

46. Verbindung gemäß Anspruch 1 mit der Struktur: 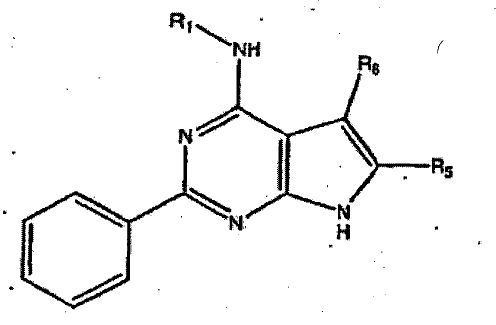 wobei R₁ 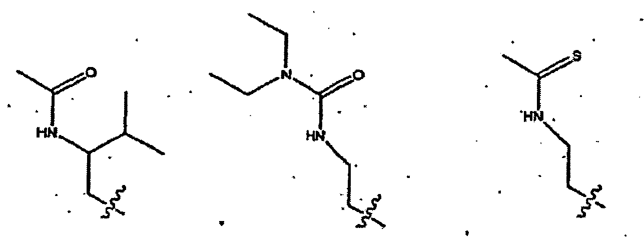 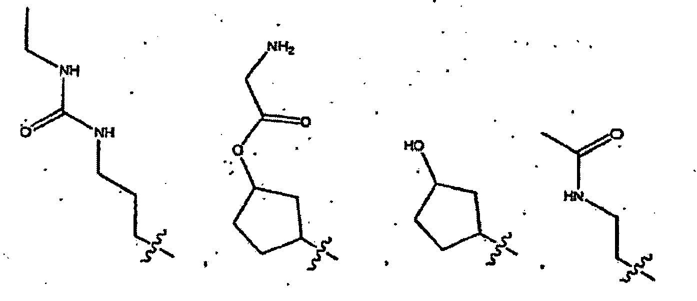 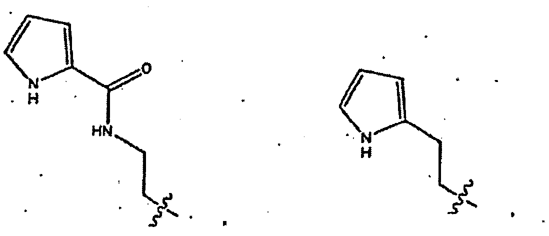 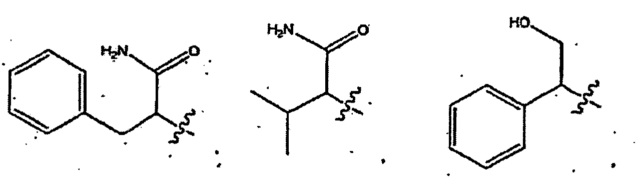 oder 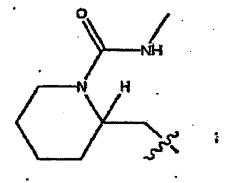 ist;
R₅ und R₆ unabhängig für H, substituiertes oder unsubstituiertes Alkyl oder Aryl stehen,
wobei der Substituent, falls vorhanden, wie in Anspruch 1 definiert ist,
wobei, wenn R₁ 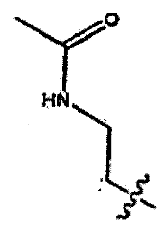 ist,
R₅ 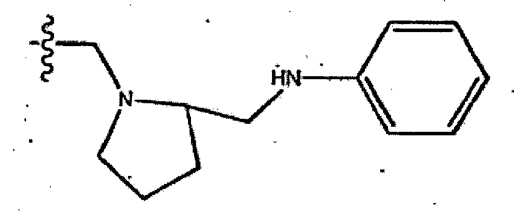 ist,
und R₆ für H steht.

47. Verbindung gemäß Anspruch 46 mit der Struktur:

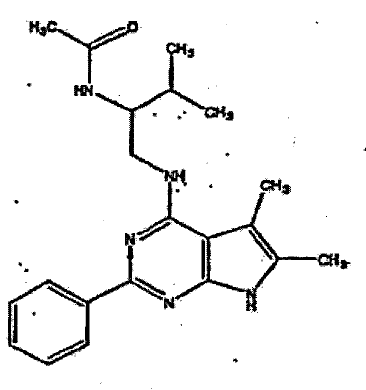

48. Verbindung gemäß Anspruch 46 mit der Struktur:

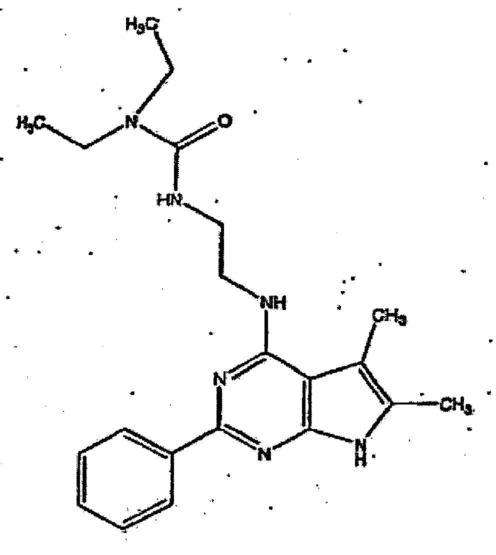

49. Verbindung gemäß Anspruch 46 mit der Struktur:

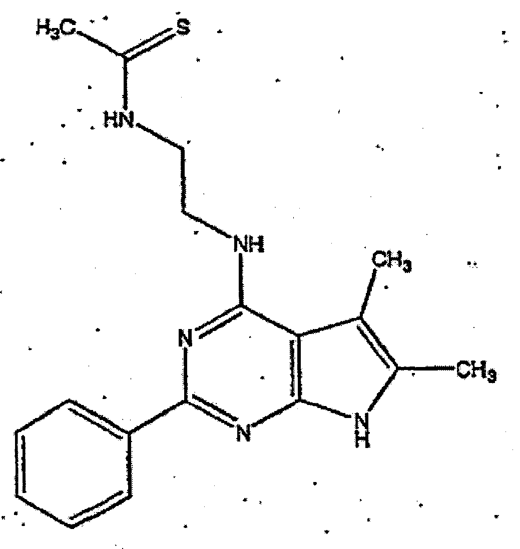

50. Verbindung gemäß Anspruch 46 mit der Struktur:

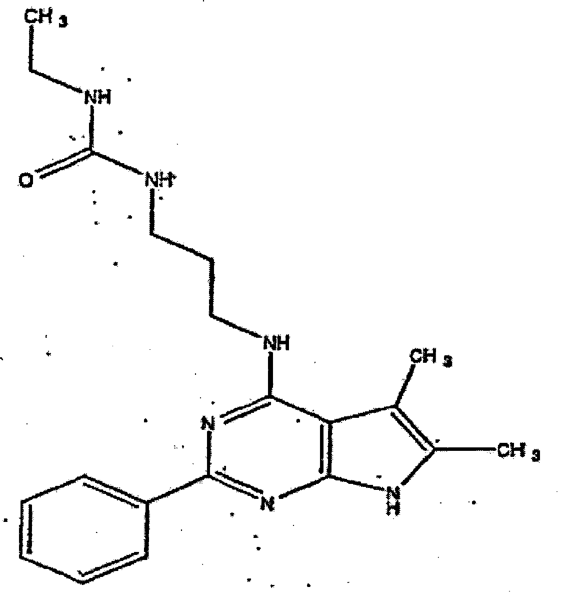

51. Verbindung gemäß Anspruch 46 mit der Struktur:

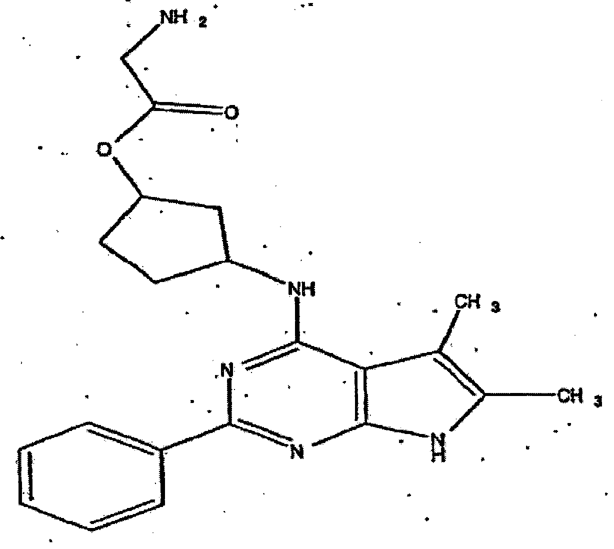

52. Verbindung gemäß Anspruch 46 mit der Struktur:

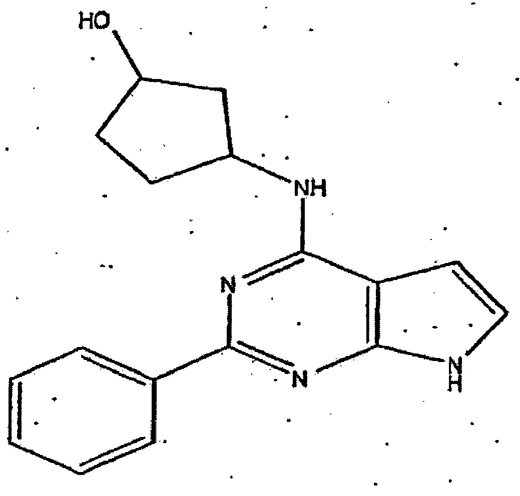

53. Verbindung gemäß Anspruch 52 mit der Struktur:

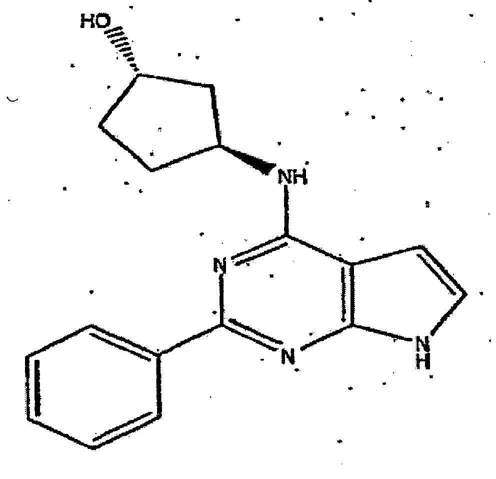

54. Verbindung gemäß Anspruch 52 mit der Struktur:

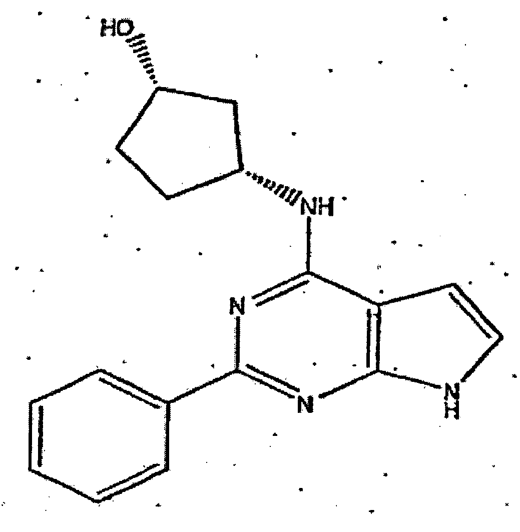

55. Verbindung gemäß Anspruch 52 mit der Struktur:

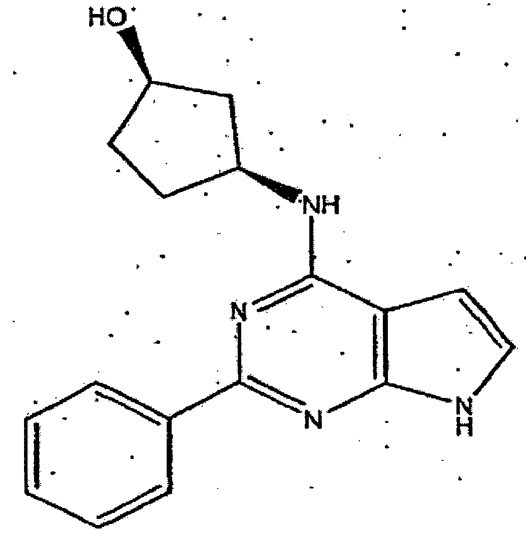

56. Verbindung gemäß Anspruch 52 mit der Struktur:

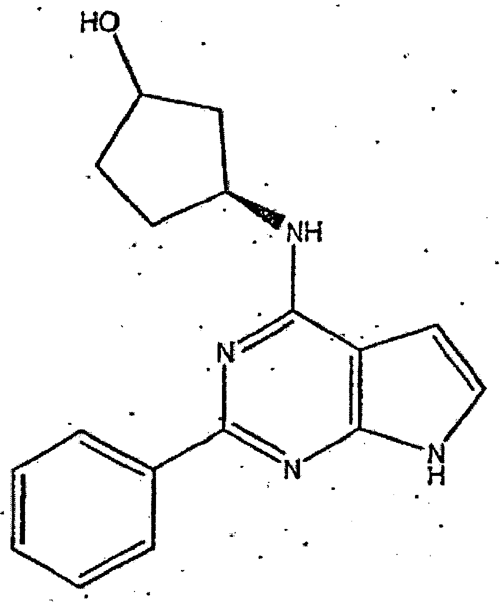

57. Verbindung gemäß Anspruch 46 mit der Struktur:

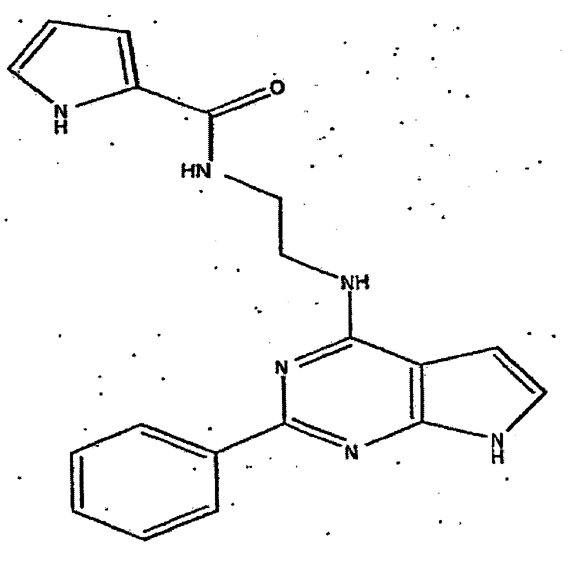

58. Verbindung gemäß Anspruch 1 mit der Struktur:

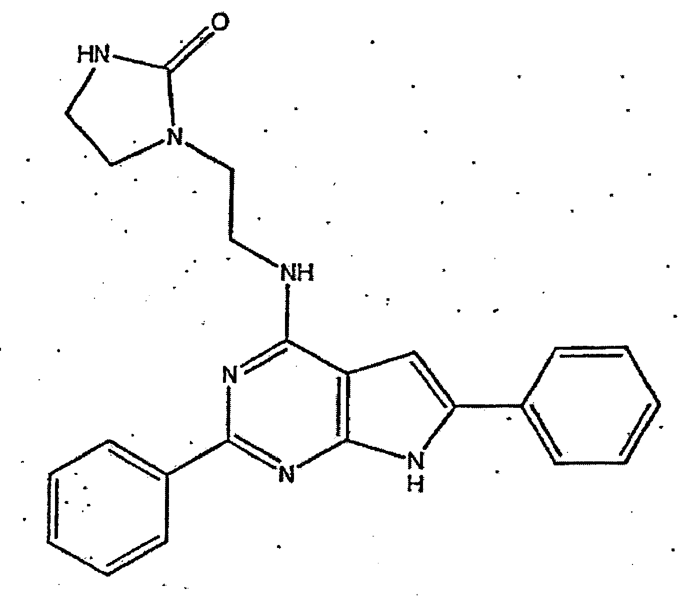

59. Verbindung gemäß Anspruch 46 mit der Struktur:

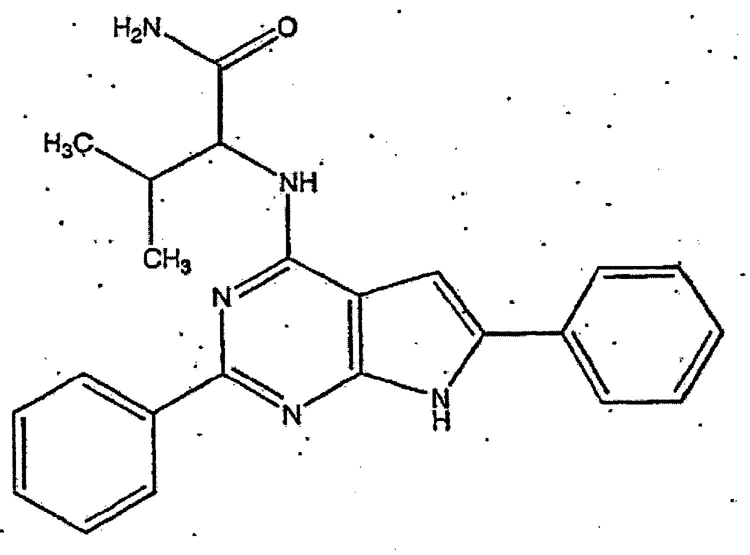

60. Verbindung gemäß Anspruch 46 mit der Struktur:

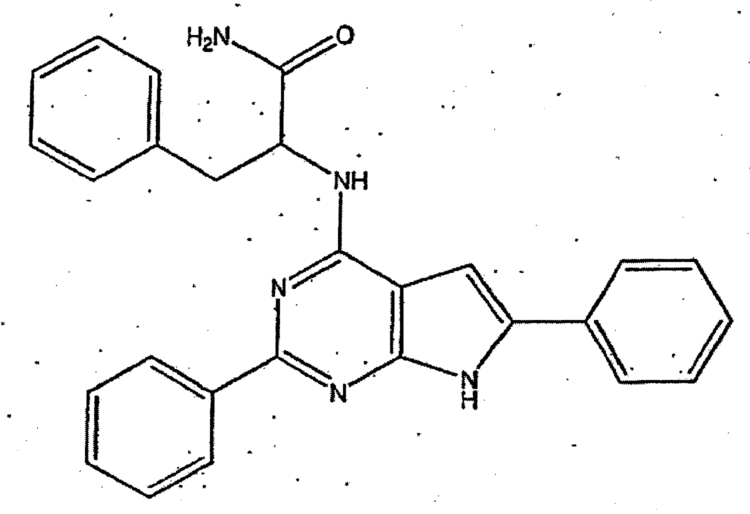

61. Verbindung gemäß Anspruch 46 mit der Struktur:

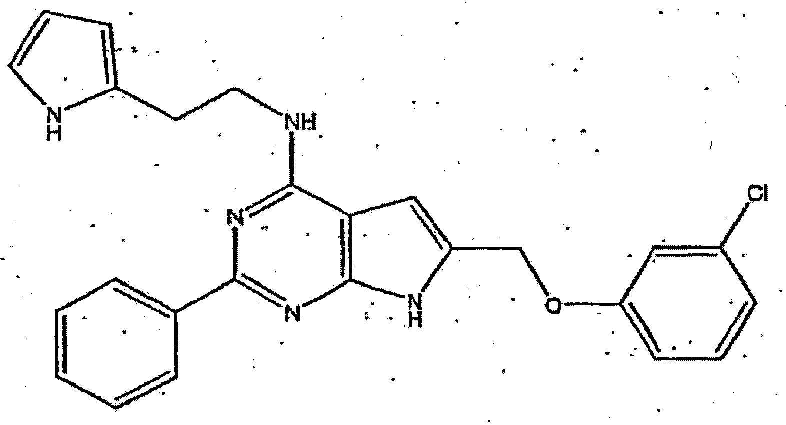

62. Verbindung gemäß Anspruch 46 mit der Struktur:

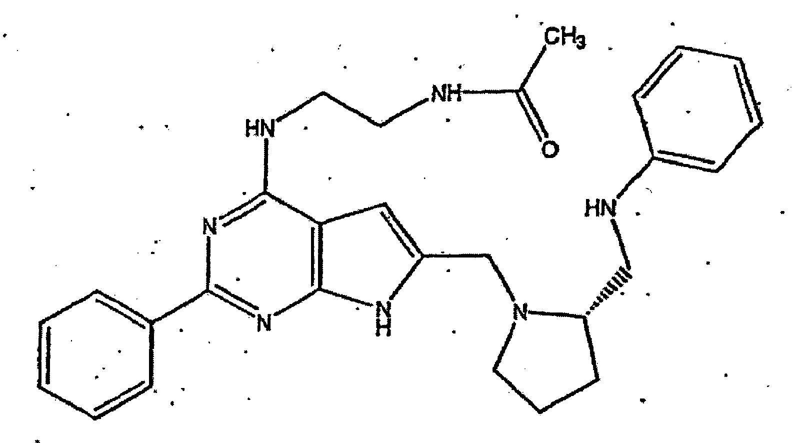

63. Verbindung gemäß Anspruch 62 mit der Struktur:

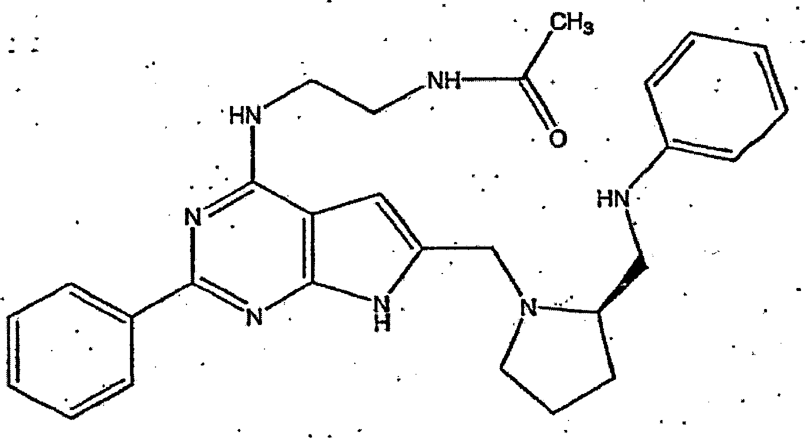

64. Verbindung gemäß Anspruch 46 mit der Struktur:

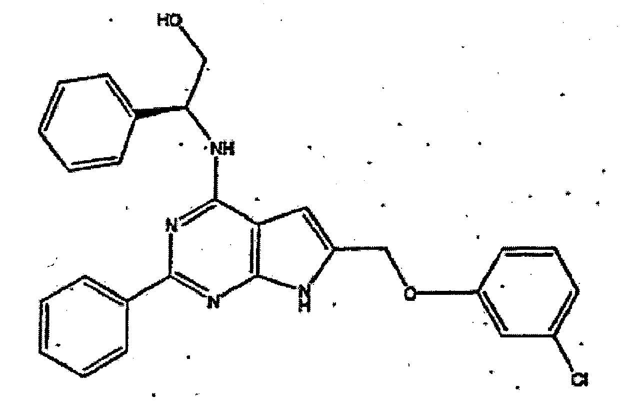

65. Verbindung gemäß Anspruch 64 mit der Struktur:

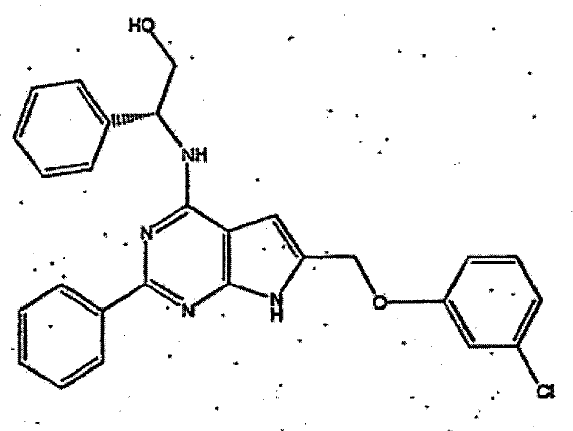

66. Verbindung gemäß Anspruch 46 mit der Struktur:

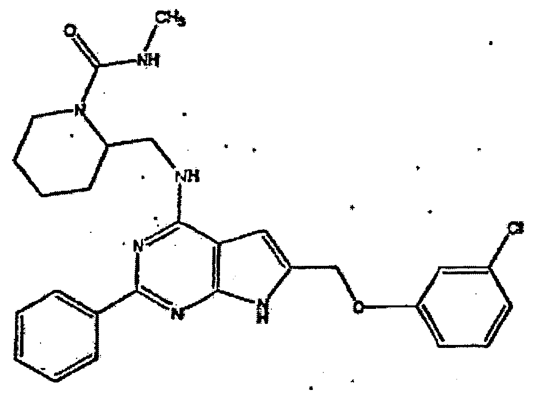

67. Verbindung gemäß Anspruch 66 mit der Struktur:

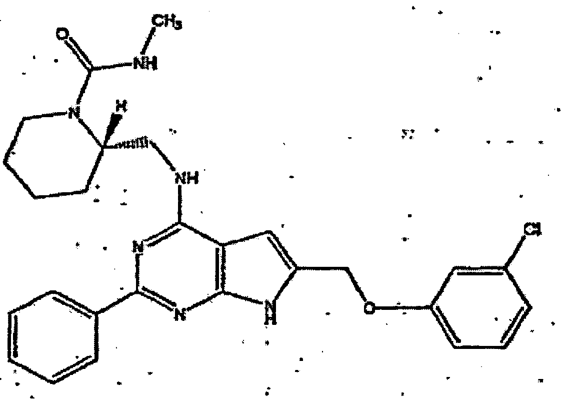

68. Verbindung gemäß Anspruch 66 mit der Struktur:

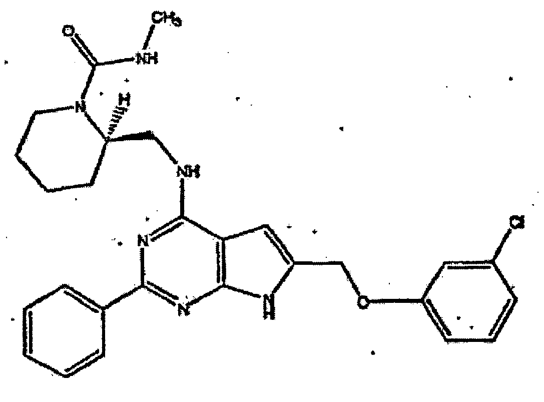

69. Verbindung gemäß Anspruch 1 mit der Struktur: 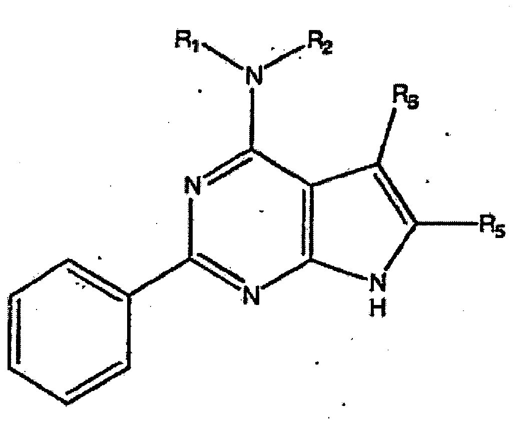 wobei R₁, R₂ und das Stickstoffatom zusammen 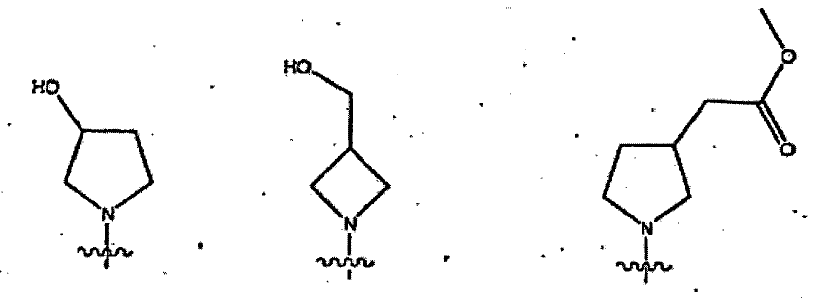 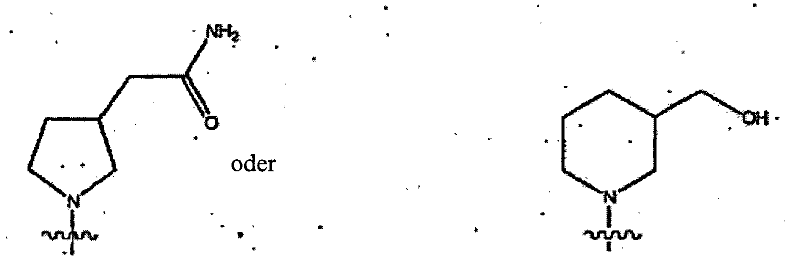 sind; und
wobei R₅ und R₆ unabhängig für H, substituiertes oder unsubstituiertes Alkyl, oder Aryl stehen,
wobei der Substituent, falls vorhanden, wie in Anspruch 1 definiert ist,
wobei, wenn R₁, R₂ und das Stickstoffatom zusammen 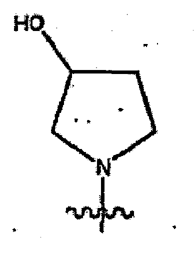 sind
R₅ Phenyl ist und R₆ für H steht;
wobei, wenn R₁, R₂ und das Stickstoffatom zusammen 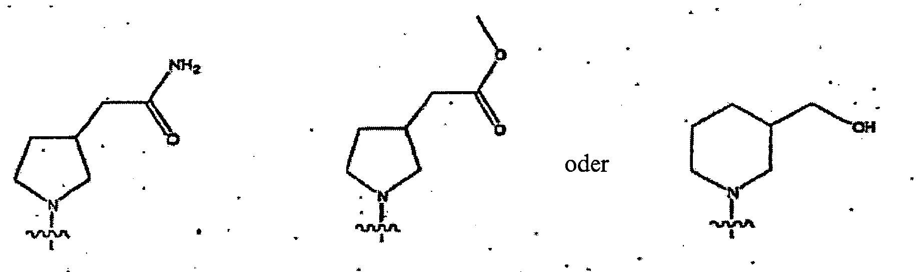 sind
R₅ 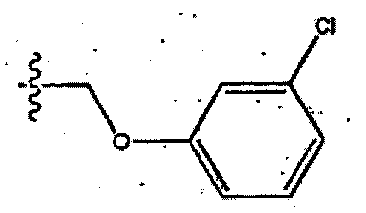 ist
und R₆ für H steht; und
wobei, wenn R₁, R₂ und das Stickstoffatom zusammen 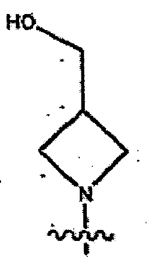 sind,
R₅ und R₆ beide H sind.

70. Verbindung gemäß Anspruch 69 mit der Struktur:

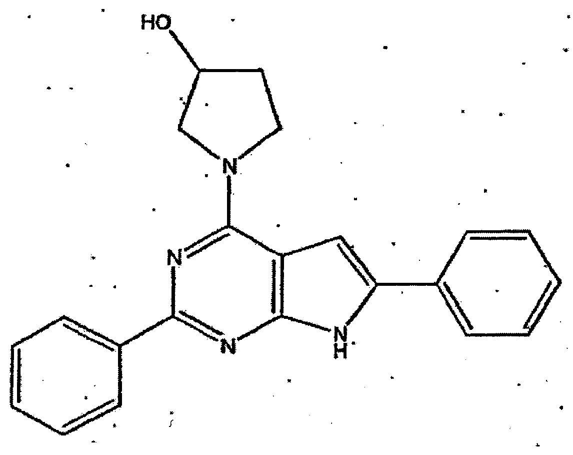

71. Verbindung gemäß Anspruch 70 mit der Struktur:

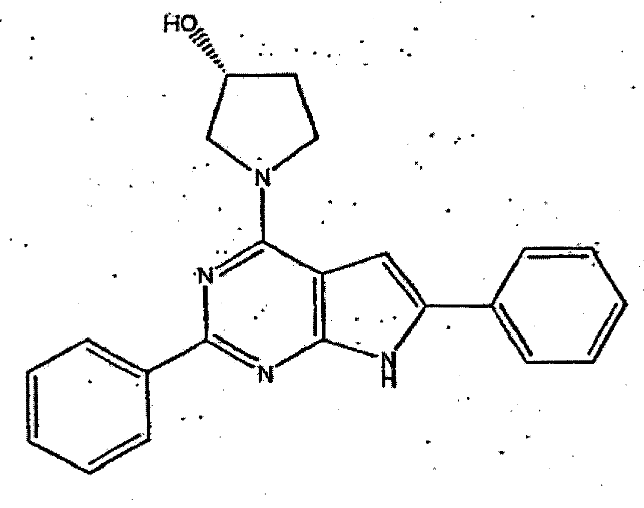

72. Verbindung gemäß Anspruch 70 mit der Struktur:

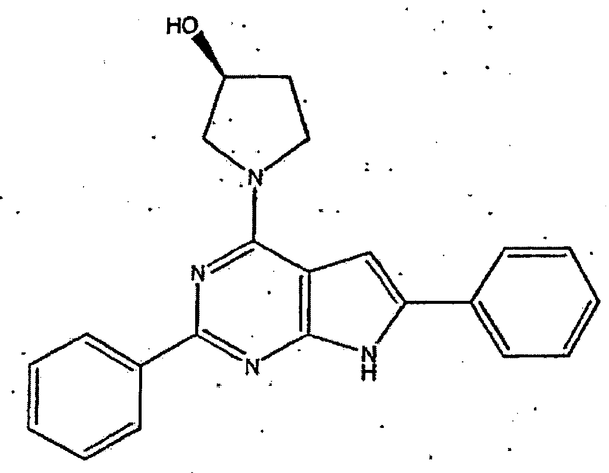

73. Verbindung gemäß Anspruch 69 mit der Struktur:

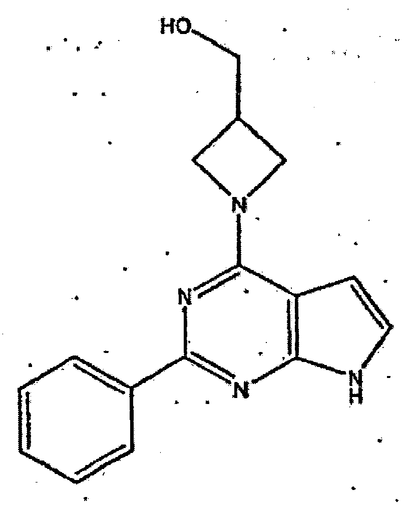

74. Verbindung gemäß Anspruch 69 mit der Struktur:

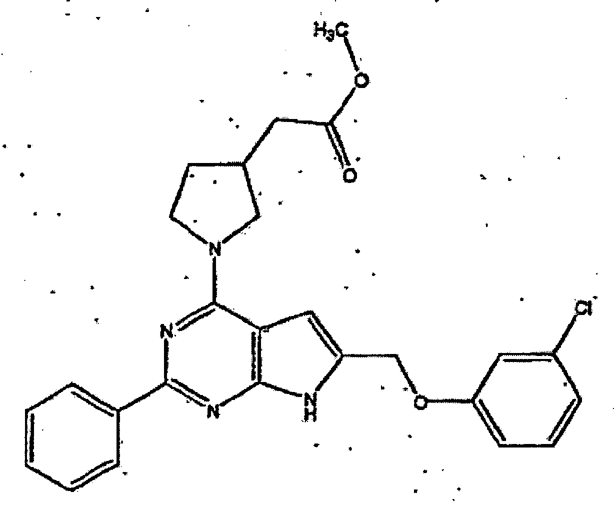

75. Verbindung gemäß Anspruch 74 mit der Struktur:

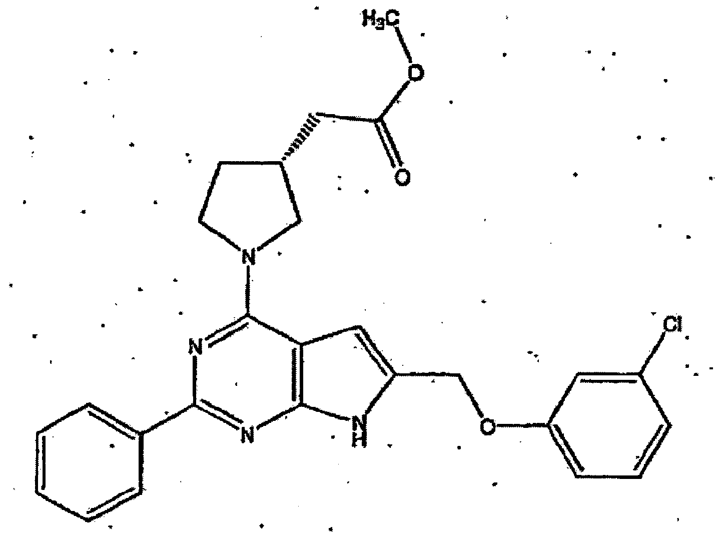

76. Verbindung gemäß Anspruch 74 mit der Struktur:

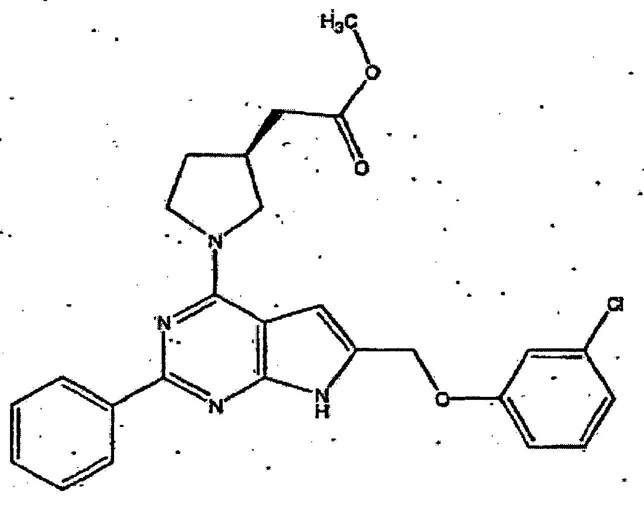

77. Verbindung gemäß Anspruch 69 mit der Struktur:

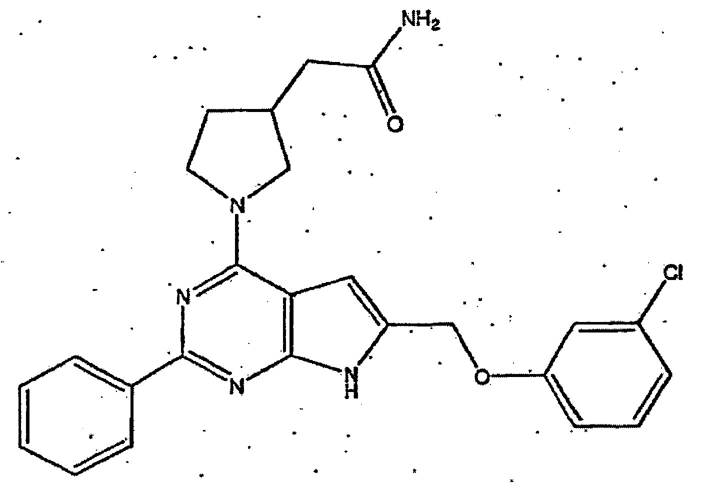

78. Verbindung gemäß Anspruch 77 mit der Struktur:

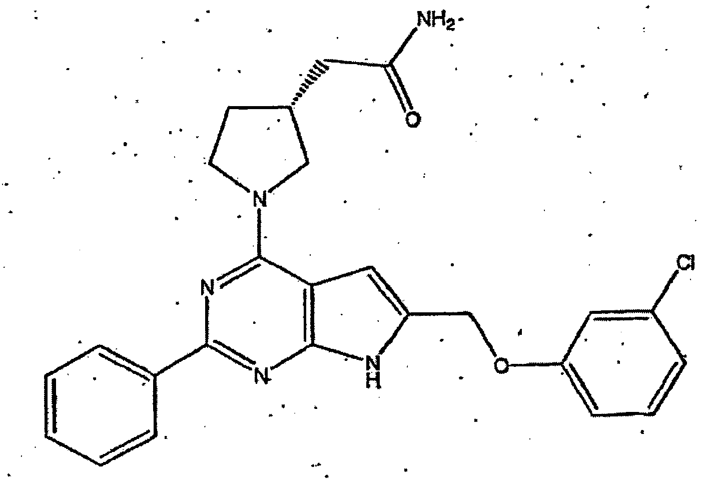

79. Verbindung gemäß Anspruch 77 mit der Struktur:

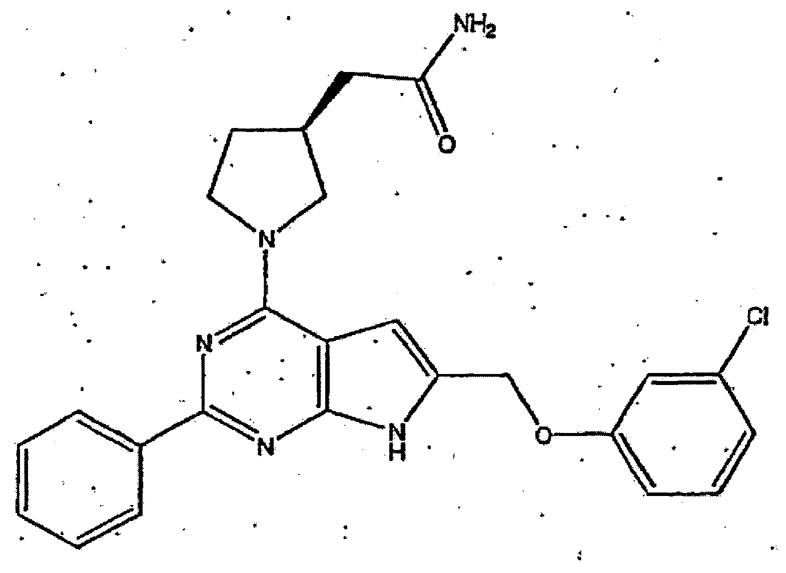

80. Verbindung gemäß Anspruch 69 mit der Struktur:

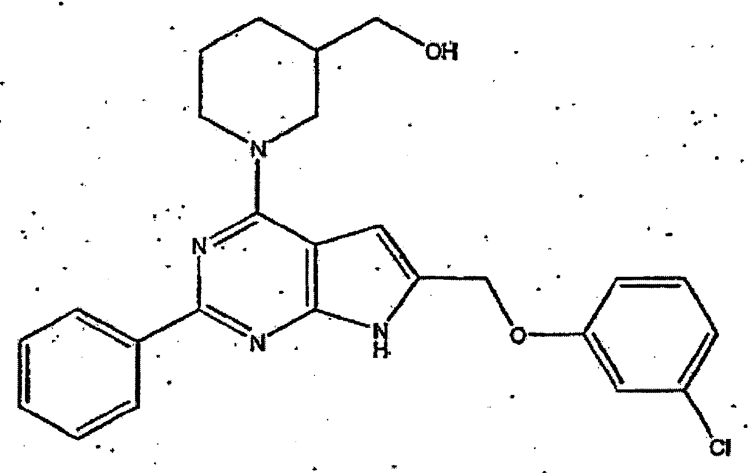

81. Verbindung gemäß Anspruch 80 mit der Struktur:

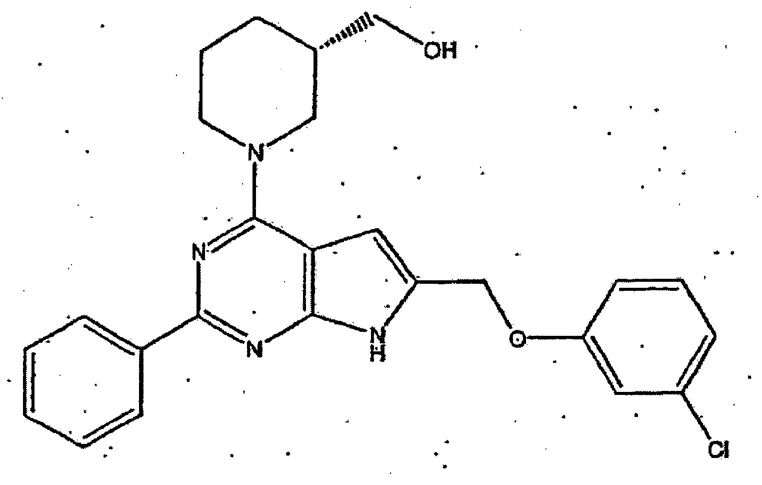

82. Verbindung gemäß Anspruch 80 mit der Struktur:

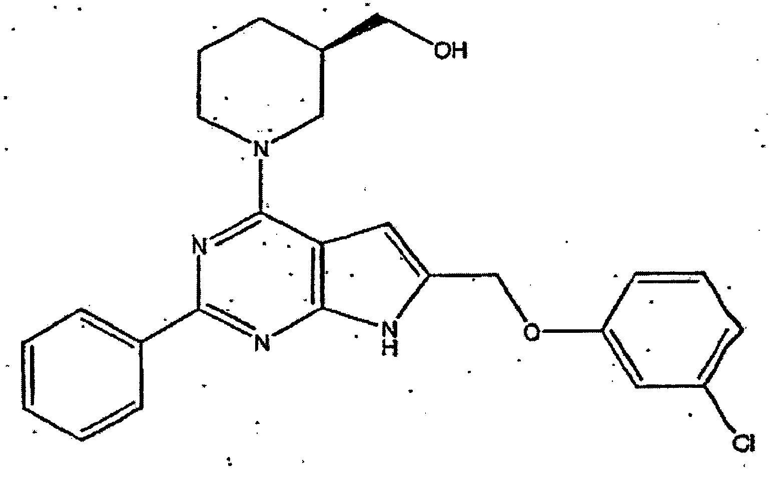

83. Verbindung gemäß Anspruch 1 mit der Struktur:

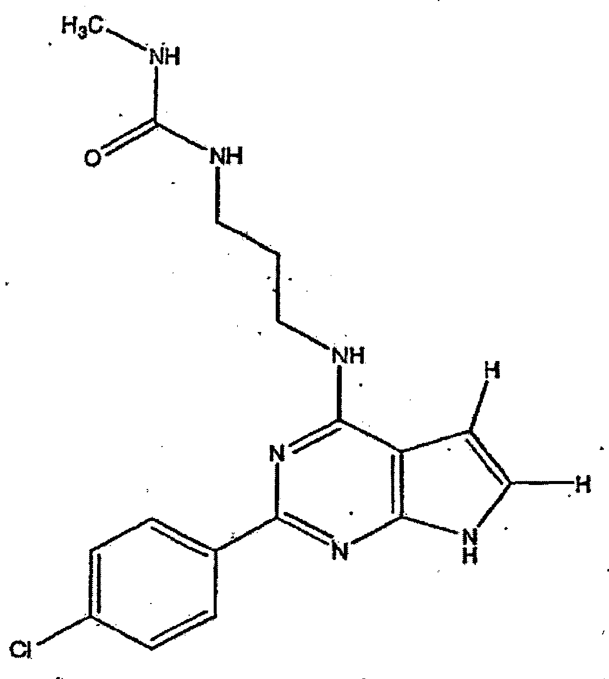

84. Verwendung einer Verbindung gemäß Anspruch 46, 58, 69 oder 83 für die Herstellung eines Medikaments, welches zur Behandlung einer Erkrankung, die in Verbindung mit einem A3-Adenosinrezeptor steht, bei einem Patienten, der solch einer Behandlung bedarf, nützlich ist.

85. Verwendung gemäß Anspruch 84 wobei der A3-Adenosinrezeptor mit Asthma, Überempfindlichkeit, Rhinitis, Heuschnupfen, Serumkrankheit, allergischer Vaskulitis, atopischer Dermatitis, Dermatitis, Psoriasis, Ekzem, idiopathischer pulmonarer Fibrose, eosinophiler Cholecystitis, chronischer Atemsweginfektion, hypereosinophilen Syndromen, eosinophiler Gastroenteritis, Ödem, Urticaria, eosinophiler Myocarditis, episodischem Angioödem mit Eosinophilie, entzündlicher Erkrankung des Darms, Colitis ulcerosa, allergischer Granulomatose, Karzinomatose, eosinophilem Granulom, familiärer Hystiozytose, Bluthochdruck, Mastzellendegranulation, Tumor, kardialer Hypoxie, zerebraler Ischämie, Diurese, Nierenversagen, neurologischer Störung, mentaler Störung, kognitiver Störung, myokardialer Ischämie, Bronchokonstriktion, Arthritis, Autoimmunerkrankung, Morbus Crohn, Graves-Krankheit, Diabetes, Multipler Sklerose, Anämie, Fertilitätsstörungen, Lupus erythematodes, Reperfusionsschaden, Gehimarteriolendurchmesser, Freisetzung von allergischen Mediatoren, Sklerodermie, Schlaganfall, globaler Ischämie, Störung des zentralen Nervensystems, kardiovaskulärer Störung, Nierenstörung, entzündlichen Störungen, gastrointestinaler Störung, Störung des Auges, allergischer Störung, Störung der Atemwege oder Störung des Immunsystems in Verbindung steht.

86. Wasserlösliches Prodrug, wobei das wasserlösliche Prodrug *in vivo* umgewandelt wird, um eine Verbindung gemäß Anspruch 2, 8, 9, 11, 21, 22, 33, 46, 58, 69 oder 83 herzustellen.

87. Prodrug gemäß Anspruch 86, wobei das Prodrug *in vivo* durch Esterase-katalysierte Hydrolyse umgewandelt wird.

88. Arzneimittel, umfassend das Prodrug gemäß Anspruch 86 und einen pharmazeutisch verträglichen Träger.

89. Arzneimittel gemäß Anspruch 88, wobei das Arzneimittel eine ophthalmische Formulierung ist.

90. Arzneimittel gemäß Anspruch 88, wobei das Arzneimittel eine periokuläre, retrobulbäre oder intraokuläre Injektionsformulierung ist.

91. Arzneimittel gemäß Anspruch 88, wobei das Arzneimittel eine systemische Formulierung ist.

92. Verfahren zur Hemmung der Aktivität eines A3-Adenosinrezeptors in einer Zelle *in vitro,* welches das Inkontaktbringen der Zelle mit einer Verbindung gemäß Anspruch 46, 58, 69 oder 83 umfaßt.

93. Verfahren gemäß Anspruch 16, 39 oder 92, wobei die Zelle eine menschliche Zelle ist.

94. Verwendung einer Verbindung gemäß Anspruch 46, 58, 69 oder 83 für die Herstellung eines Medikaments, welches bei der Behandlung einer gastrointestinalen Störung bei einem Patienten nützlich ist.

95. Verwendung gemäß Anspruch 94, wobei die Störung Diarrhöe ist.

96. Verwendung einer Verbindung gemäß Anspruch 46, 58, 69 oder 83 für die Herstellung eines Medikaments, welches bei der Behandlung von Störungen der Atemwege bei einem Patienten nützlich ist.

97. Verwendung gemäß Anspruch 96, wobei die Störung Asthma, chronisch obstruktive Lungenerkrankung, allergische Rhinitis oder eine Störung der oberen Atemwege ist.

98. Verwendung einer Verbindung gemäß Anspruch 46, 58, 69 oder 83 für die Herstellung eines Medikaments, welches bei der Behandlung von Schädigungen am Auge eines Patienten nützlich ist.

99. Verwendung gemäß Anspruch 98, wobei die Schädigung eine Schädigung der Netzhaut oder der Sehnervpapille ist.

100. Verwendung gemäß Anspruch 98, wobei die Schädigung akut oder chronisch ist.

101. Verwendung gemäß Anspruch 98, wobei die Schädigung das Ergebnis eines Glaukoms, eines Ödems, das Ergebnis von Ischämie, Hypoxie oder das eines Traumas ist.

102. Verwendung gemäß Anspruch 14, 17, 36, 84, 94, 96 oder 98, wobei der Patient ein Mensch ist.

103. Arzneimittel, umfassend eine beliebige Verbindung gemäß Anspruch 46, 58, 69 oder 83, einen pharmazeutisch verträglichen Träger und mindestens eines von Prostaglandinagonist, Muskarinagonist oder β-2-Antagonist.

104. Arzneimittel, umfassend eine therapeutisch wirksame Menge der Verbindung gemäß Anspruch 46, 58, 69 oder 83 und einen pharmazeutisch verträglichen Träger.

105. Arzneimittel gemäß Anspruch 104, wobei die therapeutisch wirksame Menge wirksam ist, um eine Störung der Atemwege oder eine gastrointestinale Störung zu behandeln.

106. Arzneimittel gemäß Anspruch 105, wobei die gastrointestinale Störung Diarrhöe ist.

107. Arzneimittel gemäß Anspruch 105, wobei die Störung der Atemwege Asthma, allergische Rhinitis oder eine chronisch obstruktive Lungenerkrankung ist.

108. Arzneimittel gemäß Anspruch 40 oder 104, wobei das Arzneimittel eine ophthalmische Formulierung ist.

109. Arzneimittel gemäß Anspruch 19, 40 oder 104, wobei das Arzneimittel eine periokuläre, retrobulbäre oder intraokuläre Injektionsformulierung ist.

110. Arzneimittel gemäß Anspruch 19, 40 oder 104, wobei das Arzneimittel eine systemische Formulierung ist.

111. Arzneimittel gemäß Anspruch 19, 40 oder 104, wobei das Arzneimittel eine chirurgische Irrigationslösung ist.

112. Verfahren zur Herstellung der Verbindung gemäß Anspruch 1, umfassend die Schritte
a) Umsetzen von 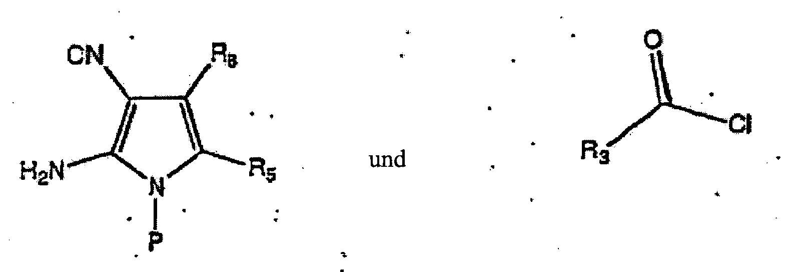 um 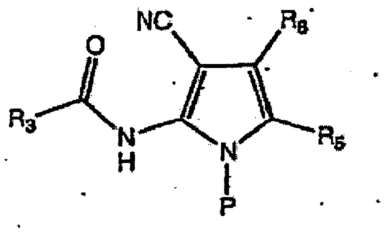 bereitzustellen,
wobei P eine entfernbare Schutzgruppe ist;
b) Behandeln des Produkts von Schritt a) unter Cyclisierungsbedingungen, um 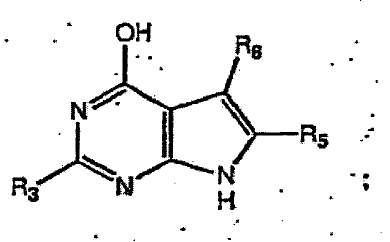 bereitzustellen;
c) Behandeln des Produkts von Schritt b) unter geeigneten Bedingungen, um 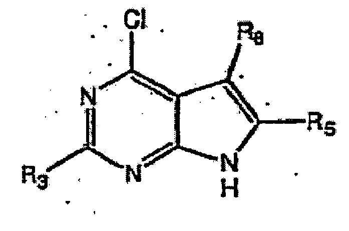 bereitzustellen; und
d) Behandeln des chlorierten Produkts von Schritt c) mit NHR₁R₂, um 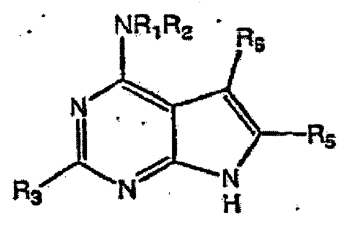 bereitzustellen,
wobei
R₁ gleich 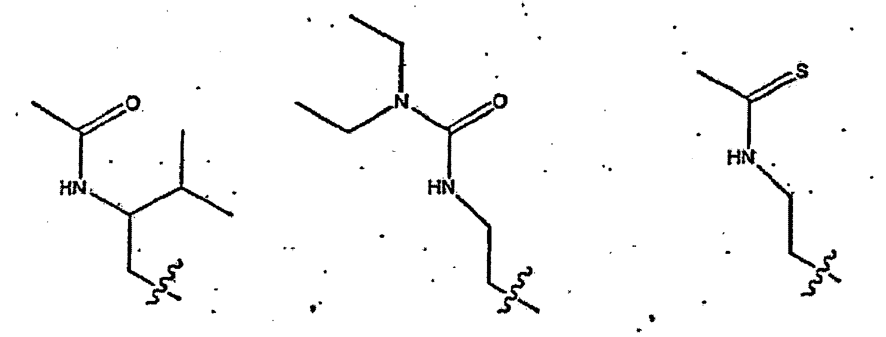 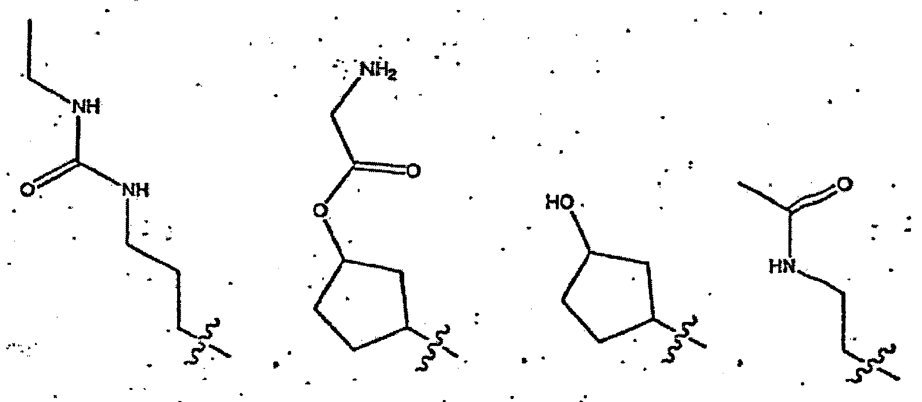 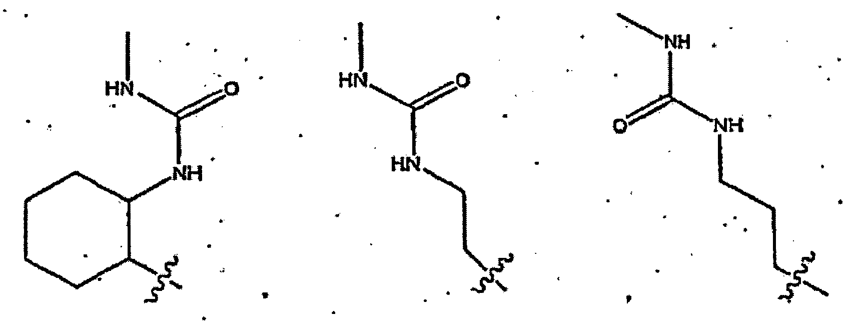 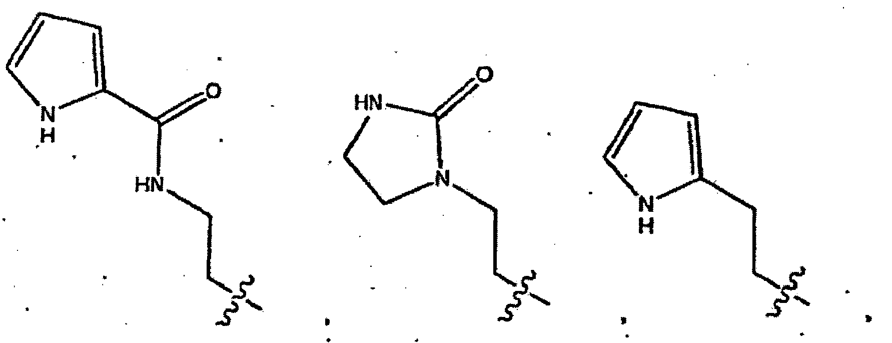 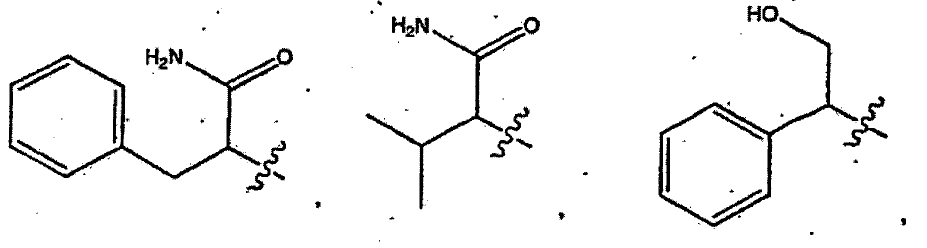 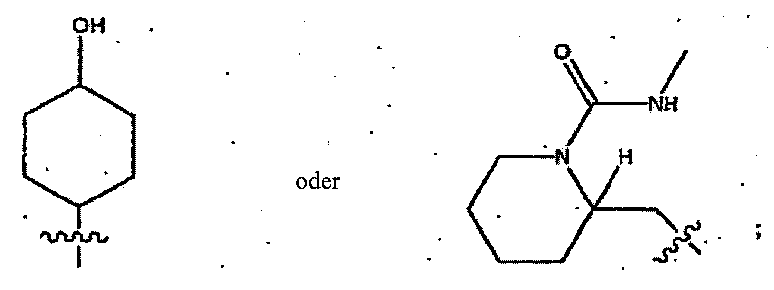 ist; und
R₂ für H steht; oder
NR₁R₂ zusammen 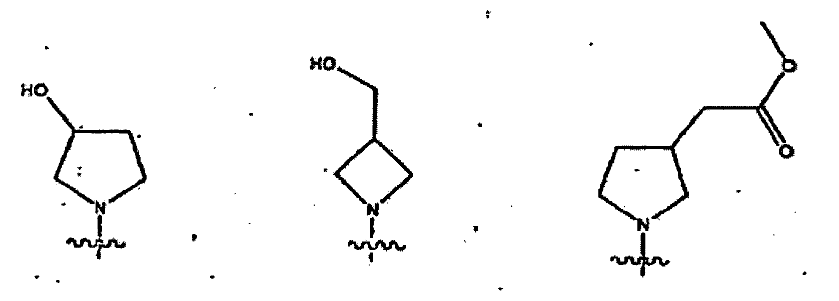 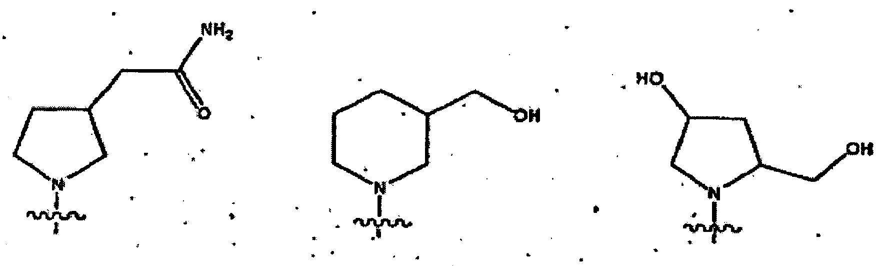 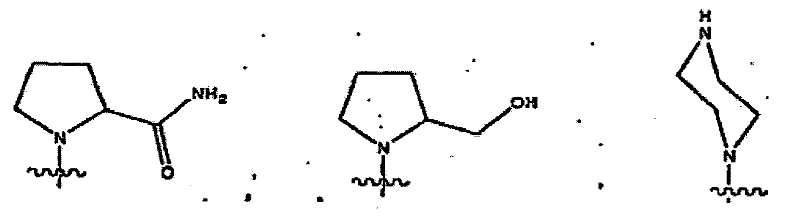 sind,
wobei R₃ wie in Anspruch 1 definiert ist;
wobei R₅ für H, CH₃, substituiertes oder unsubstituiertes Alkyl, Aryl oder Phenyl steht,
wobei der Substituent, falls vorhanden, wie in Anspruch 1 definiert ist, 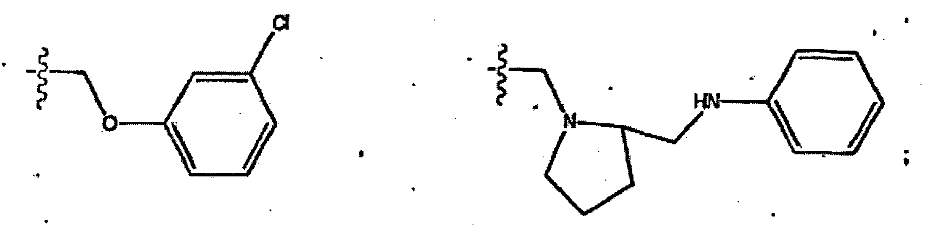 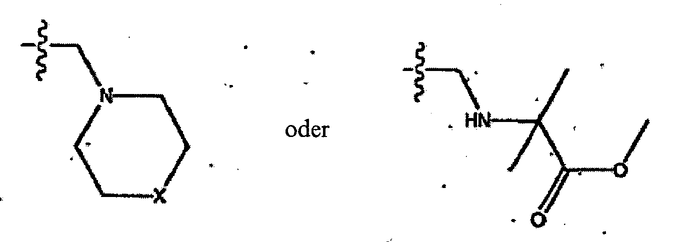 wobei X gleich O oder S ist;
und
wobei R₆ für H, CH₃, substituiertes oder unsubstituiertes Alkyl, Cycloalkyl oder 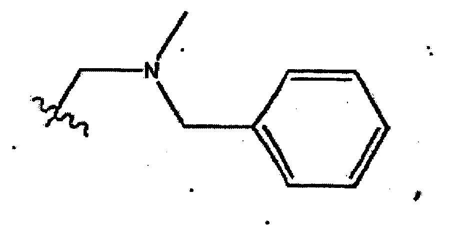 steht,
wobei, wenn R₁ gleich 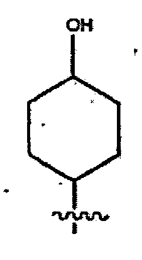 ist,
R₃ Phenyl ist;
R₅ 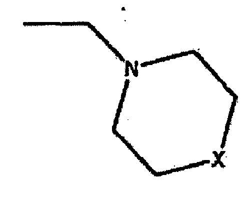 ist,
und R₆ für H steht;
wobei X für O oder S steht;
wobei, wenn R₁ gleich 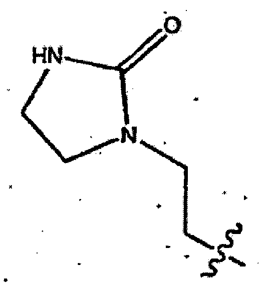 ist,
R₃ Phenyl ist;
R₅ Phenyl ist und R₆ für H steht;
wobei, wenn R₁ 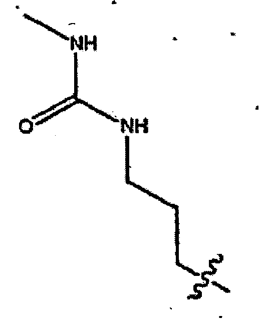 ist,
R₃ 4-Chlorphenyl ist;
R₅ und R₆ jeweils H sind;
wobei, wenn R₁ 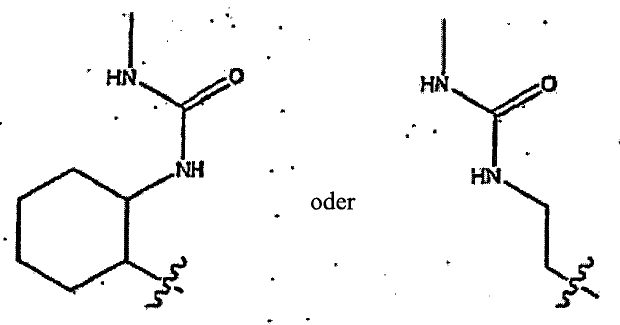 ist,
R₅ und R₆ jeweils unabhängig H oder Alkyl sind;
wobei, wenn R₁ 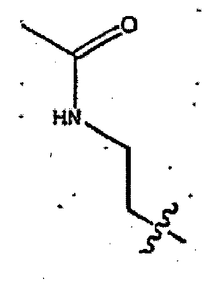 ist,
R₃ Phenyl ist; und
R₅ und R₆ beide H sind, oder
R₃ Phenyl ist;
R₅ 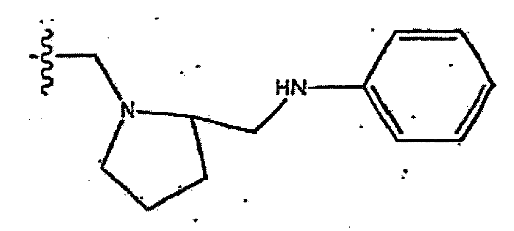 ist
und R₆ für H steht, oder
R₃ 4-Pyridyl ist;
R₅ für CH₃ steht und R₆ 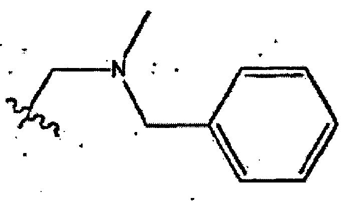 ist.

113. Verwendung der Verbindung gemäß einem der Ansprüche 2, 8, 9 oder 11 für die Herstellung eines Medikaments, welches bei der Behandlung einer Erkrankung, die in Verbindung mit einem A1-Adenosinrezeptor steht, bei einem Patienten, der einer solcher Behandlung bedarf, nützlich ist, wobei die Erkrankung in Verbindung mit dem A1-Adenosinrezeptor Antidiurese, Bradykardie, Bronchitis, Bronchokonstriktion, Alzheimer-Krankheit, Herzrhythmusstörungen, kardiale Hypoxie, Bluthochdruck, Entzündung, negative kardiale Inotropie und Dromotropie, Nierenversagen, Sedation ist, oder in Verbindung mit Transmitterfreisetzung, respiratorischer Epithelie, Kontraktion der glatten Muskulatur, die einer respiratorischen Epithelie zugrunde liegt, Vasokonstriktion oder Mastzellendegranulation steht.

114. Verwendung einer Verbindung gemäß einem der Ansprüche 2, 8, 9 oder 11 für die Herstellung eines Medikaments, welches zur Gedächtnissteigerung eines Patienten nützlich ist.

115. Verwendung einer Verbindung gemäß einem der Ansprüche 21, 22 oder 33 für die Herstellung eines Medikaments, welches zur Behandlung einer Erkrankung, die in Verbindung mit einem A2a-Adenosinrezeptor steht, bei einem Patienten, der einer solchen Behandlung bedarf, nützlich ist, wobei die Erkrankung in Verbindung mit dem A2a-Adenosinrezeptor Parkinsonsche Krankheit oder Glaukom ist.

116. Verbindung gemäß Anspruch 21, wobei, falls NR₁R₂ zusammen einen 2-Aminocarbonyl-Pyrrolidinring bilden, R₃ substituiertes Phenyl oder unsubstituiertes Pyridin ist, wobei der Substituent Halogen, Hydroxyl, Alkoxy, Alkylcarbonyloxy, Arylcarbonyloxy, Alkoxycarbonyloxy, Aryloxycarbonyloxy, Carboxylat, Alkylcarbonyl, Alkoxycarbonyl, Aminocarbonyl, Alkylthiocarbonyl, Phosphat, Phosphonat, Phosphinat, Cyano, Amino, Alkylamino, Dialkylamino, Arylamino, Diarylamino, Alkylarylamino, Acylamino, Alkylcarbonylamino, Arylcarbonylamino, Carbamoyl, Ureido, Amidino, Imino, Sulfhydryl, Alkylthio, Arylthio, Thiocarboxylat, Sulfate, Sulfonat, Sulfamoyl, Sulfonamido, Nitro, Trifluormethyl, Azido, Heterocyclyl oder Alkylaryl ist.

117. Verwendung der Verbindung gemäß einem der Ansprüche 46, 58, 69 oder 83 für die Herstellung eines Medikaments, welches bei der Behandlung einer Erkrankung, die in Verbindung mit einem A3-Adenosinrezeptor steht, bei einem Patienten, der einer solchen Behandlung bedarf, nützlich ist, wobei die Erkrankung in Verbindung mit dem A3-Adenosinrezeptor myokardiale Ischämie, Bronchitis oder Bronchokonstriktion ist.

118. Verwendung der Verbindung gemäß einem der Ansprüche 46, 58, 69 oder 83 für die Herstellung eines Medikaments, welches bei der Behandlung von Entzündungen des Auges in Verbindung mit dem A3-Adenosinrezeptor bei einem Patienten nützlich ist.

119. Verwendung der Verbindung gemäß einem der Ansprüche 46, 58, 69 oder 83 für die Herstellung eines Medikaments, welches bei der Behandlung einer Erkrankung, die in Verbindung mit einem A3-Adenosinrezeptor steht, bei einem Patienten, der einer solchen Behandlung bedarf, nützlich ist, wobei die Erkrankung in Verbindung mit dem A3-Adenosinrezeptor mit Mastzellendegranulation in Verbindung steht.

120. Verwendung einer Verbindung gemäß einem der Ansprüche 46, 58, 69 oder 83 für die Herstellung eines Medikaments, welches bei der Behandlung einer Erkrankung, die in Zusammenhang mit einem A3-Adenosinrezeptor steht, bei einem Patienten, der einer solchen Behandlung bedarf, nützlich ist, wobei die Erkrankung in Verbindung mit dem A3-Adenosinrezeptor Asthma, Glaukom, Retinopathie, okulare Ischämie oder Makulardegeneration ist.

## Revendications

1. Composé possédant la structure : 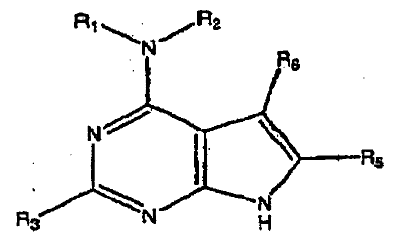 dans laquelle R₁ représente 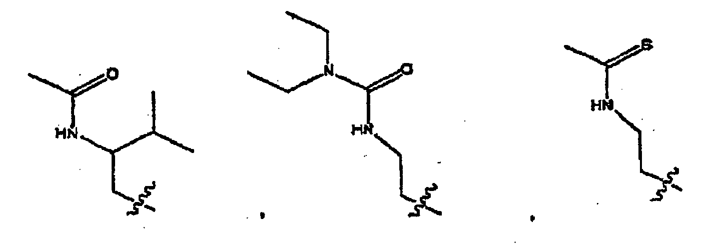 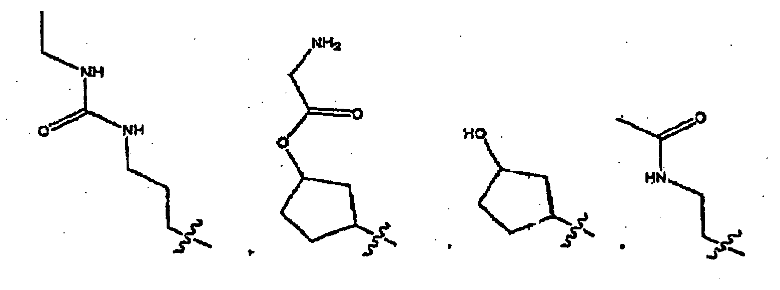 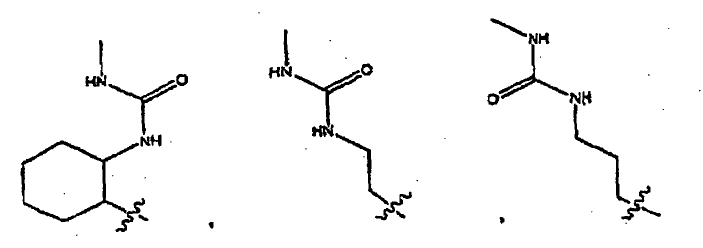 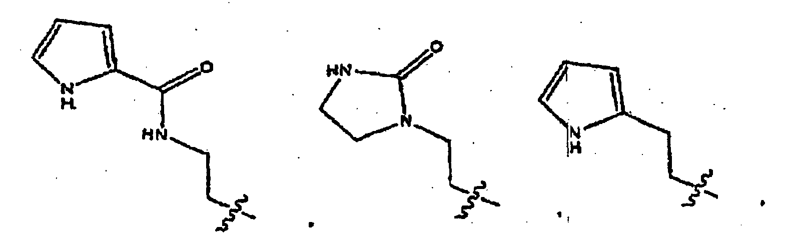 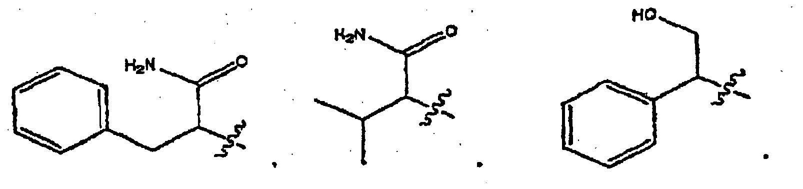 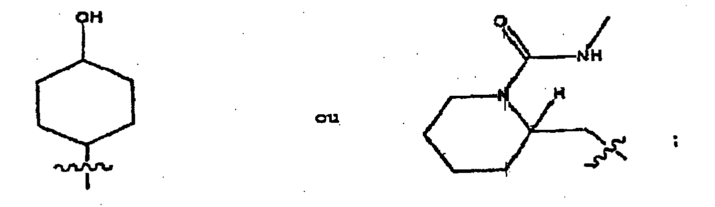 et
R₂ représente H : ou
NR₁R₂ représentent ensemble 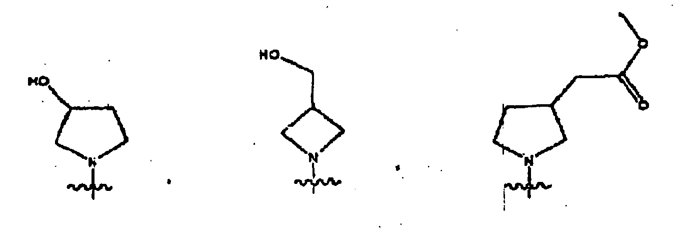 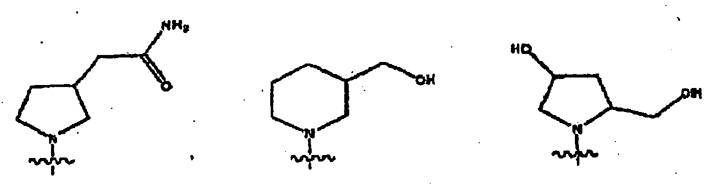 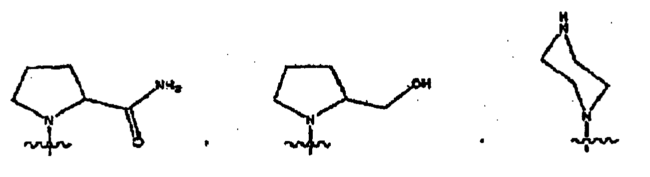 dans laquelle R₃ représente un groupe: phényle, pyrrole, thiophène, furanne, thiazole, imidazole, triazole, pyrazole, pyridine, 2(1H)-pyridone, 4(1H)-pyridone, pyrazine, pyrimidine, pyridazine, isothiazole, isoxazole, oxazole, tétrazole, naphtyle, tétraline, benzofuranne, benzothiophène, 2,3-dihydroindole, 1H-indole, indolyle, benzopyrazole, 1,3-benzodioxole, benzoxazole, purine, coumarine, chromone, quinolyle, tétrahydroquinoléine, isoquinoléine, benzimidazole, quinazoline, ptéridine, 2(1H)-quinolone, 1(2H)-isoquinolone, 1,4-benzisoxazine, benzothiazole, quinoxaline, N-oxyde de quinpléine, N-oxyde d'isoquinoléine, N-oxyde de quinoxaline, N-oxyde de quinazoline, benzoxazine, phtalazine ou cinnoline substitué ou non substitué ;
dans laquelle R₅ représente H, CH₃, un groupe alkyle, aryle ou phényle substitué ou non substitué,
ou 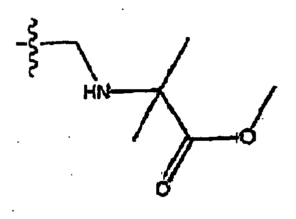 et
dans laquelle R₆ représente H, CH₃, un groupe alkyle, cycloalkyle substitué ou non substitué,
où le substituant, s'il est présent, est un atome d'halogène, un groupe hydroxyle, alcoxy, alkylcarbonyloxy, arylcarbonyloxy, alcoxycarbonyloxy, aryloxycarbonyloxy, carboxylate, alkylcarbonyle, alcoxycarbonyle, aminocarbonyle, alkylthiocarbonyle, phosphate, phosphonato, phospinato, cyano, amino, acylamino, amidino, imino, sulfhydryle, alkylthio, arylthio, thiocarboxylate, sulfates, sulfonato, sulfamoyle, sulfonamida, nitro, trifluorométhyle, azido, hétérocyclyle ou alkylaryle,
ou un de ses sels acceptable sur le plan pharmaceutique,
où quand R₁ représente 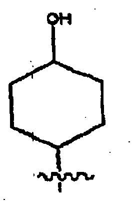 R₃ représente un groupe phényle;
R5 représente 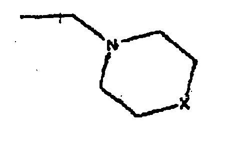 et R₆ représente H ;
où X représente O ou S ; et
où quand R1 représente 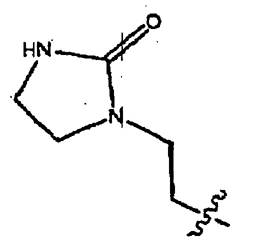 R₃ représente un groupe phényle ;
R₅ représente un groupe phényle et
R₆ représente H ;
où quand R1 représente 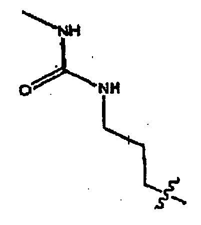 R₃ représente un groupe 4-chlorophényle ;
R₅ et R₆ représentent chacun H ;
où quand R₁ représente 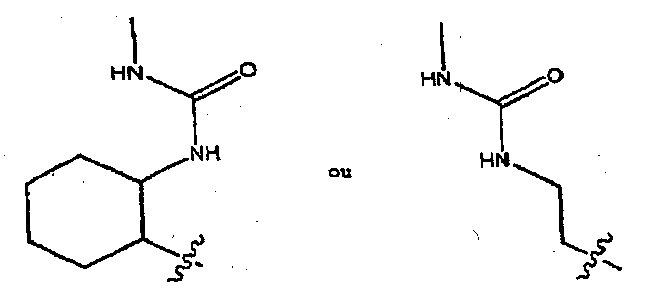 R₅ et R₆ représentent chacun, indépendamment l'un de l'autre, H ou un groupe alkyle;
où quand R1 représente 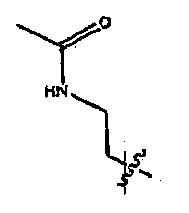 R₃ représente un groupe phényle ; et
R₅ et R₆ représentent tous deux H, ou
R₃ représente un groupe phényle ;
R5 représente 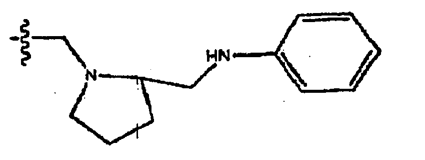 et R₆ représente H, ou
R₃ représente un groupe 4-pyridyle ;
R₅ représente CH₃ et R₆ représente

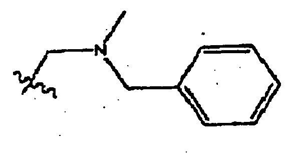

2. Composé selon la revendication 1, possédant la structure : 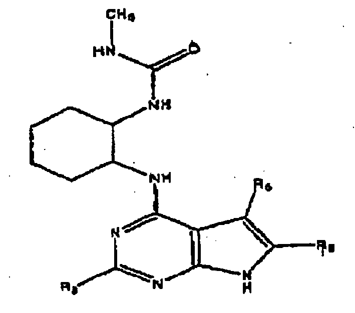 dans laquelle R₃ est tel que défini dans la revendication 1 ; dans laquelle R₅ et R₆ représentent, indépendamment l'un de l'autre, H ou un groupe alkyle.

3. Composé selon la revendication 2, possédant la structure :

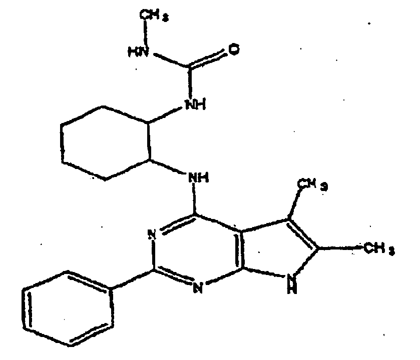

4. Composé selon la revendication 3, possédant la structure :

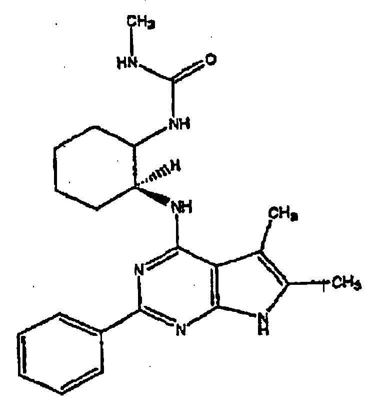

5. Composé selon la revendication 3, possédant la structure :

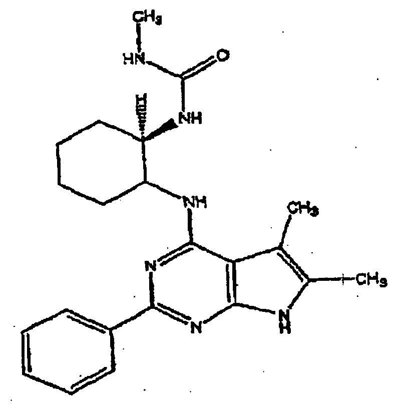

6. Composé selon la revendication 3, possédant la structure :

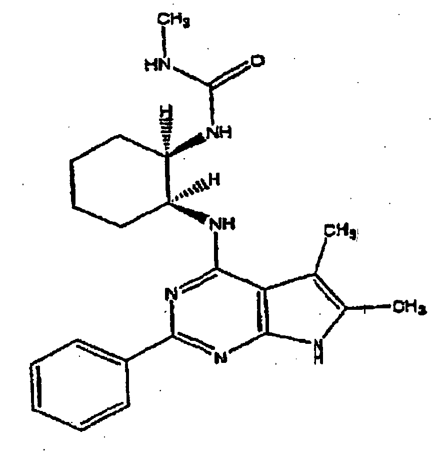

7. Composé selon la revendication 3, possédant la structure :

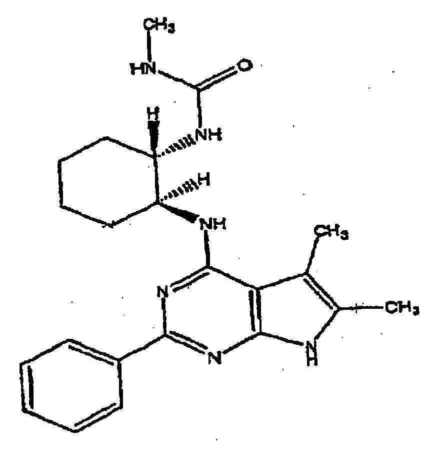

8. Composé selon la revendication 1, possédant la structure :

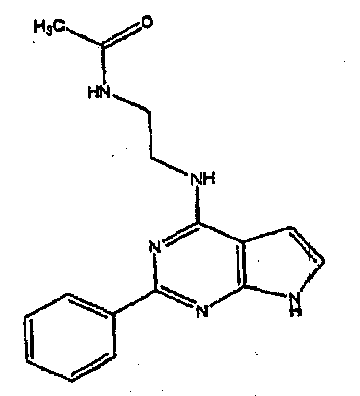

9. Composé selon la revendication 1, possédant la structure : 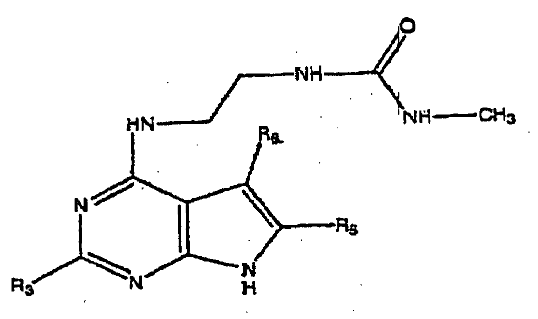 dans laquelle R₃ est tel que défini dans la revendication 1 ;
dans laquelle R₅ et R₆ représentent, indépendamment l'un de l'autre, H ou un groupe alkyle.

10. Composé selon la revendication 9, possédant la structure :

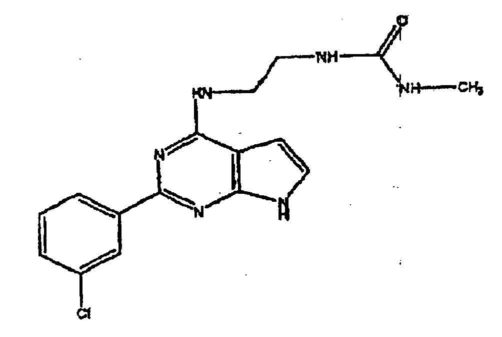

11. Composé selon la revendication 1, possédant la structure : 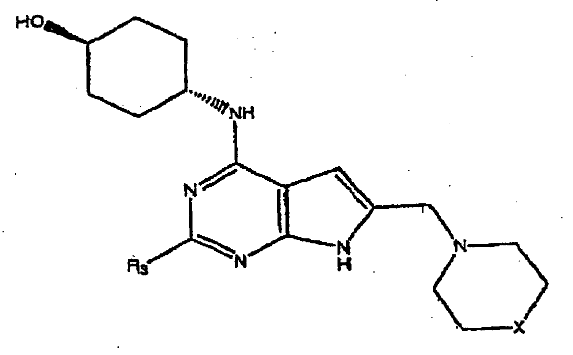 dans laquelle R₃ est tel que défini dans la revendication 1 ; dans laquelle X représente un atome d'oxygène ou de soufre.

12. Composé selon la revendication 11, possédant la structure :

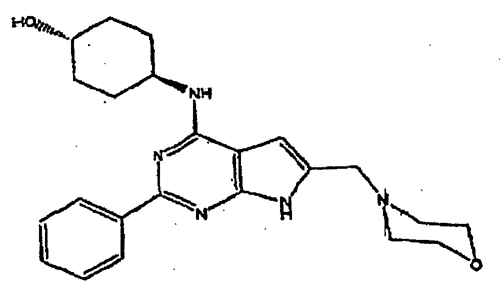

13. Composé selon la revendication 11, possédant la structure :

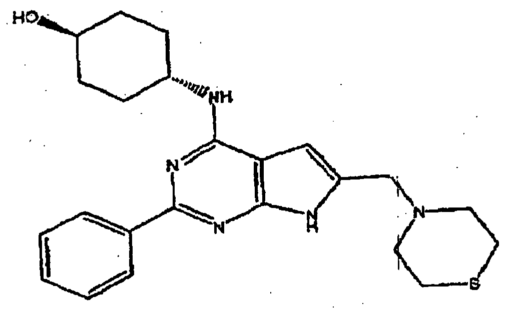

14. Utilisation d'un composé selon la revendication 2, 8, 9 ou 11, pour la fabrication d'un médicament utile pour traiter une maladie associée à un récepteur de l'adénosine A₁ chez un sujet ayant besoin d'un tel traitement.

15. Utilisation selon la revendication 14, dans laquelle ledit récepteur de l'adénosinie A₁ est associé à une maladie cognitive, une insuffisance rénale, des troubles du rythme cardiaque, des épithéliums de type respiratoire, une libération des transmetteurs, une sédation, une vasoconstriction, une bradycardie, une inotropie et une dromotropie cardiaques négatives, une bronchoconstriction, une chimiotaxie des neutrophiles, un état de reflux ou un état ulcératif.

16. Procédé pour l'inhibition de l'activité d'un récepteur de l'adénosine A1 dans une cellule *in vitro,* qui comprend la mise en contact de ladite cellule avec un composé selon les revendications 2, 8, 9 ou 11.

17. Utilisation selon la revendication 14, dans laquelle ladite maladie est un asthme, une bronchopneumopathie chronique obstructive, des rhinites allergiques ou un trouble des voies respiratoires supérieures.

18. Composition pharmaceutique comprenant le composé selon l'une quelconque des revendications 2, 8, 9 ou 11, un support acceptable sur le plan pharmaceutique et au moins l'un parmi un stéroïde, un agoniste β2, un glucocorticoïde, un antagoniste des leucotriènes ou un agoniste anticholinergique.

19. Composition pharmaceutique; comprenant une quantité efficace sur le plan thérapeutique du composé selon les revendications 2, 8, 9 ou 11 et un support acceptable sur le plan pharmaceutique.

20. Composition pharmaceutique selon la revendication 19, dans laquelle ladite quantité efficace sur le plan thérapeutique est efficace pour traiter l'asthme, les rhinites allergiques ou la bronchopneumopathie chronique obstructive.

21. Composé selon la revendication 1, possédant la structure : 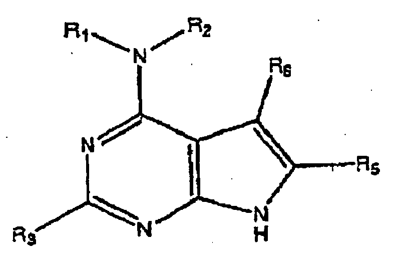 dans laquelle NR₁R₂ représente un groupe (D)-2-aminocarbonyl-N-pyrrolidinyle, (*D*)+2-hydroxyméthyl-N-pyrrolidinyle, (*D*)-2-hydroxyméthyl+trans-4-hydroxy-N-pyrrolidinyle ou N-pipérazinyle ;
dans laquelle R₃ est tel que défini dans la revendication 1;
dans laquelle R₅ représente H, un groupe phényle ou 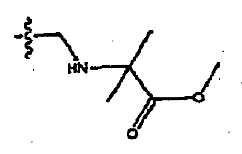 dans laquelle R₆ représente H, un groupe alkyle ou cycloalkyle.

22. Composé selon la revendication 1, possédant la structure : 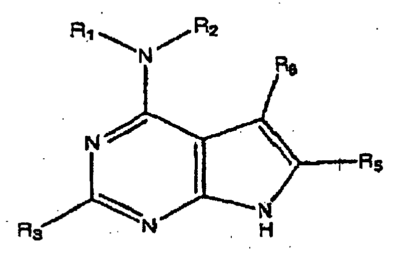 dans laquelle NR₁R₂ représente un groupe 3-hydroxyméthyl-N-pipéridinyle ;
dans laquelle R₃ représente un groupe 4-pyridyle ;
dans laquelle R₅ représente H ou un groupe alkyle ; et
dans laquelle R₆ représente H ou un groupe alkyle.

23. Composé selon la revendication 21, possédant la structure :

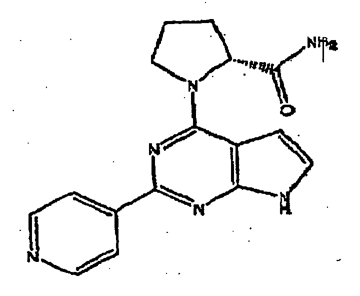

24. Composé selon la revendication 21, possédant la structure :

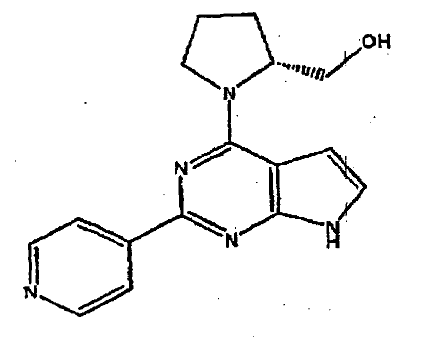

25. Composé selon la revendication 21, possédant la structure :

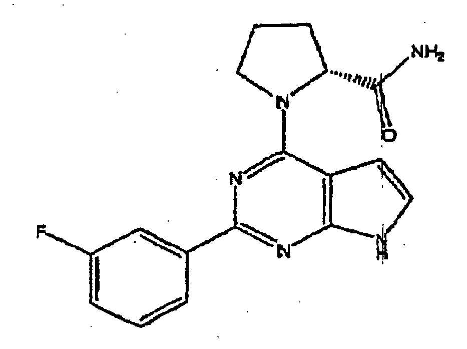

26. Composé selon la revendication 21, possédant la structure :

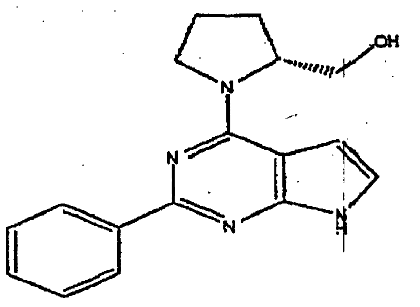

27. Composé selon la revendication 21, possédant la structure :

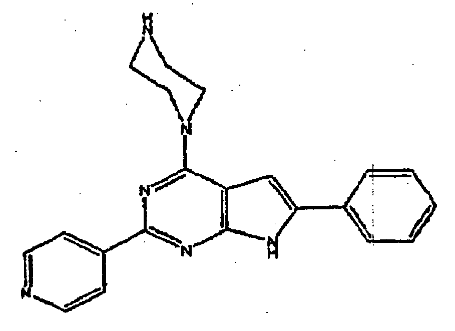

28. Composé selon la revendication 21, possédant la structure :

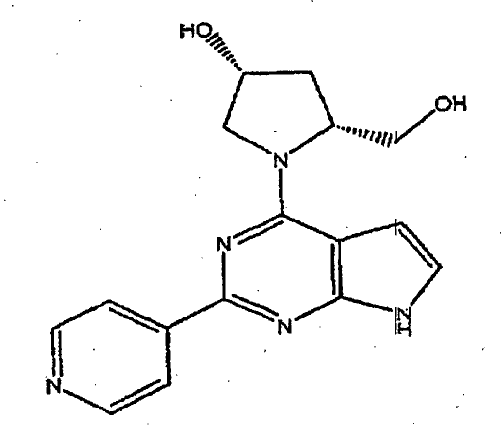

29. Composé selon la revendication 1, possédant la structure :

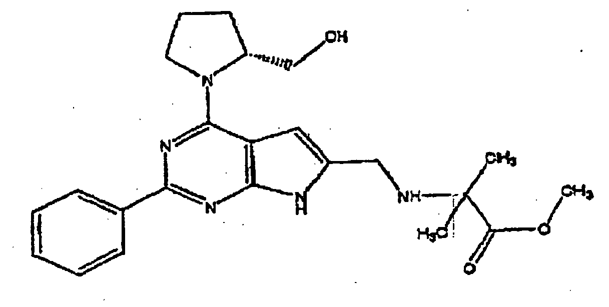

30. Composé selon la revendication 22, possédant la structure :

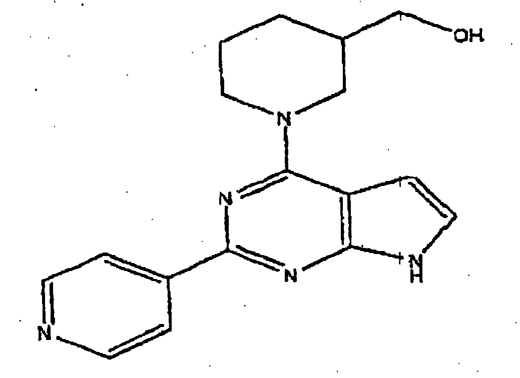

31. Composé selon la revendication 30, possédant la structure :

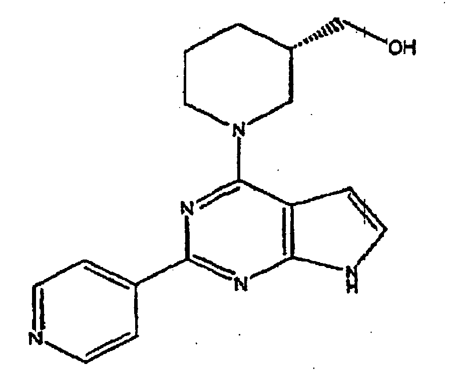

32. Composé selon la revendication 30, possédant la structure :

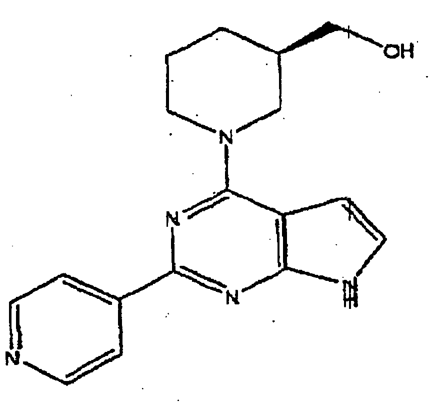

33. Composé selon la revendication 1, possédant la structure : 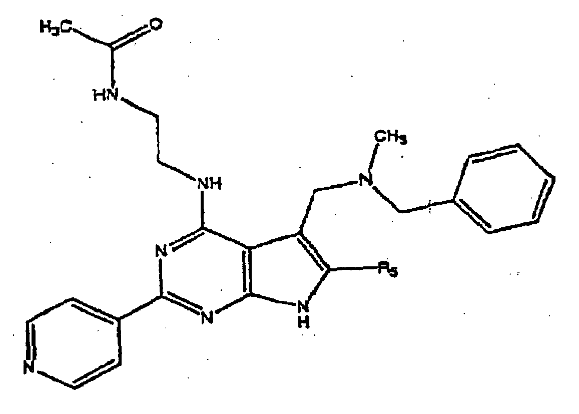 dans laquelle R₅ représente H ou un groupe méthyle.

34. Composé selon la revendication 33, possédant la structure :

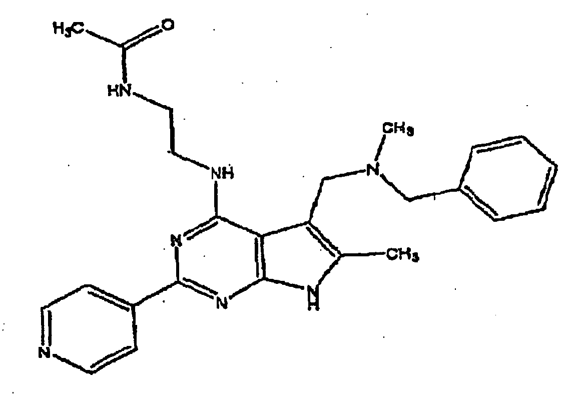

35. Composé selon la revendication 33, possédant la structure:

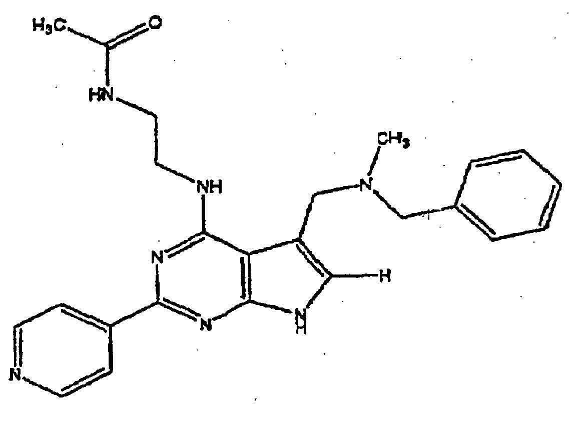

36. Utilisation d'un composé selon la revendication 21, 22 ou 33, pour la fabrication d'un médicament utile pour traiter une maladie associée à un récepteur de l'adénosine A₂ₐ chez un sujet ayant besoin d'un tel traitement.

37. Utilisation selon la revendication 36, dans laquelle ledit récepteur de l'adénosine A₂ₐ est associé à une activité locomotrice, une vasodilatation, une inhibition plaquettaire, une génération de superoxyde neutrophile, un trouble cognitif, une démence sénile ou la maladie de Parkinson.

38. Utilisation selon la revendication 36, dans laquelle le composé traite lesdites maladies en stimulant une adénylate cyclase.

39. Procédé pour l'inhibition de l'activité d'un récepteur de l'adénosine A₂ₐ dans une cellule in vitro, qui comprend la mise en contact de ladite cellule avec un composé selon les revendications 21, 22 ou 33.

40. Composition pharmaceutique, comprenant une quantité efficace sur le plan thérapeutique du composé selon les revendications 21, 22 ou 33 et un support acceptable sur le plan pharmaceutique.

41. Composition pharmaceutique selon la revendication 40, dans laquelle ladite quantité efficace sur le plan thérapeutique est efficace pour traiter la maladie de Parkinson et les maladies associées à l'activité locomotrice, la vasodilatation, l'inhibition plaquettaire, la génération de superoxyde neutrophile, le trouble cognitif ou la démence sénile.

42. Composition pharmaceutique comprenant l'un quelconque des composés selon les revendications 21, 22
ou 33, un support acceptable sur le plan pharmaceutique et au moins un stimulateur de la dopamine.

43. Composition pharmaceutique comprenant l'un quelconque des composés selon les revendications 21, 22 ou 33, un support acceptable sur le plan pharmaceutique et au moins un agent cytotoxique.

44. Composition pharmaceutique comprenant l'un quelconque des composés selon les revendications 21, 22 ou 33, un support acceptable sur le plan pharmaceutique et au moins l'un parmi un agoniste de la prostaglandine, un agoniste muscrinique ou un antagoniste des récepteurs β-2.

45. Procédé pour préparer le composé selon la revendication 33, comprenant les étapes de
a) réaction de 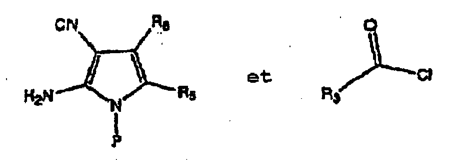 pour fournir 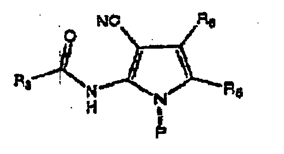 où P représente un groupe protecteur pouvant être éliminé ;
b) traitement du produit de l'étape a) dans des conditions de cyclisation pour fournir 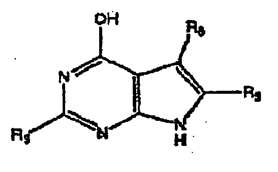
c) traitement du produit de l'étape b) dans des conditions adéquates pour fournir 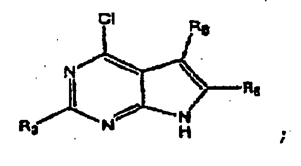 et
d) traitement du produit chloré de l'étape c) d'abord avec de la diméthylamine et du formaldéhyde, puis avec de la N-méthylbenzylamine et enfin avec NH₂R₁ pour fournir 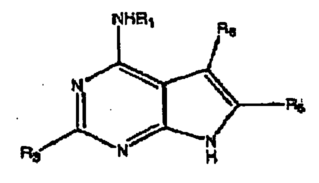
où R₁ représente un groupe acétamidoéthyle ; où R₃ représente un groupe 4-pyridyle ; où R₅ représente H ou un groupe méthyle ; où R₆ représente un groupe N-méthyl-N-benzyl-aminométhyle.

46. Composé selon la revendication 1, possédant la structure : 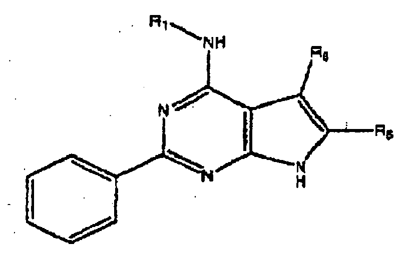 dans laquelle R₁ représente 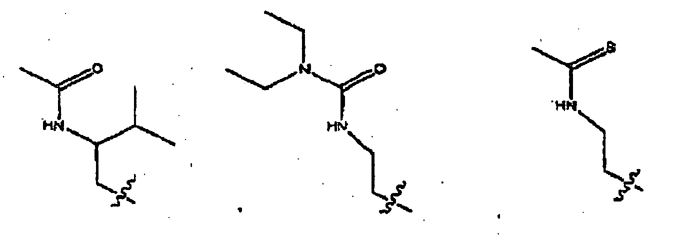 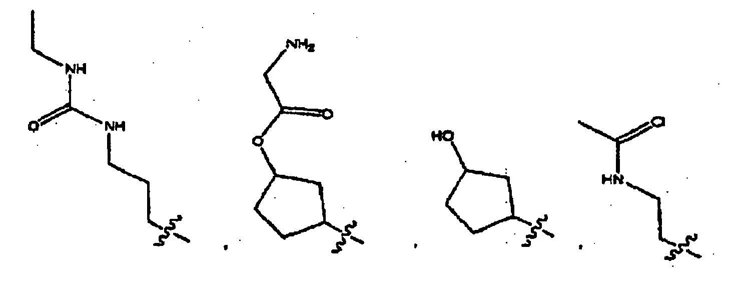 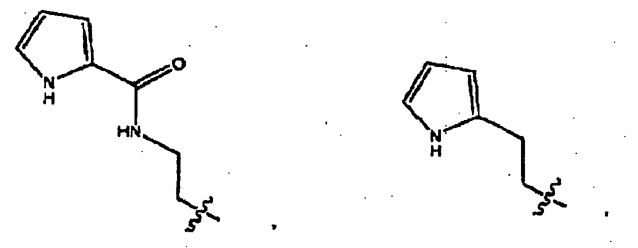 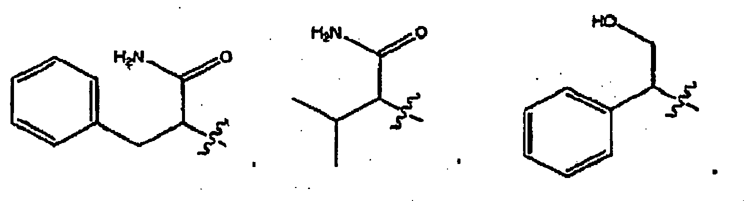 ou 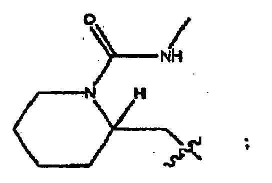 R₅ et R₆ représentent, indépendamment l'un de l'autre, H, un groupe alkyle ou aryle substitué ou non substitué,
où le substituant, s'il est présent, est tel que défini dans la revendication 1,
où quand R₁ représente 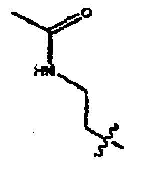 R₅ représente 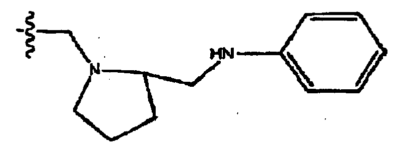 et R₆ représente H.

47. Composé selon la revendication 46, possédant la structure :

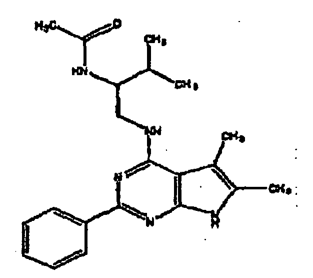

48. Composé selon la revendication 46, possédant la structure :

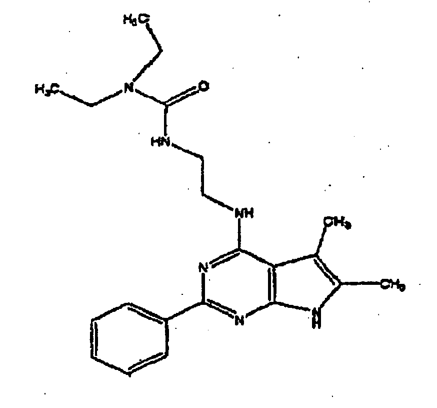

49. Composé selon la revendication 46, possédant la structure :

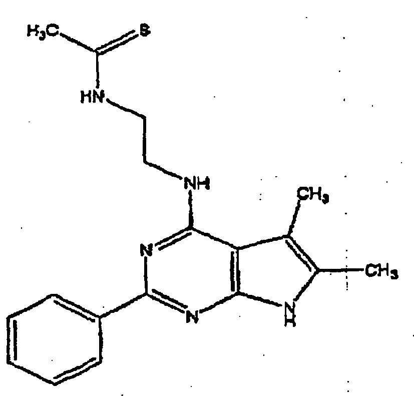

50. Composé selon la revendication 46, possédant la structure :

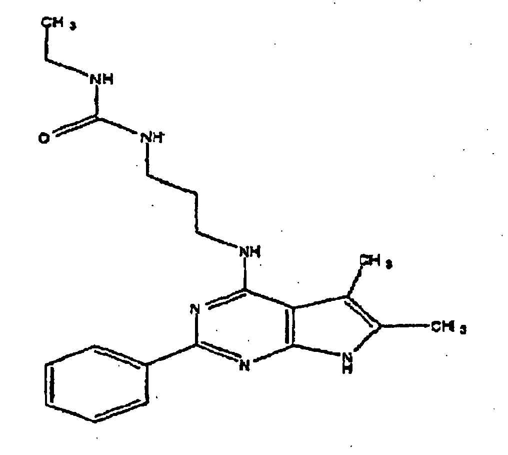

51. Composé selon la revendication 46, possédant la structure :

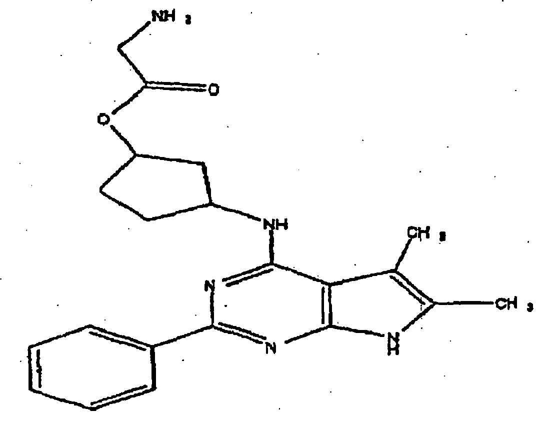

52. Composé selon la revendication 46, possédant la structure :

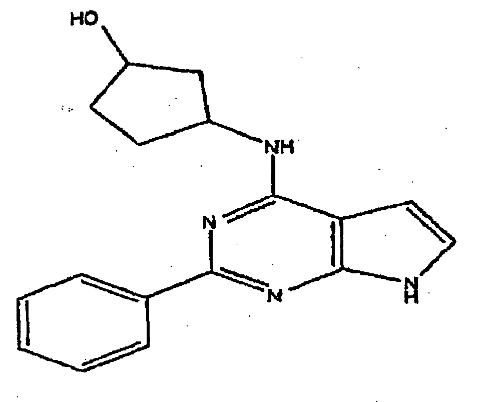

53. Composé selon la revendication 52, possédant la structure :

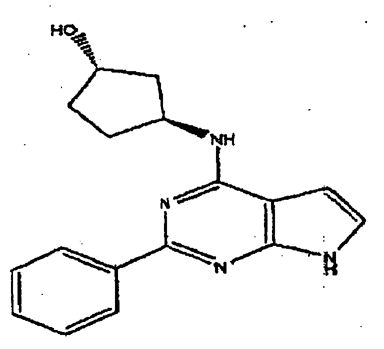

54. Composé selon la revendication 52, possédant la structure :

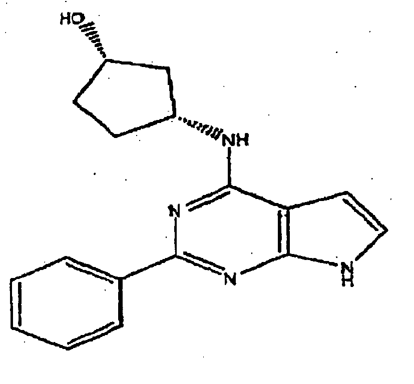

55. Composé selon la revendication 52, possédant la structure :

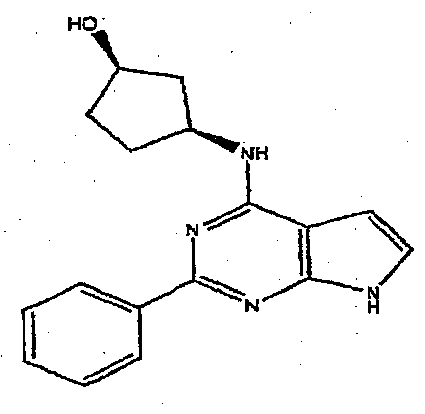

56. Composé selon la revendication 52, possédant la structure :

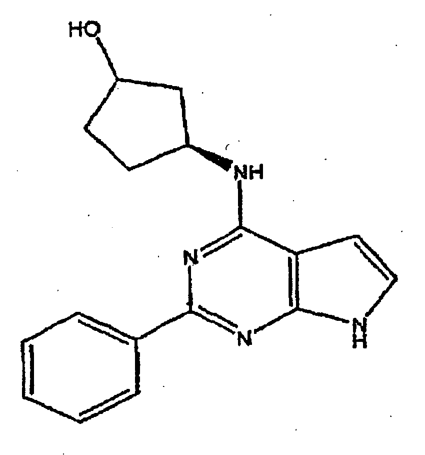

57. Composé selon la revendication 46, possédant la structure :

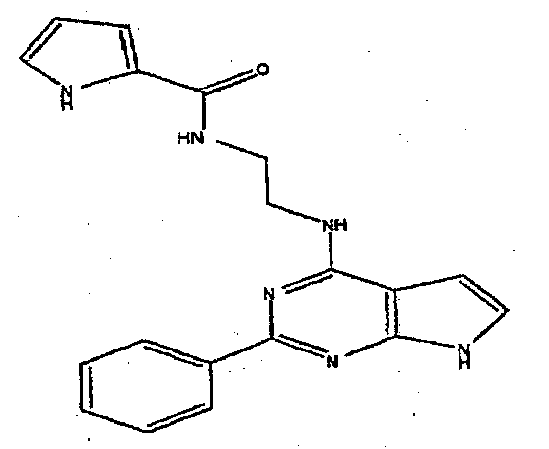

58. Composé selon la revendication 1, possédant la structure :

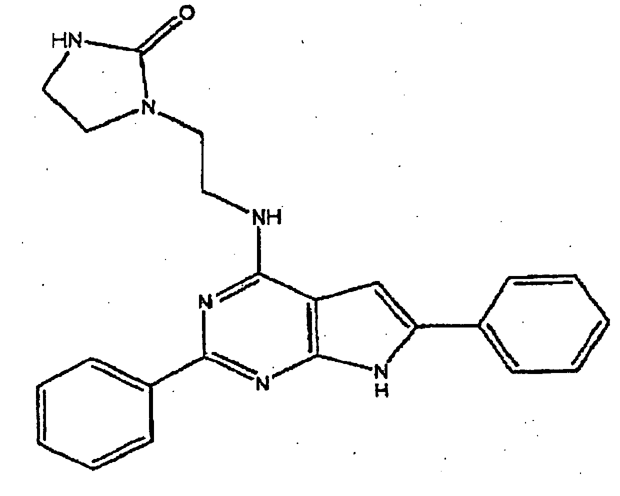

59. Composé selon la revendication 46, possédant la structure :

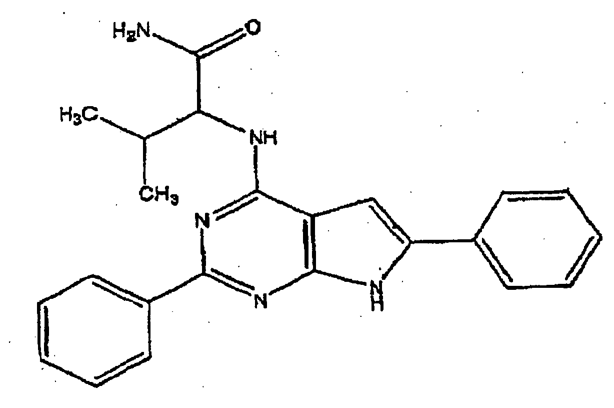

60. Composé selon la revendication 46, possédant la structure:

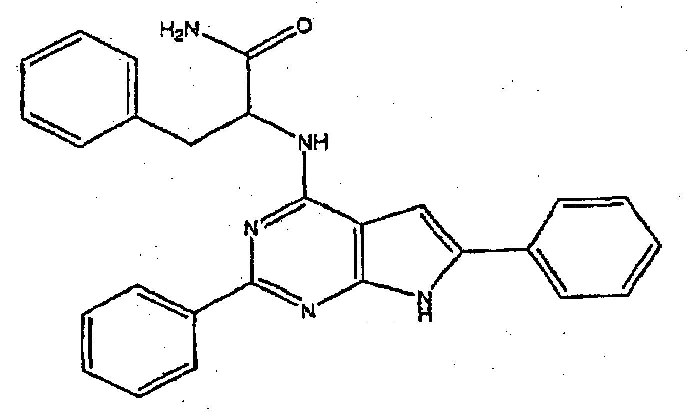

61. Composé selon la revendication 46, possédant la structure :

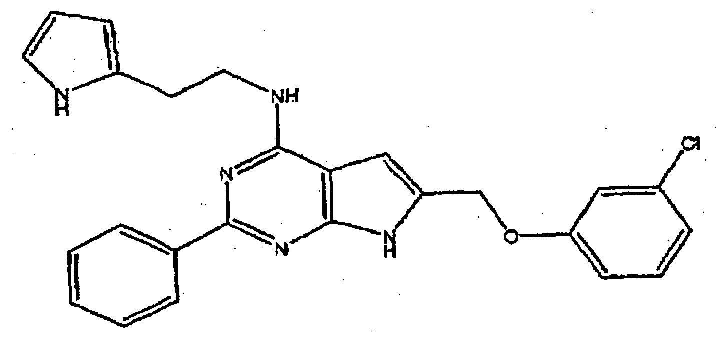

62. Composé selon la revendication 46, possédant la structure :

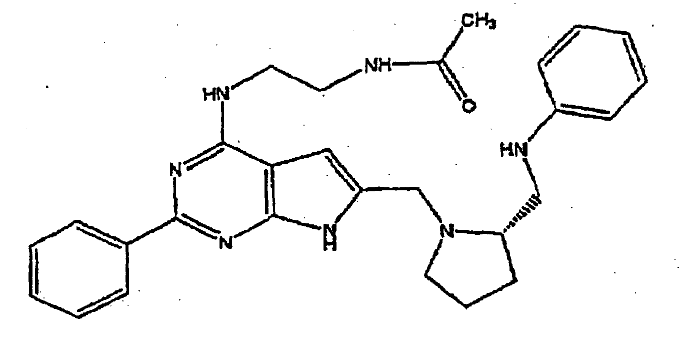

63. Composé selon la revendication 62, possédant la structure :

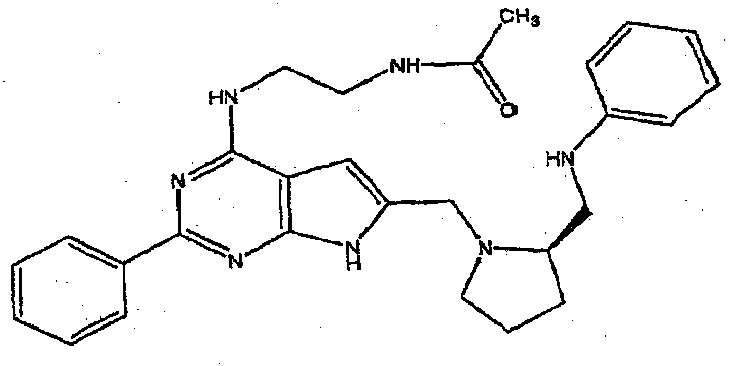

64. Composé selon la revendication 46, possédant la structure :

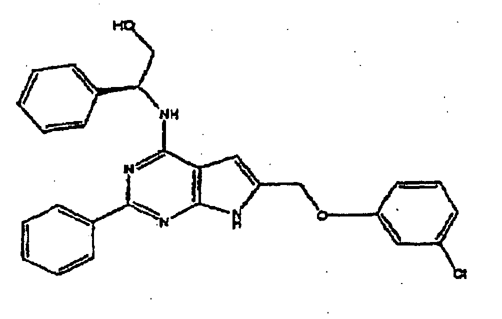

65. Composé selon la revendication 64, possédant la structure :

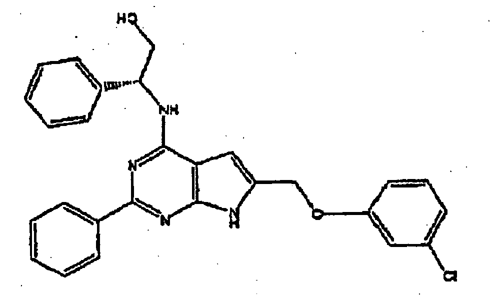

66. Composé selon la revendication 46, possédant la structure :

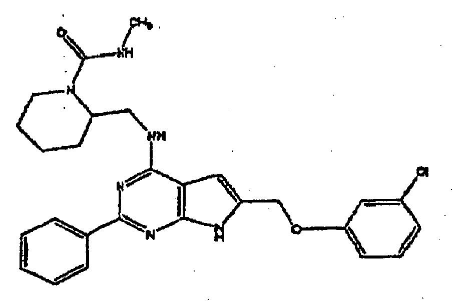

67. Composé selon la revendication 66, possédant la structure :

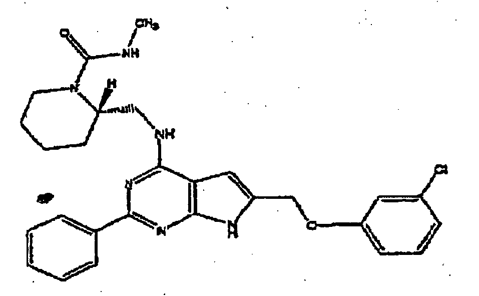

68. Composé selon la revendication 66, possédant la structure :

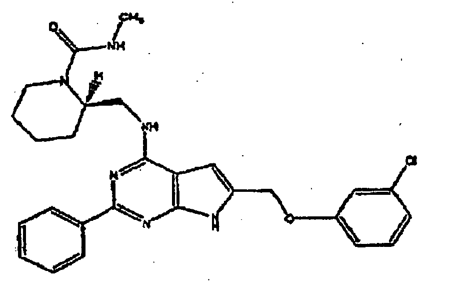

69. Composé selon la revendication 1, possédant la structure : 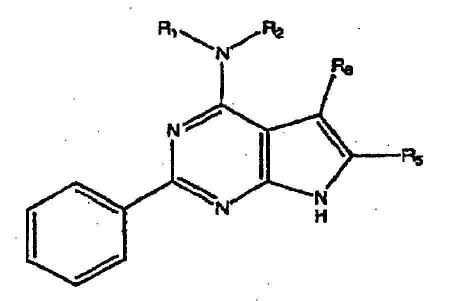 dans laquelle R₁, R₂ et l'azote représentent ensemble 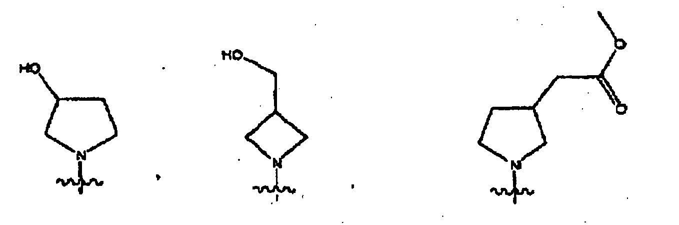 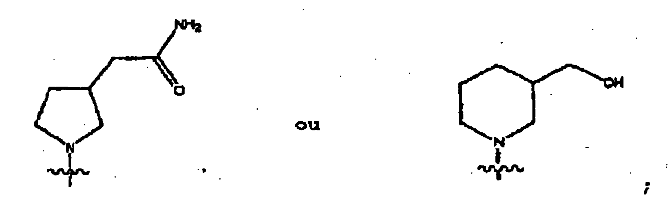 et
dans laquelle R₅ et R₆ représentent, indépendamment l'un de l'autre, H, un groupe alkyle ou aryle substitué ou non substitué,
où le substituant, s'il est présent, est tel que défini dans la revendication 1,
où quand R₁, R₂ et l'azote représentent ensemble 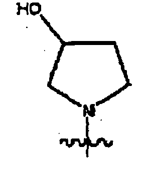 R₅ représente un groupe phényle et R₆ représente H ;
où quand R₁, R₂ et l'azote représentent ensemble 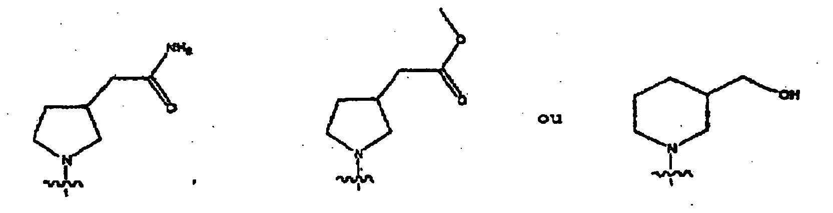 R5 représente 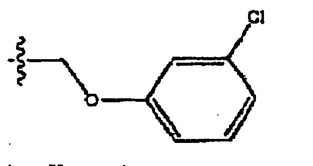 et R₆ représente H; et
où quand R₁, R₂ et l'azote représentent ensemble 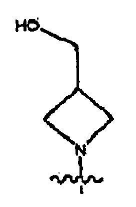 R₅ et R₆ représentent tous deux H.

70. Composé selon la revendication 69, possédant la structure :

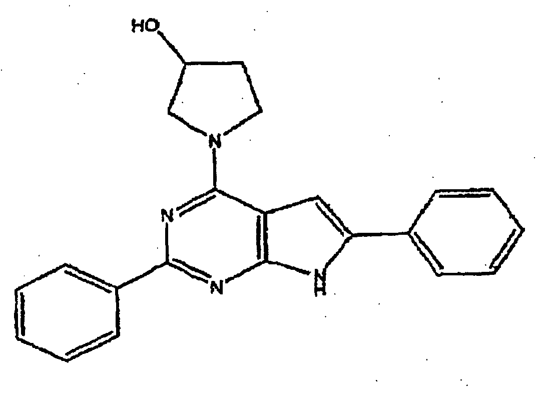

71. Composé selon la revendication 70, possédant la structure :

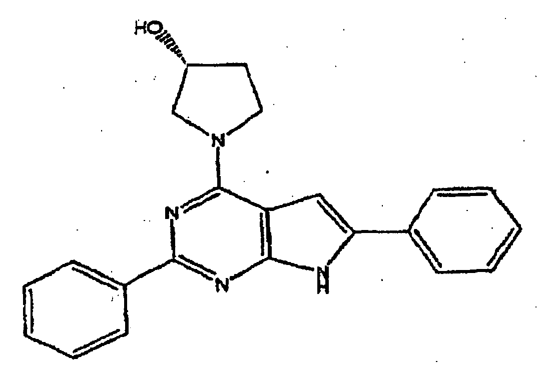

72. Composé selon la revendication 70, possédant la structure :

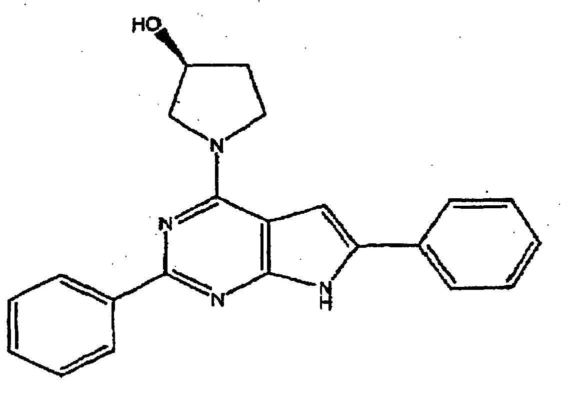

73. Composé selon la revendication 69, possédant la structure :

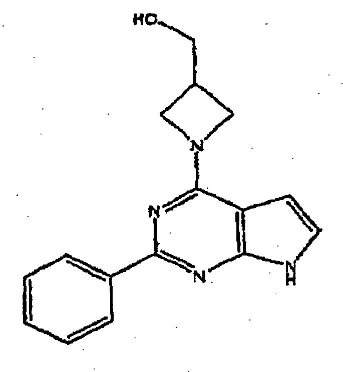

74. Composé selon la revendication 69, possédant la structure :

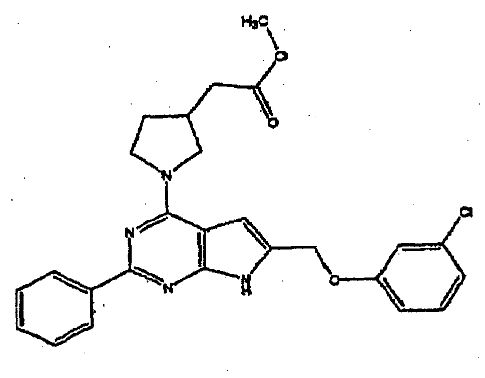

75. Composé selon la revendication 74, possédant la structure :

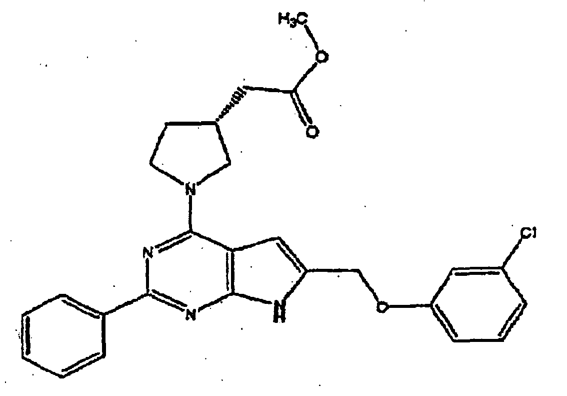

76. Composé selon la revendication 74, possédant la structure :

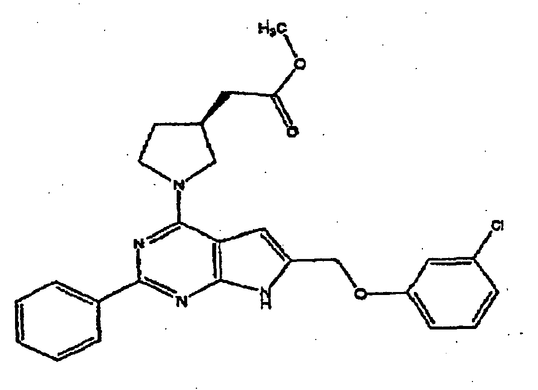

77. Composé selon la revendication 69, possédant la structure :

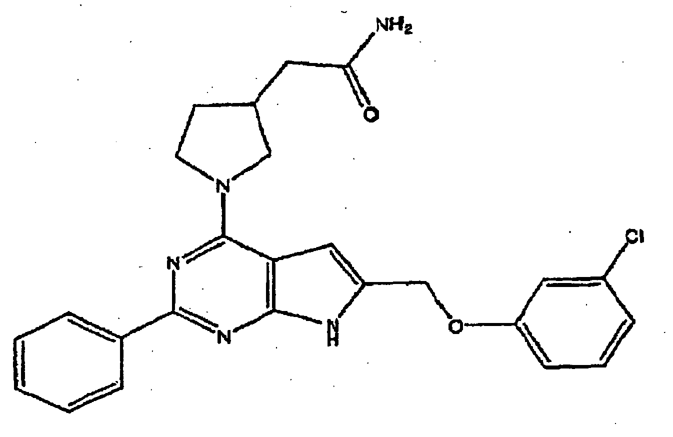

78. Composé selon la revendication 77, possédant la structure :

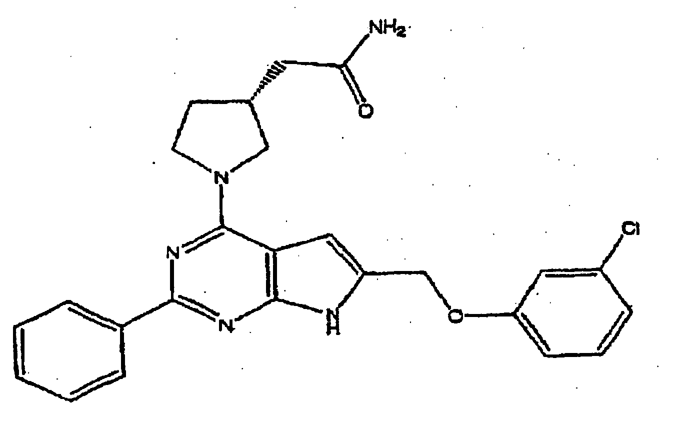

79. Composé selon la revendication 77, possédant la structure :

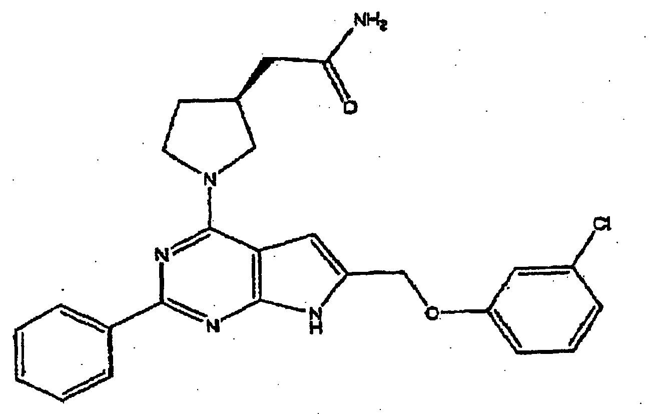

80. Composé selon la revendication 69, possédant la structure :

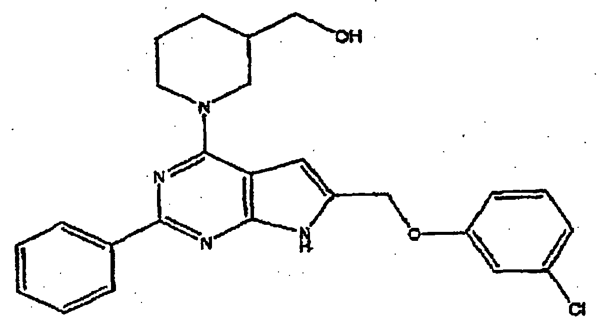

81. Composé selon la revendication 80, possédant la structure :

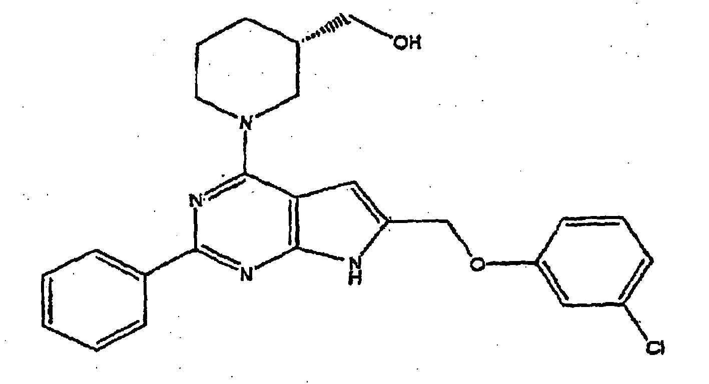

82. Composé selon la revendication 80, possédant la structure :

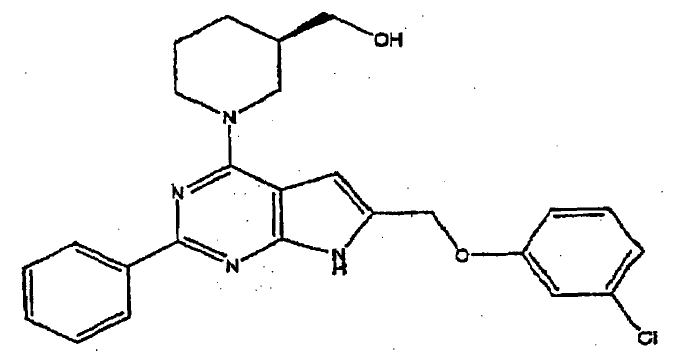

83. Composé selon la revendication 1, possédant la structure :

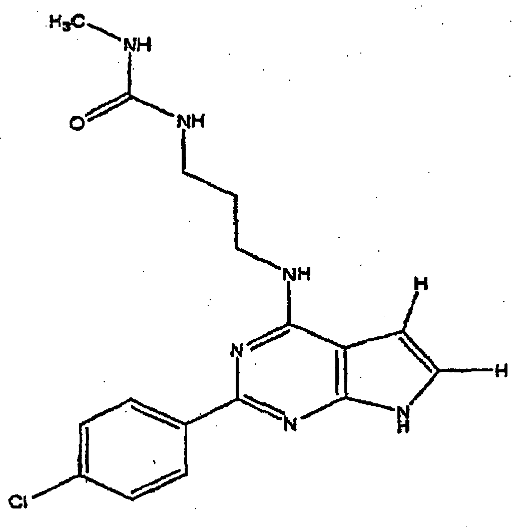

84. Utilisation d'un composé selon la revendication 46, 58, 69 ou 83, pour la fabrication d'un médicament utile pour traiter une maladie associée à un récepteur de l'adénosine A₃ chez un sujet ayant besoin d'un tel traitement.

85. Utilisation selon la revendication 84, dans laquelle ledit récepteur de l'adénosine A₃ est associé à l'asthme, l'hypersensibilité, les rhinites, le rhume des foins, la maladie du sérum, la vascularite allergique, la dermatite atopique, la dermatite, le psoriasis, l'eczéma, la fibrose pulmonaire idiopathique, la chlorécystite éosinophile, l'inflammation chronique des voies aériennes, les syndromes hyperéosinophiliques, la gastro-entérite éosinophile, l'oedème, l'urticaire, la myocardiopathie éosinophile, l'oedème angioneurotique épisodique accompagné d'éosinophilie, la maladie intestinale inflammatoire, la rectocolite hémorragique, la granulomatose allergique, la carcinomatose, le granulome éosinophile, l'histiocytose familiale, l'hypertension, la dégranulation des mastocytes, une tumeur, une hypoxie cardiaque, une ischémie cérébrale, une diurèse, une insuffisance rénale, un trouble neurologique, un trouble mental, un trouble cognitif, une ischémie du myocarde, une bronchoconstriction, une arthrite, une maladie auto-immune, la maladie de Crohn, la maladie de Grave, le diabète, la sclérose en plaques, l'anémie, les troubles de la fertilité, le lupus érythémateux, une lésion liée à une reperfusion, un diamètre des artérioles du cerveau, la libération de médiateurs allergiques, une sclérodermie, une attaque, une ischémie globale, un trouble du système nerveux central, un trouble cardio-vasculaire, un trouble rénal, un trouble inflammatoire, un trouble gastro-intestinal, un trouble oculaire, un trouble allergique, un trouble respiratoire ou un trouble immunologique.

86. Promédicament hydrosoluble, dans lequel ledit promédicament hydrosoluble est métabolisé *in vivo* pour produire le composé selon la revendication 2, 8, 9, 11, 21, 22, 33, 46, 58, 69 ou 83.

87. Promédicament selon la revendication 86, dans lequel ledit promédicament est métabolisé *in vivo* à l'aide d'une hydrolyse catalysée par une estérase.

88. Composition pharmaceutique comprenant le promédicament selon la revendication 86 et un support acceptable sur le plan pharmaceutique.

89. Composition pharmaceutique selon la revendication 88, dans laquelle ladite composition pharmaceutique est une préparation ophtalmique.

90. Composition pharmaceutique selon la revendication 88, dans laquelle ladite composition pharmaceutique est une préparation pour injection périoculaire, rétrobulbaire ou intra-oculaire.

91. Composition pharmaceutique selon la revendication 88, dans laquelle ladite composition pharmaceutique est une préparation systémique.

92. Procédé pour l'inhibition de l'activité d'un récepteur de l'adénosine A₃ dans une cellule *in vitro,* qui comprend la mise en contact de ladite cellule avec un composé selon la revendication 46, 58, 69 ou 83.

93. Procédé selon la revendication 16, 39 ou 92,
dans lequel la cellule est une cellule humaine.

94. Utilisation d'un composé selon la revendication 46, 58, 69 ou 83, pour la fabrication d'un médicament utile pour traiter un trouble gastro-intestinal chez un sujet.

95. Utilisation selon la revendication 94, dans laquelle ledit trouble est une diarrhée.

96. Utilisation d'un composé selon la revendication 46, 58, 69 ou 83, pour la fabrication d'un médicament utile pour traiter un trouble respiratoire chez un sujet.

97. Utilisation selon la revendication 96, dans laquelle ledit trouble est un asthme, une bronchopneumopathie chronique obstructive, des rhinites allergiques ou un trouble des voies respiratoires supérieures.

98. Utilisation d'un composé selon la revendication 46, 58, 69 ou 83, pour la fabrication d'un médicament utile pour traiter un dommage à l'oeil d'un sujet.

99. Utilisation selon la revendication 98, dans laquelle ledit dommage comprend une lésion de la rétine
ou de la papille optique.

100. Utilisation selon la revendication 98, dans laquelle ledit dommage est aigu ou chronique.

101. Utilisation selon la revendication 98, dans laquelle ledit dommage est la conséquence d'un glaucome, d'un oedème, d'une ischémie, d'une hypoxie ou d'un traumatisme.

102. Utilisation selon la revendication 14, 17, 36, 84, 94, 96 ou 98, dans laquelle le sujet est un humain.

103. Composition pharmaceutique comprenant l'un quelconque des composés selon les revendications 46, 58, 69 ou 83, un support acceptable sur le plan pharmaceutique et au moins l'un parmi un agoniste de la prostaglandine, un agoniste muscrinique ou un antagoniste des récepteurs β-2 .

104. Composition pharmaceutique comprenant une quantité efficace sur le plan thérapeutique du composé selon les revendications 46, 58, 69 ou 83 et un support acceptable sur le plan pharmaceutique.

105. Composition pharmaceutique selon la revendication 104, dans laquelle ladite quantité efficace sur le plan thérapeutique est efficace pour traiter un trouble respiratoire ou un trouble gastro-intestinal.

106. Composition pharmaceutique selon la revendication 105, dans laquelle ledit trouble gastro-intestinal est la diarrhée.

107. Composition pharmaceutique selon la revendication 105, dans laquelle ledit trouble respiratoire est l'asthme, les rhinites allergiques ou la bronchopneumopathie chronique obstructive.

108. Composition pharmaceutique selon la revendication 40 ou 104, dans laquelle ladite composition pharmaceutique est une préparation ophtalmique.

109. Composition pharmaceutique selon la revendication 19, 40 ou 104, dans laquelle ladite composition pharmaceutique est une préparation pour injection périoculaire, rétrobulbaire ou intra-oculaire.

110. Composition pharmaceutique selon la revendication 19, 40 ou 104, dans laquelle ladite composition pharmaceutique est une préparation systémique.

111. Composition pharmaceutique selon la revendication 19, 40 ou 104, dans laquelle ladite composition pharmaceutique est une solution d'irrigation chirurgicale.

112. Procédé pour préparer le composé selon la revendication 1, comprenant les étapes de :
a) réaction de 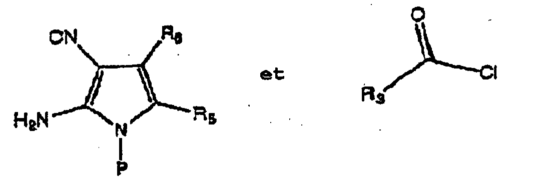 pour fournir 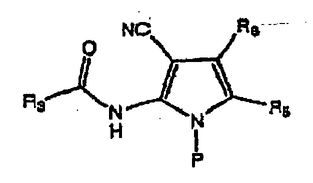 où P représente un groupe protecteur pouvant être éliminé ;
b) traitement du produit de l'étape a) dans des conditions de cyclisation pour fournir. 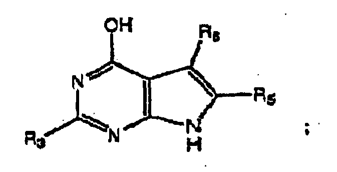
c) traitement du produit de l'étape b) dans des conditions adéquates pour fournir 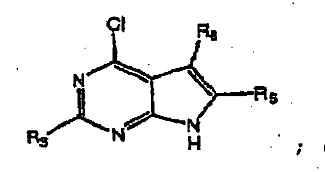 et
d) traitement du produit chloré de l'étape c) avec NHR₁R₂ pour fournir 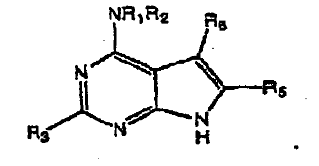
où
R₁ représente 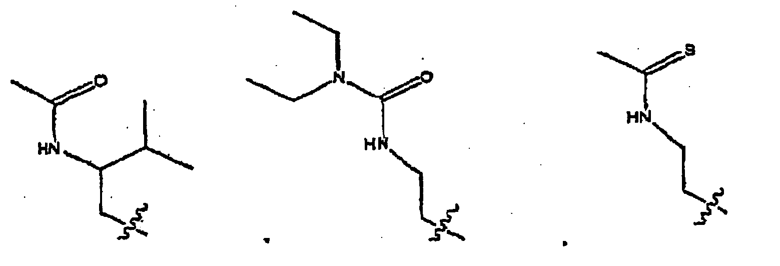 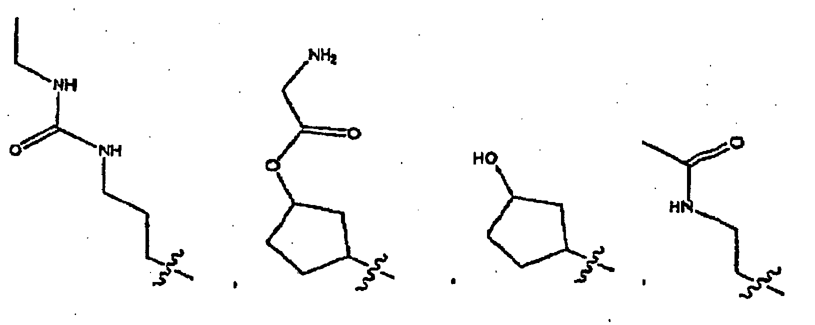 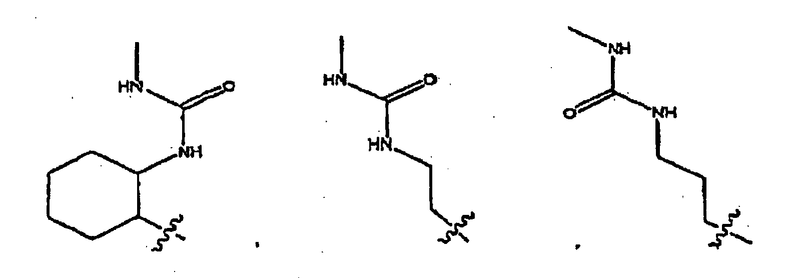 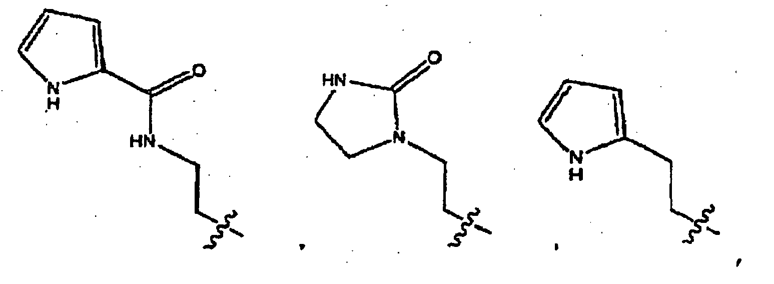 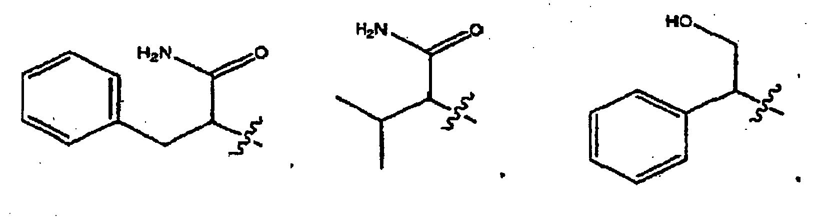 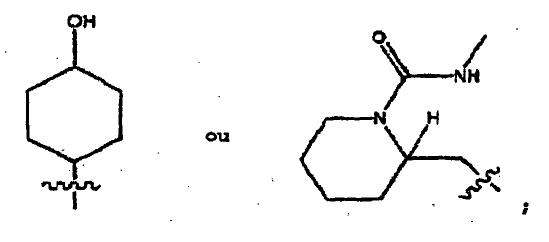 et
R₂ représente H ; ou
NR₁R₂ représentent ensemble 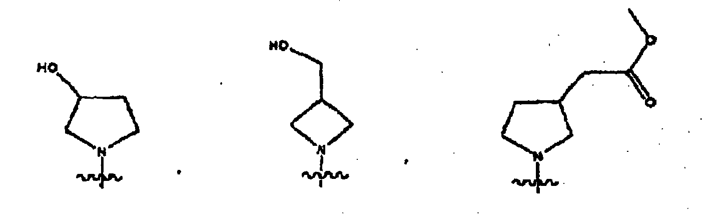 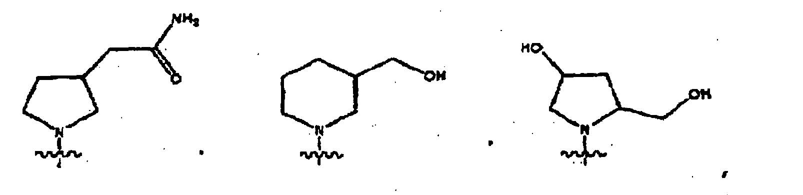 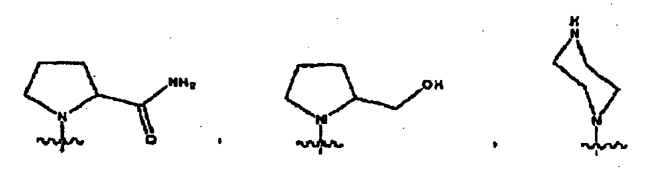 où R₃ est tel que défini dans la revendication 1 ;
où R₅ représente H, CH₃, un groupe alkyle, aryle ou phényle substitué ou non substitué,
où le substituant, s'il est présent, est tel que défini dans la revendication 1, 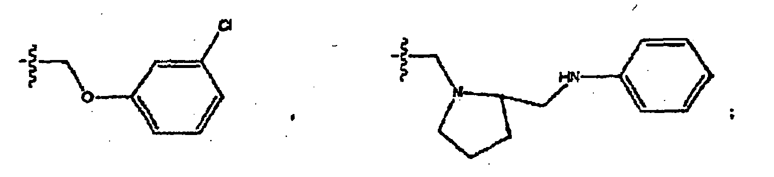 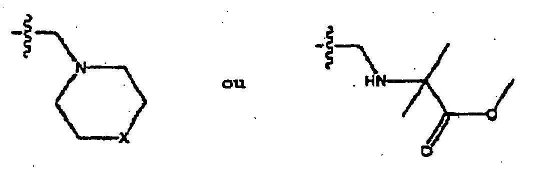 où X représente O ou S ;
et
où R₆ représente H, CH₃, un groupe alkyle, cycloalkyle
ou 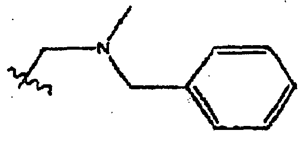 substitué ou non substitué,
où quand R₁ représente 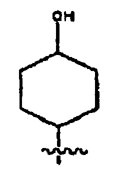 R₃ représente un groupe phényle,
R₅ représente 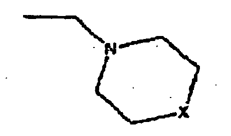 et R₆ représente H ;
où X représente O ou S ;
où quand R₁ représente 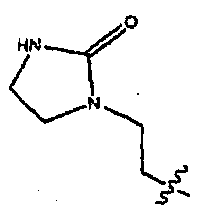 R₃ représente un groupe phényle ;
R₅ représente un groupe phényle et R₆ représente H ;
où quand R1 représente 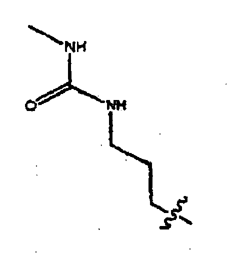 R₃ représenté un groupe 4-chlorophényle ;
R₅ et R₆ représentent chacun H ;
où quand R₁ représente 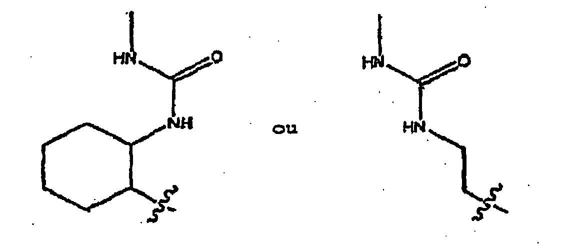 R₅ et R₆ représentent chacun, indépendamment l'un de l'autre, H ou un groupe alkyle ;
où quand R₁ représente 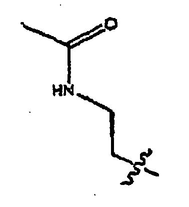 R3 représente un groupe phényle ; et
R₅ et R₆ représentent tous deux H, ou
R₃ représente un groupe phényle ;
R₅ représente 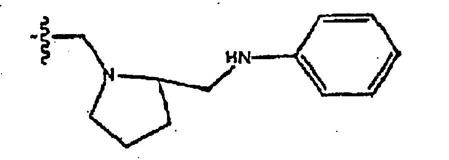 et R₆ représente H, ou
R₃ représente un groupe 4-pyridyle ;
R₅ représente CH₃ et R₆ représente

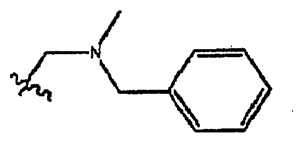

113. Utilisation du composé selon l'une quelconque des revendications 2, 8, 9 ou 11, pour la fabrication d'un médicament utile pour traiter une maladie associée à un récepteur de l'adénosine A₁ chez un sujet ayant besoin d'un tel traitement, dans laquelle la maladie associée au récepteur de l'adénosine A₁ est l'antidiurèse; la bradycardie, la bronchite, la bronchoconstriction, la maladie d'Alzheimer, les troubles du rythme cardiaque, l'hypoxie cardiaque, l'hypertension, l'inflammation, l'inotropie et la dromotropie cardiaques négatives, l'insuffisance rénale, la sédation ou est associée à la libération des transmetteurs, aux épithéliums de type respiratoire, à la contraction des épithéliums de type respiratoire sous-jacents le muscle lisse, à la vasoconstriction ou à la dégranulation des mastocytes.

114. Utilisation du composé selon l'une quelconque des revendications 2, 8, 9 ou 11, pour la fabrication d'un médicament utile pour stimuler la mémoire d'un sujet.

115. Utilisation du composé selon l'une quelconque des revendications 21, 22 ou 33, pour la fabrication d'un médicament utile pour traiter une maladie associée à un récepteur de l'adénosine A₂ₐ chez un sujet ayant besoin d'un tel traitement, dans laquelle la maladie associée au récepteur de l'adénosine A₂ₐ est la maladie de Parkinson ou un glaucome.

116. Composé selon la revendication 21, dans lequel quand NR₁R₂ forment ensemble un cycle 2-aminocarbonyl-pyrrolidine, R₃ représente un groupe phényle substitué ou pyridine non substitué
où le substituant est un atome groupe hydroxyle, alcoxy, alkylcarbonyloxy, arylcarbonyloxy, alcoxycarbonyloxy, aryloxycarbonyloxy, carboxylate, alkylcarbonyle, alcoxycarbonyle, aminocarbonyle, alkylthiocarbonyle, phosphate, phosphonato, phospinato, cyano, amino, alkylamino, dialkylamino, arylamino, diarylamino, alkylarylamino, acylamino, alkylcarbonylamino, arylcarbonylamino, carbamoyle, uréido, amidino, imino, sulfhydryle, alkylthio, arylthio, thiocarboxylate, sulfates, sulfonato, sulfamoyle, sulfonamido, nitro, trifluorométhyle, azido, hétérocyclyle ou alkylaryle.

117. Utilisation du composé selon l'une quelconque des revendications 46, 58, 69 ou 83, pour la fabrication d'un médicament utile pour traiter une maladie associée à un récepteur de l'adénosine A₃ chez un sujet ayant besoin d'un tel traitement, dans laquelle la maladie associée au récepteur de l'adénosine A₃ est une ischémie du myocarde, une bronchite ou une bronchoconstriction.

118. Utilisation du composé selon l'une quelconque des revendications 46, 58, 69 ou 83, pour la fabrication d'un médicament utile pour traiter une inflammation de l'oeil associée au récepteur de l'adénosine A₃ chez un sujet.

119. Utilisation du composé selon l'une quelconque des revendications 46, 58, 69 ou 83, pour la fabrication d'un médicament utile pour traiter une maladie associée à un récepteur de l'adénosine A₃ chez un sujet ayant besoin d'un tel traitement, dans laquelle la maladie associée au récepteur de l'adénosine A₃ est associée à une dégranulation des mastocytes.

120. Utilisation du composé selon l'une quelconque des revendications 46, 58,69 ou 83, pour la fabrication d'un médicament utile pour traiter une maladie associée à un récepteur de l'adénosine A₃ chez un sujet ayant besoin d'un tel traitement, dans laquelle la maladie associée au récepteur de l'adénosine A₃ est un asthme, un glaucome, une rétinopathie, une ischémie oculaire ou une dégénérescence maculaire.
